(19) Europäisches Patentamt
European Patent Office
Office européen des brevets

(11) EP 3 995 583 A1

(12) EUROPEAN PATENT APPLICATION

(43) Date of publication:
**11.05.2022 Bulletin 2022/19**

(21) Application number: **20206809.4**

(22) Date of filing: **10.11.2020**

(51) International Patent Classification (IPC):
***C12N 15/82*** *(2006.01)* ***C07K 14/325*** *(2006.01)*

(52) Cooperative Patent Classification (CPC):
**C12N 15/8286; C07K 14/325; C12Q 1/6895**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Centro de Tecnologia Canavieira S.A**
**01452-000 Jardim Paulistano São Paulo (BR)**

(72) Inventors:
- **YANAGUI, Karina**
  **São Paulo Piracicaba São Paulo (BR)**
- **KAUPERT NETO, Antonio Adalberto**
  **13.400-970 Piracicaba São Paulo (BR)**
- **ZAKIR PEREIRA, Ana Carolina Vieira**
  **13.400-970 Piracicaba São Paulo (BR)**
- **LORENA SERENO, Maria**
  **13.400-970 Piracicaba São Paulo (BR)**
- **CHEAVEGATTI GIANOTTO, Adriana**
  **13.400-970 Piracicaba São Paulo (BR)**
- **GENTILE, Agustina**
  **13.400-970 Piracicaba São Paulo (BR)**
- **CUTRI, Lucas**
  **13.400-970 Piracicaba São Paulo (BR)**
- **SALLES DE OLIVEIRA, Wladecir**
  **13.400-970 Piracicaba São Paulo (BR)**

(74) Representative: **Williams, Andrea**
**Marks & Clerk LLP**
**62-68 Hills Road**
**Cambridge CB2 1LA (GB)**

# (54) POLYNUCLEOTIDES, PRIMERS, AND METHODS FOR DETECTION OF TRANSGENIC EVENT, GENETIC CONSTRUCT, KIT FOR DETECTION MATERIAL FROM A PLANT SAMPLE, EVENT CTC75064-3, INSECT-RESISTANT SUGARCANE PLANT, AND METHOD FOR PRODUCING AN INSECT-RESISTANT SUGARCANE PLANT, PLANT CELL, PLANT PART OR SEED

(57) The present invention relates to the field of biotechnology. More precisely, a genetic construct and method for producing a transgenic plant event, especially a sugarcane event (*Saccharum* spp.), which is resistant to infestation by the *Diatraea saccharalis* pest, popularly known as ordinary borer, reed borer or just borer. The invention describes the event, the methods for event identification as well as the insertion detection method based on the unique region of intersection between the insert and the host genome and the flanking regions that characterize it.

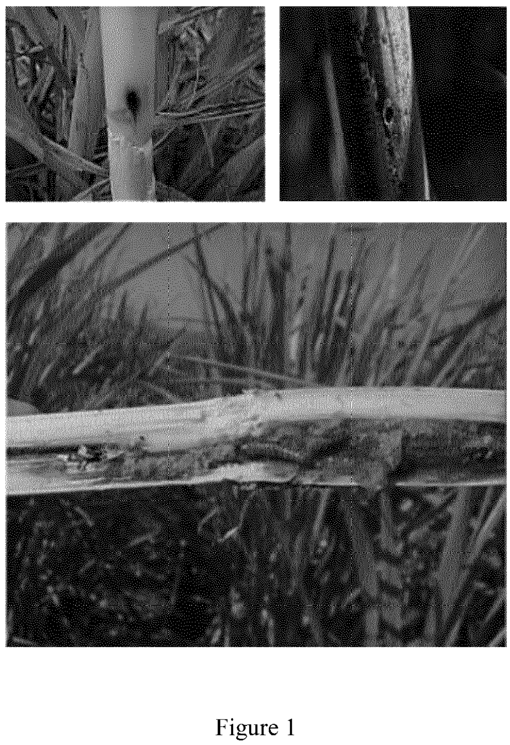

Figure 1

EP 3 995 583 A1

SEQUENCE LISTING

<110> CTC - CENTRO DE TECNOLOGIA CANAVIEIRA S.A.

<120> POLYNUCLEOTIDES, PRIMERS, AND METHODS FOR DETECTION OF TRANSGENIC EVENT, GENETIC CONSTRUCT, KIT FOR DETECTION MATERIAL FROM A PLANT SAMPLE, EVENT CTC75064-3, INSECT-RESISTANT SUGARCANE PLANT, AND METHOD FOR PRODUCING AN INSECT-RESISTANT SUGARCANE PLANT, PLANT CELL, PLANT PART OR SEED

<130> P249682-EP

<160> 116

<170> PatentIn version 3.5

<210> 1
<211> 6570
<212> DNA
<213> Artificial Sequence

<220>
<223> Genetic construct comprising Cry1Ac and nptII genes

<400> 1
tggcaggata tattgtggtg taaacaaatt gacgcttaga caacttaata acacattgcg 60

gacgttttta atgtactgaa ttagtactga tatcggtacc ttaattcggg ggatctggat 120

tttagtactg gattttggtt ttaggaatta gaaattttat tgatagaagt attttacaaa 180

tacaaataca tactaagggt ttcttatatg ctcaacacat gagcgaaacc ctataggaac 240

cctaattccc ttatctggga actactcaca cattattatg gagaaactcg agcttgtcga 300

tcgatcactc agcctcgagg gtggcggtca ctgggatgaa ctcgaacctg tcgatgatca 360

cgccggcggt gccgctgaag ttcctcacgc ccacgatgtt accgagggag gaggtgaaag 420

cgttggcgct ctcgaagtaa ccgaagtcgc tggattggag gttgtccagg gaggtagcgg 480

tagctggcac ggtgttggag aagatggagg agttacccca gttcacgttg aggtggatcg 540

gggtcacgga agcgtacctc acgcgcaccc tgtacctggt ggaggtggat gggaagtgga 600

ttggcacctc gatgtagccc ctgttctgga tgttgttgcc gctgctgttg agcctcacga 660

ggtcgccacc ggtgaagcct gggccggaaa tgacggaacc gttgaagagg aagttgccct 720

tcacggccgg gatttgggtg atgctgtcgg aggcgatgat gttgttgaac tcagcgctgc 780

ggtggatcca ggagaacatc ggagccctga tgatgctcac ggagctgttg ctgaagccgg 840

agcggaacat ggacacgtgg ctcagcctgt gggagaagcc ttgcctgggt ggcacgttgt 900

tgttctgtgg tgggatctcg tccagggagt ccacggtgcc gctcttcctg tagacagcgg 960

atggcaggtt ggaggaggtg ccgtaggcga actcggtgcc gtcgagcacg gacagttgct 1020

ggttgttgat accgatgttg aagggcctcc tgtacagggt ggaggacagg gtcctgtaga 1080

caccctgacc cagttgagcc acgatgcgtt gctgtggagc ggcgttgccc atggtgccgt 1140

agagcgggaa ggtgaactcg gggccgctga agcccactgg ggaggccatg atctggtggc 1200

cggaccagta gtactcgccc ctgtgagcgt cggtgtagat ggtgatgctg ttcaggatgt 1260

ccatcaggtg tgggctcctg atggagccct cgataccctg ggcggaaccg cggaagctac 1320

cgtcgaagtt ctccagcact gggttggtgt agatctccct ggtgagttgg gacacggtgc 1380

ggatcgggta ggtcctggag tcgtagttcg ggaagaggga cacaatgtcc agcacggtga 1440

gggtcaactc cctcctgaac tggttgtacc tgatccagtc cctggagtcc ggaccccaga 1500

cgcgctccag gccggtgttg taccagcgca cagcgtggtc ggtgtagttg ccaatcagcc 1560

tggtcaggtc gttgtagcgg ctgttgatgg tggcagcatc gaagccccac ctttggccga 1620

acacgctcac gtcgcgcagc acgctgaggt gcaggttagc ggcttgcacg tacacggaca 1680

ggagcggcac ttggtagttc tggacggcga acagtgggat agcggtggtc agggcgctgt 1740

tcatgtcgtt gaattgaatg cgcatttcct cgcggagagc tgggttggtc gggtcggcct 1800

cccactccct gaagctctcg gcgtagattt ggtagaggtt gctcaggccc tccagcctgg 1860

agatggcctg gttcctggcg aactcttcga tcctctggtt gatcagctgc tcgatttgca 1920

ccaggaaggc gtcccattgg gatggaccga agatacccca gatgatgtcc accaggccga 1980

gcacgaagcc agcacctggc acgaactcgc tgagcaggaa ctgggtcaag gacagggaga 2040

tgtcgatggg ggtgtaaccg gtctcgatgc gctcgccacc cagcacctcc acctctgggt 2100

tgctcaggca gttgtatggg atgcactcgt tgatgtttgg gttgttgtcc atggcggggt 2160

tgatcaggtt gatcacttct acctacaaaa aagctccgca cgaggctgca tttgtcacaa 2220

atcatgaaaa gaaaaactac cgatgaacaa tgctgaggga ttcaaattct acccacaaaa 2280

agaagaaaga aagatctagc acatctaagc ctgacgaagc agcagaaata tataaaaata 2340

taaaccatag tgccctttttc ccctcttcct gatcttgttt agcacggcgg aaattttaaa 2400

ccccccatca tctcccccaa caacggcgga tcgcagatct acatccgaga gccccattcc 2460

ccgcgagatc cgggccggat ccacgccggc gagagcccca gccgcgagat cccgcccctc 2520

ccgcgcaccg atctgggcgc gcacgaagcc gcctctcgcc cacccaaact accaaggcca 2580

aagatcgaga ccgagacgga aaaaaaaacg gagaaagaaa gaggagaggg gcggggtggt 2640

taccggcggc ggcggaggcc tcccttggat cttatggtgt gttgtccctg tgtgttctcc 2700

aatagtgtgg cttgagtgtg tggaagatgg ttctagagga tctgctagag tcagcttgtc 2760

agcgtgtcct ctccaaatga aatgaacttc cttatataga ggaagggtct tgcgaaggat 2820

agtgggattg tgcgtcatcc cttacgtcag tggagatatc acatcaatcc acttgctttg 2880

aagacgtggt tggaacgtct tctttttcca cgatgctcct cgtgggtggg ggtccatctt 2940

tgggaccact gtcggcagag gcatcttcaa cgatggcctt tcctttatcg caatgatggc 3000

atttgtagga gccaccttcc ttttccacta tcttcacaat aaagtgacag atagctgggc 3060

aatggaatcc gaggaggttt ccggatatta ccctttgttg aaaagtctca atcggaccat 3120
cacatcaatc cacttgcttt gaagacgtgg ttggaacgtc ttctttttcc acgatgctcc 3180
tcgtgggtgg gggtccatct ttgggaccac tgtcggcaga ggcatcttca acgatggcct 3240
ttcctttatc gcaatgatgg catttgtagg agccaccttc cttttccact atcttcacaa 3300
taaagtgaca gatagctggg caatggaatc cgaggaggtt tccggatatt accctttgtt 3360
gaaaagtctc aatcggacct gtttaaaccc tgaagcttac gcgtatgcct gcagtgcagc 3420
gtgacccggt cgtgcccctc tctagagata atgagcattg catgtctaag ttataaaaaa 3480
ttaccacata tttttttgt cacacttgtt tgaagtgcag tttatctatc tttatacata 3540
tatttaaact ttactctacg aataatataa tctatagtac tacaataata tcagtgtttt 3600
agagaatcat ataaatgaac agttagacat ggtctaaagg acaattgagt attttgacaa 3660
caggactcta cagttttatc tttttagtgt gcatgtgttc tcctttttt ttgcaaatag 3720
cttcacctat ataatacttc atccatttta ttagtacatc catttagggt ttagggttaa 3780
tggtttttat agactaattt ttttagtaca tctattttat tctattttag cctctaaatt 3840
aagaaaacta aaactctatt ttagtttttt tatttaataa tttagatata aaatagaata 3900
aaataaagtg actaaaaatt aaacaaatac cctttaagaa attaaaaaaa ctaaggaaac 3960
atttttcttg tttcgagtag ataatgccag cctgttaaac gccgtcgacg agtctaacgg 4020
acaccaacca gcgaaccagc agcgtcgcgt cgggccaagc gaagcagacg gcacggcatc 4080
tctgtcgctg cctctggacc cctctcgaga gttccgctcc accgttggac ttgctccgct 4140
gtcggcatcc agaaattgcg tggcggagcg gcagacgtga gccggcacgg caggcggcct 4200
cctcctcctc tcacggcacg gcagctacgg gggattcctt tcccaccgct ccttcgcttt 4260
cccttcctcg cccgccgtaa taaatagaca ccccctccac accctctttc cccaacctcg 4320
tgttgttcgg agcgcacaca cacacaacca gatctccccc aaatccaccc gtcggcacct 4380
ccgcttcaag gtacgccgct cgtcctcccc cccccccct ctctaccttc tctagatcgg 4440
cgttccggtc catggttagg gcccggtagt tctacttctg ttcatgtttg tgttagatcc 4500
gtgtttgtgt tagatccgtg ctgctagcgt tcgtacacgg atgcgacctg tacgtcagac 4560
acgttctgat tgctaacttg ccagtgtttc tctttgggga atcctgggat ggctctagcc 4620
gttccgcaga cgggatcgat ttcatgattt tttttgtttc gttgcatagg gtttggtttg 4680
cccttttcct ttatttcaat atatgccgtg cacttgtttg tcgggtcatc ttttcatgct 4740
tttttttgtc ttggttgtga tgatgtggtc tggttgggcg gtcgttctag atcggagtag 4800
aattctgttt caaactacct ggtggattta ttaattttgg atctgtatgt gtgtgccata 4860
catattcata gttacgaatt gaagatgatg gatggaaata tcgatctagg ataggtatac 4920

```
atgttgatgc gggttttact gatgcatata cagagatgct ttttgttcgc ttggttgtga     4980
tgatgtggtg tggttgggcg gtcgttcatt cgttctagat cggagtagaa tactgtttca     5040
aactacctgg tgtatttatt aattttggaa ctgtatgtgt gtgtcataca tcttcatagt     5100
tacgagttta agatggatgg aaatatcgat ctaggatagg tatacatgtt gatgtgggtt     5160
ttactgatgc atatacatga tggcatatgc agcatctatt catatgctct aaccttgagt     5220
acctatctat tataataaac aagtatgttt tataattatt ttgatcttga tatacttgga     5280
tgatggcata tgcagcagct atatgtggat ttttttagcc ctgccttcat acgctattta     5340
tttgcttggt actgtttctt ttgtcgatgc tcaccctgtt gtttggtgtt acttctgcag     5400
gtcgactcta gaatgtggat tgaacaagat ggattgcacg caggttctcc ggccgcttgg     5460
gtggagaggc tattcggcta tgactgggca caacagacaa tcggctgctc tgatgccgcc     5520
gtgttccggc tgtcagcgca ggggcgcccg gttctttttg tcaagaccga cctgtccggt     5580
gccctgaatg aactgcagga cgaggcagcg cggctatcgt ggctggccac gacgggcgtt     5640
ccttgcgcag ctgtgctcga cgttgtcact gaagcgggaa gggactggct gctattgggc     5700
gaagtgccgg ggcaggatct cctgtcatct caccttgctc ctgccgagaa agtatccatc     5760
atggctgatg caatgcggcg gctgcatacg cttgatccgg ctacctgccc attcgaccac     5820
caagcgaaac atcgcatcga gcgagcacgt actcggatgg aagccggtct tgtcgatcag     5880
gatgatctgg acgaagagca tcaggggctc gcgccagccg aactgttcgc caggctcaag     5940
gcgcgcatgc ccgacggcga ggatctcgtc gtgacccatg gcgatgcctg cttgccgaat     6000
atcatggtgg aaaatggccg cttttctgga ttcatcgact gtggccggct gggtgtggcg     6060
gaccgctatc aggacatagc gttggctacc cgtgatattg ctgaagagct tggcggcgaa     6120
tgggctgacc gcttcctcgt gctttacggt atcgccgctc ccgattcgca gcgcatcgcc     6180
ttctatcgcc ttcttgacga gttcttctga gatcgttcaa acatttggca ataaagtttc     6240
ttaagattga atcctgttgc cggtcttgcg atgattatca tataatttct gttgaattac     6300
gttaagcatg taataattaa catgtaatgc atgacgttat ttatgagatg ggtttttatg     6360
attagagtcc cgcaattata catttaatac gcgatagaaa acaaaatata gcgcgcaaac     6420
taggataaat tatcgcgcgc ggtgtcatct atgttactag atcggcgcgc caagggcgaa     6480
ttccagcaca ctggcggccg ttactagtgg atcgagctcg tcgactctag actcgagggc     6540
gcgccgacag gatatattgg cgggtaaacc                                     6570
```

<210>  2
<211>  6524
<212>  DNA
<213>  Artificial Sequence

<220>

<223>  T-DNA fragment event CTC75064

<400>  2
acaaattgac gcttagacaa cttaataaca cattgcggac gtttttaatg tactgaatta 60

gtactgatat cggtacctta attcgggga tctggatttt agtactggat tttggtttta 120

ggaattagaa attttattga tagaagtatt ttacaaatac aaatacatac taagggtttc 180

ttatatgctc aacacatgag cgaaacccta taggaaccct aattccctta tctgggaact 240

actcacacat tattatggag aaactcgagc ttgtcgatcg atcactcagc ctcgagggtg 300

gcggtcactg ggatgaactc gaacctgtcg atgatcacgc cggcggtgcc gctgaagttc 360

ctcacgccca cgatgttacc gagggaggag gtgaaagcgt tggcgctctc gaagtaaccg 420

aagtcgctgg attggaggtt gtccagggag gtagcggtag ctggcacggt gttggagaag 480

atggaggagt taccccagtt cacgttgagg tggatcgggg tcacggaagc gtacctcacg 540

cgcaccctgt acctggtgga ggtggatggg aagtggattg gcacctcgat gtagcccctg 600

ttctggatgt tgttgccgct gctgttgagc ctcacgaggt cgccaccggt gaagcctggg 660

ccggaaatga cggaaccgtt gaagaggaag ttgcccttca cggccgggat ttgggtgatg 720

ctgtcggagg cgatgatgtt gttgaactca gcgctgcggt ggatccagga gaacatcgga 780

gccctgatga tgctcacgga gctgttgctg aagccggagc ggaacatgga cacgtggctc 840

agcctgtggg agaagccttg cctgggtggc acgttgttgt tctgtggtgg gatctcgtcc 900

agggagtcca cggtgccgct cttcctgtag acagcggatg gcaggttgga ggaggtgccg 960

taggcgaact cggtgccgtc gagcacggac agttgctggt tgttgatacc gatgttgaag 1020

ggcctcctgt acagggtgga ggacagggtc ctgtagacac cctgacccag ttgagccacg 1080

atgcgttgct gtggagcggc gttgcccatg gtgccgtaga gcgggaaggt gaactcgggg 1140

ccgctgaagc ccactgggga ggccatgatc tggtggccgg accagtagta ctcgcccctg 1200

tgagcgtcgg tgtagatggt gatgctgttc aggatgtcca tcaggtgtgg gctcctgatg 1260

gagccctcga taccctgggc ggaaccgcgg aagctaccgt cgaagttctc cagcactggg 1320

ttggtgtaga tctccctggt gagttgggac acggtgcgga tcgggtaggt cctggagtcg 1380

tagttcggga agagggacac aatgtccagc acggtgaggg tcaactccct cctgaactgg 1440

ttgtacctga tccagtccct ggagtccgga ccccagacgc gctccaggcc ggtgttgtac 1500

cagcgcacag cgtggtcggt gtagttgcca atcagcctgg tcaggtcgtt gtagcggctg 1560

ttgatggtgg cagcatcgaa gccccacctt tggccgaaca cgctcacgtc gcgcagcacg 1620

ctgaggtgca ggttagcggc ttgcacgtac acggacagga gcggcacttg gtagttctgg 1680

acggcgaaca gtgggatagc ggtggtcagg gcgctgttca tgtcgttgaa ttgaatgcgc 1740

atttcctcgc ggagagctgg gttggtcggg tcggcctccc actccctgaa gctctcggcg 1800

tagatttggt agaggttgct caggccctcc agcctggaga tggcctggtt cctggcgaac 1860

tcttcgatcc tctggttgat cagctgctcg atttgcacca ggaaggcgtc ccattgggat 1920

ggaccgaaga taccccagat gatgtccacc aggccgagca cgaagccagc acctggcacg 1980

aactcgctga gcaggaactg ggtcaaggac agggagatgt cgatgggggt gtaaccggtc 2040

tcgatgcgct cgccacccag cacctccacc tctgggttgc tcaggcagtt gtatgggatg 2100

cactcgttga tgtttgggtt gttgtccatg gcggggttga tcaggttgat cacttctacc 2160

tacaaaaaag ctccgcacga ggctgcattt gtcacaaatc atgaaaagaa aaactaccga 2220

tgaacaatgc tgagggattc aaattctacc cacaaaaaga agaaagaaag atctagcaca 2280

tctaagcctg acgaagcagc agaaatatat aaaaatataa accatagtgc ccttttcccc 2340

tcttcctgat cttgtttagc acggcggaaa ttttaaaccc cccatcatct cccccaacaa 2400

cggcggatcg cagatctaca tccgagagcc ccattccccg cgagatccgg gccggatcca 2460

cgccggcgag agccccagcc gcgagatccc gcccctcccg cgcaccgatc tgggcgcgca 2520

cgaagccgcc tctcgcccac ccaaactacc aaggccaaag atcgagaccg agacggaaaa 2580

aaaaacggag aaagaaagag gagaggggcg gggtggttac cggcggcggc ggaggcctcc 2640

cttggatctt atggtgtgtt gtccctgtgt gttctccaat agtgtggctt gagtgtgtgg 2700

aagatggttc tagaggatct gctagagtca gcttgtcagc gtgtcctctc caaatgaaat 2760

gaacttcctt atatagagga agggtcttgc gaaggatagt gggattgtgc gtcatccctt 2820

acgtcagtgg agatatcaca tcaatccact tgctttgaag acgtggttgg aacgtcttct 2880

ttttccacga tgctcctcgt gggtgggggt ccatctttgg gaccactgtc ggcagaggca 2940

tcttcaacga tggcctttcc tttatcgcaa tgatggcatt tgtaggagcc accttccttt 3000

tccactatct tcacaataaa gtgacagata gctgggcaat ggaatccgag gaggtttccg 3060

gatattaccc tttgttgaaa agtctcaatc ggaccatcac atcaatccac ttgctttgaa 3120

gacgtggttg gaacgtcttc tttttccacg atgctcctcg tgggtggggg tccatctttg 3180

ggaccactgt cggcagaggc atcttcaacg atggcctttc ctttatcgca atgatggcat 3240

ttgtaggagc caccttcctt ttccactatc ttcacaataa agtgacagat agctgggcaa 3300

tggaatccga ggaggtttcc ggatattacc ctttgttgaa aagtctcaat cggacctgtt 3360

taaaccctga agcttacgcg tatgcctgca gtgcagcgtg acccggtcgt gcccctctct 3420

agagataatg agcattgcat gtctaagtta taaaaaatta ccacatattt tttttgtcac 3480

acttgtttga agtgcagttt atctatcttt atacatatat ttaaacttta ctctacgaat 3540

aatataatct atagtactac aataatatca gtgttttaga gaatcatata aatgaacagt 3600

tagacatggt ctaaaggaca attgagtatt ttgacaacag gactctacag ttttatcttt 3660

ttagtgtgca tgtgttctcc tttttttttg caaatagctt cacctatata atacttcatc 3720

cattttatta gtacatccat ttagggttta gggttaatgg tttttataga ctaatttttt 3780
tagtacatct attttattct attttagcct ctaaattaag aaaactaaaa ctctatttta 3840
gtttttttat ttaataattt agatataaaa tagaataaaa taaagtgact aaaaattaaa 3900
caaataccct ttaagaaatt aaaaaaacta aggaaacatt tttcttgttt cgagtagata 3960
atgccagcct gttaaacgcc gtcgacgagt ctaacggaca ccaaccagcg aaccagcagc 4020
gtcgcgtcgg gccaagcgaa gcagacggca cggcatctct gtcgctgcct ctggacccct 4080
ctcgagagtt ccgctccacc gttggacttg ctccgctgtc ggcatccaga aattgcgtgg 4140
cggagcggca gacgtgagcc ggcacggcag gcggcctcct cctcctctca cggcacggca 4200
gctacggggg attcctttcc caccgctcct tcgctttccc ttcctcgccc gccgtaataa 4260
atagacaccc cctccacacc ctctttcccc aacctcgtgt tgttcggagc gcacacacac 4320
acaaccagat ctcccccaaa tccacccgtc ggcacctccg cttcaaggta cgccgctcgt 4380
cctccccccc cccccctctc taccttctct agatcggcgt tccggtccat ggttagggcc 4440
cggtagttct acttctgttc atgtttgtgt tagatccgtg tttgtgttag atccgtgctg 4500
ctagcgttcg tacacggatg cgacctgtac gtcagacacg ttctgattgc taacttgcca 4560
gtgtttctct ttggggaatc ctgggatggc tctagccgtt ccgcagacgg gatcgatttc 4620
atgatttttt ttgtttcgtt gcatagggtt tggtttgccc ttttccttta tttcaatata 4680
tgccgtgcac ttgtttgtcg ggtcatcttt tcatgctttt ttttgtcttg gttgtgatga 4740
tgtggtctgg ttgggcggtc gttctagatc ggagtagaat tctgtttcaa actacctggt 4800
ggatttatta attttggatc tgtatgtgtg tgccatacat attcatagtt acgaattgaa 4860
gatgatggat ggaaatatcg atctaggata ggtatacatg ttgatgcggg ttttactgat 4920
gcatatacag agatgctttt tgttcgcttg gttgtgatga tgtggtgtgg ttgggcggtc 4980
gttcattcgt tctagatcgg agtagaatac tgtttcaaac tacctggtgt atttattaat 5040
tttggaactg tatgtgtgtg tcatacatct tcatagttac gagtttaaga tggatggaaa 5100
tatcgatcta ggataggtat acatgttgat gtgggtttta ctgatgcata tacatgatgg 5160
catatgcagc atctattcat atgctctaac cttgagtacc tatctattat aataaacaag 5220
tatgttttat aattattttg atcttgatat acttggatga tggcatatgc agcagctata 5280
tgtggatttt tttagccctg ccttcatacg ctatttattt gcttggtact gtttcttttg 5340
tcgatgctca ccctgttgtt tggtgttact tctgcaggtc gactctagaa tgtggattga 5400
acaagatgga ttgcacgcag gttctccggc cgcttgggtg gagaggctat tcggctatga 5460
ctgggcacaa cagacaatcg gctgctctga tgccgccgtg ttccggctgt cagcgcaggg 5520
gcgcccggtt ctttttgtca agaccgacct gtccggtgcc ctgaatgaac tgcaggacga 5580

ggcagcgcgg ctatcgtggc tggccacgac gggcgttcct tgcgcagctg tgctcgacgt 5640

tgtcactgaa gcgggaaggg actggctgct attgggcgaa gtgccggggc aggatctcct 5700

gtcatctcac cttgctcctg ccgagaaagt atccatcatg gctgatgcaa tgcggcggct 5760

gcatacgctt gatccggcta cctgcccatt cgaccaccaa gcgaaacatc gcatcgagcg 5820

agcacgtact cggatggaag ccggtcttgt cgatcaggat gatctggacg aagagcatca 5880

ggggctcgcg ccagccgaac tgttcgccag gctcaaggcg cgcatgcccg acggcgagga 5940

tctcgtcgtg acccatggcg atgcctgctt gccgaatatc atggtggaaa atggccgctt 6000

ttctggattc atcgactgtg gccggctggg tgtggcggac cgctatcagg acatagcgtt 6060

ggctacccgt gatattgctg aagagcttgg cggcgaatgg gctgaccgct tcctcgtgct 6120

ttacggtatc gccgctcccg attcgcagcg catcgccttc tatcgccttc ttgacgagtt 6180

cttctgagat cgttcaaaca tttggcaata aagtttctta agattgaatc ctgttgccgg 6240

tcttgcgatg attatcatat aatttctgtt gaattacgtt aagcatgtaa taattaacat 6300

gtaatgcatg acgttattta tgagatgggt ttttatgatt agagtcccgc aattatacat 6360

ttaatacgcg atagaaaaca aaatatagcg cgcaaactag gataaattat cgcgcgcggt 6420

gtcatctatg ttactagatc ggcgcgccaa gggcgaattc cagcacactg gcggccgtta 6480

ctagtggatc gagctcgtcg actctagact cgagggcgcg ccga 6524

<210> 3
<211> 206
<212> DNA
<213> Artificial Sequence

<220>
<223> Junction nucleotide sequence between 5′ region of insert and sugarcane genome of event CTC75064

<400> 3
gtgatcctag actttctaga aatcttagga agattactac actttagtta ttcataccaa 60

aaactaattc atagcttaac atgaccaaaa ttgctaatat ttcacaaatt gacgcttaga 120

caacttaata acacattgcg gacgttttta atgtactgaa ttagtactga tatcggtacc 180

ttaattcggg ggatctggat tttagt 206

<210> 4
<211> 206
<212> DNA
<213> Artificial sequence

<220>
<223> Junction nucleotide sequence between 3′ region of insert and sugarcane genome of event CTC75064

<400> 4
tcatctatgt tactagatcg gcgcgccaag ggcgaattcc agcacactgg cggccgttac 60

tagtggatcg agctcgtcga ctctagactc gagggcgcgc cgataccaat ttggacagaa 120

aactgacaag aacttcgaaa atacataatt ggagttctag agacacaaca aaagtgatcc 180

ttaactttat gtaaagctta ttaagt 206

<210> 5
<211> 6729
<212> DNA
<213> Artificial sequence

<220>
<223> Event CTC75064: flanking sequences and T-DNA

<400> 5
gtgatcctag actttctaga aatcttagga agattactac actttagtta ttcataccaa 60

aaactaattc atagcttaac atgaccaaaa ttgctaatat ttcacaaatt gacgcttaga 120

caacttaata acacattgcg gacgttttta atgtactgaa ttagtactga tatcggtacc 180

ttaattcggg ggatctggat tttagtactg gattttggtt ttaggaatta gaaattttat 240

tgatagaagt attttacaaa tacaaataca tactaagggt ttcttatatg ctcaacacat 300

gagcgaaacc ctataggaac cctaattccc ttatctggga actactcaca cattattatg 360

gagaaactcg agcttgtcga tcgatcactc agcctcgagg gtggcggtca ctgggatgaa 420

ctcgaacctg tcgatgatca cgccggcggt gccgctgaag ttcctcacgc ccacgatgtt 480

accgagggag gaggtgaaag cgttggcgct ctcgaagtaa ccgaagtcgc tggattggag 540

gttgtccagg gaggtagcgg tagctggcac ggtgttggag aagatggagg agttacccca 600

gttcacgttg aggtggatcg gggtcacgga agcgtacctc acgcgcaccc tgtacctggt 660

ggaggtggat gggaagtgga ttggcactcg atgtagcccc tgttctggat gttgttgccg 720

ctgctgttga gcctcacgag gtcgccaccg gtgaagcctg ggccggaaat gacggaaccg 780

ttgaagagga agttgccctt cacggccggg atttgggtga tgctgtcgga ggcgatgatg 840

ttgttgaact cagcgctgcg gtggatccag gagaacatcg gagccctgat gatgctcacg 900

gagctgttgc tgaagccgga gcggaacatg gacacgtggc tcagcctgtg ggagaagcct 960

tgcctgggtg gcacgttgtt gttctgtggt gggatctcgt ccagggagtc cacggtgccg 1020

ctcttcctgt agacagcgga tggcaggttg gaggaggtgc cgtaggcgaa ctcggtgccg 1080

tcgagcacgg acagttgctg gttgttgata ccgatgttga agggcctcct gtacagggtg 1140

gaggacaggg tcctgtagac accctgaccc agttgagcca cgatgcgttg ctgtggagcg 1200

gcgttgccca tggtgccgta gagcgggaag gtgaactcgg ggccgctgaa gcccactggg 1260

gaggccatga tctggtggcc ggaccagtag tactcgcccc tgtgagcgtc ggtgtagatg 1320

gtgatgctgt tcaggatgtc catcaggtgt gggctcctga tggagccctc gataccctgg 1380

gcggaaccgc ggaagctacc gtcgaagttc tccagcactg ggttggtgta gatctccctg 1440
gtgagttggg acacggtgcg gatcgggtag gtcctggagt cgtagttcgg gaagagggac 1500
acaatgtcca gcacggtgag ggtcaactcc ctcctgaact ggttgtacct gatccagtcc 1560
ctggagtccg gaccccagac gcgctccagg ccggtgttgt accagcgcac agcgtggtcg 1620
gtgtagttgc caatcagcct ggtcaggtcg ttgtagcggc tgttgatggt ggcagcatcg 1680
aagccccacc tttggccgaa cacgctcacg tcgcgcagca cgctgaggtg caggttagcg 1740
gcttgcacgt acacggacag gagcggcact tggtagttct ggacggcgaa cagtgggata 1800
gcggtggtca gggcgctgtt catgtcgttg aattgaatgc gcatttcctc gcggagagct 1860
gggttggtcg ggtcggcctc ccactccctg aagctctcgg cgtagatttg gtagaggttg 1920
ctcaggccct ccagcctgga gatggcctgg ttcctggcga actcttcgat cctctggttg 1980
atcagctgct cgatttgcac caggaaggcg tcccattggg atggaccgaa gataccccag 2040
atgatgtcca ccaggccgag cacgaagcca gcacctggca cgaactcgct gagcaggaac 2100
tgggtcaagg acagggagat gtcgatgggg gtgtaaccgg tctcgatgcg ctcgccaccc 2160
agcacctcca cctctgggtt gctcaggcag ttgtatggga tgcactcgtt gatgtttggg 2220
ttgttgtcca tggcggggtt gatcaggttg atcacttcta cctacaaaaa agctccgcac 2280
gaggctgcat ttgtcacaaa tcatgaaaag aaaaactacc gatgaacaat gctgagggat 2340
tcaaattcta cccacaaaaa gaagaaagaa agatctagca catctaagcc tgacgaagca 2400
gcagaaatat ataaaaatat aaaccatagt gcccttttcc cctcttcctg atcttgttta 2460
gcacggcgga aattttaaac cccccatcat ctcccccaac aacggcggat cgcagatcta 2520
catccgagag ccccattccc cgcgagatcc gggccggatc cacgccggcg agagccccag 2580
ccgcgagatc ccgcccctcc cgcgcaccga tctgggcgcg cacgaagccg cctctcgccc 2640
acccaaacta ccaaggccaa agatcgagac cgagacggaa aaaaaaacgg agaaagaaag 2700
aggagagggg cggggtggtt accggcggcg gcggaggcct cccttggatc ttatggtgtg 2760
ttgtccctgt gtgttctcca atagtgtggc ttgagtgtgt ggaagatggt tctagaggat 2820
ctgctagagt cagcttgtca gcgtgtcctc tccaaatgaa atgaacttcc ttatatagag 2880
gaagggtctt gcgaaggata gtgggattgt gcgtcatccc ttacgtcagt ggagatatca 2940
catcaatcca cttgctttga agacgtggtt ggaacgtctt ctttttccac gatgctcctc 3000
gtgggtgggg gtccatcttt gggaccactg tcggcagagg catcttcaac gatggccttt 3060
cctttatcgc aatgatggca tttgtaggag ccaccttcct tttccactat cttcacaata 3120
aagtgacaga tagctgggca atggaatccg aggaggtttc cggatattac cctttgttga 3180
aaagtctcaa tcggaccatc acatcaatcc acttgctttg aagacgtggt tggaacgtct 3240
tctttttcca cgatgctcct cgtgggtggg ggtccatctt tgggaccact gtcggcagag 3300

gcatcttcaa cgatggcctt tcctttatcg caatgatggc atttgtagga gccaccttcc 3360
ttttccacta tcttcacaat aaagtgacag atagctgggc aatggaatcc gaggaggttt 3420
ccggatatta ccctttgttg aaaagtctca atcggacctg tttaaaccct gaagcttacg 3480
cgtatgcctg cagtgcagcg tgacccggtc gtgcccctct ctagagataa tgagcattgc 3540
atgtctaagt tataaaaaat taccacatat ttttttgtc acacttgttt gaagtgcagt 3600
ttatctatct ttatacatat atttaaactt tactctacga ataatataat ctatagtact 3660
acaataatat cagtgtttta gagaatcata taaatgaaca gttagacatg gtctaaagga 3720
caattgagta ttttgacaac aggactctac agttttatct ttttagtgtg catgtgttct 3780
cctttttttt tgcaaatagc ttcacctata taatacttca tccattttat tagtacatcc 3840
atttagggtt tagggttaat ggtttttata gactaatttt tttagtacat ctattttatt 3900
ctattttagc ctctaaatta agaaaactaa aactctattt tagttttttt atttaataat 3960
ttagatataa aatagaataa aataaagtga ctaaaaatta aacaaatacc ctttaagaaa 4020
ttaaaaaaac taaggaaaca tttttcttgt ttcgagtaga taatgccagc ctgttaaacg 4080
ccgtcgacga gtctaacgga caccaaccag cgaaccagca gcgtcgcgtc gggccaagcg 4140
aagcagacgg cacggcatct ctgtcgctgc ctctggaccc ctctcgagag ttccgctcca 4200
ccgttggact tgctccgctg tcggcatcca gaaattgcgt ggcggagcgg cagacgtgag 4260
ccggcacggc aggcggcctc ctcctcctct cacggcacgg cagctacggg ggattccttt 4320
cccaccgctc cttcgctttc ccttcctcgc ccgccgtaat aaatagacac cccctccaca 4380
ccctctttcc ccaacctcgt gttgttcgga gcgcacacac acacaaccag atctccccca 4440
aatccacccg tcggcacctc cgcttcaagg tacgccgctc gtcctccccc ccccccctc 4500
tctaccttct ctagatcggc gttccggtcc atggttaggg cccggtagtt ctacttctgt 4560
tcatgtttgt gttagatccg tgtttgtgtt agatccgtgc tgctagcgtt cgtacacgga 4620
tgcgacctgt acgtcagaca cgttctgatt gctaacttgc cagtgtttct ctttggggaa 4680
tcctgggatg gctctagccg ttccgcagac gggatcgatt tcatgatttt ttttgtttcg 4740
ttgcataggg tttggtttgc ccttttcctt tatttcaata tatgccgtgc acttgtttgt 4800
cgggtcatct tttcatgctt ttttttgtct tggttgtgat gatgtggtct ggttgggcgg 4860
tcgttctaga tcggagtaga attctgtttc aaactacctg gtggatttat taattttgga 4920
tctgtatgtg tgtgccatac atattcatag ttacgaattg aagatgatgg atggaaatat 4980
cgatctagga taggtataca tgttgatgcg ggttttactg atgcatatac agagatgctt 5040
tttgttcgct tggttgtgat gatgtggtgt ggttgggcgg tcgttcattc gttctagatc 5100
ggagtagaat actgtttcaa actacctggt gtatttatta attttggaac tgtatgtgtg 5160

tgtcatacat cttcatagtt acgagtttaa gatggatgga aatatcgatc taggataggt 5220

atacatgttg atgtgggttt tactgatgca tatacatgat ggcatatgca gcatctattc 5280

atatgctcta accttgagta cctatctatt ataataaaca agtatgtttt ataattattt 5340

tgatcttgat atacttggat gatggcatat gcagcagcta tatgtggatt tttttagccc 5400

tgccttcata cgctatttat ttgcttggta ctgtttcttt tgtcgatgct caccctgttg 5460

tttggtgtta cttctgcagg tcgactctag aatgtggatt gaacaagatg gattgcacgc 5520

aggttctccg gccgcttggg tggagaggct attcggctat gactgggcac aacagacaat 5580

cggctgctct gatgccgccg tgttccggct gtcagcgcag gggcgcccgg ttctttttgt 5640

caagaccgac ctgtccggtg ccctgaatga actgcaggac gaggcagcgc ggctatcgtg 5700

gctggccacg acgggcgttc cttgcgcagc tgtgctcgac gttgtcactg aagcgggaag 5760

ggactggctg ctattgggcg aagtgccggg gcaggatctc ctgtcatctc accttgctcc 5820

tgccgagaaa gtatccatca tggctgatgc aatgcggcgg ctgcatacgc ttgatccggc 5880

tacctgccca ttcgaccacc aagcgaaaca tcgcatcgag cgagcacgta ctcggatgga 5940

agccggtctt gtcgatcagg atgatctgga cgaagagcat caggggctcg cgccagccga 6000

actgttcgcc aggctcaagg cgcgcatgcc cgacggcgag gatctcgtcg tgacccatgg 6060

cgatgcctgc ttgccgaata tcatggtgga aaatggccgc ttttctggat tcatcgactg 6120

tggccggctg ggtgtggcgg accgctatca ggacatagcg ttggctaccc gtgatattgc 6180

tgaagagctt ggcggcgaat gggctgaccg cttcctcgtg ctttacggta tcgccgctcc 6240

cgattcgcag cgcatcgcct tctatcgcct tcttgacgag ttcttctgag atcgttcaaa 6300

catttggcaa taaagtttct taagattgaa tcctgttgcc ggtcttgcga tgattatcat 6360

ataatttctg ttgaattacg ttaagcatgt aataattaac atgtaatgca tgacgttatt 6420

tatgagatgg gtttttatga ttagagtccc gcaattatac atttaatacg cgatagaaaa 6480

caaaatatag cgcgcaaact aggataaatt atcgcgcgcg gtgtcatcta tgttactaga 6540

tcggcgcgcc aagggcgaat tccagcacac tggcggccgt tactagtgga tcgagctcgt 6600

cgactctaga ctcgagggcg cgccgatacc aatttggaca gaaaactgac aagaacttcg 6660

aaaatacata attggagttc tagagacaca acaaaagtga tccttaactt tatgtaaagc 6720

ttattaagt 6729

<210> 6
<211> 19
<212> DNA
<213> Artificial sequence

<220>
<223> primer forward (Right Border)

<400> 6
caagggcgaa ttccagcac 19

<210> 7
<211> 24
<212> DNA
<213> Artificial sequence

<220>
<223> primer reverse (Right border)

<400> 7
gttcttgtca gttttctgtc caaa 24

<210> 8
<211> 26
<212> DNA
<213> Artificial sequence

<220>
<223> primer forward (Left Border)

<400> 8
catgaccaaa attgctaata tttcac 26

<210> 9
<211> 25
<212> DNA
<213> Artificial sequence

<220>
<223> primer reverse (Left Border)

<400> 9
aatccagtac taaaatccag atccc 25

<210> 10
<211> 18
<212> DNA
<213> Artificial sequence

<220>
<223> probe (Right border)

<400> 10
ccgttactag tggatcga 18

<210> 11
<211> 22
<212> DNA
<213> Artificial sequence

<220>
<223> probe (Left border)

<400> 11
cttaataaca cattgcggac gt 22

<210> 12
<211> 107
<212> DNA
<213> Artificial sequence

<220>
<223> Amplicon Event CTC75064 (Right Border)

<400> 12
caagggcgaa ttccagcaca ctggcggccg ttactagtgg atcgagctcg tcgactctag 60

actcgagggc gcgccgatac caatttggac agaaaactga caagaac 107

<210> 13
<211> 135
<212> DNA
<213> Artificial sequence

<220>
<223> Amplicon Event CTC75064 (Left Border)

<400> 13
catgaccaaa attgctaata tttcacaaat tgacgcttag acaacttaat aacacattgc 60

ggacgttttt aatgtactga attagtactg atatcggtac cttaattcgg gggatctgga 120

ttttagtact ggatt 135

<210> 14
<211> 12800
<212> DNA
<213> Artificial sequence

<220>
<223> Binary plasmid comprising cry1Ac and nptII genes

<400> 14
tggcaggata tattgtggtg taaacaaatt gacgcttaga caacttaata acacattgcg 60

gacgttttta atgtactgaa ttagtactga tatcggtacc ttaattcggg ggatctggat 120

tttagtactg gattttggtt ttaggaatta gaaattttat tgatagaagt attttacaaa 180

tacaaataca tactaagggt ttcttatatg ctcaacacat gagcgaaacc ctataggaac 240

cctaattccc ttatctggga actactcaca cattattatg gagaaactcg agcttgtcga 300

tcgatcactc agcctcgagg gtggcggtca ctgggatgaa ctcgaacctg tcgatgatca 360

cgccggcggt gccgctgaag ttcctcacgc ccacgatgtt accgagggag gaggtgaaag 420

cgttggcgct ctcgaagtaa ccgaagtcgc tggattggag gttgtccagg gaggtagcgg 480

tagctggcac ggtgttggag aagatggagg agttacccca gttcacgttg aggtggatcg 540

gggtcacgga agcgtacctc acgcgcaccc tgtacctggt ggaggtggat gggaagtgga 600

ttggcacctc gatgtagccc ctgttctgga tgttgttgcc gctgctgttg agcctcacga 660

ggtcgccacc ggtgaagcct gggccggaaa tgacggaacc gttgaagagg aagttgccct 720

tcacggccgg gatttgggtg atgctgtcgg aggcgatgat gttgttgaac tcagcgctgc 780
ggtggatcca ggagaacatc ggagccctga tgatgctcac ggagctgttg ctgaagccgg 840
agcggaacat ggacacgtgg ctcagcctgt gggagaagcc ttgcctgggt ggcacgttgt 900
tgttctgtgg tgggatctcg tccagggagt ccacggtgcc gctcttcctg tagacagcgg 960
atggcaggtt ggaggaggtg ccgtaggcga actcggtgcc gtcgagcacg gacagttgct 1020
ggttgttgat accgatgttg aagggcctcc tgtacagggt ggaggacagg gtcctgtaga 1080
caccctgacc cagttgagcc acgatgcgtt gctgtggagc ggcgttgccc atggtgccgt 1140
agagcgggaa ggtgaactcg gggccgctga agcccactgg ggaggccatg atctggtggc 1200
cggaccagta gtactcgccc ctgtgagcgt cggtgtagat ggtgatgctg ttcaggatgt 1260
ccatcaggtg tgggctcctg atggagccct cgataccctg ggcggaaccg cggaagctac 1320
cgtcgaagtt ctccagcact gggttggtgt agatctccct ggtgagttgg gacacggtgc 1380
ggatcgggta ggtcctggag tcgtagttcg ggaagaggga cacaatgtcc agcacggtga 1440
gggtcaactc cctcctgaac tggttgtacc tgatccagtc cctggagtcc ggaccccaga 1500
cgcgctccag gccggtgttg taccagcgca cagcgtggtc ggtgtagttg ccaatcagcc 1560
tggtcaggtc gttgtagcgg ctgttgatgg tggcagcatc gaagccccac ctttggccga 1620
acacgctcac gtcgcgcagc acgctgaggt gcaggttagc ggcttgcacg tacacggaca 1680
ggagcggcac ttggtagttc tggacggcga acagtgggat agcggtggtc agggcgctgt 1740
tcatgtcgtt gaattgaatg cgcatttcct cgcggagagc tgggttggtc gggtcggcct 1800
cccactccct gaagctctcg gcgtagattt ggtagaggtt gctcaggccc tccagcctgg 1860
agatggcctg gttcctggcg aactcttcga tcctctggtt gatcagctgc tcgatttgca 1920
ccaggaaggc gtcccattgg gatggaccga agatacccca gatgatgtcc accaggccga 1980
gcacgaagcc agcacctggc acgaactcgc tgagcaggaa ctgggtcaag gacagggaga 2040
tgtcgatggg ggtgtaaccg gtctcgatgc gctcgccacc cagcacctcc acctctgggt 2100
tgctcaggca gttgtatggg atgcactcgt tgatgtttgg gttgttgtcc atggcggggt 2160
tgatcaggtt gatcacttct acctacaaaa aagctccgca cgaggctgca tttgtcacaa 2220
atcatgaaaa gaaaaactac cgatgaacaa tgctgaggga ttcaaattct acccacaaaa 2280
agaagaaaga aagatctagc acatctaagc ctgacgaagc agcagaaata tataaaaata 2340
taaaccatag tgcccttttc ccctcttcct gatcttgttt agcacggcgg aaattttaaa 2400
ccccccatca tctcccccaa caacggcgga tcgcagatct acatccgaga gccccattcc 2460
ccgcgagatc cgggccggat ccacgccggc gagagcccca gccgcgagat cccgcccctc 2520
ccgcgcaccg atctgggcgc gcacgaagcc gcctctcgcc cacccaaact accaaggcca 2580

aagatcgaga ccgagacgga aaaaaaaacg gagaaagaaa gaggagaggg gcggggtggt 2640

taccggcggc ggcggaggcc tcccttggat cttatggtgt gttgtccctg tgtgttctcc 2700

aatagtgtgg cttgagtgtg tggaagatgg ttctagagga tctgctagag tcagcttgtc 2760

agcgtgtcct ctccaaatga aatgaacttc cttatataga ggaagggtct tgcgaaggat 2820

agtgggattg tgcgtcatcc cttacgtcag tggagatatc acatcaatcc acttgctttg 2880

aagacgtggt tggaacgtct tctttttcca cgatgctcct cgtgggtggg ggtccatctt 2940

tgggaccact gtcggcagag gcatcttcaa cgatggcctt tcctttatcg caatgatggc 3000

atttgtagga gccaccttcc ttttccacta tcttcacaat aaagtgacag atagctgggc 3060

aatggaatcc gaggaggttt ccggatatta ccctttgttg aaaagtctca atcggaccat 3120

cacatcaatc cacttgcttt gaagacgtgg ttggaacgtc ttctttttcc acgatgctcc 3180

tcgtgggtgg gggtccatct ttgggaccac tgtcggcaga ggcatcttca acgatggcct 3240

ttcctttatc gcaatgatgg catttgtagg agccaccttc cttttccact atcttcacaa 3300

taaagtgaca gatagctggg caatggaatc cgaggaggtt tccggatatt accctttgtt 3360

gaaaagtctc aatcggacct gtttaaaccc tgaagcttac gcgtatgcct gcagtgcagc 3420

gtgacccggt cgtgcccctc tctagagata atgagcattg catgtctaag ttataaaaaa 3480

ttaccacata ttttttttgt cacacttgtt tgaagtgcag tttatctatc tttatacata 3540

tatttaaact ttactctacg aataatataa tctatagtac tacaataata tcagtgtttt 3600

agagaatcat ataaatgaac agttagacat ggtctaaagg acaattgagt attttgacaa 3660

caggactcta cagttttatc tttttagtgt gcatgtgttc tccttttttt ttgcaaatag 3720

cttcacctat ataatacttc atccatttta ttagtacatc catttagggt ttagggttaa 3780

tggtttttat agactaattt ttttagtaca tctattttat tctattttag cctctaaatt 3840

aagaaaacta aaactctatt ttagtttttt tatttaataa tttagatata aaatagaata 3900

aaataaagtg actaaaaatt aaacaaatac cctttaagaa attaaaaaaa ctaaggaaac 3960

atttttcttg tttcgagtag ataatgccag cctgttaaac gccgtcgacg agtctaacgg 4020

acaccaacca gcgaaccagc agcgtcgcgt cgggccaagc gaagcagacg gcacggcatc 4080

tctgtcgctg cctctggacc cctctcgaga gttccgctcc accgttggac ttgctccgct 4140

gtcggcatcc agaaattgcg tggcggagcg gcagacgtga gccggcacgg caggcggcct 4200

cctcctcctc tcacggcacg gcagctacgg gggattcctt tcccaccgct ccttcgcttt 4260

cccttcctcg cccgccgtaa taaatagaca ccccctccac accctctttc cccaacctcg 4320

tgttgttcgg agcgcacaca cacacaacca gatctccccc aaatccaccc gtcggcacct 4380

ccgcttcaag gtacgccgct cgtcctcccc cccccccct ctctaccttc tctagatcgg 4440

cgttccggtc catggttagg gcccggtagt tctacttctg ttcatgtttg tgttagatcc 4500

gtgtttgtgt tagatccgtg ctgctagcgt tcgtacacgg atgcgacctg tacgtcagac 4560

acgttctgat tgctaacttg ccagtgtttc tctttgggga atcctgggat ggctctagcc 4620

gttccgcaga cgggatcgat ttcatgattt tttttgtttc gttgcatagg gtttggtttg 4680

ccctttttcct ttatttcaat atatgccgtg cacttgtttg tcgggtcatc ttttcatgct 4740

ttttttttgtc ttggttgtga tgatgtggtc tggttgggcg gtcgttctag atcggagtag 4800

aattctgttt caaactacct ggtggattta ttaattttgg atctgtatgt gtgtgccata 4860

catattcata gttacgaatt gaagatgatg gatggaaata tcgatctagg ataggtatac 4920

atgttgatgc gggttttact gatgcatata cagagatgct ttttgttcgc ttggttgtga 4980

tgatgtggtg tggttgggcg gtcgttcatt cgttctagat cggagtagaa tactgtttca 5040

aactacctgg tgtatttatt aattttggaa ctgtatgtgt gtgtcataca tcttcatagt 5100

tacgagttta agatggatgg aaatatcgat ctaggatagg tatacatgtt gatgtgggtt 5160

ttactgatgc atatacatga tggcatatgc agcatctatt catatgctct aaccttgagt 5220

acctatctat tataataaac aagtatgttt tataattatt ttgatcttga tatacttgga 5280

tgatggcata tgcagcagct atatgtggat ttttttagcc ctgccttcat acgctattta 5340

tttgcttggt actgtttctt ttgtcgatgc tcaccctgtt gtttggtgtt acttctgcag 5400

gtcgactcta gaatgtggat tgaacaagat ggattgcacg caggttctcc ggccgcttgg 5460

gtggagaggc tattcggcta tgactgggca caacagacaa tcggctgctc tgatgccgcc 5520

gtgttccggc tgtcagcgca ggggcgcccg gttctttttg tcaagaccga cctgtccggt 5580

gccctgaatg aactgcagga cgaggcagcg cggctatcgt ggctggccac gacgggcgtt 5640

ccttgcgcag ctgtgctcga cgttgtcact gaagcgggaa gggactggct gctattgggc 5700

gaagtgccgg ggcaggatct cctgtcatct caccttgctc ctgccgagaa agtatccatc 5760

atggctgatg caatgcggcg gctgcatacg cttgatccgg ctacctgccc attcgaccac 5820

caagcgaaac atcgcatcga gcgagcacgt actcggatgg aagccggtct tgtcgatcag 5880

gatgatctgg acgaagagca tcaggggctc gcgccagccg aactgttcgc caggctcaag 5940

gcgcgcatgc ccgacggcga ggatctcgtc gtgacccatg gcgatgcctg cttgccgaat 6000

atcatggtgg aaaatggccg cttttctgga ttcatcgact gtggccggct gggtgtggcg 6060

gaccgctatc aggacatagc gttggctacc cgtgatattg ctgaagagct tggcggcgaa 6120

tgggctgacc gcttcctcgt gctttacggt atcgccgctc ccgattcgca gcgcatcgcc 6180

ttctatcgcc ttcttgacga gttcttctga gatcgttcaa acatttggca ataaagtttc 6240

ttaagattga atcctgttgc cggtcttgcg atgattatca tataatttct gttgaattac 6300

gttaagcatg taataattaa catgtaatgc atgacgttat ttatgagatg ggtttttatg 6360

attagagtcc cgcaattata catttaatac gcgatagaaa acaaaatata gcgcgcaaac 6420
taggataaat tatcgcgcgc ggtgtcatct atgttactag atcggcgcgc caagggcgaa 6480
ttccagcaca ctggcggccg ttactagtgg atcgagctcg tcgactctag actcgagggc 6540
gcgccgacag gatatattgg cgggtaaacc ttagaataac ggatatttaa aagggcgtga 6600
aaaggtttat ccgttcgtcc atttgtatgt gcatgccaac cacagggttc ccctcgggat 6660
caaagtactt tgatccaacc cctccgctgc tatagtgcag tcggcttctg acgttcagtg 6720
cagccgtctt ctgaaaacga catgtcgcac aagtcctaag ttacgcgaca ggctgccgcc 6780
ctgccctttt cctggcgttt tcttgtcgcg tgttttagtc gcataaagta gaatacttgc 6840
gactagaacc ggagacatta cgccatgaac aagagcgccg ccgctggcct gctgggctat 6900
gcccgcgtca gcaccgacga ccaggacttg accaaccaac gggccgaact gcacgcggcc 6960
ggctgcacca agctgttttc cgagaagatc accggcacca ggcgcgaccg cccggagctg 7020
gccaggatgc ttgaccacct acgccctggc gacgttgtga cagtgaccag gctagaccgc 7080
ctggcccgca gcacccgcga cctactggac attgccgagc gcatccagga ggccggcgcg 7140
ggcctgcgta gcctggcaga gccgtgggcc gacaccacca cgccggccgg ccgcatggtg 7200
ttgaccgtgt tcgccggcat tgccgagttc gagcgttccc taatcatcga ccgcacccgg 7260
agcgggcgcg aggccgccaa ggcccgaggc gtgaagtttg gcccccgccc taccctcacc 7320
ccggcacaga tcgcgcacgc ccgcgagctg atcgaccagg aaggccgcac cgtgaaagag 7380
gcggctgcac tgcttggcgt gcatcgctcg accctgtacc gcgcacttga gcgcagcgag 7440
gaagtgacgc ccaccgaggc caggcggcgc ggtgccttcc gtgaggacgc attgaccgag 7500
gccgacgccc tggcggccgc cgagaatgaa cgccaagagg aacaagcatg aaaccgcacc 7560
aggacggcca ggacgaaccg tttttcatta ccgaagagat cgaggcggag atgatcgcgg 7620
ccgggtacgt gttcgagccg cccgcgcacg tctcaaccgt gcggctgcat gaaatcctgg 7680
ccggtttgtc tgatgccaag ctggcggcct ggccggccag cttggccgct gaagaaaccg 7740
agcgccgccg tctaaaaagg tgatgtgtat ttgagtaaaa cagcttgcgt catgcggtcg 7800
ctgcgtatat gatgcgatga gtaaataaac aaatacgcaa ggggaacgca tgaaggttat 7860
cgctgtactt aaccagaaag gcgggtcagg caagacgacc atcgcaaccc atctagcccg 7920
cgccctgcaa ctcgccgggg ccgatgttct gttagtcgat tccgatcccc agggcagtgc 7980
ccgcgattgg gcggccgtgc gggaagatca accgctaacc gttgtcggca tcgaccgccc 8040
gacgattgac cgcgacgtga aggccatcgg ccggcgcgac ttcgtagtga tcgacggagc 8100
gccccaggcg gcggacttgg ctgtgtccgc gatcaaggca gccgacttcg tgctgattcc 8160
ggtgcagcca agcccttacg acatatgggc caccgccgac ctggtggagc tggttaagca 8220
gcgcattgag gtcacggatg gaaggctaca agcggccttt gtcgtgtcgc gggcgatcaa 8280

aggcacgcgc atcggcggtg aggttgccga ggcgctggcc gggtacgagc tgcccattct 8340

tgagtcccgt atcacgcagc gcgtgagcta cccaggcact gccgccgccg gcacaaccgt 8400

tcttgaatca gaacccgagg gcgacgctgc ccgcgaggtc caggcgctgg ccgctgaaat 8460

taaatcaaaa ctcatttgag ttaatgaggt aaagagaaaa tgagcaaaag cacaaacacg 8520

ctaagtgccg gccgtccgag cgcacgcagc agcaaggctg caacgttggc cagcctggca 8580

gacacgccag ccatgaagcg ggtcaacttt cagttgccgg cggaggatca caccaagctg 8640

aagatgtacg cggtacgcca aggcaagacc attaccgagc tgctatctga atacatcgcg 8700

cagctaccag agtaaatgag caaatgaata aatgagtaga tgaattttag cggctaaagg 8760

aggcggcatg gaaaatcaag aacaaccagg caccgacgcc gtggaatgcc ccatgtgtgg 8820

aggaacgggc ggttggccag gcgtaagcgg ctgggttgtc tgccggccct gcaatggcac 8880

tggaaccccc aagcccgagg aatcggcgtg acggtcgcaa accatccggc ccggtacaaa 8940

tcggcgcggc gctgggtgat gacctggtgg agaagttgaa ggccgcgcag gccgcccagc 9000

ggcaacgcat cgaggcagaa gcacgccccg gtgaatcgtg gcaagcggcc gctgatcgaa 9060

tccgcaaaga atcccggcaa ccgccggcag ccggtgcgcc gtcgattagg aagccgccca 9120

agggcgacga gcaaccagat tttttcgttc cgatgctcta tgacgtgggc acccgcgata 9180

gtcgcagcat catggacgtg gccgttttcc gtctgtcgaa gcgtgaccga cgagctggcg 9240

aggtgatccg ctacgagctt ccagacgggc acgtagaggt ttccgcaggg ccggccggca 9300

tggccagtgt gtgggattac gacctggtac tgatggcggt ttcccatcta accgaatcca 9360

tgaaccgata ccgggaaggg aagggagaca agcccggccg cgtgttccgt ccacacgttg 9420

cggacgtact caagttctgc cggcgagccg atggcggaaa gcagaaagac gacctggtag 9480

aaacctgcat tcggttaaac accacgcacg ttgccatgca gcgtacgaag aaggccaaga 9540

acggccgcct ggtgacggta tccgagggtg aagccttgat tagccgctac aagatcgtaa 9600

agagcgaaac cgggcggccg gagtacatcg agatcgagct agctgattgg atgtaccgcg 9660

agatcacaga aggcaagaac ccggacgtgc tgacggttca ccccgattac tttttgatcg 9720

atcccggcat cggccgtttt ctctaccgcc tggcacgccg cgccgcaggc aaggcagaag 9780

ccagatggtt gttcaagacg atctacgaac gcagtggcag cgccggagag ttcaagaagt 9840

tctgtttcac cgtgcgcaag ctgatcgggt caaatgacct gccggagtac gatttgaagg 9900

aggaggcggg gcaggctggc ccgatcctag tcatgcgcta ccgcaacctg atcgagggcg 9960

aagcatccgc cggttcctaa tgtacggagc agatgctagg gcaaattgcc ctagcagggg 10020

aaaaaggtcg aaaaggtctc tttcctgtgg atagcacgta cattgggaac ccaaagccgt 10080

acattgggaa ccggaacccg tacattggga acccaaagcc gtacattggg aaccggtcac 10140

acatgtaagt gactgatata aaagagaaaa aaggcgattt ttccgcctaa aactctttaa 10200
aacttattaa aactcttaaa acccgcctgg cctgtgcata actgtctggc cagcgcacag 10260
ccgaagagct gcaaaaagcg cctacccttc ggtcgctgcg ctccctacgc cccgccgctt 10320
cgcgtcggcc tatcgcggcc gctggccgct caaaaatggc tggcctacgg ccaggcaatc 10380
taccagggcg cggacaagcc gcgccgtcgc cactcgaccg ccggcgccca catcaaggca 10440
ccctgcctcg cgcgtttcgg tgatgacggt gaaaacctct gacacatgca gctcccggag 10500
acggtcacag cttgtctgta agcggatgcc gggagcagac aagcccgtca gggcgcgtca 10560
gcgggtgttg gcgggtgtcg gggcgcagcc atgacccagt cacgtagcga tagcggagtg 10620
tatactggct taactatgcg gcatcagagc agattgtact gagagtgcac catatgcggt 10680
gtgaaatacc gcacagatgc gtaaggagaa aataccgcat caggcgctct tccgcttcct 10740
cgctcactga ctcgctgcgc tcggtcgttc ggctgcggcg agcggtatca gctcactcaa 10800
aggcggtaat acggttatcc acagaatcag gggataacgc aggaaagaac atgtgagcaa 10860
aaggccagca aaaggccagg aaccgtaaaa aggccgcgtt gctggcgttt ttccataggc 10920
tccgcccccc tgacgagcat cacaaaaatc gacgctcaag tcagaggtgg cgaaacccga 10980
caggactata aagataccag gcgtttcccc ctggaagctc cctcgtgcgc tctcctgttc 11040
cgaccctgcc gcttaccgga tacctgtccg cctttctccc ttcgggaagc gtggcgcttt 11100
ctcatagctc acgctgtagg tatctcagtt cggtgtaggt cgttcgctcc aagctgggct 11160
gtgtgcacga accccccgtt cagcccgacc gctgcgcctt atccggtaac tatcgtcttg 11220
agtccaaccc ggtaagacac gacttatcgc cactggcagc agccactggt aacaggatta 11280
gcagagcgag gtatgtaggc ggtgctacag agttcttgaa gtggtggcct aactacggct 11340
acactagaag gacagtattt ggtatctgcg ctctgctgaa gccagttacc ttcggaaaaa 11400
gagttggtag ctcttgatcc ggcaaacaaa ccaccgctgg tagcggtggt ttttttgttt 11460
gcaagcagca gattacgcgc agaaaaaaag gatctcaaga agatcctttg atcttttcta 11520
cggggtctga cgctcagtgg aacgaaaact cacgttaagg gattttggtc atgcattcta 11580
ggtattattt gccaacgacc ttcgtgatct cgcccttgac atagtggaca aattcttcga 11640
gctggtcggc ccgggacgcg agacggtctt cttcttggcc cagataggct tggcgcgctt 11700
cgaggatcac gggctggtat tgcgccggaa ggcgctccat cgcccagtcg gcggcgacat 11760
ccttcggcgc gatcttgccg gtaaccgccg agtaccaaat ccggctcagc gtaaggacca 11820
cattgcgctc atcgcccgcc caatccggcg gggagttcca cagggtcagc gtctcgttca 11880
gtgcttcgaa cagatcctgt tccggcaccg ggtcgaaaag ttcctcggcc gcggggccga 11940
cgagggccac gctatgctcc cgggccttgg tgagcaggat cgccagatca atgtcgatgg 12000
tggccggttc aaagataccc gccagaatat cattacgctg ccattcgccg aactggagtt 12060

cgcgtttggc cggatagcgc caggggatga tgtcatcgtg caccacaatc gtcacctcaa 12120

ccgcgcgcag gatttcgctc tcgccggggg aggcggacgt ttccagaagg tcgttgataa 12180

gcgcgcggcg cgtggtctcg tcgagacgga cggtaacggt gacaagcagg tcgatgtccg 12240

aatggggctt aaggccgccg tcaacggcgc taccatacag atgcacggcg aggagggtcg 12300

gttcgaggtg gcgctcgatg acacccacga cttccgacag ctgggtggac acctcggcga 12360

tgaccgcttc acccatttat tatttccttc ctcttttcta cagtatttaa agatacccca 12420

agaagctaat tataacaaga cgaactccaa ttcactgttc cttgcattct aaaaccttaa 12480

ataccagaaa acagcttttt caaagttgtt ttcaaagttg gcgtataaca tagtatcgac 12540

ggagccgatt ttgaaaccgc ggtgatcaca ggcagcaacg ctctgtcatc gttacaatca 12600

acatgctacc ctccgcgaga tcatccgtgt ttcaaacccg gcagcttagt tgccgttctt 12660

ccgaatagca tcggtaacat gagcaaagtc tgccgcctta caacggctct cccgctgacg 12720

ccgtcccgga ctgatgggct gcctgtatcg agtggtgatt ttgtgccgag ctgccggtcg 12780

gggagctgtt ggctggctgg 12800

<210> 15
<211> 22
<212> DNA
<213> Artificial sequence

<220>
<223> primer forward: sugarcane poly-ubiquitin gene

<400> 15
accattaccc tggaggttga ga 22

<210> 16
<211> 20
<212> DNA
<213> Artificial sequence

<220>
<223> primer reverse: sugarcane poly-ubiquitin gene

<400> 16
gtcctggatc ttcgccttca 20

<210> 17
<211> 16
<212> DNA
<213> Artificial sequence

<220>
<223> probe sugarcane poly-ubiquitin gene

<400> 17
ctctgacacc atcgac 16

<210>  18
<211>  26
<212>  DNA
<213>  Artificial sequence

<220>
<223>  Junction sequence between 5'region of insert and sugarcane genome of event CTC75064

<400>  18
ttgctaatat ttcacaaatt gacgct    26

<210>  19
<211>  26
<212>  DNA
<213>  Artificial sequence

<220>
<223>  Junction sequence between 3'region of insert and sugarcane genome of event CTC75064

<400>  19
gagggcgcgc cgataccaat ttggac    26

<210>  20
<211>  1848
<212>  DNA
<213>  Artificial sequence

<220>
<223>  Sugarcane optimized truncate cry1Ac gene (B. thurigiensis)

<400>  20
atggacaaca acccaaacat caacgagtgc atcccataca actgcctgag caacccagag    60

gtggaggtgc tgggtggcga gcgcatcgag accggttaca cccccatcga catctccctg    120

tccttgaccc agttcctgct cagcgagttc gtgccaggtg ctggcttcgt gctcggcctg    180

gtggacatca tctggggtat cttcggtcca tcccaatggg acgccttcct ggtgcaaatc    240

gagcagctga tcaaccagag gatcgaagag ttcgccagga accaggccat ctccaggctg    300

gagggcctga gcaacctcta ccaaatctac gccgagagct tcagggagtg ggaggccgac    360

ccgaccaacc cagctctccg cgaggaaatg cgcattcaat tcaacgacat gaacagcgcc    420

ctgaccaccg ctatcccact gttcgccgtc cagaactacc aagtgccgct cctgtccgtg    480

tacgtgcaag ccgctaacct gcacctcagc gtgctgcgcg acgtgagcgt gttcggccaa    540

aggtggggct tcgatgctgc caccatcaac agccgctaca acgacctgac caggctgatt    600

ggcaactaca ccgaccacgc tgtgcgctgg tacaacaccg gcctggagcg cgtctggggt    660

ccggactcca gggactggat caggtacaac cagttcagga gggagttgac cctcaccgtg    720

ctggacattg tgtccctctt cccgaactac gactccagga cctacccgat ccgcaccgtg    780

tcccaactca ccagggagat ctacaccaac ccagtgctgg agaacttcga cggtagcttc    840

cgcggttccg cccagggtat cgagggctcc atcaggagcc cacacctgat ggacatcctg 900

aacagcatca ccatctacac cgacgctcac aggggcgagt actactggtc cggccaccag 960

atcatggcct ccccagtggg cttcagcggc cccgagttca ccttcccgct ctacggcacc 1020

atgggcaacg ccgctccaca gcaacgcatc gtggctcaac tgggtcaggg tgtctacagg 1080

accctgtcct ccaccctgta caggaggccc ttcaacatcg gtatcaacaa ccagcaactg 1140

tccgtgctcg acggcaccga gttcgcctac ggcacctcct ccaacctgcc atccgctgtc 1200

tacaggaaga gcggcaccgt ggactccctg gacgagatcc caccacagaa caacaacgtg 1260

ccacccaggc aaggcttctc ccacaggctg agccacgtgt ccatgttccg ctccggcttc 1320

agcaacagct ccgtgagcat catcagggct ccgatgttct cctggatcca ccgcagcgct 1380

gagttcaaca acatcatcgc ctccgacagc atcacccaaa tcccggccgt gaagggcaac 1440

ttcctcttca acggttccgt catttccggc ccaggcttca ccggtggcga cctcgtgagg 1500

ctcaacagca gcggcaacaa catccagaac aggggctaca tcgaggtgcc aatccacttc 1560

ccatccacct ccaccaggta cagggtgcgc gtgaggtacg cttccgtgac cccgatccac 1620

ctcaacgtga actggggtaa ctcctccatc ttctccaaca ccgtgccagc taccgctacc 1680

tccctggaca acctccaatc cagcgacttc ggttacttcg agagcgccaa cgctttcacc 1740

tcctccctcg gtaacatcgt gggcgtgagg aacttcagcg gcaccgccgg cgtgatcatc 1800

gacaggttcg agttcatccc agtgaccgcc accctcgagg ctgagtga 1848

<210> 21
<211> 798
<212> DNA
<213> Artificial sequence

<220>
<223> nptII gene

<400> 21
tcagaagaac tcgtcaagaa ggcgatagaa ggcgatgcgc tgcgaatcgg gagcggcgat 60

accgtaaagc acgaggaagc ggtcagccca ttcgccgcca agctcttcag caatatcacg 120

ggtagccaac gctatgtcct gatagcggtc cgccacaccc agccggccac agtcgatgaa 180

tccagaaaag cggccatttt ccaccatgat attcggcaag caggcatcgc catgggtcac 240

gacgagatcc tcgccgtcgg gcatgcgcgc cttgagcctg gcgaacagtt cggctggcgc 300

gagcccctga tgctcttcgt ccagatcatc ctgatcgaca agaccggctt ccatccgagt 360

acgtgctcgc tcgatgcgat gtttcgcttg gtggtcgaat gggcaggtag ccggatcaag 420

cgtatgcagc cgccgcattg catcagccat gatggatact ttctcggcag gagcaaggtg 480

agatgacagg agatcctgcc ccggcacttc gcccaatagc agccagtccc ttcccgcttc 540

agtgacaacg tcgagcacag ctgcgcaagg aacgcccgtc gtggccagcc acgatagccg 600

cgctgcctcg tcctgcagtt cattcagggc accggacagg tcggtcttga caaaaagaac 660

cgggcgcccc tgcgctgaca gccggaacac ggcggcatca gagcagccga ttgtctgttg 720

tgcccagtca tagccgaata gcctctccac ccaagcggcc ggagaacctg cgtgcaatcc 780

atcttgttca atccacat 798

<210> 22
<211> 7522
<212> DNA
<213> Artificial sequence

<220>
<223> CTC75064 event: Flanking sequences and T DNA fragment

<400> 22
aacgaggtga caactagggc actacctaga taacaaacac acatacaaca tcacaatcaa 60

gcagtcattt gagaaaattc aaacaaatgg gcttaagcaa ccaacacaag acacaaggct 120

cacccaaaat aagcatttat ctcaagcatg aactaactac aagtttagct caaaacaacg 180

ctagatacgg tagccaactt agcatgtgcc tagaatctgg acagcaacgc agtagttact 240

tgtttcaacc ataactgagg ttatagacat cccacaaagg tgatcctaga ctttctagaa 300

atcttaggaa gattactaca ctttagttat tcataccaaa aactaattca tagcttaaca 360

tgaccaaaat tgctaatatt tcacaaattg acgcttagac aacttaataa cacattgcgg 420

acgtttttaa tgtactgaat tagtactgat atcggtacct taattcgggg gatctggatt 480

ttagtactgg attttggttt taggaattag aaattttatt gatagaagta ttttacaaat 540

acaaatacat actaagggtt tcttatatgc tcaacacatg agcgaaaccc tataggaacc 600

ctaattccct tatctgggaa ctactcacac attattatgg agaaactcga gcttgtcgat 660

cgatcactca gcctcgaggg tggcggtcac tgggatgaac tcgaacctgt cgatgatcac 720

gccggcggtg ccgctgaagt tcctcacgcc cacgatgtta ccgagggagg aggtgaaagc 780

gttggcgctc tcgaagtaac cgaagtcgct ggattggagg ttgtccaggg aggtagcggt 840

agctggcacg gtgttggaga agatggagga gttaccccag ttcacgttga ggtggatcgg 900

ggtcacggaa gcgtacctca cgcgcaccct gtacctggtg gaggtggatg ggaagtggat 960

tggcactcga tgtagcccct gttctggatg ttgttgccgc tgctgttgag cctcacgagg 1020

tcgccaccgg tgaagcctgg gccggaaatg acggaaccgt tgaagaggaa gttgcccttc 1080

acggccggga tttgggtgat gctgtcggag gcgatgatgt tgttgaactc agcgctgcgg 1140

tggatccagg agaacatcgg agccctgatg atgctcacgg agctgttgct gaagccggag 1200

cggaacatgg acacgtggct cagcctgtgg gagaagcctt gcctgggtgg cacgttgttg 1260

ttctgtggtg ggatctcgtc cagggagtcc acggtgccgc tcttcctgta gacagcggat 1320

ggcaggttgg aggaggtgcc gtaggcgaac tcggtgccgt cgagcacgga cagttgctgg 1380

ttgttgatac cgatgttgaa gggcctcctg tacagggtgg aggacagggt cctgtagaca 1440

ccctgaccca gttgagccac gatgcgttgc tgtggagcgg cgttgcccat ggtgccgtag 1500

agcgggaagg tgaactcggg gccgctgaag cccactgggg aggccatgat ctggtggccg 1560

gaccagtagt actcgcccct gtgagcgtcg gtgtagatgg tgatgctgtt caggatgtcc 1620

atcaggtgtg ggctcctgat ggagccctcg ataccctggg cggaaccgcg gaagctaccg 1680

tcgaagttct ccagcactgg gttggtgtag atctccctgg tgagttggga cacggtgcgg 1740

atcgggtagg tcctggagtc gtagttcggg aagagggaca caatgtccag cacggtgagg 1800

gtcaactccc tcctgaactg gttgtacctg atccagtccc tggagtccgg accccagacg 1860

cgctccaggc cggtgttgta ccagcgcaca gcgtggtcgg tgtagttgcc aatcagcctg 1920

gtcaggtcgt tgtagcggct gttgatggtg gcagcatcga agccccacct ttggccgaac 1980

acgctcacgt cgcgcagcac gctgaggtgc aggttagcgg cttgcacgta cacggacagg 2040

agcggcactt ggtagttctg gacggcgaac agtgggatag cggtggtcag ggcgctgttc 2100

atgtcgttga attgaatgcg catttcctcg cggagagctg ggttggtcgg gtcggcctcc 2160

cactccctga agctctcggc gtagatttgg tagaggttgc tcaggccctc cagcctggag 2220

atggcctggt tcctggcgaa ctcttcgatc ctctggttga tcagctgctc gatttgcacc 2280

aggaaggcgt cccattggga tggaccgaag ataccccaga tgatgtccac caggccgagc 2340

acgaagccag cacctggcac gaactcgctg agcaggaact gggtcaagga cagggagatg 2400

tcgatggggg tgtaaccggt ctcgatgcgc tcgccaccca gcacctccac ctctgggttg 2460

ctcaggcagt tgtatgggat gcactcgttg atgtttgggt tgttgtccat ggcggggttg 2520

atcaggttga tcacttctac ctacaaaaaa gctccgcacg aggctgcatt tgtcacaaat 2580

catgaaaaga aaaactaccg atgaacaatg ctgagggatt caaattctac ccacaaaaag 2640

aagaaagaaa gatctagcac atctaagcct gacgaagcag cagaaatata taaaaatata 2700

aaccatagtg cccttttccc ctcttcctga tcttgtttag cacggcggaa attttaaacc 2760

ccccatcatc tcccccaaca acggcggatc gcagatctac atccgagagc cccattcccc 2820

gcgagatccg ggccggatcc acgccggcga gagccccagc cgcgagatcc cgcccctccc 2880

gcgcaccgat ctgggcgcgc acgaagccgc ctctcgccca cccaaactac caaggccaaa 2940

gatcgagacc gagacggaaa aaaaaacgga gaaagaaaga ggagaggggc ggggtggtta 3000

ccggcggcgg cggaggcctc ccttggatct tatggtgtgt tgtccctgtg tgttctccaa 3060

tagtgtggct tgagtgtgtg gaagatggtt ctagaggatc tgctagagtc agcttgtcag 3120

cgtgtcctct ccaaatgaaa tgaacttcct tatatagagg aagggtcttg cgaaggatag 3180

tgggattgtg cgtcatccct tacgtcagtg gagatatcac atcaatccac ttgctttgaa 3240
gacgtggttg gaacgtcttc tttttccacg atgctcctcg tgggtggggg tccatctttg 3300
ggaccactgt cggcagaggc atcttcaacg atggcctttc ctttatcgca atgatggcat 3360
ttgtaggagc caccttcctt ttccactatc ttcacaataa agtgacagat agctgggcaa 3420
tggaatccga ggaggtttcc ggatattacc ctttgttgaa aagtctcaat cggaccatca 3480
catcaatcca cttgctttga agacgtggtt ggaacgtctt ctttttccac gatgctcctc 3540
gtgggtgggg gtccatcttt gggaccactg tcggcagagg catcttcaac gatggccttt 3600
cctttatcgc aatgatggca tttgtaggag ccaccttcct tttccactat cttcacaata 3660
aagtgacaga tagctgggca atggaatccg aggaggtttc cggatattac cctttgttga 3720
aaagtctcaa tcggacctgt ttaaaccctg aagcttacgc gtatgcctgc agtgcagcgt 3780
gacccggtcg tgcccctctc tagagataat gagcattgca tgtctaagtt ataaaaaatt 3840
accacatatt ttttttgtca cacttgtttg aagtgcagtt tatctatctt tatacatata 3900
tttaaacttt actctacgaa taatataatc tatagtacta caataatatc agtgttttag 3960
agaatcatat aaatgaacag ttagacatgg tctaaaggac aattgagtat tttgacaaca 4020
ggactctaca gttttatctt tttagtgtgc atgtgttctc cttttttttt gcaaatagct 4080
tcacctatat aatacttcat ccattttatt agtacatcca tttagggttt agggttaatg 4140
gtttttatag actaattttt ttagtacatc tattttattc tattttagcc tctaaattaa 4200
gaaaactaaa actctatttt agttttttta tttaataatt tagatataaa atagaataaa 4260
ataaagtgac taaaaattaa acaaataccc tttaagaaat taaaaaaact aaggaaacat 4320
ttttcttgtt tcgagtagat aatgccagcc tgttaaacgc cgtcgacgag tctaacggac 4380
accaaccagc gaaccagcag cgtcgcgtcg ggccaagcga agcagacggc acggcatctc 4440
tgtcgctgcc tctggacccc tctcgagagt tccgctccac cgttggactt gctccgctgt 4500
cggcatccag aaattgcgtg gcggagcggc agacgtgagc cggcacggca ggcggcctcc 4560
tcctcctctc acggcacggc agctacgggg gattcctttc ccaccgctcc ttcgctttcc 4620
cttcctcgcc cgccgtaata aatagacacc ccctccacac cctctttccc caacctcgtg 4680
ttgttcggag cgcacacaca cacaaccaga tctcccccaa atccacccgt cggcacctcc 4740
gcttcaaggt acgccgctcg tcctcccccc cccccctct ctaccttctc tagatcggcg 4800
ttccggtcca tggttagggc ccggtagttc tacttctgtt catgtttgtg ttagatccgt 4860
gtttgtgtta gatccgtgct gctagcgttc gtacacggat gcgacctgta cgtcagacac 4920
gttctgattg ctaacttgcc agtgtttctc tttggggaat cctgggatgg ctctagccgt 4980
tccgcagacg ggatcgattt catgattttt tttgtttcgt tgcatagggt ttggtttgcc 5040
cttttccttt atttcaatat atgccgtgca cttgtttgtc gggtcatctt ttcatgcttt 5100

tttttgtctt ggttgtgatg atgtggtctg gttgggcggt cgttctagat cggagtagaa 5160
ttctgtttca aactacctgg tggatttatt aattttggat ctgtatgtgt gtgccataca 5220
tattcatagt tacgaattga agatgatgga tggaaatatc gatctaggat aggtatacat 5280
gttgatgcgg gttttactga tgcatataca gagatgcttt ttgttcgctt ggttgtgatg 5340
atgtggtgtg gttgggcggt cgttcattcg ttctagatcg gagtagaata ctgtttcaaa 5400
ctacctggtg tatttattaa ttttggaact gtatgtgtgt gtcatacatc ttcatagtta 5460
cgagtttaag atggatggaa atatcgatct aggataggta tacatgttga tgtgggtttt 5520
actgatgcat atacatgatg gcatatgcag catctattca tatgctctaa ccttgagtac 5580
ctatctatta taataaacaa gtatgtttta taattatttt gatcttgata tacttggatg 5640
atggcatatg cagcagctat atgtggattt ttttagccct gccttcatac gctatttatt 5700
tgcttggtac tgtttctttt gtcgatgctc accctgttgt ttggtgttac ttctgcaggt 5760
cgactctaga atgtggattg aacaagatgg attgcacgca ggttctccgg ccgcttgggt 5820
ggagaggcta ttcggctatg actgggcaca acagacaatc ggctgctctg atgccgccgt 5880
gttccggctg tcagcgcagg ggcgcccggt tctttttgtc aagaccgacc tgtccggtgc 5940
cctgaatgaa ctgcaggacg aggcagcgcg gctatcgtgg ctggccacga cgggcgttcc 6000
ttgcgcagct gtgctcgacg ttgtcactga agcgggaagg gactggctgc tattgggcga 6060
agtgccgggg caggatctcc tgtcatctca ccttgctcct gccgagaaag tatccatcat 6120
ggctgatgca atgcggcggc tgcatacgct tgatccggct acctgcccat tcgaccacca 6180
agcgaaacat cgcatcgagc gagcacgtac tcggatggaa gccggtcttg tcgatcagga 6240
tgatctggac gaagagcatc aggggctcgc gccagccgaa ctgttcgcca ggctcaaggc 6300
gcgcatgccc gacggcgagg atctcgtcgt gacccatggc gatgcctgct tgccgaatat 6360
catggtggaa aatggccgct tttctggatt catcgactgt ggccggctgg gtgtggcgga 6420
ccgctatcag gacatagcgt tggctacccg tgatattgct gaagagcttg gcggcgaatg 6480
ggctgaccgc ttcctcgtgc tttacggtat cgccgctccc gattcgcagc gcatcgcctt 6540
ctatcgcctt cttgacgagt tcttctgaga tcgttcaaac atttggcaat aaagtttctt 6600
aagattgaat cctgttgccg gtcttgcgat gattatcata taatttctgt tgaattacgt 6660
taagcatgta ataattaaca tgtaatgcat gacgttattt atgagatggg tttttatgat 6720
tagagtcccg caattataca tttaatacgc gatagaaaac aaaatatagc gcgcaaacta 6780
ggataaatta tcgcgcgcgg tgtcatctat gttactagat cggcgcgcca agggcgaatt 6840
ccagcacact ggcggccgtt actagtggat cgagctcgtc gactctagac tcgagggcgc 6900
gccgatacca atttggacag aaaactgaca agaacttcga aaatacataa ttggagttct 6960

agagacacaa caaaagtgat ccttaacttt atgtaaagct tattaagttg tctataactt 7020

tcttattatc atattaagat gattctacgg ctaacaagga caaacatagc aaagaaaaca 7080

tctctgccgg atttggacag attctgtcat tctactttgc aagctcataa ctagagattt 7140

agaccatcac aattagtgct ataggacatt ttggaaagct tagaaaaagt actacacttc 7200

ttctattatc tctaagacac gattccatat gcacacaggt caaacaaacc aaacattcag 7260

atctattcag aacattgaca aaacctgacc gtaaactttt aaaattcata actctaagct 7320

caagcatgta gtcacactta tttctagtaa agctaagcac acatttttct caagcatttc 7380

ctattcaagc aacatggaac aaagcattct tgtttgactc cacacaagga agataggata 7440

ccacatcact cacaaattac tcatcatttg aacaagggtg agaggagcat agctatgcat 7500

aaggcaacaa tcatgaagat gc 7522

<210> 23
<211> 382
<212> DNA
<213> Artificial sequence

<220>
<223> CTC75064 event: Flanking sequence - Left border (5')

<400> 23
aacgaggtga caactagggc actacctaga taacaaacac acatacaaca tcacaatcaa 60

gcagtcattt gagaaaattc aaacaaatgg gcttaagcaa ccaacacaag acacaaggct 120

cacccaaaat aagcatttat ctcaagcatg aactaactac aagtttagct caaaacaacg 180

ctagatacgg tagccaactt agcatgtgcc tagaatctgg acagcaacgc agtagttact 240

tgtttcaacc ataactgagg ttatagacat cccacaaagg tgatcctaga ctttctagaa 300

atcttaggaa gattactaca ctttagttat tcataccaaa aactaattca tagcttaaca 360

tgaccaaaat tgctaatatt tc 382

<210> 24
<211> 1234
<212> DNA
<213> Artificial sequence

<220>
<223> CTC75064 event: Flanking sequence - Right border (3')

<400> 24
taccaatttg gacagaaaac tgacaagaac ttcgaaaata cataattgga gttctagaga 60

cacaacaaaa gtgatcctta actttatgta aagcttatta agttgtctat aactttctta 120

ttatcatatt aagatgattc tacggctaac aaggacaaac atagcaaaga aaacatctct 180

gccggatttg gacagattct gtcattctac tttgcaagct cataactaga gatttagacc 240

atcacaatta gtgctatagg acattttgga aagcttagaa aaagtactac acttcttcta 300

ttatctctaa gacacgattc catatgcaca caggtcaaac aaaccaaaca ttcagatcta 360

ttcagaacat tgacaaaacc tgaccgtaaa cttttaaaat tcataactct aagctcaagc 420

atgtagtcac acttatttct agtaaagcta agcacacatt tttctcaagc atttcctatt 480

caagcaacat accaatttgg acagaaaact gacaagaact tcgaaaatac ataattggag 540

ttctagagac acaacaaaag tgatccttaa ctttatgtaa agcttattaa gttgtctata 600

actttcttat tatcatatta agatgattct acggctaaca aggacaaaca tagcaaagaa 660

aacatctctg ccggatttgg acagattctg tcattctact ttgcaagctc ataactagag 720

atttagacca tcacaattag tgctatagga cattttggaa agcttagaaa aagtactaca 780

cttcttctat tatctctaag acacgattcc atatgcacac aggtcaaaca aaccaaacat 840

tcagatctat tcagaacatt gacaaaacct gaccgtaaac ttttaaaatt cataactcta 900

agctcaagca tgtagtcaca cttatttcta gtaaagctaa gcacacattt ttctcaagca 960

tttcctattc aagcaacatg gaacaaagca ttcttgtttg actccacaca aggaagatag 1020

gataccacat cactcacaaa ttactcatca tttgaacaag ggtgagagga gcatagctat 1080

gcataaggca acaatcatga agatgctgga acaaagcatt cttgtttgac tccacacaag 1140

gaagatagga taccacatca ctcacaaatt actcatcatt tgaacaaggg tgagaggagc 1200

atagctatgc ataaggcaac aatcatgaag atgc 1234

<210> 25
<211> 26041
<212> DNA
<213> Artificial sequence

<220>
<223> Cas9/crRNA/HR template construction

<400> 25
cttagaataa cggatattta aaagggcgtg aaaaggttta tccgttcgtc catttgtatg 60

tgcatgccaa ccacagggtt cccctcggga tcaaagtact ttgatccaac ccctccgctg 120

ctatagtgca gtcggcttct gacgttcagt gcagccgtct tctgaaaacg acatgtcgca 180

caagtcctaa gttacgcgac aggctgccgc cctgcccttt tcctggcgtt ttcttgtcgc 240

gtgttttagt cgcataaagt agaatacttg cgactagaac cggagacatt acgccatgaa 300

caagagcgcc gccgctggcc tgctgggcta tgcccgcgtc agcaccgacg accaggactt 360

gaccaaccaa cgggccgaac tgcacgcggc cggctgcacc aagctgtttt ccgagaagat 420

caccggcacc aggcgcgacc gcccggagct ggccaggatg cttgaccacc tacgccctgg 480

cgacgttgtg acagtgacca ggctagaccg cctggcccgc agcacccgcg acctactgga 540

cattgccgag cgcatccagg aggccggcgc gggcctgcgt agcctggcag agccgtgggc 600

cgacaccacc acgccggccg gccgcatggt gttgaccgtg ttcgccggca ttgccgagtt 660
cgagcgttcc ctaatcatcg accgcacccg gagcgggcgc gaggccgcca aggcccgagg 720
cgtgaagttt ggcccccgcc ctaccctcac cccggcacag atcgcgcacg cccgcgagct 780
gatcgaccag gaaggccgca ccgtgaaaga ggcggctgca ctgcttggcg tgcatcgctc 840
gaccctgtac cgcgcacttg agcgcagcga ggaagtgacg cccaccgagg ccaggcggcg 900
cggtgccttc cgtgaggacg cattgaccga ggccgacgcc ctggcggccg ccgagaatga 960
acgccaagag gaacaagcat gaaaccgcac caggacggcc aggacgaacc gtttttcatt 1020
accgaagaga tcgaggcgga gatgatcgcg gccgggtacg tgttcgagcc gcccgcgcac 1080
gtctcaaccg tgcggctgca tgaaatcctg gccggtttgt ctgatgccaa gctggcggcc 1140
tggccggcca gcttggccgc tgaagaaacc gagcgccgcc gtctaaaaag gtgatgtgta 1200
tttgagtaaa acagcttgcg tcatgcggtc gctgcgtata tgatgcgatg agtaaataaa 1260
caaatacgca aggggaacgc atgaaggtta tcgctgtact taaccagaaa ggcgggtcag 1320
gcaagacgac catcgcaacc catctagccc gcgccctgca actcgccggg gccgatgttc 1380
tgttagtcga ttccgatccc cagggcagtg cccgcgattg ggcggccgtg cgggaagatc 1440
aaccgctaac cgttgtcggc atcgaccgcc cgacgattga ccgcgacgtg aaggccatcg 1500
gccggcgcga cttcgtagtg atcgacggag cgccccaggc ggcggacttg gctgtgtccg 1560
cgatcaaggc agccgacttc gtgctgattc cggtgcagcc aagcccttac gacatatggg 1620
ccaccgccga cctggtggag ctggttaagc agcgcattga ggtcacggat ggaaggctac 1680
aagcggcctt tgtcgtgtcg cgggcgatca aaggcacgcg catcggcggt gaggttgccg 1740
aggcgctggc cgggtacgag ctgcccattc ttgagtcccg tatcacgcag cgcgtgagct 1800
acccaggcac tgccgccgcc ggcacaaccg ttcttgaatc agaacccgag ggcgacgctg 1860
cccgcgaggt ccaggcgctg gccgctgaaa ttaaatcaaa actcatttga gttaatgagg 1920
taaagagaaa atgagcaaaa gcacaaacac gctaagtgcc ggccgtccga gcgcacgcag 1980
cagcaaggct gcaacgttgg ccagcctggc agacacgcca gccatgaagc gggtcaactt 2040
tcagttgccg gcggaggatc acaccaagct gaagatgtac gcggtacgcc aaggcaagac 2100
cattaccgag ctgctatctg aatacatcgc gcagctacca gagtaaatga gcaaatgaat 2160
aaatgagtag atgaatttta gcggctaaag gaggcggcat ggaaaatcaa gaacaaccag 2220
gcaccgacgc cgtggaatgc cccatgtgtg gaggaacggg cggttggcca ggcgtaagcg 2280
gctgggttgt ctgccggccc tgcaatggca ctggaacccc caagcccgag gaatcggcgt 2340
gacggtcgca aaccatccgg cccggtacaa atcggcgcgg cgctgggtga tgacctggtg 2400
gagaagttga aggccgcgca ggccgcccag cggcaacgca tcgaggcaga agcacgcccc 2460
ggtgaatcgt ggcaagcggc cgctgatcga atccgcaaag aatcccggca accgccggca 2520

gccggtgcgc cgtcgattag gaagccgccc aagggcgacg agcaaccaga ttttttcgtt 2580

ccgatgctct atgacgtggg cacccgcgat agtcgcagca tcatggacgt ggccgttttc 2640

cgtctgtcga agcgtgaccg acgagctggc gaggtgatcc gctacgagct tccagacggg 2700

cacgtagagg tttccgcagg gccggccggc atggccagtg tgtgggatta cgacctggta 2760

ctgatggcgg tttcccatct aaccgaatcc atgaaccgat accgggaagg gaagggagac 2820

aagcccggcc gcgtgttccg tccacacgtt gcggacgtac tcaagttctg ccggcgagcc 2880

gatggcggaa agcagaaaga cgacctggta gaaacctgca ttcggttaaa caccacgcac 2940

gttgccatgc agcgtacgaa gaaggccaag aacggccgcc tggtgacggt atccgagggt 3000

gaagccttga ttagccgcta caagatcgta aagagcgaaa ccgggcggcc ggagtacatc 3060

gagatcgagc tagctgattg gatgtaccgc gagatcacag aaggcaagaa cccggacgtg 3120

ctgacggttc accccgatta ctttttgatc gatcccggca tcggccgttt tctctaccgc 3180

ctggcacgcc gcgccgcagg caaggcagaa gccagatggt tgttcaagac gatctacgaa 3240

cgcagtggca gcgccggaga gttcaagaag ttctgtttca ccgtgcgcaa gctgatcggg 3300

tcaaatgacc tgccggagta cgatttgaag gaggaggcgg ggcaggctgg cccgatccta 3360

gtcatgcgct accgcaacct gatcgagggc gaagcatccg ccggttccta atgtacggag 3420

cagatgctag ggcaaattgc cctagcaggg gaaaaaggtc gaaaaggtct ctttcctgtg 3480

gatagcacgt acattgggaa cccaaagccg tacattggga accggaaccc gtacattggg 3540

aacccaaagc cgtacattgg gaaccggtca cacatgtaag tgactgatat aaaagagaaa 3600

aaaggcgatt tttccgccta aaactcttta aaacttatta aaactcttaa aacccgcctg 3660

gcctgtgcat aactgtctgg ccagcgcaca gccgaagagc tgcaaaaagc gcctaccctt 3720

cggtcgctgc gctccctacg ccccgccgct tcgcgtcggc ctatcgcggc cgctggccgc 3780

tcaaaaatgg ctggcctacg gccaggcaat ctaccagggc gcggacaagc cgcgccgtcg 3840

ccactcgacc gccggcgccc acatcaaggc accctgcctc gcgcgtttcg gtgatgacgg 3900

tgaaaacctc tgacacatgc agctcccgga gacggtcaca gcttgtctgt aagcggatgc 3960

cgggagcaga caagcccgtc agggcgcgtc agcgggtgtt ggcgggtgtc ggggcgcagc 4020

catgacccag tcacgtagcg atagcggagt gtatactggc ttaactatgc ggcatcagag 4080

cagattgtac tgagagtgca ccatatgcgg tgtgaaatac cgcacagatg cgtaaggaga 4140

aaataccgca tcaggcgctc ttccgcttcc tcgctcactg actcgctgcg ctcggtcgtt 4200

cggctgcggc gagcggtatc agctcactca aaggcggtaa tacggttatc cacagaatca 4260

ggggataacg caggaaagaa catgtgagca aaaggccagc aaaaggccag gaaccgtaaa 4320

aaggccgcgt tgctggcgtt tttccatagg ctccgccccc ctgacgagca tcacaaaaat 4380

cgacgctcaa gtcagaggtg gcgaaacccg acaggactat aaagatacca ggcgtttccc 4440
cctggaagct ccctcgtgcg ctctcctgtt ccgaccctgc cgcttaccgg atacctgtcc 4500
gcctttctcc cttcgggaag cgtggcgctt tctcatagct cacgctgtag gtatctcagt 4560
tcggtgtagg tcgttcgctc caagctgggc tgtgtgcacg aaccccccgt tcagcccgac 4620
cgctgcgcct tatccggtaa ctatcgtctt gagtccaacc cggtaagaca cgacttatcg 4680
ccactggcag cagccactgg taacaggatt agcagagcga ggtatgtagg cggtgctaca 4740
gagttcttga agtggtggcc taactacggc tacactagaa ggacagtatt tggtatctgc 4800
gctctgctga agccagttac cttcggaaaa agagttggta gctcttgatc cggcaaacaa 4860
accaccgctg gtagcggtgg tttttttgtt tgcaagcagc agattacgcg cagaaaaaaa 4920
ggatctcaag aagatccttt gatcttttct acggggtctg acgctcagtg gaacgaaaac 4980
tcacgttaag ggattttggt catgcattct aggtattatt tgccaacgac cttcgtgatc 5040
tcgcccttga catagtggac aaattcttcg agctggtcgg cccgggacgc gagacggtct 5100
tcttcttggc ccagataggc ttggcgcgct tcgaggatca cgggctggta ttgcgccgga 5160
aggcgctcca tcgcccagtc ggcggcgaca tccttcggcg cgatcttgcc ggtaaccgcc 5220
gagtaccaaa tccggctcag cgtaaggacc acattgcgct catcgcccgc ccaatccggc 5280
ggggagttcc acagggtcag cgtctcgttc agtgcttcga acagatcctg ttccggcacc 5340
gggtcgaaaa gttcctcggc cgcggggccg acgagggcca cgctatgctc ccgggccttg 5400
gtgagcagga tcgccagatc aatgtcgatg gtggccggtt caaagatacc cgccagaata 5460
tcattacgct gccattcgcc gaactggagt tcgcgtttgg ccggatagcg ccaggggatg 5520
atgtcatcgt gcaccacaat cgtcacctca accgcgcgca ggatttcgct ctcgccgggg 5580
gaggcggacg tttccagaag gtcgttgata agcgcgcggc gcgtggtctc gtcgagacgg 5640
acggtaacgg tgacaagcag gtcgatgtcc gaatggggct taaggccgcc gtcaacggcg 5700
ctaccataca gatgcacggc gaggagggtc ggttcgaggt ggcgctcgat gacacccacg 5760
acttccgaca gctgggtgga cacctcggcg atgaccgctt cacccattta ttatttcctt 5820
cctcttttct acagtattta aagatacccc aagaagctaa ttataacaag acgaactcca 5880
attcactgtt ccttgcattc taaaacctta aataccagaa aacagctttt tcaaagttgt 5940
tttcaaagtt ggcgtataac atagtatcga cggagccgat tttgaaaccg cggtgatcac 6000
aggcagcaac gctctgtcat cgttacaatc aacatgctac cctccgcgag atcatccgtg 6060
tttcaaaccc ggcagcttag ttgccgttct tccgaatagc atcggtaaca tgagcaaagt 6120
ctgccgcctt acaacggctc tcccgctgac gccgtcccgg actgatgggc tgcctgtatc 6180
gagtggtgat tttgtgccga gctgccggtc ggggagctgt tggctggctg gtggcaggat 6240
atattgtggt gtaaacaaat tgacgcttag acaacttaat aacacattgc ggacgttttt 6300

aatgtactga attagtactg ataaatggcg cgccaagctt tggcaaacag ctattatggg 6360
tattatgggt ggtaccacgc gtcgatccac tagtaacggc cgccagtgtg ctggaattcg 6420
cccttggcgc gccgatctag taacatagat gacaccgcgc gcgataattt atcctagttt 6480
gcgcgctata ttttgttttc tatcgcgtat taaatgtata attgcgggac tctaatcata 6540
aaaacccatc tcataaataa cgtcatgcat tacatgttaa ttattacatg cttaacgtaa 6600
ttcaacagaa attatatgat aatcatcgca agaccggcaa caggattcaa tcttaagaaa 6660
ctttattgcc aaatgtttga acgatctcag aagaactcgt caagaaggcg atagaaggcg 6720
atgcgctgcg aatcgggagc ggcgataccg taaagcacga ggaagcggtc agcccattcg 6780
ccgccaagct cttcagcaat atcacgggta gccaacgcta tgtcctgata gcggtccgcc 6840
acacccagcc ggccacagtc gatgaatcca gaaaagcggc cattttccac catgatattc 6900
ggcaagcagg catcgccatg ggtcacgacg agatcctcgc cgtcgggcat gcgcgccttg 6960
agcctggcga acagttcggc tggcgcgagc ccctgatgct cttcgtccag atcatcctga 7020
tcgacaagac cggcttccat ccgagtacgt gctcgctcga tgcgatgttt cgcttggtgg 7080
tcgaatgggc aggtagccgg atcaagcgta tgcagccgcc gcattgcatc agccatgatg 7140
gatactttct cggcaggagc aaggtgagat gacaggagat cctgccccgg cacttcgccc 7200
aatagcagcc agtcccttcc cgcttcagtg acaacgtcga gcacagctgc gcaaggaacg 7260
cccgtcgtgg ccagccacga tagccgcgct gcctcgtcct gcagttcatt cagggcaccg 7320
gacaggtcgg tcttgacaaa aagaaccggg cgcccctgcg ctgacagccg gaacacggcg 7380
gcatcagagc agccgattgt ctgttgtgcc cagtcatagc cgaatagcct ctccacccaa 7440
gcggccggag aacctgcgtg caatccatct tgttcaatcc acatggtggt gtgacctgca 7500
gaagtaacac caaacaacag ggtgagcatc gacaaaagaa acagtaccaa gcaaataaat 7560
agcgtatgaa ggcagggcta aaaaaatcca catatagctg ctgcatatgc catcatccaa 7620
gtatatcaag atcaaaataa ttataaaaca tacttgttta ttataataga taggtactca 7680
aggttagagc atatgaatag atgctgcata tgccatcatg tatatgcatc agtaaaaccc 7740
acatcaacat gtatacctat cctagatcga tatttccatc catcttaaac tcgtaactat 7800
gaagatgtat gacacacaca tacagttcca aaattaataa atacaccagg tagtttgaaa 7860
cagtattcta ctccgatcta gaacgaatga acgaccgccc aaccacacca catcatcaca 7920
accaagcgaa caaaaagcat ctctgtatat gcatcagtaa aacccgcatc aacatgtata 7980
cctatcctag atcgatattt ccatccatca tcttcaattc gtaactatga atatgtatgg 8040
cacacacata cagatccaaa attaataaat ccaccaggta gtttgaaaca gaattctact 8100
ccgatctaga acgaccgccc aaccagacca catcatcaca accaagacaa aaaaaagcat 8160

gaaaagatga cccgacaaac aagtgcacgg catatattga aataaaggaa aagggcaaac 8220

caaaccctat gcaacgaaac aaaaaaaatc atgaaatcga tcccgtctgc ggaacggcta 8280

gagccatccc aggattcccc aaagagaaac actggcaagt tagcaatcag aacgtgtctg 8340

acgtacaggt cgcatccgtg tacgaacgct agcagcacgg atctaacaca aacacggatc 8400

taacacaaac atgaacagaa gtagaactac cgggccctaa ccatggaccg gaacgccgat 8460

ctagagaagg tagagagggg ggggggggga ggacgagcgg cgtaccttga agcggaggtg 8520

ccgacgggtg gatttggggg agatctggtt gtgtgtgtgt gcgctccgaa caacacgagg 8580

ttggggaaag agggtgtgga gggggtgtct atttattacg gcgggcgagg aagggaaagc 8640

gaaggagcgg tgggaaagga atcccccgta gctgccgtgc cgtgagagga ggaggaggcc 8700

gcctgccgtg ccggctcacg tctgccgctc cgccacgcaa tttctggatg ccgacagcgg 8760

agcaagtcca acggtggagc ggaactctcg agaggggtcc agaggcagcg acagagatgc 8820

cgtgccgtct gcttcgcttg gcccgacgcg acgctgctgg ttcgctggtt ggtgtccgtt 8880

agactcgtcg acggcgttta acaggctggc attatctact cgaaacaaga aaaatgtttc 8940

cttagttttt ttaatttctt aaagggtatt tgtttaattt ttagtcactt tattttattc 9000

tattttatat ctaaattatt aaataaaaaa actaaaatag agttttagtt ttcttaattt 9060

agaggctaaa atagaataaa atagatgtac taaaaaaatt agtctataaa aaccattaac 9120

cctaaaccct aaatggatgt actaataaaa tggatgaagt attatatagg tgaagctatt 9180

tgcaaaaaaa aaggagaaca catgcacact aaaaagataa aactgtagag tcctgttgtc 9240

aaaatactca attgtccttt agaccatgtc taactgttca tttatatgat tctctaaaac 9300

actgatatta ttgtagtact atagattata ttattcgtag agtaaagttt aaatatatgt 9360

ataaagatag ataaactgca cttcaaacaa gtgtgacaaa aaaaatatgt ggtaattttt 9420

tataacttag acatgcaatg ctcattatct ctagagaggg gcacgaccgg gtcacgctgc 9480

actgcaggga tccgatctag taacatagat gacaccgcgc gcgataattt atcctagttt 9540

gcgcgctata ttttgttttc tatcgcgtat taaatgtata attgcgggac tctaatcata 9600

aaaacccatc tcataaataa cgtcatgcat tacatgttaa ttattacatg cttaacgtaa 9660

ttcaacagaa attatatgat aatcatcgca agaccggcaa caggattcaa tcttaagaaa 9720

ctttattgcc aaatgtttga acgatcccta ggacgatctc acttgtacag ctcgtccatg 9780

ccgtgggtga tgccagctgc ggtgacgaac tccagcagga ccatgtggtc gcgcttctcg 9840

ttggggtcct tgctcagagc ggactgggtg ctcaggtagt ggttgtcggg cagcagcacg 9900

ggaccgtcgc cgatgggcgt gttctgctgg tagtggtcgg cgagctggac gctgccgtcc 9960

tcgatgttgt ggcggatctt gaagttgacc ttgatgccgt tcttctgctt gtcagccatg 10020

atgtagacgt tgtggctgtt gtagttgtac tccagcttgt gccccaggat gttgccgtcc 10080

tccttgaagt cgatgccctt cagctcgatg cggttcacca gggtgtcgcc ctcgaacttc 10140
acctcggctc gggtcttgta gttgccgtcg tccttgaaga agatggtgcg ctcctggacg 10200
tagccttcgg gcatggcgga cttgaagaag tcgtgctgct tcatgtggtc ggggtagcgg 10260
ctgaagcact gcacgccgta ggtgaaggtg gtcacgaggg tgggccaggg cacgggcagc 10320
ttgccggtgg tgcagatgaa cttcagggtc agcttgccgt aggtggcgtc gccctcgccc 10380
tcgccgctga cgctgaactt gtggccgttc acgtcgccgt ccagctcgac caggatgggc 10440
accaccccag tgaacagctc ctcgcccttg ctcactacaa aaaagctccg cacgaggctg 10500
catttgtcac aaatcatgaa aagaaaaact accgatgaac aatgctgagg gattcaaatt 10560
ctacccacaa aaagaagaaa gaaagatcta gcacatctaa gcctgacgaa gcagcagaaa 10620
tatataaaaa tataaaccat agtgcccttt tcccctcttc ctgatcttgt ttagcacggc 10680
ggaaatttta aaccccccat catctccccc aacaacggcg gatcgcagat ctacatccga 10740
gagccccatt ccccgcgaga tccgggccgg atccacgccg gcgagagccc cagccgcgag 10800
atcccgcccc tcccgcgcac cgatctgggc gcgcacgaag ccgcctctcg cccacccaaa 10860
ctaccaaggc caaagatcga gaccgagacg gaaaaaaaaa cggagaaaga aagaggagag 10920
gggcggggtg gttaccggcg gcggcggagg cctcccttgg atcttatggt gtgttgtccc 10980
tgtgtgttct ccaatagtgt ggcttgagtg tgtggaagat ggttctagag gatctgctag 11040
agtcagcttg tcagcgtgtc ctctccaaat gaaatgaact tccttatata gaggaagggt 11100
cttgcgaagg atagtgggat tgtgcgtcat cccttacgtc agtggagata tcacatcaat 11160
ccacttgctt tgaagacgtg gttggaacgt cttctttttc cacgatgctc ctcgtgggtg 11220
ggggtccatc tttgggacca ctgtcggcag aggcatcttc aacgatggcc tttcctttat 11280
cgcaatgatg gcatttgtag gagccacctt cctttccac tatcttcaca ataaagtgac 11340
agatagctgg gcaatggaat ccgaggaggt ttccggatat taccctttgt tgaaaagtct 11400
caatcggacc atcacatcaa tccacttgct ttgaagacgt ggttggaacg tcttcttttt 11460
ccacgatgct cctcgtgggt gggggtccat ctttgggacc actgtcggca gaggcatctt 11520
caacgatggc ctttccttta tcgcaatgat ggcatttgta ggagccacct tcctttcca 11580
ctatcttcac aataaagtga cagatagctg ggcaatggaa tccgaggagg tttccggata 11640
ttaccctttg ttgaaaagtc tcaatcggac cccctcagcc tgcagtgcag cgtgacccgg 11700
tcgtgcccct ctctagagat aatgagcatt gcatgtctaa gttataaaaa attaccacat 11760
atttttttg tcacacttgt ttgaagtgca gtttatctat ctttatacat atatttaaac 11820
tttactctac gaataatata atctatagta ctacaataat atcagtgttt tagagaatca 11880
tataaatgaa cagttagaca tggtctaaag gacaattgag tattttgaca acaggactct 11940

acagttttat ctttttagtg tgcatgtgtt ctcctttttt tttgcaaata gcttcaccta 12000
tataatactt catccatttt attagtacat ccatttaggg tttagggtta atggttttta 12060
tagactaatt tttttagtac atctatttta ttctatttta gcctctaaat taagaaaact 12120
aaaactctat tttagttttt ttatttaata atttagatat aaaatagaat aaaataaagt 12180
gactaaaaat taaacaaata ccctttaaga aattaaaaaa actaaggaaa catttttctt 12240
gtttcgagta gataatgcca gcctgttaaa cgccgtcgac gagtctaacg gacaccaacc 12300
agcgaaccag cagcgtcgcg tcgggccaag cgaagcagac ggcacggcat ctctgtcgct 12360
gcctctggac ccctctcgag agttccgctc caccgttgga cttgctccgc tgtcggcatc 12420
cagaaattgc gtggcggagc ggcagacgtg agccggcacg gcaggcggcc tcctcctcct 12480
ctcacggcac ggcagctacg gggggattcct ttcccaccgc tccttcgctt tcccttcctc 12540
gcccgccgta ataaatagac accccctcca caccctcttt ccccaacctc gtgttgttcg 12600
gagcgcacac acacacaacc agatctcccc caaatccacc cgtcggcacc tccgcttcaa 12660
ggtacgccgc tcgtcctccc cccccccccc tctctacctt ctctagatcg gcgttccggt 12720
ccatggttag ggcccggtag ttctacttct gttcatgttt gtgttagatc cgtgtttgtg 12780
ttagatccgt gctgctagcg ttcgtacacg gatgcgacct gtacgtcaga cacgttctga 12840
ttgctaactt gccagtgttt ctctttgggg aatcctggga tggctctagc cgttccgcag 12900
acgggatcga tttcatgatt ttttttgttt cgttgcatag ggtttggttt gcccttttcc 12960
tttatttcaa tatatgccgt gcacttgttt gtcgggtcat cttttcatgc ttttttttgt 13020
cttggttgtg atgatgtggt ctggttgggc ggtcgttcta gatcggagta gaattctgtt 13080
tcaaactacc tggtggattt attaattttg gatctgtatg tgtgtgccat acatattcat 13140
agttacgaat tgaagatgat ggatggaaat atcgatctag gataggtata catgttgatg 13200
cgggttttac tgatgcatat acagagatgc tttttgttcg cttggttgtg atgatgtggt 13260
gtggttgggc ggtcgttcat tcgttctaga tcggagtaga atactgtttc aaactacctg 13320
gtgtatttat taattttgga actgtatgtg tgtgtcatac atcttcatag ttacgagttt 13380
aagatggatg gaaatatcga tctaggatag gtatacatgt tgatgtgggt tttactgatg 13440
catatacatg atggcatatg cagcatctat tcatatgctc taaccttgag tacctatcta 13500
ttataataaa caagtatgtt ttataattat tttgatcttg atatacttgg atgatggcat 13560
atgcagcagc tatatgtgga tttttttagc cctgccttca tacgctattt atttgcttgg 13620
tactgtttct tttgtcgatg ctcaccctgt tgtttggtgt tacttctgca ggcgatcgcc 13680
acaccaccat gccgaagaag aagcgcaagg tcatggacaa gaagtactcc atcggcctgg 13740
acatcggcac caacagcgtg ggctgggccg tcatcaccga cgagtacaag gtgccctcca 13800
agaagttcaa ggtcctcggc aacaccgaca ggcacagcat caagaagaac ctgatcggcg 13860

ccctgctgtt cgactccggc gagactgcgg aggctaccag gctgaagcgc actgctcgca 13920
ggcgctacac caggcgcaag aaccgcatct gctacctcca ggagattttc tccaacgaga 13980
tggccaaggt ggacgactcc ttcttccacc gcctggagga gagcttcctg gtcgaggaag 14040
acaagaagca cgagcgccac cctatcttcg gcaacatcgt ggacgaggtc gcctaccacg 14100
agaagtaccc aaccatctac cacctccgca agaagctggt ggactccacc gacaaggccg 14160
acctgaggct catctacctg gccctcgccc acatgatcaa gttccgcggc cacttcctca 14220
tcgagggcga cctgaacccg gacaacagcg acgtggacaa gctcttcatc cagctggtcc 14280
agacctacaa ccagctgttc gaggagaacc ccatcaacgc ctccggcgtg gacgctaagg 14340
ctatcctcag cgctaggctg tccaagagca ggcgcctgga gaacctcatc gcccagctcc 14400
cgggcgagaa gaagaacggc ctcttcggca acctgatcgc tctgtccctc ggcctgaccc 14460
ccaacttcaa gagcaacttc gacctggccg aggacgccaa gctccagctg tccaaggaca 14520
cctacgacga cgacctcgac aacctgctcg cccagatcgg cgaccagtac gccgacctct 14580
tcctggccgc caagaacctc tccgacgcca tcctgctcag cgacatcctg agggtgaaca 14640
ccgagatcac caaggccccg ctgtccgcca gcatgatcaa gcgctacgac gagcaccacc 14700
aggacctcac tctcctgaag gccctcgtcc gccagcagct gcccgagaag tacaaggaga 14760
ttttcttcga ccagagcaag aacggctacg cgggctacat cgatggcggc gcctcccagg 14820
aagagttcta caagttcatc aagcctatcc tggagaagat ggacggcacc gaggagctgc 14880
tcgtgaagct gaaccgcgag gacctgctcc gcaagcagag gaccttcgac aacggcagca 14940
tccctcacca gatccacctg ggcgagctgc acgctatcct ccgccgccag gaagacttct 15000
acccattcct gaaggacaac cgcgagaaga tcgagaagat cctcaccttc cgcatcccgt 15060
actacgtggg cccccctggcc cgcggcaact ccaggttcgc ctggatgacc aggaagagcg 15120
aggagaccat caccccgtgg aacttcgagg aagtggtgga caagggcgcc tccgctcaga 15180
gcttcatcga gcgcatgacc aacttcgaca agaacctccc taacgagaag gtgctgccaa 15240
agcactccct gctctacgag tacttcaccg tctacaacga gctgaccaag gtgaagtatg 15300
tgaccgaggg catgaggaag cccgccttcc tcagcggcga gcagaagaag gccatcgtgg 15360
acctgctctt caagaccaac cgcaaggtga ccgtcaagca gctgaaggaa gactacttca 15420
agaagatcga gtgcttcgac tccgtggaga tcagcggcgt ggaggaccgc ttcaacgcct 15480
ccctcggcac ctaccacgac ctgctcaaga tcatcaagga caaggacttc ctcgacaacg 15540
aggagaacga ggacatcctg gaggacatcg tgctcaccct gaccctcttc gaggaccgcg 15600
agatgatcga ggagaggctc aagacctacg cccacctgtt cgacgacaag gtcatgaagc 15660
agctgaagag gcgcaggtac actggctggg gccgcctcag caggaagctg atcaacggca 15720

tcagggacaa gcagtccggc aagaccatcc tggacttcct caagagcgac ggcttcgcca 15780
accgcaactt catgcagctc atccacgacg actccctgac cttcaaggaa gacatccaga 15840
aggctcaggt gtccggccag ggcgacagcc tccacgagca catcgctaac ctggcgggca 15900
gccctgccat caagaagggc atcctccaga ccgtgaaggt ggtggacgag ctggtgaagg 15960
tcatgggccg ccacaagcca gagaacatcg tcatcgagat ggccagggag aaccagacca 16020
cccagaaggg tcagaagaac tcccgcgaga ggatgaagag gatcgaggaa ggcatcaagg 16080
agctgggcag ccagatcctg aaggagcacc cggtggagaa cacccagctc cagaacgaga 16140
agctgtacct ctactacctg cagaacggcc gcgacatgta tgtggaccag gagctggaca 16200
tcaacaggct gtccgactac gacgtggacc acatcgtccc tcagtccttc ctcaaggacg 16260
acagcatcga caacaaggtg ctgacccgca gcgacaagaa caggggcaag tccgacaacg 16320
tcccaagcga ggaagtggtc aagaagatga agaactactg gcgccagctg ctcaacgcca 16380
agctcatcac ccagcgcaag ttcgacaacc tgactaaggc ggagaggggc ggcctgtccg 16440
agctggacaa ggctggcttc atcaagcgcc agctcgtgga gaccaggcag atcaccaagc 16500
acgtcgccca gatcctggac agcaggatga acaccaagta cgacgagaac gacaagctca 16560
tccgcgaggt gaaggtcatc accctcaagt ccaagctggt gagcgacttc cgcaaggact 16620
tccagttcta caaggtcagg gagatcaaca actaccacca cgcccacgat gcttacctca 16680
acgcggtggt gggcaccgcc ctcatcaaga agtaccctaa gctggagagc gagttcgtgt 16740
acggcgacta caaggtgtac gacgtccgca agatgatcgc caagtccgag caggagatcg 16800
gcaaggccac cgccaagtac ttcttctaca gcaacatcat gaacttcttc aagaccgaga 16860
tcaccctcgc caacggcgag atccgcaaga ggccactgat cgagaccaac ggcgagactg 16920
gcgagatcgt gtgggacaag ggcagggact tcgccaccgt gaggaaggtc ctgtccatgc 16980
ctcaggtgaa catcgtcaag aagaccgagg tccagaccgg cggcttctcc aaggagagca 17040
tcctcccaaa gcgcaacagc gacaagctga tcgccaggaa gaaggactgg gacccgaaga 17100
agtacggtgg cttcgactcc cctactgtgg cttacagcgt cctggtggtc gccaaggtgg 17160
agaagggcaa gtccaagaag ctgaagagcg tcaaggagct gctcggcatc accatcatgg 17220
agaggtccag cttcgagaag aacccgatcg acttcctgga ggccaagggc tacaaggaag 17280
tgaagaagga cctgatcatc aagctgccca agtacagcct gttcgagctg gagaacggcc 17340
gcaagaggat gctcgcctcc gctggcgagc tgcagaaggg caacgagctg gccctcccgt 17400
ccaagtatgt gaacttcctg tacctcgcct cccactacga gaagctgaag ggcagccccg 17460
aggacaacga gcagaagcag ctcttcgtcg agcagcacaa gcactacctg gacgagatca 17520
tcgagcagat cagcgagttc agcaagcgcg tgatcctcgc cgacgccaac ctcgacaagg 17580
tcctgtccgc ctacaacaag caccgcgaca agcctatcag ggagcaggcc gagaacatca 17640

tccacctgtt caccctcacc aacctgggcg ccccagctgc cttcaagtac ttcgacacca 17700

ccatcgaccg caagaggtac accagcacca aggaagtgct ggacgccacc ctgatccacc 17760

agtccatcac cggcctgtac gagactcgca tcgacctcag ccagctgggc ggcgacccga 17820

agaagaagcg caaagtctga gggaccctcg atcgacaagc tcgagtttct ccataataat 17880

gtgtgagtag ttcccagata agggaattag ggttcctata gggtttcgct catgtgttga 17940

gcatataaga aacccttagt atgtatttgt atttgtaaaa tacttctatc aataaaattt 18000

ctaattccta aaaccaaaat ccagtactaa aatccagatc ccccgaatta acctgcaggg 18060

gcggcaggga gagttttaac attgactagc gtgctgataa tttgtgagaa ataataattg 18120

acaagtagat actgacattt gagaagagct tctgaactgt tattagtaac aaaaatggaa 18180

agctgatgca cggaaaaagg aaagaaaaag ccatactttt ttttaggtag gaaaagaaaa 18240

agccatacga gactgatgtc tctcagatgg gccgggatct gtctatctag caggcagcag 18300

ccctaccaac ctcacgggcc agcaattacg agtccttcta aaacgtcccg ccgagggcgc 18360

gtggccgtgc tgtgcagcag cacgtctaac attagtccca cctcgccagt ttacagggag 18420

cagaaccagc ttataagcgg aggcgcggca ccaagaagca aagtctagga tcacctttgt 18480

gttttagagc tagaaatagc aagttaaaat aaggctagtc cgttatcaac ttgaaaaagt 18540

ggcaccgagt cggtgctttt ttttcctcga ggaagtctag gatcaccttt gtgggaacga 18600

ggtgacaact agggcactac ctagataaca aacacacata caacatcaca atcaagcagt 18660

catttgagaa aattcaaaca aatgggctta agcaaccaac acaagacaca aggctcaccc 18720

aaaataagca tttatctcaa gcatgaacta actacaagtt tagctcaaaa caacgctaga 18780

tacggtagcc aacttagcat gtgcctagaa tctggacagc aacgcagtag ttacttgttt 18840

caaccataac tgaggttata gacatgccac aaaggtgatc ctagactttc tagaaatctt 18900

aggaagatta ctacacttta gttattcata ccaaaaacta attcatagct taacatgacc 18960

aaaattgcta atatttcaca aattgacgct tagacaactt aataacacat tgcggacgtt 19020

tttaatgtac tgaattagta ctgatatcgg taccttaatt cgggggatct ggattttagt 19080

actggatttt ggttttagga attagaaatt ttattgatag aagtatttta caaatacaaa 19140

tacatactaa gggtttctta tatgctcaac acatgagcga aaccctatag gaaccctaat 19200

tcccttatct gggaactact cacacattat tatggagaaa ctcgagcttg tcgatcgatc 19260

actcagcctc gagggtggcg gtcactggga tgaactcgaa cctgtcgatg atcacgccgg 19320

cggtgccgct gaagttcctc acgcccacga tgttaccgag ggaggaggtg aaagcgttgg 19380

cgctctcgaa gtaaccgaag tcgctggatt ggaggttgtc cagggaggta gcggtagctg 19440

gcacggtgtt ggagaagatg gaggagttac cccagttcac gttgaggtgg atcggggtca 19500

cggaagcgta cctcacgcgc accctgtacc tggtggaggt ggatgggaag tggattggca 19560

ctcgatgtag cccctgttct ggatgttgtt gccgctgctg ttgagcctca cgaggtcgcc 19620

accggtgaag cctgggccgg aaatgacgga accgttgaag aggaagttgc ccttcacggc 19680

cgggatttgg gtgatgctgt cggaggcgat gatgttgttg aactcagcgc tgcggtggat 19740

ccaggagaac atcggagccc tgatgatgct cacggagctg ttgctgaagc cggagcggaa 19800

catggacacg tggctcagcc tgtgggagaa gccttgcctg ggtggcacgt tgttgttctg 19860

tggtgggatc tcgtccaggg agtccacggt gccgctcttc ctgtagacag cggatggcag 19920

gttggaggag gtgccgtagg cgaactcggt gccgtcgagc acggacagtt gctggttgtt 19980

gataccgatg ttgaagggcc tcctgtacag ggtggaggac agggtcctgt agacaccctg 20040

acccagttga gccacgatgc gttgctgtgg agcggcgttg cccatggtgc cgtagagcgg 20100

gaaggtgaac tcggggccgc tgaagcccac tggggaggcc atgatctggt ggccggacca 20160

gtagtactcg cccctgtgag cgtcggtgta gatggtgatg ctgttcagga tgtccatcag 20220

gtgtgggctc ctgatggagc cctcgatacc ctgggcggaa ccgcggaagc taccgtcgaa 20280

gttctccagc actgggttgg tgtagatctc cctggtgagt tgggacacgg tgcggatcgg 20340

gtaggtcctg gagtcgtagt tcgggaagag ggacacaatg tccagcacgg tgagggtcaa 20400

ctccctcctg aactggttgt acctgatcca gtccctggag tccggacccc agacgcgctc 20460

caggccggtg ttgtaccagc gcacagcgtg gtcggtgtag ttgccaatca gcctggtcag 20520

gtcgttgtag cggctgttga tggtggcagc atcgaagccc cacctttggc cgaacacgct 20580

cacgtcgcgc agcacgctga ggtgcaggtt agcggcttgc acgtacacgg acaggagcgg 20640

cacttggtag ttctggacgg cgaacagtgg gatagcggtg gtcagggcgc tgttcatgtc 20700

gttgaattga atgcgcattt cctcgcggag agctgggttg gtcgggtcgg cctcccactc 20760

cctgaagctc tcggcgtaga tttggtagag gttgctcagg ccctccagcc tggagatggc 20820

ctggttcctg gcgaactctt cgatcctctg gttgatcagc tgctcgattt gcaccaggaa 20880

ggcgtcccat tgggatggac cgaagatacc ccagatgatg tccaccaggc cgagcacgaa 20940

gccagcacct ggcacgaact cgctgagcag gaactgggtc aaggacaggg agatgtcgat 21000

gggggtgtaa ccggtctcga tgcgctcgcc acccagcacc tccacctctg ggttgctcag 21060

gcagttgtat gggatgcact cgttgatgtt tgggttgttg tccatggcgg ggttgatcag 21120

gttgatcact tctacctaca aaaaagctcc gcacgaggct gcatttgtca caaatcatga 21180

aaagaaaaac taccgatgaa caatgctgag ggattcaaat tctacccaca aaaagaagaa 21240

agaaagatct agcacatcta agcctgacga agcagcagaa atatataaaa atataaacca 21300

tagtgccctt ttcccctctt cctgatcttg tttagcacgg cggaaatttt aaacccccca 21360

tcatctcccc caacaacggc ggatcgcaga tctacatccg agagccccat tccccgcgag 21420

atccgggccg gatccacgcc ggcgagagcc ccagccgcga gatcccgccc ctcccgcgca 21480

ccgatctggg cgcgcacgaa gccgcctctc gcccacccaa actaccaagg ccaaagatcg 21540

agaccgagac ggaaaaaaaa acggagaaag aaagaggaga ggggcggggt ggttaccggc 21600

ggcggcggag gcctcccttg gatcttatgg tgtgttgtcc ctgtgtgttc tccaatagtg 21660

tggcttgagt gtgtggaaga tggttctaga ggatctgcta gagtcagctt gtcagcgtgt 21720

cctctccaaa tgaaatgaac ttccttatat agaggaaggg tcttgcgaag gatagtggga 21780

ttgtgcgtca tcccttacgt cagtggagat atcacatcaa tccacttgct ttgaagacgt 21840

ggttggaacg tcttcttttt ccacgatgct cctcgtgggt gggggtccat ctttgggacc 21900

actgtcggca gaggcatctt caacgatggc ctttccttta tcgcaatgat ggcatttgta 21960

ggagccacct tccttttcca ctatcttcac aataaagtga cagatagctg ggcaatggaa 22020

tccgaggagg tttccggata ttaccctttg ttgaaaagtc tcaatcggac catcacatca 22080

atccacttgc tttgaagacg tggttggaac gtcttctttt tccacgatgc tcctcgtggg 22140

tgggggtcca tctttgggac cactgtcggc agaggcatct tcaacgatgg cctttccttt 22200

atcgcaatga tggcatttgt aggagccacc ttccttttcc actatcttca caataaagtg 22260

acagatagct gggcaatgga atccgaggag gtttccggat attacccttt gttgaaaagt 22320

ctcaatcgga cctgtttaaa ccctgaagct tacgcgtatg cctgcagtgc agcgtgaccc 22380

ggtcgtgccc ctctctagag ataatgagca ttgcatgtct aagttataaa aaattaccac 22440

atattttttt tgtcacactt gtttgaagtg cagtttatct atctttatac atatatttaa 22500

actttactct acgaataata taatctatag tactacaata atatcagtgt tttagagaat 22560

catataaatg aacagttaga catggtctaa aggacaattg agtattttga caacaggact 22620

ctacagtttt atcttttttag tgtgcatgtg ttctcctttt tttttgcaaa tagcttcacc 22680

tatataatac ttcatccatt ttattagtac atccatttag ggtttagggt taatggtttt 22740

tatagactaa tttttttagt acatctattt tattctattt tagcctctaa attaagaaaa 22800

ctaaaactct attttagttt ttttatttaa taatttagat ataaaataga ataaaataaa 22860

gtgactaaaa attaaacaaa taccctttaa gaaattaaaa aaactaagga aacatttttc 22920

ttgtttcgag tagataatgc cagcctgtta aacgccgtcg acgagtctaa cggacaccaa 22980

ccagcgaacc agcagcgtcg cgtcgggcca agcgaagcag acggcacggc atctctgtcg 23040

ctgcctctgg acccctctcg agagttccgc tccaccgttg gacttgctcc gctgtcggca 23100

tccagaaatt gcgtggcgga gcggcagacg tgagccggca cggcaggcgg cctcctcctc 23160

ctctcacggc acggcagcta cgggggattc ctttcccacc gctccttcgc tttcccttcc 23220

tcgcccgccg taataaatag acaccccctc cacaccctct ttccccaacc tcgtgttgtt 23280

cggagcgcac acacacacaa ccagatctcc cccaaatcca cccgtcggca cctccgcttc 23340
aaggtacgcc gctcgtcctc cccccccccc cctctctacc ttctctagat cggcgttccg 23400
gtccatggtt agggcccggt agttctactt ctgttcatgt ttgtgttaga tccgtgtttg 23460
tgttagatcc gtgctgctag cgttcgtaca cggatgcgac ctgtacgtca gacacgttct 23520
gattgctaac ttgccagtgt ttctctttgg ggaatcctgg gatggctcta gccgttccgc 23580
agacgggatc gatttcatga ttttttttgt ttcgttgcat agggtttggt ttgccctttt 23640
cctttatttc aatatatgcc gtgcacttgt ttgtcgggtc atcttttcat gctttttttt 23700
gtcttggttg tgatgatgtg gtctggttgg gcggtcgttc tagatcggag tagaattctg 23760
tttcaaacta cctggtggat ttattaattt tggatctgta tgtgtgtgcc atacatattc 23820
atagttacga attgaagatg atggatggaa atatcgatct aggataggta tacatgttga 23880
tgcgggtttt actgatgcat atacagagat gctttttgtt cgcttggttg tgatgatgtg 23940
gtgtggttgg gcggtcgttc attcgttcta gatcggagta gaatactgtt tcaaactacc 24000
tggtgtattt attaattttg gaactgtatg tgtgtgtcat acatcttcat agttacgagt 24060
ttaagatgga tggaaatatc gatctaggat aggtatacat gttgatgtgg gttttactga 24120
tgcatataca tgatggcata tgcagcatct attcatatgc tctaaccttg agtacctatc 24180
tattataata aacaagtatg ttttataatt attttgatct tgatatactt ggatgatggc 24240
atatgcagca gctatatgtg gattttttta gccctgcctt catacgctat ttatttgctt 24300
ggtactgttt cttttgtcga tgctcaccct gttgtttggt gttacttctg caggtcgact 24360
ctagaatgtg gattgaacaa gatggattgc acgcaggttc tccggccgct tgggtggaga 24420
ggctattcgg ctatgactgg gcacaacaga caatcggctg ctctgatgcc gccgtgttcc 24480
ggctgtcagc gcaggggcgc ccggttcttt ttgtcaagac cgacctgtcc ggtgccctga 24540
atgaactgca ggacgaggca gcgcggctat cgtggctggc cacgacgggc gttccttgcg 24600
cagctgtgct cgacgttgtc actgaagcgg gaagggactg gctgctattg ggcgaagtgc 24660
cggggcagga tctcctgtca tctcaccttg ctcctgccga gaaagtatcc atcatggctg 24720
atgcaatgcg gcggctgcat acgcttgatc cggctacctg cccattcgac caccaagcga 24780
aacatcgcat cgagcgagca cgtactcgga tggaagccgg tcttgtcgat caggatgatc 24840
tggacgaaga gcatcagggg ctcgcgccag ccgaactgtt cgccaggctc aaggcgcgca 24900
tgcccgacgg cgaggatctc gtcgtgaccc atggcgatgc ctgcttgccg aatatcatgg 24960
tggaaaatgg ccgcttttct ggattcatcg actgtggccg gctgggtgtg gcggaccgct 25020
atcaggacat agcgttggct acccgtgata ttgctgaaga gcttggcggc gaatgggctg 25080
accgcttcct cgtgctttac ggtatcgccg ctcccgattc gcagcgcatc gccttctatc 25140
gccttcttga cgagttcttc tgagatcgtt caaacatttg gcaataaagt ttcttaagat 25200

tgaatcctgt tgccggtctt gcgatgatta tcatataatt tctgttgaat tacgttaagc 25260

atgtaataat taacatgtaa tgcatgacgt tatttatgag atgggttttt atgattagag 25320

tcccgcaatt atacatttaa tacgcgatag aaaacaaaat atagcgcgca aactaggata 25380

aattatcgcg cgcggtgtca tctatgttac tagatcggcg cgccaagggc gaattccagc 25440

acactggcgg ccgttactag tggatcgagc tcgtcgactc tagactcgag ggcgcgccga 25500

taccaatttg gacagaaaac tgacaagaac ttcgaaaata cataattgga gttctagaga 25560

cacaacaaaa gtgatcctta actttatgta aagcttatta agttgtctat aactttctta 25620

ttatcatatt aagatgattc tacggctaac aaggacaaac atagcaaaga aaacatctct 25680

gccggatttg gacagattct gtcattctac tttgcaagct cataactaga gatttagacc 25740

atcacaatta gtgctatagg acattttgga aagcttagaa aaagtactac acttcttcta 25800

ttatctctaa gacacgattc catatgcaca caggtcaaac aaaccaaaca ttcagatcta 25860

ttcagaacat tgacaaaacc tgcccacaaa ggtgatccta gacttggtac cactagtatt 25920

aattaagttt aaacggcgcg ccaagggcga attccagcac actggcggcc gttactagtg 25980

gatcgagctc gtcgactcta gactcgaggg cgcgcctgac aggatatatt ggcgggtaaa 26040

c 26041

<210> 26
<211> 16771
<212> DNA
<213> Artificial sequence

<220>
<223> HR_template construct

<400> 26
cttagaataa cggatattta aaagggcgtg aaaaggttta tccgttcgtc catttgtatg 60

tgcatgccaa ccacagggtt cccctcggga tcaaagtact ttgatccaac ccctccgctg 120

ctatagtgca gtcggcttct gacgttcagt gcagccgtct tctgaaaacg acatgtcgca 180

caagtcctaa gttacgcgac aggctgccgc cctgcccttt tcctggcgtt ttcttgtcgc 240

gtgttttagt cgcataaagt agaatacttg cgactagaac cggagacatt acgccatgaa 300

caagagcgcc gccgctggcc tgctgggcta tgcccgcgtc agcaccgacg accaggactt 360

gaccaaccaa cgggccgaac tgcacgcggc cggctgcacc aagctgtttt ccgagaagat 420

caccggcacc aggcgcgacc gcccggagct ggccaggatg cttgaccacc tacgccctgg 480

cgacgttgtg acagtgacca ggctagaccg cctggcccgc agcacccgcg acctactgga 540

cattgccgag cgcatccagg aggccggcgc gggcctgcgt agcctggcag agccgtgggc 600

cgacaccacc acgccggccg gccgcatggt gttgaccgtg ttcgccggca ttgccgagtt 660

cgagcgttcc ctaatcatcg accgcacccg gagcgggcgc gaggccgcca aggcccgagg 720
cgtgaagttt ggccccccgcc ctaccctcac cccggcacag atcgcgcacg cccgcgagct 780
gatcgaccag gaaggccgca ccgtgaaaga ggcggctgca ctgcttggcg tgcatcgctc 840
gaccctgtac cgcgcacttg agcgcagcga ggaagtgacg cccaccgagg ccaggcggcg 900
cggtgccttc cgtgaggacg cattgaccga ggccgacgcc ctggcggccg ccgagaatga 960
acgccaagag gaacaagcat gaaaccgcac caggacggcc aggacgaacc gtttttcatt 1020
accgaagaga tcgaggcgga gatgatcgcg gccgggtacg tgttcgagcc gcccgcgcac 1080
gtctcaaccg tgcggctgca tgaaatcctg gccggtttgt ctgatgccaa gctggcggcc 1140
tggccggcca gcttggccgc tgaagaaacc gagcgccgcc gtctaaaaag gtgatgtgta 1200
tttgagtaaa acagcttgcg tcatgcggtc gctgcgtata tgatgcgatg agtaaataaa 1260
caaatacgca aggggaacgc atgaaggtta tcgctgtact taaccagaaa ggcgggtcag 1320
gcaagacgac catcgcaacc catctagccc gcgccctgca actcgccggg gccgatgttc 1380
tgttagtcga ttccgatccc cagggcagtg cccgcgattg ggcggccgtg cgggaagatc 1440
aaccgctaac cgttgtcggc atcgaccgcc cgacgattga ccgcgacgtg aaggccatcg 1500
gccggcgcga cttcgtagtg atcgacggag cgccccaggc ggcggacttg gctgtgtccg 1560
cgatcaaggc agccgacttc gtgctgattc cggtgcagcc aagcccttac gacatatggg 1620
ccaccgccga cctggtggag ctggttaagc agcgcattga ggtcacggat ggaaggctac 1680
aagcggcctt tgtcgtgtcg cgggcgatca aaggcacgcg catcggcggt gaggttgccg 1740
aggcgctggc cgggtacgag ctgcccattc ttgagtcccg tatcacgcag cgcgtgagct 1800
acccaggcac tgccgccgcc ggcacaaccg ttcttgaatc agaacccgag ggcgacgctg 1860
cccgcgaggt ccaggcgctg gccgctgaaa ttaaatcaaa actcatttga gttaatgagg 1920
taaagagaaa atgagcaaaa gcacaaacac gctaagtgcc ggccgtccga gcgcacgcag 1980
cagcaaggct gcaacgttgg ccagcctggc agacacgcca gccatgaagc gggtcaactt 2040
tcagttgccg gcggaggatc acaccaagct gaagatgtac gcggtacgcc aaggcaagac 2100
cattaccgag ctgctatctg aatacatcgc gcagctacca gagtaaatga gcaaatgaat 2160
aaatgagtag atgaatttta gcggctaaag gaggcggcat ggaaaatcaa gaacaaccag 2220
gcaccgacgc cgtggaatgc cccatgtgtg gaggaacggg cggttggcca ggcgtaagcg 2280
gctgggttgt ctgccggccc tgcaatggca ctggaacccc caagcccgag gaatcggcgt 2340
gacggtcgca aaccatccgg cccggtacaa atcggcgcgg cgctgggtga tgacctggtg 2400
gagaagttga aggccgcgca ggccgcccag cggcaacgca tcgaggcaga agcacgcccc 2460
ggtgaatcgt ggcaagcggc cgctgatcga atccgcaaag aatcccggca accgccggca 2520
gccggtgcgc cgtcgattag gaagccgccc aagggcgacg agcaaccaga tttttcgtt 2580

ccgatgctct atgacgtggg cacccgcgat agtcgcagca tcatggacgt ggccgttttc 2640
cgtctgtcga agcgtgaccg acgagctggc gaggtgatcc gctacgagct tccagacggg 2700
cacgtagagg tttccgcagg gccggccggc atggccagtg tgtgggatta cgacctggta 2760
ctgatggcgg tttcccatct aaccgaatcc atgaaccgat accgggaagg gaagggagac 2820
aagcccggcc gcgtgttccg tccacacgtt gcggacgtac tcaagttctg ccggcgagcc 2880
gatggcggaa agcagaaaga cgacctggta gaaacctgca ttcggttaaa caccacgcac 2940
gttgccatgc agcgtacgaa gaaggccaag aacggccgcc tggtgacggt atccgagggt 3000
gaagccttga ttagccgcta caagatcgta aagagcgaaa ccgggcggcc ggagtacatc 3060
gagatcgagc tagctgattg gatgtaccgc gagatcacag aaggcaagaa cccggacgtg 3120
ctgacggttc accccgatta ctttttgatc gatcccggca tcggccgttt tctctaccgc 3180
ctggcacgcc gcgccgcagg caaggcagaa gccagatggt tgttcaagac gatctacgaa 3240
cgcagtggca gcgccggaga gttcaagaag ttctgtttca ccgtgcgcaa gctgatcggg 3300
tcaaatgacc tgccggagta cgatttgaag gaggaggcgg ggcaggctgg cccgatccta 3360
gtcatgcgct accgcaacct gatcgagggc gaagcatccg ccggttccta atgtacggag 3420
cagatgctag ggcaaattgc cctagcaggg gaaaaaggtc gaaaaggtct ctttcctgtg 3480
gatagcacgt acattgggaa cccaaagccg tacattggga accggaaccc gtacattggg 3540
aacccaaagc cgtacattgg gaaccggtca cacatgtaag tgactgatat aaaagagaaa 3600
aaaggcgatt tttccgccta aaactcttta aaacttatta aaactcttaa aacccgcctg 3660
gcctgtgcat aactgtctgg ccagcgcaca gccgaagagc tgcaaaaagc gcctaccctt 3720
cggtcgctgc gctccctacg ccccgccgct tcgcgtcggc ctatcgcggc cgctggccgc 3780
tcaaaaatgg ctggcctacg gccaggcaat ctaccagggc gcggacaagc cgcgccgtcg 3840
ccactcgacc gccggcgccc acatcaaggc accctgcctc gcgcgtttcg gtgatgacgg 3900
tgaaaacctc tgacacatgc agctcccgga gacggtcaca gcttgtctgt aagcggatgc 3960
cgggagcaga caagcccgtc agggcgcgtc agcgggtgtt ggcgggtgtc ggggcgcagc 4020
catgacccag tcacgtagcg atagcggagt gtatactggc ttaactatgc ggcatcagag 4080
cagattgtac tgagagtgca ccatatgcgg tgtgaaatac cgcacagatg cgtaaggaga 4140
aaataccgca tcaggcgctc ttccgcttcc tcgctcactg actcgctgcg ctcggtcgtt 4200
cggctgcggc gagcggtatc agctcactca aaggcggtaa tacggttatc cacagaatca 4260
ggggataacg caggaaagaa catgtgagca aaaggccagc aaaaggccag gaaccgtaaa 4320
aaggccgcgt tgctggcgtt tttccatagg ctccgccccc ctgacgagca tcacaaaaat 4380
cgacgctcaa gtcagaggtg gcgaaacccg acaggactat aaagatacca ggcgtttccc 4440

cctggaagct ccctcgtgcg ctctcctgtt ccgaccctgc cgcttaccgg atacctgtcc 4500
gcctttctcc cttcgggaag cgtggcgctt tctcatagct cacgctgtag gtatctcagt 4560
tcggtgtagg tcgttcgctc caagctgggc tgtgtgcacg aaccccccgt tcagcccgac 4620
cgctgcgcct tatccggtaa ctatcgtctt gagtccaacc cggtaagaca cgacttatcg 4680
ccactggcag cagccactgg taacaggatt agcagagcga ggtatgtagg cggtgctaca 4740
gagttcttga agtggtggcc taactacggc tacactagaa ggacagtatt tggtatctgc 4800
gctctgctga agccagttac cttcggaaaa agagttggta gctcttgatc cggcaaacaa 4860
accaccgctg gtagcggtgg tttttttgtt tgcaagcagc agattacgcg cagaaaaaaa 4920
ggatctcaag aagatccttt gatcttttct acggggtctg acgctcagtg gaacgaaaac 4980
tcacgttaag ggattttggt catgcattct aggtattatt tgccaacgac cttcgtgatc 5040
tcgcccttga catagtggac aaattcttcg agctggtcgg cccgggacgc gagacggtct 5100
tcttcttggc ccagataggc ttggcgcgct tcgaggatca cgggctggta ttgcgccgga 5160
aggcgctcca tcgcccagtc ggcggcgaca tccttcggcg cgatcttgcc ggtaaccgcc 5220
gagtaccaaa tccggctcag cgtaaggacc acattgcgct catcgcccgc ccaatccggc 5280
ggggagttcc acagggtcag cgtctcgttc agtgcttcga acagatcctg ttccggcacc 5340
gggtcgaaaa gttcctcggc cgcggggccg acgagggcca cgctatgctc ccgggccttg 5400
gtgagcagga tcgccagatc aatgtcgatg gtggccggtt caaagatacc cgccagaata 5460
tcattacgct gccattcgcc gaactggagt tcgcgtttgg ccggatagcg ccaggggatg 5520
atgtcatcgt gcaccacaat cgtcacctca accgcgcgca ggatttcgct ctcgccgggg 5580
gaggcggacg tttccagaag gtcgttgata agcgcgcggc gcgtggtctc gtcgagacgg 5640
acggtaacgg tgacaagcag gtcgatgtcc gaatggggct taaggccgcc gtcaacggcg 5700
ctaccataca gatgcacggc gaggagggtc ggttcgaggt ggcgctcgat gacacccacg 5760
acttccgaca gctgggtgga cacctcggcg atgaccgctt cacccattta ttatttcctt 5820
cctcttttct acagtattta aagatacccc aagaagctaa ttataacaag acgaactcca 5880
attcactgtt ccttgcattc taaaacctta aataccagaa aacagctttt tcaaagttgt 5940
tttcaaagtt ggcgtataac atagtatcga cggagccgat tttgaaaccg cggtgatcac 6000
aggcagcaac gctctgtcat cgttacaatc aacatgctac cctccgcgag atcatccgtg 6060
tttcaaaccc ggcagcttag ttgccgttct tccgaatagc atcggtaaca tgagcaaagt 6120
ctgccgcctt acaacggctc tcccgctgac gccgtcccgg actgatgggc tgcctgtatc 6180
gagtggtgat tttgtgccga gctgccggtc ggggagctgt tggctggctg gtggcaggat 6240
atattgtggt gtaaacaaat tgacgcttag acaacttaat aacacattgc ggacgttttt 6300
aatgtactga attagtactg ataaatggcg cgccaagctt tggcaaacag ctattatggg 6360

tattatgggt ggtaccacgc gtcggatccg atctagtaac atagatgaca ccgcgcgcga 6420

taatttatcc tagtttgcgc gctatatttt gttttctatc gcgtattaaa tgtataattg 6480

cgggactcta atcataaaaa cccatctcat aaataacgtc atgcattaca tgttaattat 6540

tacatgctta acgtaattca acagaaatta tatgataatc atcgcaagac cggcaacagg 6600

attcaatctt aagaaacttt attgccaaat gtttgaacga tccctaggac gatctcactt 6660

gtagagctcg tccatgccgt agaggaacag gtgatggcgg ccctcggacc tctcgtactg 6720

ctcaacaata gtgtaatcct cgttatggct agtaatatcc agcttagtat ccacgtagta 6780

gtagccaggg agctgaaccg gcttcttggc catgtagata gtcttgaact ccaccaggta 6840

atggccacca tccttcagct tgagagcctg atgaatctcg cccttcagaa cgccatccct 6900

agggtagagc ctctcagtgg aggcctccca ccccatggtc ttcttctgca tgacagggcc 6960

gtctggaggg aagttggtgc cgcgcatctt caccttgtag atgagggtgc catcttggag 7020

ggagctatcc tgagtcacag taaccaggcc accatcttca aagttcatga cgcgttccca 7080

cttgaagcct tctgggaagg agagcttctt gtaatcagga atatcagcag ggtgcttcac 7140

gtaggctttg gagccgtaca tgaactgagg ggagaggata tcccaggcga aagggagagg 7200

gccgccctta gtcactttga gcttagcagt ctgggtgcct tcataagggc ggccctcgcc 7260

ctcaccttca atctcgaact catggccgtt catggagccc tccatcctga ccttgaagcg 7320

catgaactcc ttgatcacgg ccatgttgtt gtcctcgctg gaggcggtgc cgctggagcc 7380

gctgccagtg gagccagtgc cgtggccgag gaacaggtgg tgcctgccct cgctgcgctc 7440

gtactgctcc acgatggtgt agtcctcgtt gtgggaggtg atgtcgagct tggtgtcgac 7500

gtagtagtag cctggcagct gcacaggctt cttggccatg tagatggtct tgaactcgac 7560

caggtagtgg ccaccgtcct tgagcttcag ggcctggtgg atctcgccct tgagcacgcc 7620

gtccctgggg tacagcctct cagtgctagc ctcccagccc atggtcttct tctgcatgac 7680

cgggccatcg ggcgggaagt tagtgcccct catcttcacc ttgtagatga gggtgccatc 7740

ctggaggctg gagtcctgag tgacggtcac gaggccaccg tcctcgaagt tcatgacgcg 7800

ctcccacttg aagccctcgg ggaaggacag cttcttgtag tcggggatgt cggccgggtg 7860

cttcacgtag gccttgctgc cgtacatgaa ctgcggggag aggatgtccc aagcgaatgg 7920

cagcgggccg cccttagtga ccttgagctt ggcggtctgg gtgccctcgt aaggcctgcc 7980

ctcgccctcg ccctcgatct cgaactcgtg gccgttcatg ctgccctcca tcctcacctt 8040

gaagcgcatg aactccttga tcacttcctc gcccttggac accactacaa aaaagctccg 8100

cacgaggctg catttgtcac aaatcatgaa aagaaaaact accgatgaac aatgctgagg 8160

gattcaaatt ctacccacaa aaagaagaaa gaaagatcta gcacatctaa gcctgacgaa 8220

gcagcagaaa tatataaaaa tataaaccat agtgcccttt tcccctcttc ctgatcttgt 8280
ttagcacggc ggaaatttta aaccccccat catctccccc aacaacggcg gatcgcagat 8340
ctacatccga gagccccatt ccccgcgaga tccgggccgg atccacgccg gcgagagccc 8400
cagccgcgag atcccgcccc tcccgcgcac cgatctgggc gcgcacgaag ccgcctctcg 8460
cccacccaaa ctaccaaggc caaagatcga gaccgagacg gaaaaaaaaa cggagaaaga 8520
aagaggagag ggcggggtg gttaccggcg gcggcggagg cctcccttgg atcttatggt 8580
gtgttgtccc tgtgtgttct ccaatagtgt ggcttgagtg tgtggaagat ggttctagag 8640
gatctgctag agtcagcttg tcagcgtgtc ctctccaaat gaaatgaact tccttatata 8700
gaggaagggt cttgcgaagg atagtgggat tgtgcgtcat cccttacgtc agtggagata 8760
tcacatcaat ccacttgctt tgaagacgtg gttggaacgt cttctttttc cacgatgctc 8820
ctcgtgggtg ggggtccatc tttgggacca ctgtcggcag aggcatcttc aacgatggcc 8880
tttcctttat cgcaatgatg gcatttgtag gagccacctt ccttttccac tatcttcaca 8940
ataaagtgac agatagctgg gcaatggaat ccgaggaggt ttccggatat taccctttgt 9000
tgaaaagtct caatcggacc atcacatcaa tccacttgct ttgaagacgt ggttggaacg 9060
tcttcttttt ccacgatgct cctcgtgggt gggggtccat ctttgggacc actgtcggca 9120
gaggcatctt caacgatggc ctttccttta tcgcaatgat ggcatttgta ggagccacct 9180
tccttttcca ctatcttcac aataaagtga cagatagctg ggcaatggaa tccgaggagg 9240
tttccggata ttaccctttg ttgaaaagtc tcaatcggac cccctcagcc tgcacctcga 9300
ggaagtctag gatcaccttt gtgggaacga ggtgacaact agggcactac ctagataaca 9360
aacacacata caacatcaca atcaagcagt catttgagaa aattcaaaca aatgggctta 9420
agcaaccaac acaagacaca aggctcaccc aaaataagca tttatctcaa gcatgaacta 9480
actacaagtt tagctcaaaa caacgctaga tacggtagcc aacttagcat gtgcctagaa 9540
tctggacagc aacgcagtag ttacttgttt caaccataac tgaggttata gacataccac 9600
aaaggtgatc ctagactttc tagaaatctt aggaagatta ctacacttta gttattcata 9660
ccaaaaacta attcatagct taacatgacc aaaattgcta atatttcaca aattgacgct 9720
tagacaactt aataacacat tgcggacgtt tttaatgtac tgaattagta ctgatatcgg 9780
taccttaatt cgggggatct ggattttagt actggatttt ggttttagga attagaaatt 9840
ttattgatag aagtatttta caaatacaaa tacatactaa gggtttctta tatgctcaac 9900
acatgagcga aaccctatag gaaccctaat tcccttatct gggaactact cacacattat 9960
tatggagaaa ctcgagcttg tcgatcgatc actcagcctc gagggtggcg gtcactggga 10020
tgaactcgaa cctgtcgatg atcacgccgg cggtgccgct gaagttcctc acgcccacga 10080
tgttaccgag ggaggaggtg aaagcgttgg cgctctcgaa gtaaccgaag tcgctggatt 10140

ggaggttgtc cagggaggta gcggtagctg gcacggtgtt ggagaagatg gaggagttac 10200

cccagttcac gttgaggtgg atcggggtca cggaagcgta cctcacgcgc accctgtacc 10260

tggtggaggt ggatgggaag tggattggca ctcgatgtag cccctgttct ggatgttgtt 10320

gccgctgctg ttgagcctca cgaggtcgcc accggtgaag cctgggccgg aaatgacgga 10380

accgttgaag aggaagttgc ccttcacggc cgggatttgg gtgatgctgt cggaggcgat 10440

gatgttgttg aactcagcgc tgcggtggat ccaggagaac atcggagccc tgatgatgct 10500

cacggagctg ttgctgaagc cggagcggaa catggacacg tggctcagcc tgtgggagaa 10560

gccttgcctg ggtggcacgt tgttgttctg tggtgggatc tcgtccaggg agtccacggt 10620

gccgctcttc ctgtagacag cggatggcag gttggaggag gtgccgtagg cgaactcggt 10680

gccgtcgagc acggacagtt gctggttgtt gataccgatg ttgaagggcc tcctgtacag 10740

ggtggaggac agggtcctgt agacaccctg acccagttga gccacgatgc gttgctgtgg 10800

agcggcgttg cccatggtgc cgtagagcgg gaaggtgaac tcggggccgc tgaagcccac 10860

tggggaggcc atgatctggt ggccggacca gtagtactcg cccctgtgag cgtcggtgta 10920

gatggtgatg ctgttcagga tgtccatcag gtgtgggctc ctgatggagc cctcgatacc 10980

ctgggcggaa ccgcggaagc taccgtcgaa gttctccagc actgggttgg tgtagatctc 11040

cctggtgagt tgggacacgg tgcggatcgg gtaggtcctg gagtcgtagt tcgggaagag 11100

ggacacaatg tccagcacgg tgagggtcaa ctccctcctg aactggttgt acctgatcca 11160

gtccctggag tccggacccc agacgcgctc caggccggtg ttgtaccagc gcacagcgtg 11220

gtcggtgtag ttgccaatca gcctggtcag gtcgttgtag cggctgttga tggtggcagc 11280

atcgaagccc cacctttggc cgaacacgct cacgtcgcgc agcacgctga ggtgcaggtt 11340

agcggcttgc acgtacacgg acaggagcgg cacttggtag ttctggacgg cgaacagtgg 11400

gatagcggtg gtcagggcgc tgttcatgtc gttgaattga atgcgcattt cctcgcggag 11460

agctgggttg gtcgggtcgg cctcccactc cctgaagctc tcggcgtaga tttggtagag 11520

gttgctcagg ccctccagcc tggagatggc ctggttcctg gcgaactctt cgatcctctg 11580

gttgatcagc tgctcgattt gcaccaggaa ggcgtcccat tgggatggac cgaagatacc 11640

ccagatgatg tccaccaggc cgagcacgaa gccagcacct ggcacgaact cgctgagcag 11700

gaactgggtc aaggacaggg agatgtcgat gggggtgtaa ccggtctcga tgcgctcgcc 11760

acccagcacc tccacctctg ggttgctcag gcagttgtat gggatgcact cgttgatgtt 11820

tgggttgttg tccatggcgg ggttgatcag gttgatcact tctacctaca aaaaagctcc 11880

gcacgaggct gcatttgtca caaatcatga aaagaaaaac taccgatgaa caatgctgag 11940

ggattcaaat tctacccaca aaaagaagaa agaaagatct agcacatcta agcctgacga 12000

agcagcagaa atatataaaa atataaacca tagtgccctt ttcccctctt cctgatcttg 12060

tttagcacgg cggaaatttt aaacccccca tcatctcccc caacaacggc ggatcgcaga 12120

tctacatccg agagccccat tccccgcgag atccgggccg gatccacgcc ggcgagagcc 12180

ccagccgcga gatcccgccc ctcccgcgca ccgatctggg cgcgcacgaa gccgcctctc 12240

gcccacccaa actaccaagg ccaaagatcg agaccgagac ggaaaaaaaa acggagaaag 12300

aaagaggaga ggggcggggt ggttaccggc ggcggcggag gcctcccttg gatcttatgg 12360

tgtgttgtcc ctgtgtgttc tccaatagtg tggcttgagt gtgtggaaga tggttctaga 12420

ggatctgcta gagtcagctt gtcagcgtgt cctctccaaa tgaaatgaac ttccttatat 12480

agaggaaggg tcttgcgaag gatagtggga ttgtgcgtca tcccttacgt cagtggagat 12540

atcacatcaa tccacttgct ttgaagacgt ggttggaacg tcttcttttt ccacgatgct 12600

cctcgtgggt gggggtccat ctttgggacc actgtcggca gaggcatctt caacgatggc 12660

ctttccttta tcgcaatgat ggcatttgta ggagccacct tccttttcca ctatcttcac 12720

aataaagtga cagatagctg ggcaatggaa tccgaggagg tttccggata ttaccctttg 12780

ttgaaaagtc tcaatcggac catcacatca atccacttgc tttgaagacg tggttggaac 12840

gtcttctttt tccacgatgc tcctcgtggg tgggggtcca tctttgggac cactgtcggc 12900

agaggcatct tcaacgatgg cctttccttt atcgcaatga tggcatttgt aggagccacc 12960

ttccttttcc actatcttca caataaagtg acagatagct gggcaatgga atccgaggag 13020

gtttccggat attacccttt gttgaaaagt ctcaatcgga cctgtttaaa ccctgaagct 13080

tacgcgtatg cctgcagtgc agcgtgaccc ggtcgtgccc ctctctagag ataatgagca 13140

ttgcatgtct aagttataaa aaattaccac atattttttt tgtcacactt gtttgaagtg 13200

cagtttatct atctttatac atatatttaa actttactct acgaataata taatctatag 13260

tactacaata atatcagtgt tttagagaat catataaatg aacagttaga catggtctaa 13320

aggacaattg agtattttga caacaggact ctacagtttt atctttttag tgtgcatgtg 13380

ttctcctttt tttttgcaaa tagcttcacc tatataatac ttcatccatt ttattagtac 13440

atccatttag ggtttagggt taatggtttt tatagactaa tttttttagt acatctattt 13500

tattctattt tagcctctaa attaagaaaa ctaaaactct attttagttt ttttatttaa 13560

taatttagat ataaaataga ataaaataaa gtgactaaaa attaaacaaa taccctttaa 13620

gaaattaaaa aaactaagga aacatttttc ttgtttcgag tagataatgc cagcctgtta 13680

aacgccgtcg acgagtctaa cggacaccaa ccagcgaacc agcagcgtcg cgtcgggcca 13740

agcgaagcag acggcacggc atctctgtcg ctgcctctgg acccctctcg agagttccgc 13800

tccaccgttg gacttgctcc gctgtcggca tccagaaatt gcgtggcgga gcggcagacg 13860

tgagccggca cggcaggcgg cctcctcctc ctctcacggc acggcagcta cgggggattc 13920

ctttcccacc gctccttcgc tttcccttcc tcgcccgccg taataaatag acaccccctc 13980

cacaccctct ttccccaacc tcgtgttgtt cggagcgcac acacacacaa ccagatctcc 14040

cccaaatcca cccgtcggca cctccgcttc aaggtacgcc gctcgtcctc cccccccccc 14100

cctctctacc ttctctagat cggcgttccg gtccatggtt agggcccggt agttctactt 14160

ctgttcatgt ttgtgttaga tccgtgtttg tgttagatcc gtgctgctag cgttcgtaca 14220

cggatgcgac ctgtacgtca gacacgttct gattgctaac ttgccagtgt ttctctttgg 14280

ggaatcctgg gatggctcta gccgttccgc agacgggatc gatttcatga ttttttttgt 14340

ttcgttgcat agggtttggt ttgccctttt cctttatttc aatatatgcc gtgcacttgt 14400

ttgtcgggtc atcttttcat gctttttttt gtcttggttg tgatgatgtg gtctggttgg 14460

gcggtcgttc tagatcggag tagaattctg tttcaaacta cctggtggat ttattaattt 14520

tggatctgta tgtgtgtgcc atacatattc atagttacga attgaagatg atggatggaa 14580

atatcgatct aggataggta tacatgttga tgcgggtttt actgatgcat atacagagat 14640

gctttttgtt cgcttggttg tgatgatgtg gtgtggttgg gcggtcgttc attcgttcta 14700

gatcggagta gaatactgtt tcaaactacc tggtgtattt attaattttg gaactgtatg 14760

tgtgtgtcat acatcttcat agttacgagt ttaagatgga tggaaatatc gatctaggat 14820

aggtatacat gttgatgtgg gttttactga tgcatataca tgatggcata tgcagcatct 14880

attcatatgc tctaaccttg agtacctatc tattataata aacaagtatg ttttataatt 14940

attttgatct tgatatactt ggatgatggc atatgcagca gctatatgtg gattttttta 15000

gccctgcctt catacgctat ttatttgctt ggtactgttt cttttgtcga tgctcaccct 15060

gttgtttggt gttacttctg caggtcgact ctagaatgtg gattgaacaa gatggattgc 15120

acgcaggttc tccggccgct tgggtggaga ggctattcgg ctatgactgg gcacaacaga 15180

caatcggctg ctctgatgcc gccgtgttcc ggctgtcagc gcaggggcgc ccggttcttt 15240

ttgtcaagac cgacctgtcc ggtgccctga atgaactgca ggacgaggca gcgcggctat 15300

cgtggctggc cacgacgggc gttccttgcg cagctgtgct cgacgttgtc actgaagcgg 15360

gaagggactg gctgctattg ggcgaagtgc cggggcagga tctcctgtca tctcaccttg 15420

ctcctgccga gaaagtatcc atcatggctg atgcaatgcg gcggctgcat acgcttgatc 15480

cggctacctg cccattcgac caccaagcga aacatcgcat cgagcgagca cgtactcgga 15540

tggaagccgg tcttgtcgat caggatgatc tggacgaaga gcatcagggg ctcgcgccag 15600

ccgaactgtt cgccaggctc aaggcgcgca tgcccgacgg cgaggatctc gtcgtgaccc 15660

atggcgatgc ctgcttgccg aatatcatgg tggaaaatgg ccgcttttct ggattcatcg 15720

actgtggccg gctgggtgtg gcggaccgct atcaggacat agcgttggct acccgtgata 15780

ttgctgaaga gcttggcggc gaatgggctg accgcttcct cgtgctttac ggtatcgccg 15840

ctcccgattc gcagcgcatc gccttctatc gccttcttga cgagttcttc tgagatcgtt 15900

caaacatttg gcaataaagt ttcttaagat tgaatcctgt tgccggtctt gcgatgatta 15960

tcatataatt tctgttgaat tacgttaagc atgtaataat taacatgtaa tgcatgacgt 16020

tatttatgag atgggttttt atgattagag tcccgcaatt atacatttaa tacgcgatag 16080

aaaacaaaat atagcgcgca aactaggata aattatcgcg cgcggtgtca tctatgttac 16140

tagatcggcg cgccaagggc gaattccagc acactggcgg ccgttactag tggatcgagc 16200

tcgtcgactc tagactcgag ggcgcgccga taccaatttg gacagaaaac tgacaagaac 16260

ttcgaaaata cataattgga gttctagaga cacaacaaaa gtgatcctta actttatgta 16320

aagcttatta agttgtctat aactttctta ttatcatatt aagatgattc tacggctaac 16380

aaggacaaac atagcaaaga aaacatctct gccggatttg gacagattct gtcattctac 16440

tttgcaagct cataactaga gatttagacc atcacaatta gtgctatagg acattttgga 16500

aagcttagaa aaagtactac acttcttcta ttatctctaa gacacgattc catatgcaca 16560

caggtcaaac aaaccaaaca ttcagatcta ttcagaacat tgacaaaacc tgcccacaaa 16620

ggtgatccta gacttggtac cactagtatt aattaagttt aaacggcgcg ccaagggcga 16680

attccagcac actggcggcc gttactagtg gatcgagctc gtcgactcta gactcgaggg 16740

cgcgcctgac aggatatatt ggcgggtaaa c 16771

<210> 27
<211> 18754
<212> DNA
<213> Artificial sequence

<220>
<223> Cas9/crRNA template construction

<400> 27
cttagaataa cggatattta aaagggcgtg aaaaggttta tccgttcgtc catttgtatg 60

tgcatgccaa ccacagggtt cccctcggga tcaaagtact ttgatccaac ccctccgctg 120

ctatagtgca gtcggcttct gacgttcagt gcagccgtct tctgaaaacg acatgtcgca 180

caagtcctaa gttacgcgac aggctgccgc cctgcccttt tcctggcgtt ttcttgtcgc 240

gtgttttagt cgcataaagt agaatacttg cgactagaac cggagacatt acgccatgaa 300

caagagcgcc gccgctggcc tgctgggcta tgcccgcgtc agcaccgacg accaggactt 360

gaccaaccaa cgggccgaac tgcacgcggc cggctgcacc aagctgtttt ccgagaagat 420

caccggcacc aggcgcgacc gcccggagct ggccaggatg cttgaccacc tacgccctgg 480

cgacgttgtg acagtgacca ggctagaccg cctggcccgc agcacccgcg acctactgga 540

cattgccgag cgcatccagg aggccggcgc gggcctgcgt agcctggcag agccgtgggc 600

cgacaccacc acgccggccg gccgcatggt gttgaccgtg ttcgccggca ttgccgagtt 660
cgagcgttcc ctaatcatcg accgcacccg gagcgggcgc gaggccgcca aggcccgagg 720
cgtgaagttt ggcccccgcc ctaccctcac cccggcacag atcgcgcacg cccgcgagct 780
gatcgaccag gaaggccgca ccgtgaaaga ggcggctgca ctgcttggcg tgcatcgctc 840
gaccctgtac cgcgcacttg agcgcagcga ggaagtgacg cccaccgagg ccaggcggcg 900
cggtgccttc cgtgaggacg cattgaccga ggccgacgcc ctggcggccg ccgagaatga 960
acgccaagag gaacaagcat gaaaccgcac caggacggcc aggacgaacc gtttttcatt 1020
accgaagaga tcgaggcgga gatgatcgcg gccgggtacg tgttcgagcc gcccgcgcac 1080
gtctcaaccg tgcggctgca tgaaatcctg gccggtttgt ctgatgccaa gctggcggcc 1140
tggccggcca gcttggccgc tgaagaaacc gagcgccgcc gtctaaaaag gtgatgtgta 1200
tttgagtaaa acagcttgcg tcatgcggtc gctgcgtata tgatgcgatg agtaaataaa 1260
caaatacgca aggggaacgc atgaaggtta tcgctgtact taaccagaaa ggcgggtcag 1320
gcaagacgac catcgcaacc catctagccc gcgccctgca actcgccggg gccgatgttc 1380
tgttagtcga ttccgatccc cagggcagtg cccgcgattg ggcggccgtg cgggaagatc 1440
aaccgctaac cgttgtcggc atcgaccgcc cgacgattga ccgcgacgtg aaggccatcg 1500
gccggcgcga cttcgtagtg atcgacggag cgccccaggc ggcggacttg gctgtgtccg 1560
cgatcaaggc agccgacttc gtgctgattc cggtgcagcc aagcccttac gacatatggg 1620
ccaccgccga cctggtggag ctggttaagc agcgcattga ggtcacggat ggaaggctac 1680
aagcggcctt tgtcgtgtcg cgggcgatca aaggcacgcg catcggcggt gaggttgccg 1740
aggcgctggc cgggtacgag ctgcccattc ttgagtcccg tatcacgcag cgcgtgagct 1800
acccaggcac tgccgccgcc ggcacaaccg ttcttgaatc agaacccgag ggcgacgctg 1860
cccgcgaggt ccaggcgctg gccgctgaaa ttaaatcaaa actcatttga gttaatgagg 1920
taaagagaaa atgagcaaaa gcacaaacac gctaagtgcc ggccgtccga gcgcacgcag 1980
cagcaaggct gcaacgttgg ccagcctggc agacacgcca gccatgaagc gggtcaactt 2040
tcagttgccg gcggaggatc acaccaagct gaagatgtac gcggtacgcc aaggcaagac 2100
cattaccgag ctgctatctg aatacatcgc gcagctacca gagtaaatga gcaaatgaat 2160
aaatgagtag atgaatttta gcggctaaag gaggcggcat ggaaaatcaa gaacaaccag 2220
gcaccgacgc cgtggaatgc cccatgtgtg gaggaacggg cggttggcca ggcgtaagcg 2280
gctgggttgt ctgccggccc tgcaatggca ctggaacccc caagcccgag gaatcggcgt 2340
gacggtcgca aaccatccgg cccggtacaa atcggcgcgg cgctgggtga tgacctggtg 2400
gagaagttga aggccgcgca ggccgcccag cggcaacgca tcgaggcaga agcacgcccc 2460

ggtgaatcgt ggcaagcggc cgctgatcga atccgcaaag aatcccggca accgccggca 2520

gccggtgcgc cgtcgattag gaagccgccc aagggcgacg agcaaccaga tttttttcgtt 2580

ccgatgctct atgacgtggg cacccgcgat agtcgcagca tcatggacgt ggccgttttc 2640

cgtctgtcga agcgtgaccg acgagctggc gaggtgatcc gctacgagct tccagacggg 2700

cacgtagagg tttccgcagg gccggccggc atggccagtg tgtgggatta cgacctggta 2760

ctgatggcgg tttcccatct aaccgaatcc atgaaccgat accgggaagg gaagggagac 2820

aagcccggcc gcgtgttccg tccacacgtt gcggacgtac tcaagttctg ccggcgagcc 2880

gatggcggaa agcagaaaga cgacctggta gaaacctgca ttcggttaaa caccacgcac 2940

gttgccatgc agcgtacgaa gaaggccaag aacggccgcc tggtgacggt atccgagggt 3000

gaagccttga ttagccgcta caagatcgta aagagcgaaa ccgggcggcc ggagtacatc 3060

gagatcgagc tagctgattg gatgtaccgc gagatcacag aaggcaagaa cccggacgtg 3120

ctgacggttc accccgatta ctttttgatc gatcccggca tcggccgttt tctctaccgc 3180

ctggcacgcc gcgccgcagg caaggcagaa gccagatggt tgttcaagac gatctacgaa 3240

cgcagtggca gcgccggaga gttcaagaag ttctgtttca ccgtgcgcaa gctgatcggg 3300

tcaaatgacc tgccggagta cgatttgaag gaggaggcgg ggcaggctgg cccgatccta 3360

gtcatgcgct accgcaacct gatcgagggc gaagcatccg ccggttccta atgtacggag 3420

cagatgctag ggcaaattgc cctagcaggg gaaaaaggtc gaaaaggtct ctttcctgtg 3480

gatagcacgt acattgggaa cccaaagccg tacattggga accggaaccc gtacattggg 3540

aacccaaagc cgtacattgg gaaccggtca cacatgtaag tgactgatat aaaagagaaa 3600

aaaggcgatt tttccgccta aaactcttta aaacttatta aaactcttaa aacccgcctg 3660

gcctgtgcat aactgtctgg ccagcgcaca gccgaagagc tgcaaaaagc gcctaccctt 3720

cggtcgctgc gctccctacg ccccgccgct tcgcgtcggc ctatcgcggc cgctggccgc 3780

tcaaaaatgg ctggcctacg gccaggcaat ctaccagggc gcggacaagc cgcgccgtcg 3840

ccactcgacc gccggcgccc acatcaaggc accctgcctc gcgcgtttcg gtgatgacgg 3900

tgaaaacctc tgacacatgc agctcccgga gacggtcaca gcttgtctgt aagcggatgc 3960

cgggagcaga caagcccgtc agggcgcgtc agcgggtgtt ggcgggtgtc ggggcgcagc 4020

catgacccag tcacgtagcg atagcggagt gtatactggc ttaactatgc ggcatcagag 4080

cagattgtac tgagagtgca ccatatgcgg tgtgaaatac cgcacagatg cgtaaggaga 4140

aaataccgca tcaggcgctc ttccgcttcc tcgctcactg actcgctgcg ctcggtcgtt 4200

cggctgcggc gagcggtatc agctcactca aaggcggtaa tacggttatc cacagaatca 4260

ggggataacg caggaaagaa catgtgagca aaaggccagc aaaaggccag gaaccgtaaa 4320

aaggccgcgt tgctggcgtt tttccatagg ctccgccccc ctgacgagca tcacaaaaat 4380

cgacgctcaa gtcagaggtg gcgaaacccg acaggactat aaagatacca ggcgtttccc 4440
cctggaagct ccctcgtgcg ctctcctgtt ccgaccctgc cgcttaccgg atacctgtcc 4500
gcctttctcc cttcgggaag cgtggcgctt tctcatagct cacgctgtag gtatctcagt 4560
tcggtgtagg tcgttcgctc caagctgggc tgtgtgcacg aaccccccgt tcagcccgac 4620
cgctgcgcct tatccggtaa ctatcgtctt gagtccaacc cggtaagaca cgacttatcg 4680
ccactggcag cagccactgg taacaggatt agcagagcga ggtatgtagg cggtgctaca 4740
gagttcttga agtggtggcc taactacggc tacactagaa ggacagtatt tggtatctgc 4800
gctctgctga agccagttac cttcggaaaa agagttggta gctcttgatc cggcaaacaa 4860
accaccgctg gtagcggtgg tttttttgtt tgcaagcagc agattacgcg cagaaaaaaa 4920
ggatctcaag aagatccttt gatcttttct acggggtctg acgctcagtg gaacgaaaac 4980
tcacgttaag ggattttggt catgcattct aggtattatt tgccaacgac cttcgtgatc 5040
tcgcccttga catagtggac aaattcttcg agctggtcgg cccgggacgc gagacggtct 5100
tcttcttggc ccagataggc ttggcgcgct tcgaggatca cgggctggta ttgcgccgga 5160
aggcgctcca tcgcccagtc ggcggcgaca tccttcggcg cgatcttgcc ggtaaccgcc 5220
gagtaccaaa tccggctcag cgtaaggacc acattgcgct catcgcccgc ccaatccggc 5280
ggggagttcc acagggtcag cgtctcgttc agtgcttcga acagatcctg ttccggcacc 5340
gggtcgaaaa gttcctcggc cgcggggccg acgagggcca cgctatgctc ccgggccttg 5400
gtgagcagga tcgccagatc aatgtcgatg gtggccggtt caaagatacc cgccagaata 5460
tcattacgct gccattcgcc gaactggagt tcgcgtttgg ccggatagcg ccaggggatg 5520
atgtcatcgt gcaccacaat cgtcacctca accgcgcgca ggatttcgct ctcgccgggg 5580
gaggcggacg tttccagaag gtcgttgata agcgcgcggc gcgtggtctc gtcgagacgg 5640
acggtaacgg tgacaagcag gtcgatgtcc gaatggggct taaggccgcc gtcaacggcg 5700
ctaccataca gatgcacggc gaggagggtc ggttcgaggt ggcgctcgat gacacccacg 5760
acttccgaca gctgggtgga cacctcggcg atgaccgctt cacccattta ttatttcctt 5820
cctcttttct acagtattta aagatacccc aagaagctaa ttataacaag acgaactcca 5880
attcactgtt ccttgcattc taaaacctta aataccagaa aacagctttt tcaaagttgt 5940
tttcaaagtt ggcgtataac atagtatcga cggagccgat tttgaaaccg cggtgatcac 6000
aggcagcaac gctctgtcat cgttacaatc aacatgctac cctccgcgag atcatccgtg 6060
tttcaaaccc ggcagcttag ttgccgttct tccgaatagc atcggtaaca tgagcaaagt 6120
ctgccgcctt acaacggctc tcccgctgac gccgtcccgg actgatgggc tgcctgtatc 6180
gagtggtgat tttgtgccga gctgccggtc ggggagctgt tggctggctg gtggcaggat 6240

atattgtggt gtaaacaaat tgacgcttag acaacttaat aacacattgc ggacgttttt 6300

aatgtactga attagtactg ataaatggcg cgccaagctt tggcaaacag ctattatggg 6360

tattatgggt ggtaccacgc gtcgatccac tagtaacggc cgccagtgtg ctggaattcg 6420

cccttggcgc gccgatctag taacatagat gacaccgcgc gcgataattt atcctagttt 6480

gcgcgctata ttttgttttc tatcgcgtat taaatgtata attgcgggac tctaatcata 6540

aaaacccatc tcataaataa cgtcatgcat tacatgttaa ttattacatg cttaacgtaa 6600

ttcaacagaa attatatgat aatcatcgca agaccggcaa caggattcaa tcttaagaaa 6660

ctttattgcc aaatgtttga acgatctcag aagaactcgt caagaaggcg atagaaggcg 6720

atgcgctgcg aatcgggagc ggcgataccg taaagcacga ggaagcggtc agcccattcg 6780

ccgccaagct cttcagcaat atcacgggta gccaacgcta tgtcctgata gcggtccgcc 6840

acacccagcc ggccacagtc gatgaatcca gaaaagcggc cattttccac catgatattc 6900

ggcaagcagg catcgccatg ggtcacgacg agatcctcgc cgtcgggcat gcgcgccttg 6960

agcctggcga acagttcggc tggcgcgagc ccctgatgct cttcgtccag atcatcctga 7020

tcgacaagac cggcttccat ccgagtacgt gctcgctcga tgcgatgttt cgcttggtgg 7080

tcgaatgggc aggtagccgg atcaagcgta tgcagccgcc gcattgcatc agccatgatg 7140

gatactttct cggcaggagc aaggtgagat gacaggagat cctgccccgg cacttcgccc 7200

aatagcagcc agtcccttcc cgcttcagtg acaacgtcga gcacagctgc gcaaggaacg 7260

cccgtcgtgg ccagccacga tagccgcgct gcctcgtcct gcagttcatt cagggcaccg 7320

gacaggtcgg tcttgacaaa aagaaccggg cgcccctgcg ctgacagccg gaacacggcg 7380

gcatcagagc agccgattgt ctgttgtgcc cagtcatagc cgaatagcct ctccacccaa 7440

gcggccggag aacctgcgtg caatccatct tgttcaatcc acatggtggt gtgacctgca 7500

gaagtaacac caaacaacag ggtgagcatc gacaaaagaa acagtaccaa gcaaataaat 7560

agcgtatgaa ggcagggcta aaaaaatcca catatagctg ctgcatatgc catcatccaa 7620

gtatatcaag atcaaaataa ttataaaaca tacttgttta ttataataga taggtactca 7680

aggttagagc atatgaatag atgctgcata tgccatcatg tatatgcatc agtaaaaccc 7740

acatcaacat gtatacctat cctagatcga tatttccatc catcttaaac tcgtaactat 7800

gaagatgtat gacacacaca tacagttcca aaattaataa atacaccagg tagtttgaaa 7860

cagtattcta ctccgatcta gaacgaatga acgaccgccc aaccacacca catcatcaca 7920

accaagcgaa caaaaagcat ctctgtatat gcatcagtaa aacccgcatc aacatgtata 7980

cctatcctag atcgatattt ccatccatca tcttcaattc gtaactatga atatgtatgg 8040

cacacacata cagatccaaa attaataaat ccaccaggta gtttgaaaca gaattctact 8100

ccgatctaga acgaccgccc aaccagacca catcatcaca accaagacaa aaaaaagcat 8160

gaaaagatga cccgacaaac aagtgcacgg catatattga aataaaggaa aagggcaaac 8220
caaaccctat gcaacgaaac aaaaaaaatc atgaaatcga tcccgtctgc ggaacggcta 8280
gagccatccc aggattcccc aaagagaaac actggcaagt tagcaatcag aacgtgtctg 8340
acgtacaggt cgcatccgtg tacgaacgct agcagcacgg atctaacaca aacacggatc 8400
taacacaaac atgaacagaa gtagaactac cgggccctaa ccatggaccg gaacgccgat 8460
ctagagaagg tagagagggg ggggggggga ggacgagcgg cgtaccttga agcggaggtg 8520
ccgacgggtg gatttggggg agatctggtt gtgtgtgtgt gcgctccgaa caacacgagg 8580
ttggggaaag agggtgtgga gggggtgtct atttattacg gcgggcgagg aagggaaagc 8640
gaaggagcgg tgggaaagga atcccccgta gctgccgtgc cgtgagagga ggaggaggcc 8700
gcctgccgtg ccggctcacg tctgccgctc cgccacgcaa tttctggatg ccgacagcgg 8760
agcaagtcca acggtggagc ggaactctcg agaggggtcc agaggcagcg acagagatgc 8820
cgtgccgtct gcttcgcttg gcccgacgcg acgctgctgg ttcgctggtt ggtgtccgtt 8880
agactcgtcg acggcgttta acaggctggc attatctact cgaaacaaga aaaatgtttc 8940
cttagttttt ttaatttctt aaagggtatt tgtttaattt ttagtcactt tattttattc 9000
tattttatat ctaaattatt aaataaaaaa actaaaatag agttttagtt ttcttaattt 9060
agaggctaaa atagaataaa atagatgtac taaaaaaatt agtctataaa aaccattaac 9120
cctaaaccct aaatggatgt actaataaaa tggatgaagt attatatagg tgaagctatt 9180
tgcaaaaaaa aaggagaaca catgcacact aaaaagataa aactgtagag tcctgttgtc 9240
aaaatactca attgtccttt agaccatgtc taactgttca tttatatgat tctctaaaac 9300
actgatatta ttgtagtact atagattata ttattcgtag agtaaagttt aaatatatgt 9360
ataaagatag ataaactgca cttcaaacaa gtgtgacaaa aaaaatatgt ggtaattttt 9420
tataacttag acatgcaatg ctcattatct ctagagaggg gcacgaccgg gtcacgctgc 9480
actgcaggga tccgatctag taacatagat gacaccgcgc gcgataattt atcctagttt 9540
gcgcgctata ttttgttttc tatcgcgtat taaatgtata attgcgggac tctaatcata 9600
aaaacccatc tcataaataa cgtcatgcat tacatgttaa ttattacatg cttaacgtaa 9660
ttcaacagaa attatatgat aatcatcgca agaccggcaa caggattcaa tcttaagaaa 9720
ctttattgcc aaatgtttga acgatcccta ggacgatctc acttgtacag ctcgtccatg 9780
ccgtgggtga tgccagctgc ggtgacgaac tccagcagga ccatgtggtc gcgcttctcg 9840
ttggggtcct tgctcagagc ggactgggtg ctcaggtagt ggttgtcggg cagcagcacg 9900
ggaccgtcgc cgatgggcgt gttctgctgg tagtggtcgg cgagctggac gctgccgtcc 9960
tcgatgttgt ggcggatctt gaagttgacc ttgatgccgt tcttctgctt gtcagccatg 10020

atgtagacgt tgtggctgtt gtagttgtac tccagcttgt gccccaggat gttgccgtcc 10080
tccttgaagt cgatgccctt cagctcgatg cggttcacca gggtgtcgcc ctcgaacttc 10140
acctcggctc gggtcttgta gttgccgtcg tccttgaaga agatggtgcg ctcctggacg 10200
tagccttcgg gcatggcgga cttgaagaag tcgtgctgct tcatgtggtc ggggtagcgg 10260
ctgaagcact gcacgccgta ggtgaaggtg gtcacgaggg tgggccaggg cacgggcagc 10320
ttgccggtgg tgcagatgaa cttcagggtc agcttgccgt aggtggcgtc gccctcgccc 10380
tcgccgctga cgctgaactt gtggccgttc acgtcgccgt ccagctcgac caggatgggc 10440
accaccccag tgaacagctc ctcgcccttg ctcactacaa aaaagctccg cacgaggctg 10500
catttgtcac aaatcatgaa aagaaaaact accgatgaac aatgctgagg gattcaaatt 10560
ctacccacaa aaagaagaaa gaaagatcta gcacatctaa gcctgacgaa gcagcagaaa 10620
tatataaaaa tataaaccat agtgcccttt tcccctcttc ctgatcttgt ttagcacggc 10680
ggaaatttta aacccccccat catctccccc aacaacggcg gatcgcagat ctacatccga 10740
gagccccatt ccccgcgaga tccgggccgg atccacgccg gcgagagccc cagccgcgag 10800
atcccgcccc tcccgcgcac cgatctgggc gcgcacgaag ccgcctctcg cccacccaaa 10860
ctaccaaggc caaagatcga gaccgagacg gaaaaaaaaa cggagaaaga aagaggagag 10920
gggcggggtg gttaccggcg gcggcggagg cctcccttgg atcttatggt gtgttgtccc 10980
tgtgtgttct ccaatagtgt ggcttgagtg tgtggaagat ggttctagag gatctgctag 11040
agtcagcttg tcagcgtgtc ctctccaaat gaaatgaact tccttatata gaggaagggt 11100
cttgcgaagg atagtgggat tgtgcgtcat cccttacgtc agtggagata tcacatcaat 11160
ccacttgctt tgaagacgtg gttggaacgt cttctttttc cacgatgctc ctcgtgggtg 11220
ggggtccatc tttgggacca ctgtcggcag aggcatcttc aacgatggcc tttcctttat 11280
cgcaatgatg gcatttgtag gagccacctt ccttttccac tatcttcaca ataaagtgac 11340
agatagctgg gcaatggaat ccgaggaggt ttccggatat taccctttgt tgaaaagtct 11400
caatcggacc atcacatcaa tccacttgct ttgaagacgt ggttggaacg tcttcttttt 11460
ccacgatgct cctcgtgggt gggggtccat ctttgggacc actgtcggca gaggcatctt 11520
caacgatggc ctttccttta tcgcaatgat ggcatttgta ggagccacct tccttttcca 11580
ctatcttcac aataaagtga cagatagctg ggcaatggaa tccgaggagg tttccggata 11640
ttaccctttg ttgaaaagtc tcaatcggac cccctcagcc tgcagtgcag cgtgacccgg 11700
tcgtgcccct ctctagagat aatgagcatt gcatgtctaa gttataaaaa attaccacat 11760
attttttttg tcacacttgt ttgaagtgca gtttatctat ctttatacat atatttaaac 11820
tttactctac gaataatata atctatagta ctacaataat atcagtgttt tagagaatca 11880
tataaatgaa cagttagaca tggtctaaag gacaattgag tattttgaca acaggactct 11940

acagttttat ctttttagtg tgcatgtgtt ctcctttttt tttgcaaata gcttcaccta 12000

tataatactt catccatttt attagtacat ccatttaggg tttagggtta atggttttta 12060

tagactaatt tttttagtac atctatttta ttctatttta gcctctaaat taagaaaact 12120

aaaactctat tttagttttt ttatttaata atttagatat aaaatagaat aaaataaagt 12180

gactaaaaat taaacaaata ccctttaaga aattaaaaaa actaaggaaa catttttctt 12240

gtttcgagta gataatgcca gcctgttaaa cgccgtcgac gagtctaacg gacaccaacc 12300

agcgaaccag cagcgtcgcg tcgggccaag cgaagcagac ggcacggcat ctctgtcgct 12360

gcctctggac ccctctcgag agttccgctc caccgttgga cttgctccgc tgtcggcatc 12420

cagaaattgc gtggcggagc ggcagacgtg agccggcacg gcaggcggcc tcctcctcct 12480

ctcacggcac ggcagctacg gggggattcct ttcccaccgc tccttcgctt tcccttcctc 12540

gcccgccgta ataaatagac accccctcca caccctcttt ccccaacctc gtgttgttcg 12600

gagcgcacac acacacaacc agatctcccc caaatccacc cgtcggcacc tccgcttcaa 12660

ggtacgccgc tcgtcctccc cccccccccc tctctacctt ctctagatcg gcgttccggt 12720

ccatggttag ggcccggtag ttctacttct gttcatgttt gtgttagatc cgtgtttgtg 12780

ttagatccgt gctgctagcg ttcgtacacg gatgcgacct gtacgtcaga cacgttctga 12840

ttgctaactt gccagtgttt ctctttgggg aatcctggga tggctctagc cgttccgcag 12900

acgggatcga tttcatgatt ttttttgttt cgttgcatag ggtttggttt gccctttcc 12960

tttatttcaa tatatgccgt gcacttgttt gtcgggtcat cttttcatgc ttttttttgt 13020

cttggttgtg atgatgtggt ctggttgggc ggtcgttcta gatcggagta gaattctgtt 13080

tcaaactacc tggtggattt attaattttg gatctgtatg tgtgtgccat acatattcat 13140

agttacgaat tgaagatgat ggatggaaat atcgatctag gataggtata catgttgatg 13200

cgggttttac tgatgcatat acagagatgc tttttgttcg cttggttgtg atgatgtggt 13260

gtggttgggc ggtcgttcat tcgttctaga tcggagtaga atactgtttc aaactacctg 13320

gtgtatttat taattttgga actgtatgtg tgtgtcatac atcttcatag ttacgagttt 13380

aagatggatg gaaatatcga tctaggatag gtatacatgt tgatgtgggt tttactgatg 13440

catatacatg atggcatatg cagcatctat tcatatgctc taaccttgag tacctatcta 13500

ttataataaa caagtatgtt ttataattat tttgatcttg atatacttgg atgatggcat 13560

atgcagcagc tatatgtgga tttttttagc cctgccttca tacgctattt atttgcttgg 13620

tactgtttct tttgtcgatg ctcaccctgt tgtttggtgt tacttctgca ggcgatcgcc 13680

acaccaccat gccgaagaag aagcgcaagg tcatggacaa gaagtactcc atcggcctgg 13740

acatcggcac caacagcgtg ggctgggccg tcatcaccga cgagtacaag gtgccctcca 13800

agaagttcaa ggtcctcggc aacaccgaca ggcacagcat caagaagaac ctgatcggcg 13860

ccctgctgtt cgactccggc gagactgcgg aggctaccag gctgaagcgc actgctcgca 13920

ggcgctacac caggcgcaag aaccgcatct gctacctcca ggagattttc tccaacgaga 13980

tggccaaggt ggacgactcc ttcttccacc gcctggagga gagcttcctg gtcgaggaag 14040

acaagaagca cgagcgccac cctatcttcg gcaacatcgt ggacgaggtc gcctaccacg 14100

agaagtaccc aaccatctac cacctccgca agaagctggt ggactccacc gacaaggccg 14160

acctgaggct catctacctg gccctcgccc acatgatcaa gttccgcggc cacttcctca 14220

tcgagggcga cctgaacccg gacaacagcg acgtggacaa gctcttcatc cagctggtcc 14280

agacctacaa ccagctgttc gaggagaacc ccatcaacgc ctccggcgtg gacgctaagg 14340

ctatcctcag cgctaggctg tccaagagca ggcgcctgga gaacctcatc gcccagctcc 14400

cgggcgagaa gaagaacggc ctcttcggca acctgatcgc tctgtccctc ggcctgaccc 14460

ccaacttcaa gagcaacttc gacctggccg aggacgccaa gctccagctg tccaaggaca 14520

cctacgacga cgacctcgac aacctgctcg cccagatcgg cgaccagtac gccgacctct 14580

tcctggccgc caagaacctc tccgacgcca tcctgctcag cgacatcctg agggtgaaca 14640

ccgagatcac caaggccccg ctgtccgcca gcatgatcaa gcgctacgac gagcaccacc 14700

aggacctcac tctcctgaag gccctcgtcc gccagcagct gcccgagaag tacaaggaga 14760

ttttcttcga ccagagcaag aacggctacg cgggctacat cgatggcggc gcctcccagg 14820

aagagttcta caagttcatc aagcctatcc tggagaagat ggacggcacc gaggagctgc 14880

tcgtgaagct gaaccgcgag gacctgctcc gcaagcagag gaccttcgac aacggcagca 14940

tccctcacca gatccacctg ggcgagctgc acgctatcct ccgccgccag gaagacttct 15000

acccattcct gaaggacaac cgcgagaaga tcgagaagat cctcaccttc cgcatcccgt 15060

actacgtggg ccccctggcc cgcggcaact ccaggttcgc ctggatgacc aggaagagcg 15120

aggagaccat caccccgtgg aacttcgagg aagtggtgga caagggcgcc tccgctcaga 15180

gcttcatcga gcgcatgacc aacttcgaca agaacctccc taacgagaag gtgctgccaa 15240

agcactccct gctctacgag tacttcaccg tctacaacga gctgaccaag gtgaagtatg 15300

tgaccgaggg catgaggaag cccgccttcc tcagcggcga gcagaagaag gccatcgtgg 15360

acctgctctt caagaccaac cgcaaggtga ccgtcaagca gctgaaggaa gactacttca 15420

agaagatcga gtgcttcgac tccgtggaga tcagcggcgt ggaggaccgc ttcaacgcct 15480

ccctcggcac ctaccacgac ctgctcaaga tcatcaagga caaggacttc ctcgacaacg 15540

aggagaacga ggacatcctg gaggacatcg tgctcaccct gaccctcttc gaggaccgcg 15600

agatgatcga ggagaggctc aagacctacg cccacctgtt cgacgacaag gtcatgaagc 15660

agctgaagag gcgcaggtac actggctggg gccgcctcag caggaagctg atcaacggca 15720

tcagggacaa gcagtccggc aagaccatcc tggacttcct caagagcgac ggcttcgcca 15780

accgcaactt catgcagctc atccacgacg actccctgac cttcaaggaa gacatccaga 15840

aggctcaggt gtccggccag ggcgacagcc tccacgagca catcgctaac ctggcgggca 15900

gccctgccat caagaagggc atcctccaga ccgtgaaggt ggtggacgag ctggtgaagg 15960

tcatgggccg ccacaagcca gagaacatcg tcatcgagat ggccagggag aaccagacca 16020

cccagaaggg tcagaagaac tcccgcgaga ggatgaagag gatcgaggaa ggcatcaagg 16080

agctgggcag ccagatcctg aaggagcacc cggtggagaa cacccagctc cagaacgaga 16140

agctgtacct ctactacctg cagaacggcc gcgacatgta tgtggaccag gagctggaca 16200

tcaacaggct gtccgactac gacgtggacc acatcgtccc tcagtccttc ctcaaggacg 16260

acagcatcga caacaaggtg ctgacccgca gcgacaagaa caggggcaag tccgacaacg 16320

tcccaagcga ggaagtggtc aagaagatga agaactactg gcgccagctg ctcaacgcca 16380

agctcatcac ccagcgcaag ttcgacaacc tgactaaggc ggagaggggc ggcctgtccg 16440

agctggacaa ggctggcttc atcaagcgcc agctcgtgga gaccaggcag atcaccaagc 16500

acgtcgccca gatcctggac agcaggatga acaccaagta cgacgagaac gacaagctca 16560

tccgcgaggt gaaggtcatc accctcaagt ccaagctggt gagcgacttc cgcaaggact 16620

tccagttcta caaggtcagg gagatcaaca actaccacca cgcccacgat gcttacctca 16680

acgcggtggt gggcaccgcc ctcatcaaga agtaccctaa gctggagagc gagttcgtgt 16740

acggcgacta caaggtgtac gacgtccgca agatgatcgc caagtccgag caggagatcg 16800

gcaaggccac cgccaagtac ttcttctaca gcaacatcat gaacttcttc aagaccgaga 16860

tcaccctcgc caacggcgag atccgcaaga ggccactgat cgagaccaac ggcgagactg 16920

gcgagatcgt gtgggacaag ggcagggact tcgccaccgt gaggaaggtc ctgtccatgc 16980

ctcaggtgaa catcgtcaag aagaccgagg tccagaccgg cggcttctcc aaggagagca 17040

tcctcccaaa gcgcaacagc gacaagctga tcgccaggaa gaaggactgg gacccgaaga 17100

agtacggtgg cttcgactcc cctactgtgg cttacagcgt cctggtggtc gccaaggtgg 17160

agaagggcaa gtccaagaag ctgaagagcg tcaaggagct gctcggcatc accatcatgg 17220

agaggtccag cttcgagaag aacccgatcg acttcctgga ggccaagggc tacaaggaag 17280

tgaagaagga cctgatcatc aagctgccca agtacagcct gttcgagctg gagaacggcc 17340

gcaagaggat gctcgcctcc gctggcgagc tgcagaaggg caacgagctg gccctcccgt 17400

ccaagtatgt gaacttcctg tacctcgcct cccactacga gaagctgaag ggcagccccg 17460

aggacaacga gcagaagcag ctcttcgtcg agcagcacaa gcactacctg gacgagatca 17520

tcgagcagat cagcgagttc agcaagcgcg tgatcctcgc cgacgccaac ctcgacaagg 17580

tcctgtccgc ctacaacaag caccgcgaca agcctatcag ggagcaggcc gagaacatca 17640

tccacctgtt caccctcacc aacctgggcg ccccagctgc cttcaagtac ttcgacacca 17700

ccatcgaccg caagaggtac accagcacca aggaagtgct ggacgccacc ctgatccacc 17760

agtccatcac cggcctgtac gagactcgca tcgacctcag ccagctgggc ggcgacccga 17820

agaagaagcg caaagtctga gggaccctcg atcgacaagc tcgagtttct ccataataat 17880

gtgtgagtag ttcccagata agggaattag ggttcctata gggtttcgct catgtgttga 17940

gcatataaga aacccttagt atgtatttgt atttgtaaaa tacttctatc aataaaattt 18000

ctaattccta aaaccaaaat ccagtactaa aatccagatc ccccgaatta acctgcaggg 18060

gcggcaggga gagttttaac attgactagc gtgctgataa tttgtgagaa ataataattg 18120

acaagtagat actgacattt gagaagagct tctgaactgt tattagtaac aaaaatggaa 18180

agctgatgca cggaaaaagg aaagaaaaag ccatactttt ttttaggtag gaaaagaaaa 18240

agccatacga gactgatgtc tctcagatgg gccgggatct gtctatctag caggcagcag 18300

ccctaccaac ctcacgggcc agcaattacg agtccttcta aaacgtcccg ccgagggcgc 18360

gtggccgtgc tgtgcagcag cacgtctaac attagtccca cctcgccagt ttacagggag 18420

cagaaccagc ttataagcgg aggcgcggca ccaagaagca aagtctagga tcacctttgt 18480

gttttagagc tagaaatagc aagttaaaat aaggctagtc cgttatcaac ttgaaaaagt 18540

ggcaccgagt cggtgctttt ttttcctcga ggggcgatag gaacacgtac aacggccgtt 18600

gtacgtgttc ctatcgccgg taccactagt attaattaag tttaaacggc gcgccaaggg 18660

cgaattccag cacactggcg gccgttacta gtggatcgag ctcgtcgact ctagactcga 18720

gggcgcgcct gacaggatat attggcgggt aaac 18754

<210> 28
<211> 20
<212> DNA
<213> Artificial sequence

<220>
<223> Cas9 - crRNA

<400> 28
aagtctagga tcacctttgt 20

<210> 29
<211> 7519
<212> DNA
<213> Artificial sequence

<220>
<223> Complementary sequence of SEQ ID NO 22 (reverse-complement)

<400> 29
gcatcttcat gattgttgcc ttatgcatag ctatgctcct ctcacccttg ttcaaatgat 60

gagtaatttg tgagtgatgt ggtatcctat cttccttgtg tggagtcaaa caagaatgct 120
ttgttccatg ttgcttgaat aggaaatgct tgagaaaaat gtgtgcttag ctttactaga 180
aataagtgtg actacatgct tgagcttaga gttatgaatt ttaaaagttt acggtcaggt 240
tttgtcaatg ttctgaatag atctgaatgt ttggtttgtt tgacctgtgt gcatatggaa 300
tcgtgtctta gagataatag aagaagtgta gtactttttc taagctttcc aaaatgtcct 360
atagcactaa ttgtgatggt ctaaatctct agttatgagc ttgcaaagta gaatgacaga 420
atctgtccaa atccggcaga gatgttttct ttgctatgtt tgtccttgtt agccgtagaa 480
tcatcttaat atgataataa gaaagttata gacaacttaa taagctttac ataaagttaa 540
ggatcacttt tgttgtgtct ctagaactcc aattatgtat tttcgaagtt cttgtcagtt 600
ttctgtccaa attggtatcg gcgcgccctc gagtctagag tcgacgagct cgatccacta 660
gtaacggccg ccagtgtgct ggaattcgcc cttggcgcgc cgatctagta acatagatga 720
caccgcgcgc gataatttat cctagtttgc gcgctatatt ttgttttcta tcgcgtatta 780
aatgtataat tgcgggactc taatcataaa aacccatctc ataaataacg tcatgcatta 840
catgttaatt attacatgct taacgtaatt caacagaaat tatatgataa tcatcgcaag 900
accggcaaca ggattcaatc ttaagaaact ttattgccaa atgtttgaac gatctcagaa 960
gaactcgtca agaaggcgat agaaggcgat gcgctgcgaa tcgggagcgg cgataccgta 1020
aagcacgagg aagcggtcag cccattcgcc gccaagctct tcagcaatat cacgggtagc 1080
caacgctatg tcctgatagc ggtccgccac acccagccgg ccacagtcga tgaatccaga 1140
aaagcggcca ttttccacca tgatattcgg caagcaggca tcgccatggg tcacgacgag 1200
atcctcgccg tcgggcatgc gcgccttgag cctggcgaac agttcggctg gcgcgagccc 1260
ctgatgctct tcgtccagat catcctgatc gacaagaccg gcttccatcc gagtacgtgc 1320
tcgctcgatg cgatgtttcg cttggtggtc gaatgggcag gtagccggat caagcgtatg 1380
cagccgccgc attgcatcag ccatgatgga tactttctcg gcaggagcaa ggtgagatga 1440
caggagatcc tgccccggca cttcgcccaa tagcagccag tcccttcccg cttcagtgac 1500
aacgtcgagc acagctgcgc aaggaacgcc cgtcgtggcc agccacgata gccgcgctgc 1560
ctcgtcctgc agttcattca gggcaccgga caggtcggtc ttgacaaaaa gaaccgggcg 1620
cccctgcgct gacagccgga acacggcggc atcagagcag ccgattgtct gttgtgccca 1680
gtcatagccg aatagcctct ccacccaagc ggccggagaa cctgcgtgca atccatcttg 1740
ttcaatccac attctagagt cgacctgcag aagtaacacc aaacaacagg gtgagcatcg 1800
acaaaagaaa cagtaccaag caaataaata gcgtatgaag gcagggctaa aaaaatccac 1860
atatagctgc tgcatatgcc atcatccaag tatatcaaga tcaaaataat tataaaacat 1920

acttgtttat tataatagat aggtactcaa ggttagagca tatgaataga tgctgcatat 1980
gccatcatgt atatgcatca gtaaaaccca catcaacatg tatacctatc ctagatcgat 2040
atttccatcc atcttaaact cgtaactatg aagatgtatg acacacacat acagttccaa 2100
aattaataaa tacaccaggt agtttgaaac agtattctac tccgatctag aacgaatgaa 2160
cgaccgccca accacaccac atcatcacaa ccaagcgaac aaaaagcatc tctgtatatg 2220
catcagtaaa acccgcatca acatgtatac ctatcctaga tcgatatttc catccatcat 2280
cttcaattcg taactatgaa tatgtatggc acacacatac agatccaaaa ttaataaatc 2340
caccaggtag tttgaaacag aattctactc cgatctagaa cgaccgccca accagaccac 2400
atcatcacaa ccaagacaaa aaaaagcatg aaaagatgac ccgacaaaca agtgcacggc 2460
atatattgaa ataaaggaaa agggcaaacc aaaccctatg caacgaaaca aaaaaaatca 2520
tgaaatcgat cccgtctgcg gaacggctag agccatccca ggattcccca aagagaaaca 2580
ctggcaagtt agcaatcaga acgtgtctga cgtacaggtc gcatccgtgt acgaacgcta 2640
gcagcacgga tctaacacaa acacggatct aacacaaaca tgaacagaag tagaactacc 2700
gggccctaac catggaccgg aacgccgatc tagagaaggt agagaggggg ggggggggag 2760
gacgagcggc gtaccttgaa gcggaggtgc cgacgggtgg atttggggga gatctggttg 2820
tgtgtgtgtg cgctccgaac aacacgaggt tggggaaaga gggtgtggag gggtgtcta 2880
tttattacgg cgggcgagga agggaaagcg aaggagcggt gggaaaggaa tcccccgtag 2940
ctgccgtgcc gtgagaggag gaggaggccg cctgccgtgc cggctcacgt ctgccgctcc 3000
gccacgcaat ttctggatgc cgacagcgga gcaagtccaa cggtggagcg gaactctcga 3060
gaggggtcca gaggcagcga cagagatgcc gtgccgtctg cttcgcttgg cccgacgcga 3120
cgctgctggt tcgctggttg gtgtccgtta gactcgtcga cggcgtttaa caggctggca 3180
ttatctactc gaaacaagaa aaatgtttcc ttagtttttt taatttctta aagggtattt 3240
gtttaatttt tagtcacttt attttattct attttatatc taaattatta aataaaaaaa 3300
ctaaaataga gttttagttt tcttaattta gaggctaaaa tagaataaaa tagatgtact 3360
aaaaaaatta gtctataaaa accattaacc ctaaacccta aatggatgta ctaataaaat 3420
ggatgaagta ttatataggt gaagctattt gcaaaaaaaa aggagaacac atgcacacta 3480
aaaagataaa actgtagagt cctgttgtca aaatactcaa ttgtccttta gaccatgtct 3540
aactgttcat ttatatgatt ctctaaaaca ctgatattat tgtagtacta tagattatat 3600
tattcgtaga gtaaagttta aatatatgta taaagataga taaactgcac ttcaaacaag 3660
tgtgacaaaa aaaatatgtg gtaatttttt ataacttaga catgcaatgc tcattatctc 3720
tagagagggg cacgaccggg tcacgctgca ctgcaggcat acgcgtaagc ttcagggttt 3780
aaacaggtcc gattgagact tttcaacaaa gggtaatatc cggaaacctc ctcggattcc 3840

attgcccagc tatctgtcac tttattgtga agatagtgga aaaggaaggt ggctcctaca 3900

aatgccatca ttgcgataaa ggaaaggcca tcgttgaaga tgcctctgcc gacagtggtc 3960

ccaaagatgg acccccaccc acgaggagca tcgtggaaaa agaagacgtt ccaaccacgt 4020

cttcaaagca agtggattga tgtgatggtc cgattgagac ttttcaacaa agggtaatat 4080

ccggaaacct cctcggattc cattgcccag ctatctgtca ctttattgtg aagatagtgg 4140

aaaaggaagg tggctcctac aaatgccatc attgcgataa aggaaaggcc atcgttgaag 4200

atgcctctgc cgacagtggt cccaaagatg gacccccacc cacgaggagc atcgtggaaa 4260

aagaagacgt tccaaccacg tcttcaaagc aagtggattg atgtgatatc tccactgacg 4320

taagggatga cgcacaatcc cactatcctt cgcaagaccc ttcctctata taaggaagtt 4380

catttcattt ggagaggaca cgctgacaag ctgactctag cagatcctct agaaccatct 4440

tccacacact caagccacac tattggagaa cacacaggga caacacacca taagatccaa 4500

gggaggcctc cgccgccgcc ggtaaccacc ccgcccctct cctctttctt tctccgtttt 4560

tttttccgtc tcggtctcga tctttggcct tggtagtttg ggtgggcgag aggcggcttc 4620

gtgcgcgccc agatcggtgc gcgggagggg cgggatctcg cggctggggc tctcgccggc 4680

gtggatccgg cccggatctc gcggggaatg gggctctcgg atgtagatct gcgatccgcc 4740

gttgttgggg gagatgatgg ggggtttaaa atttccgccg tgctaaacaa gatcaggaag 4800

aggggaaaag ggcactatgg tttatatttt tatatatttc tgctgcttcg tcaggcttag 4860

atgtgctaga tctttctttc ttctttttgt gggtagaatt tgaatccctc agcattgttc 4920

atcggtagtt tttcttttca tgatttgtga caaatgcagc ctcgtgcgga gctttttttgt 4980

aggtagaagt gatcaacctg atcaaccccg ccatggacaa caacccaaac atcaacgagt 5040

gcatcccata caactgcctg agcaacccag aggtggaggt gctgggtggc gagcgcatcg 5100

agaccggtta cacccccatc gacatctccc tgtccttgac ccagttcctg ctcagcgagt 5160

tcgtgccagg tgctggcttc gtgctcggcc tggtggacat catctggggt atcttcggtc 5220

catcccaatg ggacgccttc ctggtgcaaa tcgagcagct gatcaaccag aggatcgaag 5280

agttcgccag gaaccaggcc atctccaggc tggagggcct gagcaacctc taccaaatct 5340

acgccgagag cttcagggag tgggaggccg acccgaccaa cccagctctc cgcgaggaaa 5400

tgcgcattca attcaacgac atgaacagcg ccctgaccac cgctatccca ctgttcgccg 5460

tccagaacta ccaagtgccg ctcctgtccg tgtacgtgca agccgctaac ctgcacctca 5520

gcgtgctgcg cgacgtgagc gtgttcggcc aaaggtgggg cttcgatgct gccaccatca 5580

acagccgcta caacgacctg accaggctga ttggcaacta caccgaccac gctgtgcgct 5640

ggtacaacac cggcctggag cgcgtctggg gtccggactc cagggactgg atcaggtaca 5700

accagttcag gagggagttg accctcaccg tgctggacat tgtgtccctc ttcccgaact 5760

acgactccag gacctacccg atccgcaccg tgtcccaact caccagggag atctacacca 5820

acccagtgct ggagaacttc gacggtagct tccgcggttc cgcccagggt atcgagggct 5880

ccatcaggag cccacacctg atggacatcc tgaacagcat caccatctac accgacgctc 5940

acaggggcga gtactactgg tccggccacc agatcatggc ctccccagtg ggcttcagcg 6000

gccccgagtt caccttcccg ctctacggca ccatgggcaa cgccgctcca cagcaacgca 6060

tcgtggctca actgggtcag ggtgtctaca ggaccctgtc ctccaccctg tacaggaggc 6120

ccttcaacat cggtatcaac aaccagcaac tgtccgtgct cgacggcacc gagttcgcct 6180

acggcacctc ctccaacctg ccatccgctg tctacaggaa gagcggcacc gtggactccc 6240

tggacgagat cccaccacag aacaacaacg tgccacccag gcaaggcttc tcccacaggc 6300

tgagccacgt gtccatgttc cgctccggct tcagcaacag ctccgtgagc atcatcaggg 6360

ctccgatgtt ctcctggatc caccgcagcg ctgagttcaa caacatcatc gcctccgaca 6420

gcatcaccca aatcccggcc gtgaagggca acttcctctt caacggttcc gtcatttccg 6480

gcccaggctt caccggtggc gacctcgtga ggctcaacag cagcggcaac aacatccaga 6540

acaggggcta catcgagtgc caatccactt cccatccacc tccaccaggt acagggtgcg 6600

cgtgaggtac gcttccgtga ccccgatcca cctcaacgtg aactggggta actcctccat 6660

cttctccaac accgtgccag ctaccgctac ctccctggac aacctccaat ccagcgactt 6720

cggttacttc gagagcgcca acgctttcac ctcctccctc ggtaacatcg tgggcgtgag 6780

gaacttcagc ggcaccgccg gcgtgatcat cgacaggttc gagttcatcc cagtgaccgc 6840

caccctcgag gctgagtgat cgatcgacaa gctcgagttt ctccataata atgtgtgagt 6900

agttcccaga taagggaatt agggttccta tagggtttcg ctcatgtgtt gagcatataa 6960

gaaaccctta gtatgtattt gtatttgtaa aatacttcta tcaataaaat ttctaattcc 7020

taaaaccaaa atccagtact aaaatccaga tcccccgaat taaggtaccg atatcagtac 7080

taattcagta cattaaaaac gtccgcaatg tgttattaag ttgtctaagc gtcaatttgt 7140

gaaatattag caattttggt catgttaagc tatgaattag tttttggtat gaataactaa 7200

agtgtagtaa tcttcctaag atttctagaa agtctaggat cacctttgtg ggatgtctat 7260

aacctcagtt atggttgaaa caagtaacta ctgcgttgct gtccagattc taggcacatg 7320

ctaagttggc taccgtatct agcgttgttt tgagctaaac ttgtagttag ttcatgcttg 7380

agataaatgc ttattttggg tgagccttgt gtcttgtgtt ggttgcttaa gcccatttgt 7440

ttgaattttc tcaaatgact gcttgattgt gatgttgtat gtgtgtttgt tatctaggta 7500

gtgccctagt tgccacctc 7519

<210> 30
<211> 379
<212> DNA
<213> Artificial sequence

<220>
<223> Complementary sequence of SEQ ID NO 23 (reverse-complement)

<400> 30
gaaatattag caattttggt catgttaagc tatgaattag tttttggtat gaataactaa 60

agtgtagtaa tcttcctaag atttctagaa agtctaggat cacctttgtg ggatgtctat 120

aacctcagtt atggttgaaa caagtaacta ctgcgttgct gtccagattc taggcacatg 180

ctaagttggc taccgtatct agcgttgttt tgagctaaac ttgtagttag ttcatgcttg 240

agataaatgc ttattttggg tgagccttgt gtcttgtgtt ggttgcttaa gcccatttgt 300

ttgaattttc tcaaatgact gcttgattgt gatgttgtat gtgtgtttgt tatctaggta 360

gtgccctagt tgccacctc 379

<210> 31
<211> 617
<212> DNA
<213> Artificial sequence

<220>
<223> Complementary sequence of SEQ ID NO 24 (reverse-complement)

<400> 31
gcatcttcat gattgttgcc ttatgcatag ctatgctcct ctcacccttg ttcaaatgat 60

gagtaatttg tgagtgatgt ggtatcctat cttccttgtg tggagtcaaa caagaatgct 120

ttgttccatg ttgcttgaat aggaaatgct tgagaaaaat gtgtgcttag ctttactaga 180

aataagtgtg actacatgct tgagcttaga gttatgaatt ttaaaagttt acggtcaggt 240

tttgtcaatg ttctgaatag atctgaatgt ttggtttgtt tgacctgtgt gcatatggaa 300

tcgtgtctta gagataatag aagaagtgta gtactttttc taagctttcc aaaatgtcct 360

atagcactaa ttgtgatggt ctaaatctct agttatgagc ttgcaaagta gaatgacaga 420

atctgtccaa atccggcaga gatgttttct ttgctatgtt tgtccttgtt agccgtagaa 480

tcatcttaat atgataataa gaaagttata gacaacttaa taagctttac ataaagttaa 540

ggatcacttt tgttgtgtct ctagaactcc aattatgtat tttcgaagtt cttgtcagtt 600

ttctgtccaa attggta 617

<210> 32
<211> 26
<212> DNA
<213> Artificial sequence

<220>
<223> Complemmentary sequence of SEQ ID NO 18 (reverse-complement)

<400> 32
agcgtcaatt tgtgaaatat tagcaa 26

<210> 33
<211> 26
<212> DNA
<213> Artificial sequence

<220>
<223> Complementary sequence of SEQ ID NO 19 (reverse-complement)

<400> 33
gtccaaattg gtatcggcgc gccctc 26

<210> 34
<211> 615
<212> PRT
<213> B. thurigiensis

<400> 34

Met Asp Asn Asn Pro Asn Ile Asn Glu Cys Ile Pro Tyr Asn Cys Leu
1 5 10 15

Ser Asn Pro Glu Val Glu Val Leu Gly Gly Glu Arg Ile Glu Thr Gly
20 25 30

Tyr Thr Pro Ile Asp Ile Ser Leu Ser Leu Thr Gln Phe Leu Leu Ser
35 40 45

Glu Phe Val Pro Gly Ala Gly Phe Val Leu Gly Leu Val Asp Ile Ile
50 55 60

Trp Gly Ile Phe Gly Pro Ser Gln Trp Asp Ala Phe Leu Val Gln Ile
65 70 75 80

Glu Gln Leu Ile Asn Gln Arg Ile Glu Glu Phe Ala Arg Asn Gln Ala
85 90 95

Ile Ser Arg Leu Glu Gly Leu Ser Asn Leu Tyr Gln Ile Tyr Ala Glu
100 105 110

Ser Phe Arg Glu Trp Glu Ala Asp Pro Thr Asn Pro Ala Leu Arg Glu
115 120 125

Glu Met Arg Ile Gln Phe Asn Asp Met Asn Ser Ala Leu Thr Thr Ala
130 135 140

Ile Pro Leu Phe Ala Val Gln Asn Tyr Gln Val Pro Leu Leu Ser Val
145 150 155 160

```
Tyr Val Gln Ala Ala Asn Leu His Leu Ser Val Leu Arg Asp Val Ser
                165                 170                 175

Val Phe Gly Gln Arg Trp Gly Phe Asp Ala Ala Thr Ile Asn Ser Arg
            180                 185                 190

Tyr Asn Asp Leu Thr Arg Leu Ile Gly Asn Tyr Thr Asp His Ala Val
        195                 200                 205

Arg Trp Tyr Asn Thr Gly Leu Glu Arg Val Trp Gly Pro Asp Ser Arg
    210                 215                 220

Asp Trp Ile Arg Tyr Asn Gln Phe Arg Arg Glu Leu Thr Leu Thr Val
225                 230                 235                 240

Leu Asp Ile Val Ser Leu Phe Pro Asn Tyr Asp Ser Arg Thr Tyr Pro
                245                 250                 255

Ile Arg Thr Val Ser Gln Leu Thr Arg Glu Ile Tyr Thr Asn Pro Val
            260                 265                 270

Leu Glu Asn Phe Asp Gly Ser Phe Arg Gly Ser Ala Gln Gly Ile Glu
        275                 280                 285

Gly Ser Ile Arg Ser Pro His Leu Met Asp Ile Leu Asn Ser Ile Thr
    290                 295                 300

Ile Tyr Thr Asp Ala His Arg Gly Glu Tyr Tyr Trp Ser Gly His Gln
305                 310                 315                 320

Ile Met Ala Ser Pro Val Gly Phe Ser Gly Pro Glu Phe Thr Phe Pro
                325                 330                 335

Leu Tyr Gly Thr Met Gly Asn Ala Ala Pro Gln Gln Arg Ile Val Ala
            340                 345                 350

Gln Leu Gly Gln Gly Val Tyr Arg Thr Leu Ser Ser Thr Leu Tyr Arg
        355                 360                 365

Arg Pro Phe Asn Ile Gly Ile Asn Asn Gln Gln Leu Ser Val Leu Asp
    370                 375                 380

Gly Thr Glu Phe Ala Tyr Gly Thr Ser Ser Asn Leu Pro Ser Ala Val
385                 390                 395                 400

Tyr Arg Lys Ser Gly Thr Val Asp Ser Leu Asp Glu Ile Pro Pro Gln
                405                 410                 415
```

```
Asn Asn Asn Val Pro Pro Arg Gln Gly Phe Ser His Arg Leu Ser His
            420                 425                 430

Val Ser Met Phe Arg Ser Gly Phe Ser Asn Ser Ser Val Ser Ile Ile
        435                 440                 445

Arg Ala Pro Met Phe Ser Trp Ile His Arg Ser Ala Glu Phe Asn Asn
    450                 455                 460

Ile Ile Ala Ser Asp Ser Ile Thr Gln Ile Pro Ala Val Lys Gly Asn
465                 470                 475                 480

Phe Leu Phe Asn Gly Ser Val Ile Ser Gly Pro Gly Phe Thr Gly Gly
                485                 490                 495

Asp Leu Val Arg Leu Asn Ser Ser Gly Asn Asn Ile Gln Asn Arg Gly
            500                 505                 510

Tyr Ile Glu Val Pro Ile His Phe Pro Ser Thr Ser Thr Arg Tyr Arg
        515                 520                 525

Val Arg Val Arg Tyr Ala Ser Val Thr Pro Ile His Leu Asn Val Asn
    530                 535                 540

Trp Gly Asn Ser Ser Ile Phe Ser Asn Thr Val Pro Ala Thr Ala Thr
545                 550                 555                 560

Ser Leu Asp Asn Leu Gln Ser Ser Asp Phe Gly Tyr Phe Glu Ser Ala
                565                 570                 575

Asn Ala Phe Thr Ser Ser Leu Gly Asn Ile Val Gly Val Arg Asn Phe
            580                 585                 590

Ser Gly Thr Ala Gly Val Ile Ile Asp Arg Phe Glu Phe Ile Pro Val
        595                 600                 605

Thr Ala Thr Leu Glu Ala Glu
    610                 615


<210>  35
<211>  265
<212>  PRT
<213>  Artificial sequence

<220>
<223>  nptII protein

<400>  35
```

Met Trp Ile Glu Gln Asp Gly Leu His Ala Gly Ser Pro Ala Ala Trp
1               5                   10                  15

Val Glu Arg Leu Phe Gly Tyr Asp Trp Ala Gln Gln Thr Ile Gly Cys
            20                  25                  30

Ser Asp Ala Ala Val Phe Arg Leu Ser Ala Gln Gly Arg Pro Val Leu
        35                  40                  45

Phe Val Lys Thr Asp Leu Ser Gly Ala Leu Asn Glu Leu Gln Asp Glu
    50                  55                  60

Ala Ala Arg Leu Ser Trp Leu Ala Thr Thr Gly Val Pro Cys Ala Ala
65                  70                  75                  80

Val Leu Asp Val Val Thr Glu Ala Gly Arg Asp Trp Leu Leu Leu Gly
                85                  90                  95

Glu Val Pro Gly Gln Asp Leu Leu Ser Ser His Leu Ala Pro Ala Glu
            100                 105                 110

Lys Val Ser Ile Met Ala Asp Ala Met Arg Arg Leu His Thr Leu Asp
        115                 120                 125

Pro Ala Thr Cys Pro Phe Asp His Gln Ala Lys His Arg Ile Glu Arg
    130                 135                 140

Ala Arg Thr Arg Met Glu Ala Gly Leu Val Asp Gln Asp Asp Leu Asp
145                 150                 155                 160

Glu Glu His Gln Gly Leu Ala Pro Ala Glu Leu Phe Ala Arg Leu Lys
                165                 170                 175

Ala Arg Met Pro Asp Gly Glu Asp Leu Val Val Thr His Gly Asp Ala
            180                 185                 190

Cys Leu Pro Asn Ile Met Val Glu Asn Gly Arg Phe Ser Gly Phe Ile
        195                 200                 205

Asp Cys Gly Arg Leu Gly Val Ala Asp Arg Tyr Gln Asp Ile Ala Leu
    210                 215                 220

Ala Thr Arg Asp Ile Ala Glu Glu Leu Gly Gly Glu Trp Ala Asp Arg
225                 230                 235                 240

Phe Leu Val Leu Tyr Gly Ile Ala Ala Pro Asp Ser Gln Arg Ile Ala
                245                 250                 255

```
Phe Tyr Arg Leu Leu Asp Glu Phe Phe
            260                 265


<210>  36
<211>  6524
<212>  DNA
<213>  Artificial sequence

<220>
<223>  Complementary sequence of SEQ ID NO 02 (reverse-complement)

<400>  36
tcggcgcgcc ctcgagtcta gagtcgacga gctcgatcca ctagtaacgg ccgccagtgt      60

gctggaattc gcccttggcg cgccgatcta gtaacataga tgacaccgcg cgcgataatt     120

tatcctagtt tgcgcgctat attttgtttt ctatcgcgta ttaaatgtat aattgcggga     180

ctctaatcat aaaaacccat ctcataaata acgtcatgca ttacatgtta attattacat     240

gcttaacgta attcaacaga aattatatga taatcatcgc aagaccggca acaggattca     300

atcttaagaa actttattgc caaatgtttg aacgatctca gaagaactcg tcaagaaggc     360

gatagaaggc gatgcgctgc gaatcgggag cggcgatacc gtaaagcacg aggaagcggt     420

cagcccattc gccgccaagc tcttcagcaa tatcacgggt agccaacgct atgtcctgat     480

agcggtccgc cacacccagc cggccacagt cgatgaatcc agaaaagcgg ccattttcca     540

ccatgatatt cggcaagcag gcatcgccat gggtcacgac gagatcctcg ccgtcgggca     600

tgcgcgcctt gagcctggcg aacagttcgg ctggcgcgag cccctgatgc tcttcgtcca     660

gatcatcctg atcgacaaga ccggcttcca tccgagtacg tgctcgctcg atgcgatgtt     720

tcgcttggtg gtcgaatggg caggtagccg gatcaagcgt atgcagccgc cgcattgcat     780

cagccatgat ggatactttc tcggcaggag caaggtgaga tgacaggaga tcctgccccg     840

gcacttcgcc caatagcagc cagtcccttc ccgcttcagt gacaacgtcg agcacagctg     900

cgcaaggaac gcccgtcgtg gccagccacg atagccgcgc tgcctcgtcc tgcagttcat     960

tcagggcacc ggacaggtcg gtcttgacaa aaagaaccgg gcgcccctgc gctgacagcc    1020

ggaacacggc ggcatcagag cagccgattg tctgttgtgc ccagtcatag ccgaatagcc    1080

tctccaccca agcggccgga gaacctgcgt gcaatccatc ttgttcaatc cacattctag    1140

agtcgacctg cagaagtaac accaaacaac agggtgagca tcgacaaaag aaacagtacc    1200

aagcaaataa atagcgtatg aaggcagggc taaaaaaatc cacatatagc tgctgcatat    1260

gccatcatcc aagtatatca agatcaaaat aattataaaa catacttgtt tattataata    1320

gataggtact caaggttaga gcatatgaat agatgctgca tatgccatca tgtatatgca    1380

tcagtaaaac ccacatcaac atgtatacct atcctagatc gatatttcca tccatcttaa    1440
```

actcgtaact atgaagatgt atgacacaca catacagttc caaaattaat aaatacacca 1500
ggtagtttga aacagtattc tactccgatc tagaacgaat gaacgaccgc ccaaccacac 1560
cacatcatca caaccaagcg aacaaaaagc atctctgtat atgcatcagt aaaacccgca 1620
tcaacatgta tacctatcct agatcgatat ttccatccat catcttcaat tcgtaactat 1680
gaatatgtat ggcacacaca tacagatcca aaattaataa atccaccagg tagtttgaaa 1740
cagaattcta ctccgatcta gaacgaccgc ccaaccagac cacatcatca caaccaagac 1800
aaaaaaaagc atgaaaagat gacccgacaa acaagtgcac ggcatatatt gaaataaagg 1860
aaaagggcaa accaaaccct atgcaacgaa acaaaaaaaa tcatgaaatc gatcccgtct 1920
gcggaacggc tagagccatc ccaggattcc ccaaagagaa acactggcaa gttagcaatc 1980
agaacgtgtc tgacgtacag gtcgcatccg tgtacgaacg ctagcagcac ggatctaaca 2040
caaacacgga tctaacacaa acatgaacag aagtagaact accgggccct aaccatggac 2100
cggaacgccg atctagagaa ggtagagagg gggggggggg gaggacgagc ggcgtacctt 2160
gaagcggagg tgccgacggg tggatttggg ggagatctgg ttgtgtgtgt gtgcgctccg 2220
aacaacacga ggttggggaa agagggtgtg gagggggtgt ctatttatta cggcgggcga 2280
ggaagggaaa gcgaaggagc ggtgggaaag gaatcccccg tagctgccgt gccgtgagag 2340
gaggaggagg ccgcctgccg tgccggctca cgtctgccgc tccgccacgc aatttctgga 2400
tgccgacagc ggagcaagtc caacggtgga gcggaactct cgagaggggt ccagaggcag 2460
cgacagagat gccgtgccgt ctgcttcgct tggcccgacg cgacgctgct ggttcgctgg 2520
ttggtgtccg ttagactcgt cgacggcgtt taacaggctg gcattatcta ctcgaaacaa 2580
gaaaaatgtt tccttagttt ttttaatttc ttaaagggta tttgtttaat ttttagtcac 2640
tttattttat tctattttat atctaaatta ttaaataaaa aaactaaaat agagttttag 2700
ttttcttaat ttagaggcta aaatagaata aaatagatgt actaaaaaaa ttagtctata 2760
aaaaccatta accctaaacc ctaaatggat gtactaataa aatggatgaa gtattatata 2820
ggtgaagcta tttgcaaaaa aaaaggagaa cacatgcaca ctaaaaagat aaaactgtag 2880
agtcctgttg tcaaaatact caattgtcct ttagaccatg tctaactgtt catttatatg 2940
attctctaaa acactgatat tattgtagta ctatagatta tattattcgt agagtaaagt 3000
ttaaatatat gtataaagat agataaactg cacttcaaac aagtgtgaca aaaaaaatat 3060
gtggtaattt tttataactt agacatgcaa tgctcattat ctctagagag gggcacgacc 3120
gggtcacgct gcactgcagg catacgcgta agcttcaggg tttaaacagg tccgattgag 3180
acttttcaac aaagggtaat atccggaaac ctcctcggat tccattgccc agctatctgt 3240
cactttattg tgaagatagt ggaaaaggaa ggtggctcct acaaatgcca tcattgcgat 3300
aaaggaaagg ccatcgttga agatgcctct gccgacagtg gtcccaaaga tggaccccca 3360

cccacgagga gcatcgtgga aaaagaagac gttccaacca cgtcttcaaa gcaagtggat 3420
tgatgtgatg gtccgattga gacttttcaa caaagggtaa tatccggaaa cctcctcgga 3480
ttccattgcc cagctatctg tcactttatt gtgaagatag tggaaaagga aggtggctcc 3540
tacaaatgcc atcattgcga taaaggaaag gccatcgttg aagatgcctc tgccgacagt 3600
ggtcccaaag atggaccccc acccacgagg agcatcgtgg aaaaagaaga cgttccaacc 3660
acgtcttcaa agcaagtgga ttgatgtgat atctccactg acgtaaggga tgacgcacaa 3720
tcccactatc cttcgcaaga cccttcctct atataaggaa gttcatttca tttggagagg 3780
acacgctgac aagctgactc tagcagatcc tctagaacca tcttccacac actcaagcca 3840
cactattgga gaacacacag ggacaacaca ccataagatc caagggaggc ctccgccgcc 3900
gccggtaacc accccgcccc tctcctcttt ctttctccgt ttttttttcc gtctcggtct 3960
cgatctttgg ccttggtagt ttgggtgggc gagaggcggc ttcgtgcgcg cccagatcgg 4020
tgcgcgggag gggcgggatc tcgcggctgg ggctctcgcc ggcgtggatc cggcccggat 4080
ctcgcgggga atggggctct cggatgtaga tctgcgatcc gccgttgttg ggggagatga 4140
tggggggttt aaaatttccg ccgtgctaaa caagatcagg aagaggggaa aagggcacta 4200
tggtttatat ttttatatat ttctgctgct tcgtcaggct tagatgtgct agatctttct 4260
ttcttctttt tgtgggtaga atttgaatcc ctcagcattg ttcatcggta gtttttcttt 4320
tcatgatttg tgacaaatgc agcctcgtgc ggagcttttt tgtaggtaga agtgatcaac 4380
ctgatcaacc ccgccatgga caacaaccca aacatcaacg agtgcatccc atacaactgc 4440
ctgagcaacc cagaggtgga ggtgctgggt ggcgagcgca tcgagaccgg ttacaccccc 4500
atcgacatct ccctgtcctt gacccagttc ctgctcagcg agttcgtgcc aggtgctggc 4560
ttcgtgctcg gcctggtgga catcatctgg ggtatcttcg gtccatccca atgggacgcc 4620
ttcctggtgc aaatcgagca gctgatcaac cagaggatcg aagagttcgc caggaaccag 4680
gccatctcca ggctggaggg cctgagcaac ctctaccaaa tctacgccga gagcttcagg 4740
gagtgggagg ccgacccgac caacccagct ctccgcgagg aaatgcgcat tcaattcaac 4800
gacatgaaca gcgccctgac caccgctatc ccactgttcg ccgtccagaa ctaccaagtg 4860
ccgctcctgt ccgtgtacgt gcaagccgct aacctgcacc tcagcgtgct gcgcgacgtg 4920
agcgtgttcg gccaaaggtg gggcttcgat gctgccacca tcaacagccg ctacaacgac 4980
ctgaccaggc tgattggcaa ctacaccgac cacgctgtgc gctggtacaa caccggcctg 5040
gagcgcgtct ggggtccgga ctccagggac tggatcaggt acaaccagtt caggagggag 5100
ttgaccctca ccgtgctgga cattgtgtcc ctcttcccga actacgactc caggacctac 5160
ccgatccgca ccgtgtccca actcaccagg gagatctaca ccaacccagt gctggagaac 5220

ttcgacggta gcttccgcgg ttccgcccag ggtatcgagg gctccatcag gagcccacac 5280

ctgatggaca tcctgaacag catcaccatc tacaccgacg ctcacagggg cgagtactac 5340

tggtccggcc accagatcat ggcctcccca gtgggcttca gcggccccga gttcaccttc 5400

ccgctctacg gcaccatggg caacgccgct ccacagcaac gcatcgtggc tcaactgggt 5460

cagggtgtct acaggaccct gtcctccacc ctgtacagga ggcccttcaa catcggtatc 5520

aacaaccagc aactgtccgt gctcgacggc accgagttcg cctacggcac ctcctccaac 5580

ctgccatccg ctgtctacag gaagagcggc accgtggact ccctggacga gatcccacca 5640

cagaacaaca acgtgccacc caggcaaggc ttctcccaca ggctgagcca cgtgtccatg 5700

ttccgctccg gcttcagcaa cagctccgtg agcatcatca gggctccgat gttctcctgg 5760

atccaccgca gcgctgagtt caacaacatc atcgcctccg acagcatcac ccaaatcccg 5820

gccgtgaagg gcaacttcct cttcaacggt tccgtcattt ccggcccagg cttcaccggt 5880

ggcgacctcg tgaggctcaa cagcagcggc aacaacatcc agaacagggg ctacatcgag 5940

gtgccaatcc acttcccatc cacctccacc aggtacaggg tgcgcgtgag gtacgcttcc 6000

gtgaccccga tccacctcaa cgtgaactgg ggtaactcct ccatcttctc caacaccgtg 6060

ccagctaccg ctacctccct ggacaacctc caatccagcg acttcggtta cttcgagagc 6120

gccaacgctt tcacctcctc cctcggtaac atcgtgggcg tgaggaactt cagcggcacc 6180

gccggcgtga tcatcgacag gttcgagttc atcccagtga ccgccaccct cgaggctgag 6240

tgatcgatcg acaagctcga gtttctccat aataatgtgt gagtagttcc cagataaggg 6300

aattagggtt cctatagggt ttcgctcatg tgttgagcat ataagaaacc cttagtatgt 6360

atttgtattt gtaaaatact tctatcaata aaatttctaa ttcctaaaac caaaatccag 6420

tactaaaatc cagatccccc gaattaaggt accgatatca gtactaattc agtacattaa 6480

aaacgtccgc aatgtgttat taagttgtct aagcgtcaat ttgt 6524

<210> 37
<211> 6729
<212> DNA
<213> Artificial sequence

<220>
<223> Complementary sequence of SEQ ID NO 05 (reverse-complement)

<400> 37
acttaataag ctttacataa agttaaggat cacttttgtt gtgtctctag aactccaatt 60

atgtattttc gaagttcttg tcagttttct gtccaaattg gtatcggcgc gccctcgagt 120

ctagagtcga cgagctcgat ccactagtaa cggccgccag tgtgctggaa ttcgcccttg 180

gcgcgccgat ctagtaacat agatgacacc gcgcgcgata atttatccta gtttgcgcgc 240

tatattttgt tttctatcgc gtattaaatg tataattgcg ggactctaat cataaaaacc 300

catctcataa ataacgtcat gcattacatg ttaattatta catgcttaac gtaattcaac 360
agaaattata tgataatcat cgcaagaccg gcaacaggat tcaatcttaa gaaactttat 420
tgccaaatgt ttgaacgatc tcagaagaac tcgtcaagaa ggcgatagaa ggcgatgcgc 480
tgcgaatcgg gagcggcgat accgtaaagc acgaggaagc ggtcagccca ttcgccgcca 540
agctcttcag caatatcacg ggtagccaac gctatgtcct gatagcggtc cgccacaccc 600
agccggccac agtcgatgaa tccagaaaag cggccatttt ccaccatgat attcggcaag 660
caggcatcgc catgggtcac gacgagatcc tcgccgtcgg gcatgcgcgc cttgagcctg 720
gcgaacagtt cggctggcgc gagcccctga tgctcttcgt ccagatcatc ctgatcgaca 780
agaccggctt ccatccgagt acgtgctcgc tcgatgcgat gtttcgcttg gtggtcgaat 840
gggcaggtag ccggatcaag cgtatgcagc cgccgcattg catcagccat gatggatact 900
ttctcggcag gagcaaggtg agatgacagg agatcctgcc ccggcacttc gcccaatagc 960
agccagtccc ttcccgcttc agtgacaacg tcgagcacag ctgcgcaagg aacgcccgtc 1020
gtggccagcc acgatagccg cgctgcctcg tcctgcagtt cattcagggc accggacagg 1080
tcggtcttga caaaaagaac cgggcgcccc tgcgctgaca gccggaacac ggcggcatca 1140
gagcagccga ttgtctgttg tgcccagtca tagccgaata gcctctccac ccaagcggcc 1200
ggagaacctg cgtgcaatcc atcttgttca atccacattc tagagtcgac ctgcagaagt 1260
aacaccaaac aacagggtga gcatcgacaa aagaaacagt accaagcaaa taaatagcgt 1320
atgaaggcag ggctaaaaaa atccacatat agctgctgca tatgccatca tccaagtata 1380
tcaagatcaa aataattata aaacatactt gtttattata atagataggt actcaaggtt 1440
agagcatatg aatagatgct gcatatgcca tcatgtatat gcatcagtaa aacccacatc 1500
aacatgtata cctatcctag atcgatattt ccatccatct taaactcgta actatgaaga 1560
tgtatgacac acacatacag ttccaaaatt aataaataca ccaggtagtt tgaaacagta 1620
ttctactccg atctagaacg aatgaacgac cgcccaacca caccacatca tcacaaccaa 1680
gcgaacaaaa agcatctctg tatatgcatc agtaaaaccc gcatcaacat gtatacctat 1740
cctagatcga tatttccatc catcatcttc aattcgtaac tatgaatatg tatggcacac 1800
acatacagat ccaaaattaa taaatccacc aggtagtttg aaacagaatt ctactccgat 1860
ctagaacgac cgcccaacca gaccacatca tcacaaccaa gacaaaaaaa agcatgaaaa 1920
gatgacccga caaacaagtg cacggcatat attgaaataa aggaaaaggg caaaccaaac 1980
cctatgcaac gaaacaaaaa aaatcatgaa atcgatcccg tctgcggaac ggctagagcc 2040
atcccaggat tccccaaaga gaaacactgg caagttagca atcagaacgt gtctgacgta 2100
caggtcgcat ccgtgtacga acgctagcag cacggatcta acacaaacac ggatctaaca 2160

caaacatgaa cagaagtaga actaccgggc cctaaccatg gaccggaacg ccgatctaga 2220
gaaggtagag aggggggggg ggggaggacg agcggcgtac cttgaagcgg aggtgccgac 2280
gggtggattt gggggagatc tggttgtgtg tgtgtgcgct ccgaacaaca cgaggttggg 2340
gaaagagggt gtggaggggg tgtctattta ttacggcggg cgaggaaggg aaagcgaagg 2400
agcggtggga aaggaatccc ccgtagctgc cgtgccgtga gaggaggagg aggccgcctg 2460
ccgtgccggc tcacgtctgc cgctccgcca cgcaatttct ggatgccgac agcggagcaa 2520
gtccaacggt ggagcggaac tctcgagagg ggtccagagg cagcgacaga gatgccgtgc 2580
cgtctgcttc gcttggcccg acgcgacgct gctggttcgc tggttggtgt ccgttagact 2640
cgtcgacggc gtttaacagg ctggcattat ctactcgaaa caagaaaaat gtttccttag 2700
tttttttaat ttcttaaagg gtatttgttt aatttttagt cactttattt tattctattt 2760
tatatctaaa ttattaaata aaaaaactaa aatagagttt tagttttctt aatttagagg 2820
ctaaaataga ataaaataga tgtactaaaa aaattagtct ataaaaacca ttaaccctaa 2880
accctaaatg gatgtactaa taaaatggat gaagtattat ataggtgaag ctatttgcaa 2940
aaaaaaagga gaacacatgc acactaaaaa gataaaactg tagagtcctg ttgtcaaaat 3000
actcaattgt cctttagacc atgtctaact gttcatttat atgattctct aaaacactga 3060
tattattgta gtactataga ttatattatt cgtagagtaa agtttaaata tatgtataaa 3120
gatagataaa ctgcacttca aacaagtgtg acaaaaaaaa tatgtggtaa ttttttataa 3180
cttagacatg caatgctcat tatctctaga gaggggcacg accgggtcac gctgcactgc 3240
aggcatacgc gtaagcttca gggtttaaac aggtccgatt gagacttttc aacaaagggt 3300
aatatccgga aacctcctcg gattccattg cccagctatc tgtcacttta ttgtgaagat 3360
agtggaaaag gaaggtggct cctacaaatg ccatcattgc gataaaggaa aggccatcgt 3420
tgaagatgcc tctgccgaca gtggtcccaa agatggaccc ccacccacga ggagcatcgt 3480
ggaaaaagaa gacgttccaa ccacgtcttc aaagcaagtg gattgatgtg atggtccgat 3540
tgagactttt caacaaaggg taatatccgg aaacctcctc ggattccatt gcccagctat 3600
ctgtcacttt attgtgaaga tagtggaaaa ggaaggtggc tcctacaaat gccatcattg 3660
cgataaagga aaggccatcg ttgaagatgc ctctgccgac agtggtccca aagatggacc 3720
cccacccacg aggagcatcg tggaaaaaga agacgttcca accacgtctt caaagcaagt 3780
ggattgatgt gatatctcca ctgacgtaag ggatgacgca caatcccact atccttcgca 3840
agacccttcc tctatataag gaagttcatt tcatttggag aggacacgct gacaagctga 3900
ctctagcaga tcctctagaa ccatcttcca cacactcaag ccacactatt ggagaacaca 3960
cagggacaac acaccataag atccaaggga ggcctccgcc gccgccggta accaccccgc 4020
ccctctcctc tttctttctc cgtttttttt tccgtctcgg tctcgatctt tggccttggt 4080

agtttgggtg ggcgagaggc ggcttcgtgc gcgcccagat cggtgcgcgg gaggggcggg 4140

atctcgcggc tggggctctc gccggcgtgg atccggcccg gatctcgcgg ggaatggggc 4200

tctcggatgt agatctgcga tccgccgttg ttgggggaga tgatgggggg tttaaaattt 4260

ccgccgtgct aaacaagatc aggaagaggg gaaaagggca ctatggttta tatttttata 4320

tatttctgct gcttcgtcag gcttagatgt gctagatctt tctttcttct tttgtgggt 4380

agaatttgaa tccctcagca ttgttcatcg gtagtttttc ttttcatgat ttgtgacaaa 4440

tgcagcctcg tgcggagctt ttttgtaggt agaagtgatc aacctgatca accccgccat 4500

ggacaacaac ccaaacatca acgagtgcat cccatacaac tgcctgagca acccagaggt 4560

ggaggtgctg ggtggcgagc gcatcgagac cggttacacc cccatcgaca tctccctgtc 4620

cttgacccag ttcctgctca gcgagttcgt gccaggtgct ggcttcgtgc tcggcctggt 4680

ggacatcatc tggggtatct tcggtccatc ccaatgggac gccttcctgg tgcaaatcga 4740

gcagctgatc aaccagagga tcgaagagtt cgccaggaac caggccatct ccaggctgga 4800

gggcctgagc aacctctacc aaatctacgc cgagagcttc agggagtggg aggccgaccc 4860

gaccaaccca gctctccgcg aggaaatgcg cattcaattc aacgacatga acagcgccct 4920

gaccaccgct atcccactgt tcgccgtcca gaactaccaa gtgccgctcc tgtccgtgta 4980

cgtgcaagcc gctaacctgc acctcagcgt gctgcgcgac gtgagcgtgt tcggccaaag 5040

gtggggcttc gatgctgcca ccatcaacag ccgctacaac gacctgacca ggctgattgg 5100

caactacacc gaccacgctg tgcgctggta caacaccggc ctggagcgcg tctggggtcc 5160

ggactccagg gactggatca ggtacaacca gttcaggagg gagttgaccc tcaccgtgct 5220

ggacattgtg tccctcttcc cgaactacga ctccaggacc tacccgatcc gcaccgtgtc 5280

ccaactcacc agggagatct acaccaaccc agtgctggag aacttcgacg gtagcttccg 5340

cggttccgcc cagggtatcg agggctccat caggagccca cacctgatgg acatcctgaa 5400

cagcatcacc atctacaccg acgctcacag gggcgagtac tactggtccg gccaccagat 5460

catggcctcc ccagtgggct tcagcggccc cgagttcacc ttcccgctct acggcaccat 5520

gggcaacgcc gctccacagc aacgcatcgt ggctcaactg ggtcagggtg tctacaggac 5580

cctgtcctcc accctgtaca ggaggccctt caacatcggt atcaacaacc agcaactgtc 5640

cgtgctcgac ggcaccgagt tcgcctacgg cacctcctcc aacctgccat ccgctgtcta 5700

caggaagagc ggcaccgtgg actccctgga cgagatccca ccacagaaca acaacgtgcc 5760

acccaggcaa ggcttctccc acaggctgag ccacgtgtcc atgttccgct ccggcttcag 5820

caacagctcc gtgagcatca tcagggctcc gatgttctcc tggatccacc gcagcgctga 5880

gttcaacaac atcatcgcct ccgacagcat cacccaaatc ccggccgtga agggcaactt 5940

cctcttcaac ggttccgtca tttccggccc aggcttcacc ggtggcgacc tcgtgaggct 6000

caacagcagc ggcaacaaca tccagaacag gggctacatc gagtgccaat ccacttccca 6060

tccacctcca ccaggtacag ggtgcgcgtg aggtacgctt ccgtgacccc gatccacctc 6120

aacgtgaact ggggtaactc ctccatcttc tccaacaccg tgccagctac cgctacctcc 6180

ctggacaacc tccaatccag cgacttcggt tacttcgaga gcgccaacgc tttcacctcc 6240

tccctcggta acatcgtggg cgtgaggaac ttcagcggca ccgccggcgt gatcatcgac 6300

aggttcgagt tcatcccagt gaccgccacc ctcgaggctg agtgatcgat cgacaagctc 6360

gagtttctcc ataataatgt gtgagtagtt cccagataag ggaattaggg ttcctatagg 6420

gtttcgctca tgtgttgagc atataagaaa cccttagtat gtatttgtat ttgtaaaata 6480

cttctatcaa taaaatttct aattcctaaa accaaaatcc agtactaaaa tccagatccc 6540

ccgaattaag gtaccgatat cagtactaat tcagtacatt aaaaacgtcc gcaatgtgtt 6600

attaagttgt ctaagcgtca atttgtgaaa tattagcaat tttggtcatg ttaagctatg 6660

aattagtttt tggtatgaat aactaaagtg tagtaatctt cctaagattt ctagaaagtc 6720

taggatcac 6729

<210> 38
<211> 206
<212> DNA
<213> Artificial sequence

<220>
<223> Complementary sequence of SEQ ID NO 03 (reverse-complement)

<400> 38
actaaaatcc agatcccccg aattaaggta ccgatatcag tactaattca gtacattaaa 60

aacgtccgca atgtgttatt aagttgtcta agcgtcaatt tgtgaaatat tagcaatttt 120

ggtcatgtta agctatgaat tagtttttgg tatgaataac taaagtgtag taatcttcct 180

aagatttcta gaaagtctag gatcac 206

<210> 39
<211> 206
<212> DNA
<213> Artificial sequence

<220>
<223> Complementary sequence of SEQ ID NO 04 (reverse-complement)

<400> 39
acttaataag ctttacataa agttaaggat cacttttgtt gtgtctctag aactccaatt 60

atgtattttc gaagttcttg tcagttttct gtccaaattg gtatcggcgc gccctcgagt 120

ctagagtcga cgagctcgat ccactagtaa cggccgccag tgtgctggaa ttcgcccttg 180

gcgcgccgat ctagtaacat agatga 206

<210> 40
<211> 20
<212> DNA
<213> Artificial sequence

<220>
<223> Artificial sequence

<400> 40
gaggtgccaa tccacttccc 20

<210> 41
<211> 23
<212> DNA
<213> Artificial sequence

<220>
<223> Artificial sequence

<400> 41
gagttacccc agttcacgtt gag 23

<210> 42
<211> 17
<212> DNA
<213> Artificial sequence

<220>
<223> Artificial sequence

<400> 42
acctccacca ggtacag 17

<210> 43
<211> 21
<212> DNA
<213> Artificial sequence

<220>
<223> Artificial sequence

<400> 43
catcccatac aactgcctga g 21

<210> 44
<211> 21
<212> DNA
<213> Artificial sequence

<220>
<223> Artificial sequence

<400> 44
ctgggtcaag gacagggaga t 21

<210> 45

<211> 14
<212> DNA
<213> Artificial sequence

<220>
<223> Artificial sequence

<400> 45
ccggttacac cccc 14

<210> 46
<211> 22
<212> DNA
<213> Artificial sequence

<220>
<223> Artificial sequence

<400> 46
ctctaccaaa tctacgccga ga 22

<210> 47
<211> 21
<212> DNA
<213> Artificial sequence

<220>
<223> Artificial sequence

<400> 47
catgtcgttg aattgaatgc g 21

<210> 48
<211> 14
<212> DNA
<213> Artificial sequence

<220>
<223> Artificial sequence

<400> 48
tctccgcgag gaaa 14

<210> 49
<211> 20
<212> DNA
<213> Artificial sequence

<220>
<223> Artificial sequence

<400> 49
caccgtgctg gacattgtgt 20

<210> 50
<211> 18
<212> DNA
<213> Artificial sequence

<220>
<223> Artificial sequence

<400> 50
tgagttggga cacggtgc 18

<210> 51
<211> 19
<212> DNA
<213> Artificial sequence

<220>
<223> Artificial sequence

<400> 51
ctcttcccga actacgact 19

<210> 52
<211> 20
<212> DNA
<213> Artificial sequence

<220>
<223> Artificial sequence

<400> 52
accctgtcct ccaccctgta 20

<210> 53
<211> 20
<212> DNA
<213> Artificial sequence

<220>
<223> Artificial sequence

<400> 53
aggttggagg aggtgccgta 20

<210> 54
<211> 18
<212> DNA
<213> Artificial sequence

<220>
<223> Artificial sequence

<400> 54
ccttcaacat cggtatca 18

<210> 55
<211> 20
<212> DNA
<213> Artificial sequence

<220>
<223> Artificial sequence

<400> 55
tgccgaatat catggtggaa 20

<210> 56
<211> 19
<212> DNA
<213> Artificial sequence

<220>
<223> Artificial sequence

<400> 56
cggccacagt cgatgaatc 19

<210> 57
<211> 13
<212> DNA
<213> Artificial sequence

<220>
<223> Artificial sequence

<400> 57
tggccgcttt tct 13

<210> 58
<211> 23
<212> DNA
<213> Artificial sequence

<220>
<223> Artificial sequence

<400> 58
atgactgggc acaacagaca atc 23

<210> 59
<211> 18
<212> DNA
<213> Artificial sequence

<220>
<223> Artificial sequence

<400> 59
cggacaggtc ggtcttga 18

<210> 60
<211> 15
<212> DNA
<213> Artificial sequence

<220>
<223> Artificial sequence

<400> 60
ctgctctgat gccgc 15

<210> 61
<211> 23
<212> DNA
<213> Artificial sequence

<220>
<223> Artificial sequence

<400> 61
ctgccgagaa agtatccatc atg 23

<210> 62
<211> 20
<212> DNA
<213> Artificial sequence

<220>
<223> Artificial sequence

<400> 62
gatgtttcgc ttggtggtcg 20

<210> 63
<211> 18
<212> DNA
<213> Artificial sequence

<220>
<223> Artificial sequence

<400> 63
ctgatgcaat gcggcggc 18

<210> 64
<211> 18
<212> DNA
<213> Artificial sequence

<220>
<223> Artificial sequence

<400> 64
gccgcttgta gccttcca 18

<210> 65
<211> 16
<212> DNA
<213> Artificial sequence

<220>
<223> Artificial sequence

<400> 65
ccgccgacct ggtgga 16

<210> 66

<211>  15
<212>  DNA
<213>  Artificial sequence

<220>
<223>  Artificial sequence

<400>  66
cgtgacctca atgcg    15

<210>  67
<211>  20
<212>  DNA
<213>  Artificial sequence

<220>
<223>  Artificial sequence

<400>  67
cacaatcgtc acctcaaccg    20

<210>  68
<211>  21
<212>  DNA
<213>  Artificial sequence

<220>
<223>  Artificial sequence

<400>  68
atcaacgacc ttctggaaac g    21

<210>  69
<211>  18
<212>  DNA
<213>  Artificial sequence

<220>
<223>  Artificial sequence

<400>  69
cgcaggattt cgctctcg    18

<210>  70
<211>  20
<212>  DNA
<213>  Artificial sequence

<220>
<223>  Artificial sequence

<400>  70
acggaaccgt tgaagaggaa    20

<210>  71
<211>  24
<212>  DNA
<213>  Artificial sequence

<220>
<223> Artificial sequence

<400> 71
atgtgtgagt agttcccaga taag 24

<210> 72
<211> 20
<212> DNA
<213> Artificial sequence

<220>
<223> Artificial sequence

<400> 72
gatccaggag aacatcggag 20

<210> 73
<211> 24
<212> DNA
<213> Artificial sequence

<220>
<223> Artificial sequence

<400> 73
ctatagggtt tcgctcatgt gttg 24

<210> 74
<211> 20
<212> DNA
<213> Artificial sequence

<220>
<223> Artificial sequence

<400> 74
aattatacat ttaatacgcg 20

<210> 75
<211> 20
<212> DNA
<213> Artificial sequence

<220>
<223> Artificial sequence

<400> 75
cgcggtgtca tctatgttac 20

<210> 76
<211> 24
<212> DNA
<213> Artificial sequence

<220>
<223> Artificial sequence

<400> 76
tcaagaaggc gatagaaggc gatg 24

<210> 77
<211> 21
<212> DNA
<213> Artificial sequence

<220>
<223> Artificial sequence

<400> 77
aataacgtca tgcattacat g 21

<210> 78
<211> 20
<212> DNA
<213> Artificial sequence

<220>
<223> Artificial sequence

<400> 78
gataatcatc gcaagaccgg 20

<210> 79
<211> 20
<212> DNA
<213> Artificial sequence

<220>
<223> Artificial sequence

<400> 79
tcgtcgactc tagactcgag 20

<210> 80
<211> 26
<212> DNA
<213> Artificial sequence

<220>
<223> Artificial sequence

<400> 80
aactttattc taggataaag ctatgt 26

<210> 81
<211> 25
<212> DNA
<213> Artificial sequence

<220>
<223> Artificial sequence

<400> 81
cagataaggg aattagggtt cctat 25

<210> 82
<211> 25
<212> DNA
<213> Artificial sequence

<220>
<223> Artificial sequence

<400> 82
acatacaaca tcacaatcaa gcagt 25

<210> 83
<211> 25
<212> DNA
<213> Artificial sequence

<220>
<223> Artificial sequence

<400> 83
agagtcctgt tgtcaaaata ctcaa 25

<210> 84
<211> 25
<212> DNA
<213> Artificial sequence

<220>
<223> Artificial sequence

<400> 84
ctcaaaacaa cgctagatac ggtag 25

<210> 85
<211> 25
<212> DNA
<213> Artificial sequence

<220>
<223> Artificial sequence

<400> 85
gcatcttcat gattgttgcc ttatg 25

<210> 86
<211> 25
<212> DNA
<213> Artificial sequence

<220>
<223> Artificial sequence

<400> 86
atgattagag tcccgcaatt ataca 25

<210> 87

<211> 25
<212> DNA
<213> Artificial sequence

<220>
<223> Artificial sequence

<400> 87
cttgaatagg aaatgcttga gaaaa 25

<210> 88
<211> 25
<212> DNA
<213> Artificial sequence

<220>
<223> Artificial sequence

<400> 88
cttgagtgtg tggaagatgg ttcta 25

<210> 89
<211> 25
<212> DNA
<213> Artificial sequence

<220>
<223> Artificial sequence

<400> 89
gatctgaatg tttggtttgt ttgac 25

<210> 90
<211> 24
<212> DNA
<213> Artificial sequence

<220>
<223> Artificial sequence

<400> 90
gtttgtcctt gttagccgta gaat 24

<210> 91
<211> 25
<212> DNA
<213> Artificial sequence

<220>
<223> Artificial sequence

<400> 91
tgatgcatat acagagatgc ttttt 25

<210> 92
<211> 25
<212> DNA
<213> Artificial sequence

<220>
<223>  Artificial sequence

<400>  92
ttcatccatt ttattagtac atcca                                          25

<210>  93
<211>  18
<212>  DNA
<213>  Artificial sequence

<220>
<223>  Artificial sequence

<400>  93
acggatgcga cctgtacg                                                  18

<210>  94
<211>  20
<212>  DNA
<213>  Artificial sequence

<220>
<223>  Artificial sequence

<400>  94
tcagtaaaac ccacatcaac                                                20

<210>  95
<211>  21
<212>  DNA
<213>  Artificial sequence

<220>
<223>  Artificial sequence

<400>  95
gaccacatca tcacaaccaa g                                              21

<210>  96
<211>  21
<212>  DNA
<213>  Artificial sequence

<220>
<223>  Artificial sequence

<400>  96
gctccgaaca acacgaggtt g                                              21

<210>  97
<211>  22
<212>  DNA
<213>  Artificial sequence

<220>
<223>  Artificial sequence

<400> 97
atgaagtatt atataggtga ag 22

<210> 98
<211> 20
<212> DNA
<213> Artificial sequence

<220>
<223> Artificial sequence

<400> 98
agaggctatt cggctatgac 20

<210> 99
<211> 20
<212> DNA
<213> Artificial sequence

<220>
<223> Artificial sequence

<400> 99
cagccgaact gttcgccagg 20

<210> 100
<211> 20
<212> DNA
<213> Artificial sequence

<220>
<223> Artificial sequence

<400> 100
agcacgagga agcggtcagc 20

<210> 101
<211> 20
<212> DNA
<213> Artificial sequence

<220>
<223> Artificial sequence

<400> 101
tgagatgaca ggagatcctg 20

<210> 102
<211> 22
<212> DNA
<213> Artificial sequence

<220>
<223> Artificial sequence

<400> 102
caacagctcc gtgagcatca tc 22

<210>  103
<211>  20
<212>  DNA
<213>  Artificial sequence

<220>
<223>  Artificial sequence

<400>  103
ccagcgactt cggttacttc                                                   20

<210>  104
<211>  21
<212>  DNA
<213>  Artificial sequence

<220>
<223>  Artificial sequence

<400>  104
ctctcggcgt agatttggta g                                                 21

<210>  105
<211>  20
<212>  DNA
<213>  Artificial sequence

<220>
<223>  Artificial sequence

<400>  105
caacaaccca aacatcaacg                                                   20

<210>  106
<211>  20
<212>  DNA
<213>  Artificial sequence

<220>
<223>  Artificial sequence

<400>  106
gggtcctgta gacaccctga                                                   20

<210>  107
<211>  22
<212>  DNA
<213>  Artificial sequence

<220>
<223>  Artificial sequence

<400>  107
gctcaccctg ttgtttggtg tt                                                22

<210>  108

<211> 19
<212> DNA
<213> Artificial sequence

<220>
<223> Artificial sequence

<400> 108
cggccacagt cgatgaatc 19

<210> 109
<211> 23
<212> DNA
<213> Artificial sequence

<220>
<223> Artificial sequence

<400> 109
tgaaaagaaa aactaccgat gaa 23

<210> 110
<211> 20
<212> DNA
<213> Artificial sequence

<220>
<223> Artificial sequence

<400> 110
tgatgtgatg gtccgattga 20

<210> 111
<211> 19
<212> DNA
<213> Artificial sequence

<220>
<223> Artificial sequence

<400> 111
tcccctcggg atcaaagta 19

<210> 112
<211> 20
<212> DNA
<213> Artificial sequence

<220>
<223> Artificial sequence

<400> 112
tagcgtgttt gtgcttttgc 20

<210> 113
<211> 20
<212> DNA
<213> Artificial sequence

```
<220>
<223>  Artificial sequence

<400>  113
ggccgctgaa attaaatcaa                                                    20


<210>  114
<211>  20
<212>  DNA
<213>  Artificial sequence

<220>
<223>  Artificial sequence

<400>  114
gagtcagtga gcgaggaagc                                                    20


<210>  115
<211>  20
<212>  DNA
<213>  Artificial sequence

<220>
<223>  Artificial sequence

<400>  115
cggtgaaaac ctctgacaca                                                    20


<210>  116
<211>  20
<212>  DNA
<213>  Artificial sequence

<220>
<223>  Artificial sequence

<400>  116
atacaggcag cccatcagtc                                                    20
```

## Description

Technical Field

The present invention relates to the field of biotechnology. More specifically, it is described a genetic construct and a method for producing a transgenic plant event, especially a sugarcane *(Saccharum* spp.) event, which expresses the CrylAc toxin conferring resistance to infestation by the pest *Diatraea saccharalis,* popularly known as common borer, cane borer or just borer. The invention describes a method for detecting the event and material derived from the event resistant to cane borer infestation, as well as polynucleotides, primers, probes and the flanking regions identifying such an event.

## Background

Sugarcane *(Saccharum* spp.) is a grass belonging to the botanical family Poaceae, originating from Southeast Asia, more precisely from the large central region of New Guinea and Indonesia. It is one of the most important plant species grown in the tropical and subtropical regions, with an area of over 23 million hectares spread over 121 countries (FAO Statistical Yearbook 2012 p. 233).

Sugarcane is a source of raw material for the production of sugar, wine, molasses, rum, cachaça (Brazil's national distillate) and ethanol for fuel. The bagasse that is produced after the sugarcane milling can be used for baling, supplying heat energy, processing at mills, producing electricity (that is typically sold to the consumer's electric grid), or as raw material for the production of sugarcane second-generation ethanol (BR 11 2014 02385-1). Thus, the sugarcane agro-industry has great economic and social importance by generating millions of jobs in the area and fostering foreign exchange through the commercialization of sugar and ethanol and sustainable and optimal use of plant biomass.

More recently, with the advent of global warming and the subsequent desire for alternatives to fossil fuels (biofuels), worldwide interest in sugarcane has increased significantly. The use of sugarcane-based ethanol as a renewable energy source has been considered extremely important for reducing greenhouse gases and dependence on fossil fuels, thus making it a key element in efforts to control global climate change (Savage, 2011).

Due to the economic and social importance of sugarcane, an increasing amount of research has been directed toward defining best agricultural practices for its cultivation and improving the quality of cultivated varieties. Efforts to improve sugarcane agronomic characteristics have focused on increasing sugar production and accumulation, increasing tolerance to biotic and abiotic stresses, resistance and tolerance to pests and pathogens, and developing alternative technologies for the production of sugarcane ethanol from lignocellulosic biomass (PI 0802153-8; PI 0904538-4; PI 1101295-1).

The complexity of the polyploid and aneuploid genome of modem sugarcane varieties, coupled with their relatively restricted genetic base and low fertility, impose great difficulties and numerous limitations on the selection of plants with desirable agronomic characteristics using conventional breeding (Souza et al, 2011; D'Hont & Glaszmann, 2005, Basel, v. 109, no. 1-3, p. 27-33; Cheavegatti-Gianotto et al., 2011). Therefore, high production costs, the necessity of manual labor, and long product-to-market timelines may prevent the sugarcane industry from meeting the growing demands of the global market.

The sugar cane production has been increased over the last 20 years as a result not just of the increase on productivity of plantations, but also as a result of the increase on diversity of cultivated varieties, allowing the expansion of cultivated area to other geographical regions. This geographical expansion demands the development of adapted varieties to different edaphoclimatic conditions, rendering the sustainability of sugarcane market supported by the continuously suppling to the market of new and local adapted germplasm and by the capacity to control diseases and pests.

The conventional breeding methods are important for continuously supplying new varieties, but not sufficient to attend the need of the modern market since part of characteristics are not founded in the genetic background of the varieties, being fundamental the development of efficient methods to introduce exogenous genes. Due to the limitations of conventional breeding methods and the increasing need to rapidly and efficiently incorporate desirable traits, the use of genetic engineering (biotechnology) in sugarcane breeding programs has gained prominence, in particular due to the commercial success of incorporating desirable agronomic traits through genetic engineering into other plant species (soybean, corn, canola, beet and cotton, for example).

Plant genetic engineering involves the transfer of genes-of-interest into plant cells (genetic transformation) in such a way that a fertile and agronomically superior progeny maintain and stably express the gene responsible for the desired trait.

Despite the potential of sugarcane genetic modification (incorporation of desirable characteristics) by genetic engineering [virus resistance (Guo et al., 2015; Zhu et al., 2011), insects (Kalunke, Kolge, Babu, & Prasad, 2009; Weng et al., 2011), herbicides (Enríquez-Obregon, Vazquez-Padron, Prieto-Samsonov, De la Riva, & Selman-Housein, 1998; van der Vyver, Conradie, Kossmann, & Lloyd, 2013), drought tolerance (Molinari et al., 2007; Reis et al., 2014), salinity (Kumar, Uzma, Khan, Abbas, & Ali, 2014) and aluminum toxicity (Ribeiro, 2016), increased production and accumulation of sugar (Bewg, Poovaiah, Lan, Ralph, & Coleman, 2016; Mudge et al., 2013)], this approach is limited by intrinsic characteristics of sugarcane. Unlike maize, rice, wheat and other commercial cereals, sugarcane exhibits difficulties in tissue culture propagation, low rates of induction and regeneration of embryogenic calluses, and the impossibility of using the zygotic embryo as a target tissue in genetic transformation [(Anderson & Birch, 2012; Basnayake, Moyle, & Birch, 2011; Molinari et al., 2007)]. Low rates of transformation efficiency and high variability between sugarcane genotypes are frequently observed, and there are still numerous challenges to overcome in order to successfully incorporate desirable agronomic traits into sugarcane using genetic engineering.

Sugarcane is considered a recalcitrant species for genetic transformation, and although several genetic engineering approaches have been evaluated for this species, there are still no standard protocols that guarantee the production of transgenic events (Smith et al. 1992; Rathius & Birch 1992.; Chen et al. 1987; Arencibia 1998; Manickavasagam et al. 2004; Elliott et al. 1998).

In addition to the inherent limitations of the species that prevent the application of existing genetic engineering techniques, the complexity of the sugarcane genome (high ploidy level and aneuploidy), prevents trait introgression into specific cultivars through backcrossing (reconstitution of a specific genotype), as is commonly performed in other crops of commercial interest. Thus, for the "insertion" of the same trait in more than one germplasm, enabling gains in productivity in different geographical regions, it is necessary to carry out a new genetic transformation, incurring the same costs and risks associated with the first event obtained for that trait. Even today, there is very little predictability of success for the insertion of the same characteristic in different varieties of sugar cane, being an extremely genotype-dependent process, which makes the construction of a portfolio of genetically modified products for this species challenging.

The genotypic complexity of the species also significantly impacts the characterization of the events generated in order to ensure the necessary characteristics for their commercialization. The unambiguous identification of transgenic events is fundamental to ensure their traceability and monitoring, which is a regulatory requirement for their commercialization. The high polyploidy of the sugarcane genome and the high number of repeated regions, coupled with the lack of information on their organization and structure, make the characterization of the transgenic events generated even more difficult.

There are several technical challenges to be overcome in the field of sugarcane breeding to increase the predictability of results, even when applying widely known conventional and/or molecular/genetic techniques. Despite all the technical challenges for obtaining more productive varieties of sugarcane, there is no doubt about the urgency of obtaining improved varieties that have characteristics that significantly impact crop productivity and therefore its market.

Historically, agricultural pests are one of the main factors that cause losses in agriculture. In Brazil, the main sugarcane pest is the species *Diatraea saccharalis* (first described by Fabricius in 1794), popularly known as the common borer, cane borer or just borer. It is a member of the Crambidae family and Lepidoptera order. The borer is found practically everywhere the crop is cultivated, an area of approximately 10 million hectares for the 2019/20 crop season (CONAB, 2019).

After mating, the female sugarcane borer lays 200 to 400 eggs distributed on either side of the leaves as well as the leaf sheaths. After hatching, neonate larvae feed on the leaf parenchyma, migrating to the sheath region for shelter. They remain in this region for 7 to 10 days, feeding by scraping the leaf sheath or bark of the young internodes. After an ecdysis, the caterpillars pierce the stalk, penetrating inside. The insect creates tunnels inside the stalk, usually upwards as it feeds. Inside the culm, the caterpillar goes through approximately six ecdysis before becoming a winged adult (DINARDO-MIRANDA, 2014). This is the developmental stage of the insect that causes economic damage to the crop (Figure 1).

The attack of the sugarcane borer also causes serious secondary damage to the quality of the raw material used for sugar and alcohol production, because the drilling of the sugarcane stalk by the borer creates favorable conditions for fungal entry and opportunistic bacteria, especially *Fusarium moniliform* and *Colletotrichum falcatum,* causing red rot (Figure 2). Bacteria associated with the red rot raw material produce undesirable fermentations, resulting in products foreign to industrial alcoholic fermentation. Moreover, these bacteria also produce organic acids and gums (dextrans) from the sugars contained in the wort, which negatively affect the viability of yeast cells, requiring their replacement in fermentation reactors (Prececti and Terán 1983; Prececti et al., 1988; BOTELHO and MACEDO, 2002). Another problem arising from the presence of bacteria in fermentation reactors is the possibility of yeast flocculation occurring. In this case, the contaminating bacteria form mucilage that aggregates the yeast cells, causing them to flocculate. Finally, plants attacked by the borer/red rot complex also have high levels of phenolic compounds (METCALF and LUCKMANN, 1994; PRICE, 1997).

Assuming a 4% borer Infestation Infection Index (typical for Brazilian sugarcane fields), average agricultural losses, and pest control costs, it is estimated that the borer causes economic losses of more than R$5 billion annually to the sugar and ethanol production industries.

The sugarcane borer is difficult to control with chemical insecticides due to the feeding behavior of the larva in the stalk, which prevents the insecticide from effectively contacting the insect. As an alternative to chemical insecticides, insecticidal proteins, mainly identified from the bacterium *Bacillus thuringiensis* (Bt), have been used to control agricultural pests, including *Diatraea* sp. Among the insecticidal proteins derived from Bt strains, Cry crystalline proteins stand out for their specific toxicity to larvae of common lepidopteran, dipteran and coleopteran species. These proteins, produced as protoxins (65-149 KDa), are solubilized and activated in the intestines of susceptible insects by proteolysis and bind to the intestinal cell membrane, inducing osmotic lysis of the epithelium, which causes the insect to die.

Cry proteins are classified into several groups according to sequence homology, among them, the protein group classified as Cry1 presents high specificity against lepidopteran insects, making it an excellent candidate for introduction into sugarcane germplasm to produce sugarcane borer resistant varieties. The heterologous expression of Cry1 proteins in sugarcane varieties, although challenging, has great potential for sugarcane borer control, reducing economic losses to the sugarcane industry, as well as the release of chemical insecticides in the environment.

Therefore, there remains a need to develop strategies to mitigate the damage caused to sugarcane crops by pest infestation, especially by infestation by the sugarcane borer pest. By offering to sugarcane growers varieties with both high yield and borer resistance traits to different edaphoclimatic environments, agricultural biotechnology makes an important contribution to the sugarcane industry and to the sugarcane growers.

The event CTC75064-3 disclosed in the present invention was generated to provide to the sugarcane growers a new variety, resistant to sugarcane borer pest and with the genetic background of RB 867515 (Plant Variety Protection/PVP - protocol number - 21806.000439/2000-45; PVP Certificate № 271). In the 2018/19 season, RB 867515 sugarcane variety was grown on approximately 1,730 thousand hectares in the Brazilian Center-South region. The cultivation of this variety in the Northeast region was approximately 57 thousand hectares. Considering the sugarcane cultivated area (CONAB, 2019), this variety has a market share in Brazil of approximately 21%.

<u>Embodiments</u>

In a first embodiment the invention provides polynucleotides that unambiguously identify the event CTC75064-3.

The invention also identifies primers pairs and probes able to identify polynucleotides that characterize the event CTC75064-3.

In a third embodiment the invention provides methods for detecting plant material derived from the CTC75064-3 event.

Other embodiment of the invention defines a kit for detecting the presence of event CTC75064-3 in a sample of plant material.

The fifth embodiment of the present invention is a genetic construct capable of imparting to a sugarcane (*Saccharum* spp.) plant, resistance to insect infestation, particularly by *Diatraea saccharalis* pest.

Also, the invention provides a genetically modified sugarcane, a plant part, plant cell, plant tissue, or seed comprising the genetic construct of interest located at a site defined in the genome of the transformed sugarcane plant, characterized by specific flanking sequences.

The seventh embodiment of the present invention is to provide a commodity product.

The eighth embodiment of the present invention is a method of producing an insect resistant plant.

Finally, the ninth and tenth embodiments of the invention are to provide a method of making and cultivating an insect resistant sugarcane plant and/or plant cell, plant part, or seed.

## Summary of the Invention

The first embodiment is achieved by providing polynucleotides comprising at least 14 to 26 contiguous nucleotides of sequences selected from the group consisting of SEQ ID NO: 5, SEQ ID NO: 12, SEQ ID NO: 13, SEQ ID NO: 18, SEQ ID NO: 19, and SEQ ID NO: 22.

The second embodiment of the invention is achieved by providing primers pairs wherein the forward primer consists of SEQ ID NO: 6 and the reverse primer consists of SEQ ID NO: 7 and/or the forward primer consists of SEQ ID NO: 8 and the reverse primer is SEQ ID NO: 9.

The third embodiment is achieved by a method of detecting plant material derived from event CTC75064-3 comprising the steps of:

a) obtaining a sample for analysis;
b) extracting DNA from the sample;
c) providing primer pairs comprising at least a forward and a reverse primer;
d) amplifying a region between the primer pair; and detecting the presence of a product from amplification.

Also to achieve the third embodiment the primer pairs in step c) are designed to bind to a polynucleotide comprising contiguous nucleotides of sequences selected from the group consisting of SEQ ID NO: 22 and SEQ ID NO:

29, wherein at least one pair of primers comprises contiguous nucleotides of sequences selected from the group consisting of SEQ ID NO: 23, SEQ ID NO: 24, SEQ ID NO: 30 and SEQ ID NO: 31.

Still to achieve the third embodiment, the present invention describes a method of detecting plant material derived from event CTC75064-3 comprising the steps of:

a) obtaining a plant material sample for analysis;
b) extracting DNA or RNA from the sample;
c) providing a probe designed to bind to the complement of a polynucleotide comprising at least 14 contiguous nucleotides of sequences selected from the group consisting of SEQ ID NO: 18, SEQ ID NO: 19, SEQ ID NO: 32 and SEQ ID NO: 33;
d) hybridizing said probe with the sample; and
e) detecting the actual hybridization of the probe.

The fourth embodiment of the invention is evidenced by a kit for detecting the presence of event CTC75064-3 in a plant sample, the kit comprising a means to detect the presence of a polynucleotide comprising, at least, 14 contiguous nucleotides of SEQ ID NO: 18 and/or of SEQ ID NO: 19 and/or a pesticidal crystal protein (Cry).

The fifth embodiment is achieved through providing a genetic construct comprising SEQ ID NO: 1 or 2.

The sixth embodiment is achieved through a genetically modified sugarcane (Saccharum spp.) plant, a plant part, plant cell, plant tissue, or seed comprising SEQ ID NO: 18 or SEQ ID NO: 19.

The seventh embodiment is achieved by providing a commodity product produced from the genetic modified sugarcane from the present invention.

The eighth embodiment is achieved by a method of producing an insect resistant plant comprising SEQ ID NO: 20 and SEQ ID NO: 21

The ninth embodiment of the present invention provides a method of making an insect resistant sugarcane plant comprising introducing a genetic modification to a sugarcane

(*Saccharum* spp.) plant comprising SEQ ID NO: 5 or SEQ ID NO: 22 to produce a genetically modified sugarcane (*Saccharum spp*.) plant of event CTC75064-3.

Finally, the tenth embodiment of the invention describes a method of cultivating a genetically modified sugarcane *(Saccharum* spp.) plant of event CTC75064-3, comprising growing a genetically modified sugarcane *(Saccharum* spp.) plant of event CTC75064-3 comprising SEQ ID NO: 5 or SEQ ID NO: 22 under conditions comprising insect infestation.

**Brief Description of the Figures**

Figure 1 exemplifies the damage to sugarcane stalks caused by *D. saccharalis* (sugarcane borer).

Figure 2 exemplifies the red rot-borer complex due to *D. saccharalis* (cane borer) attack.

Figure 3 represents the map of the T-DNA introduced in the event of the present invention.

Figure 4 represents the plasmid used as a base for constructing the plasmid used in the present invention.

Figure 5 represents the resulting plasmid used to obtain the event of interest.

Figure 6 is the graph of qPCR amplification via Taqman® (relative fluorescence x cycle) for the event of interest.

Figure 7 is the qPCR melting curve via SYBR GREEN ™ (relative fluorescence x temperature) for the event of interest. The arrows indicate the specific amplification peak of the event of interest and the baseline indications of no amplification for the negative events and controls.

Figure 8 is the result of comparing means of Cry1Ac protein expression in leaves (Fresh tissue) of the event of the invention during the sugarcane cultivation cycle. Combined analysis for Barrinha, Piracicaba, Valparaiso (SP), Quirinopolis (GO), Mandaguaçu (PR) and Juazeiro (BA). Bars followed by the same letter do not differ by T test at the 5% probability level. Bars means SE.

Figure 9 is the result of comparing means of CrylAc protein expression in leaves (Dry tissue) of the event of the invention during the sugarcane cultivation cycle. Combined analysis for Barrinha, Piracicaba, Valparaiso (SP), Quirinopolis (GO), Mandaguaçu (PR) and Juazeiro (BA). Bars followed by the same letter do not differ by T test at the 5% probability level. Bars means SE.

Figure 10 is the expression means of CrylAc protein in fresh (A) and dry base (B) of the stalk, leaf and root tissues of CTC75064-3 event. Bars followed by the same letter do not differ by T test at the 5% probability level. The bars represent the mean ± standard error.

Figure 11 is the result of comparing means of NptII protein expression in leaves (Fresh tissue) of the event of the invention during the sugarcane cultivation cycle. Combined analysis for Barrinha, Piracicaba, Valparaiso (SP), Quirinopolis (GO), Mandaguaçu (PR) and Juazeiro (BA) locations. Bars followed by the same letter do not differ by T test at the 5% probability level.

Figure 12 is the result of comparing means of NptII protein expression in leaves (Dry tissue) of the event of the invention during the sugarcane cultivation cycle. Combined analysis for Barrinha, Piracicaba, Valparaiso (SP), Quirinópolis (GO), Mandaguaçu (PR) and Juazeiro (BA) locations. Bars followed by the same letter do not differ by T test at the 5% probability level.

Figure 13 is the expression means of NptII protein in fresh (A) and dry base (B) of the stalk, leaf and root tissues of CTC75064-3 event. Bars followed by the same letter do not differ by T test at the 5% probability level. The bars represent the mean ± standard error.

Figure 14 is the result of the Western blot methodology for identifying the CrylAc protein. M: molecular weight marker (kDa). R1-R4: biological repeats of the event of the invention; WT (1): negative control-total protein extracted from parental cultivar. CP: 1ng of purified CrylAc protein; WT+CP: 1ng of purified CrylAc protein diluted in total proteins of WT; ø: empty lane.

Figure 15 is the result of the Western blot methodology for identification of *NptII.* M protein: molecular weight marker (kDa). R1 and R2: biological repeats of the event of the invention. CP: 5ng *NptII* protein; WT+CP: 5ng *NptII* protein diluted in total protein extracted from parental cultivar. WT: total protein extracted from parental cultivar.

Figure 16 represents: **A)** Infestation index (I.I.%), **B)** Relative length of damage from event CTC75064-3 and from controls (plants in the first harvest). The letters represent the result of the analysis of variance by the T test and the bars refer to the standard error.

Figure 17 represents Borer Control Effectiveness of event CTC75064-3 from the 4 locations (1.1% = infestation index). The bar refers to the standard error of the analysis.

Figure 18 shows in A: larvae mortality in the Leaf disc assay with plant material from the insect (sugarcane borer) resistant CTC75064-3 event in comparison to the parental non-genetically modified CTC75-TC (RB867515); B: Borer Control Effectiveness of event CTC75064-3; C: exemplary results of larval size and development after seven days of feeding in the Leaf disc assay. On left are exemplary larvae that were fed with plant material from the insect resistant CTC75064-3 event, and on right (B) are exemplary larvae that were fed with plant material from the parental non-genetically modified CTC75-TC (RB867515).

Figure 19 demonstrates examples of cassettes for generation of event CTC75064-3 through gene editing approach. Cassette A comprises a sugarcane codon optimized Cas9 driven by pZmUbi promoter and T-35s terminator; Cassette B comprises crRNA for Cas9 driven by wheat U3 promoter and Cassette C comprises the HR template comprising CTC75064-3 T-DNA region (SEQ ID NO 2) with homologous arms for site directed integration.

Figure 20 represents an example of a gene editing construction comprising Cas 9 and crRNA Cassettes.

Figure 21 shows an example of a gene editing construction comprising the HR template comprising CTC75064-3 T-DNA region (SEQ ID NO 2) with homologous arms for site directed integration.

Figure 22 represents a gene editing construction comprising the all the cassettes for generation of event CTC75064-3: a codon optimized Cas9 driven by pZmUbi promoter and T-35s terminator; a crRNA for Cas9 driven by wheat U3 promoter and the HR template comprising CTC75064-3 T-DNA region (SEQ ID NO 2) with homologous arms for site directed integration.

Figure 23 A) is a schematic representation of Southern blot strategy using *Hind*III and *EcoRV* restriction enzymes for identifying the T-DNA inserted in the event CTC75064-3. Gray arrows below the T-DNA scheme indicate the predicted sizes of the T-DNA fragments from CTC75064-3 event generated. B) is a schematic representation of Southern blot strategy for detecting vector (backbone) fragments using the restriction enzyme *Sph*I.

Figure 24 represents the Southern blot using the restriction enzymes *Hind*III (left) and *EcoRV* (right). Results for the cry probe that recognizes the *cryIAc* gene. M: molecular weight marker; WT: negative control; 1C: positive control 1 copy. T0: plant from the 1st harvest. T1: plant from the 2nd harvest; T2: plant from the 3rd harvest; T3: plant from the 4th harvest.

Figure 25 represents Southern blot using the restriction enzymes *EcoRV* (left) and *Hind*III (right). Results for the 35s probe that recognizes 35s promoter. M: molecular weight marker; WT: negative control; 1C: positive control 1 copy. T0: plant from the 1st harvest. T1: plant from the 2nd harvest; T2: plant from the 3rd harvest; T3: plant from the 4th harvest.

Figure 26 represents the Southern blot using the restriction enzymes *Hind*III (left) and *EcoRV* (right). Results for the *nptII* probe that recognizes the *nptII*gene. M: molecular weight marker; WT: negative control; 1C: positive control 1 copy; T0: plant from the 1st harvest. T1: plant from the 2nd harvest; T2: plant from the 3rd harvest; T3: plant from the 4th harvest

Figure 27 A) represents the Southern blot using the restriction enzyme *Sph*I and the backbone probes BB1 and BB3. M: molecular weight marker; WT: negative control; 1C: positive control 1 copy with pCTC146; 1C*: positive control 1 copy with pCTC302. BB1: positive control for the probe backbone 1; BB2: positive control for the backbone 2 probe; BB3: positive control for the backbone probe 3. The red arrows indicate two repetitions of the CTC75064-3 event. Lanes 1: other events. B) represents the Southern blot using the *Sph*I restriction enzyme and the BB2 backbone probe. M: molecular weight marker; WT: negative control; 1C: positive control 1 copy with pCTC146; 1C*: positive control 1 copy with pCTC302. BB1: positive control for the probe backbone 1; BB2: positive control for the backbone 2 probe; BB3: positive control for the backbone probe 3. The red arrows indicate two repetitions of the CTC75064-3 event. Lines 1 are other events.

Figure 28 represents the confirmation of the integrity of the flanking regions of the T-DNA inserted in the event CTC75064-3. (A) Confirmation of integrity of the left border (5'). (B) confirmation of integrity of the right border (3'). Event: DNA from event CTC75064-3; WT: DNA CT 7515 parental (RB 867515 variety); C-: negative control.

## Detailed Description of the Invention

First, the term "event" refers to the transgenic plant produced through genetic transformation that stably expresses the desired trait conferred by the introduced transgene. More particularly, in the present case, the term "event" is considered to be the transgenic plant, preferably a sugarcane transgenic plant *(Saccharum* spp.) which, after genetic modification, expresses the characteristic of pest resistance, particularly resistance to the pest *Diatraea saccharalis* (sugarcane borer). In a preferred embodiment, the transgenic sugarcane produced through the genetic transformation is designated 'CTC75064-3' and may alternately be referred to as "CTC75064-3 event".

Also, for reference purposes, unless expressly mentioned otherwise, "LB region" means the left border (edge) of T-DNA transfer (5'), and "RB region" means the right border (edge) of T-DNA transfer (3').

Additionally, all biological sequences describe herein, except otherwise explicitly stated, encompass sequences having at least 80%, preferably 85%, 90%, 95%, 98%, 99% or 100% of identity with the described sequences.

Finally, "plant material" means any and all plant tissue or derivatives thereof, such as, but not limited to, seeds, stems, stalks, leaves, straws, bark, roots, cells, molecules of plant origin, among others. In addition, "plant material" may include any product of a plant or derivative thereof, for example, but not limited to, sap, sugar, ethanol, among others.

Recombinant DNA technology has enabled the isolation of genes and their stable insertion into a host genome. This technique, also called genetic transformation, can be defined as the controlled introduction of nucleic acids ("DNA" or DNA) into a recipient genome, excluding introduction by fertilization. It is a controlled process where a defined DNA fragment is introduced into the host (or recipient) genome and must be integrated into it. The stable insertion of these molecules into a host genome gives rise to an individual with the genome equal or substantially equal to the recipient (host) of the recombinant molecule, but with a new and particular feature. "Substantially equal" means a genome with more than 80%, preferably 85%, 90%, 95%, 98%, 99% or 100% of identity in relation to the recipient.

There are several plant genetic transformation techniques grouped into two main categories: indirect and direct gene transfer. Indirect transfer is when exogenous DNA is inserted into the genome by the action of a biological vector, while direct transfer is based on physical-biochemical processes.

Different tissues and/or cells could be used according to the genetic transformation technique and according to the species or genotypes to be transformed. Generally, these tissues or cells include, without limitation, embryogenic callus, callus, protoplasts, embryos, somatic embryos, meristematic tissues, and any other part, tissue or cell of plant with regenerative capacity.

Indirect transformation is based on the bacterium-mediated system of the genus *Agrobacterium* and has been the most widely used method for obtaining transgenic plants. Advantages to this method include the ability to transfer relatively long DNA segments without rearrangement while maintaining low copy number integration of the transgenes, thus ensuring greater genotypic stability for the generated events. Several *Agrobacterium* species and strains, plasmids and protocols have been developed and adapted for genetic transformation of several plant species. The advantages of these methods include higher probabilities to single copy events, stable integration, and genetic heritage of the introduced genetic traits, as well as, consistent genic expression through generations and lower rates of gene silencing.

*Agrobacterium tumefaciens* and *A. rhizogenes* are gram negative soil phytopathogenic bacteria belonging to the Rhizobiaceae family that cause diseases in dicotyledons, known as crown and hairy root galls, respectively. In this plant-pathogen interaction there is a process of natural gene transfer between the agrobacterium and the plant cell wherein fragments of bacterial DNA are transferred into the plant cell (T-DNA), integrating with the nuclear genome. In its natural form, the bacterium transfers T-DNA ("transferred DNA"), which is part of the bacterial plasmid called Ti ("tumor-inducing") and integrates into the genome of infected plant cells. The T-DNA fragment that is transferred to the plant cell is comprised of genes involved in the constitutive biosynthesis of phytohormones (auxins and cytokinins), which alter the normal developmental program of infected tissue and cause tumor formation. In addition, it also contains oncogenes for the synthesis of sugars and amino acids called opines, which serve as carbon and nitrogen sources for bacteria (Oger et al. 1997). Repeated ends of 25 base pairs (bp) at the right and left borders delimit the T-DNA and are essential for its transfer. Phenolic compounds released by injured plant tissues activate specific regions (vir regions), initiating the process of transfer of T-DNA to the plant cell. *Agrobacterium* also has chromosomal (chv) genes that promote binding between bacterial and host cells, allowing the formation of the pore passage of the T-DNA-containing complex (Sheng & Citovsky. 1996).

Since the segment to be transferred is defined by its borders, any sequence flanked by the borders can be transferred to a plant by means of agrobacteria, making it possible to manipulate these sequences in order to transfer coding sequences of interest. The replacement or deletion of the coding regions of wild-type T-DNA (oncogenes) allows for the generation of non-oncogenic (disarmed) *Agrobacterium* strains, which can carry the sequences of interest. The modified T-DNA is able to transfer the sequences of interest to plants because the virulence genes (vir region) remain intact.

Additionally, the *Agrobacterium* indirect transformation system allows for the transfer of artificial plasmid constructs to plants as long as the constructs contain such T-DNA borders, which enables the flexibility to use molecular tools and materials developed for other bacterial strains.

These artificial plasmid constructs have promoters from different origins, as for example, plant promoters, viral promoters, bacterial and or chimeric promoters, besides genes that confer antibiotic resistance, herbicide resistance or tolerance, or enzymatic activity (phosphomannose isomerase (PMI)/mannose (Man)), so these markers can be used for the selection of transformed cells or plants.

These constructions also can contain auxiliaries genes which interfere with relevant morphogenesis signaling pathways, enhancing the efficiency of the genetic transformation process and regeneration of vegetal tissues. Included, without limitations, LEAFY COTYLEDON1 (Lotan et al., 1998), Lecl (Lowe et al., 2002), LEAFY COTYLEDON2 (Stone et al., 2001), WUSCHEL (WUS; Zuo et al., 2002), e BABY BOOM (BBM; Boutilier et al., 2002), among others.

In a first aspect of the present invention, foreign or exogenous DNA to be introduced into the plant is cloned into a binary plasmid between the left and right border consensus sequences (T-DNA). The binary plasmid is transferred to an *Agrobacterium* cell, which is subsequently used to infect plant tissue. The T-DNA region of the vector comprising the exogenous DNA is inserted into the plant genome. The marker gene expression cassette and the characteristic gene expression cassette may be present in the same region of T-DNA, in different regions of T-DNA on the same plasmid, or in different regions of T-DNA on different plasmids. In one embodiment of the present invention, the cassettes are present in the same region as the T-DNA. One of skill in the art is familiar with the methods of indirect transformation by *Agrobacterium.*

Alternatively, direct DNA transfer can be used to directly introduce DNA into a plant cell. One method of direct DNA transfer is to bombard plant cells with a vector comprising DNA for insertion using a particle gun (particle-mediated biolistic transformation). Other methods for transformation of plant cells include protoplast transformation (optionally in the presence of polyethylene glycols); ultrasound treatment of plant tissues, cells, or protoplasts in a medium comprising the polynucleotide or the vector; microinjection of the polynucleotide or vector into plant material; microinjection, vacuum infiltration, sonication, use of silicon carbide, chemical transformation with PEG, electroporation of plant cells and the like. Between the disadvantages of direct transformation are challenges related to regeneration of plant tissue and the low transgene expression.

In addition, genetic transformation could be performed by site direct insertion through homologous recombination mediated by nucleases (genome editing). In recent years, genome editing technology based on use of engineered or chimeric nucleases has enabling the generation of genetically modified organisms in a more precise and specific way. The introduction of exogenous or foreign genes occur by homologous recombination through introduction of a Homologous recombination template (HR) having the exogeneous DNA linked to a DNA fragment homologous to the genome of the receptor organism. Between the tools available are the chimeric enzymatic system CRISPR (clustered, regularly interspaced, short palindromic repeats) - Cas, the Zinc finger (ZFN)nucleases and TAL effector nucleases (TALENs). Crispr-Cas systems are enzymatic systems comprising two main components: an endonuclease (Cas) and a guide-RNA (single-guide RNA - sgRNA; a guide to the specific cleavage site of Cas endonuclease). The guide RNA may also comprise of two components: a Crispr RNA (crRNA) - a sequence of 17-20 mer complementary to specific DNA genomic sequences and, optionally, of a tracrRNA. The specific cleavage performed by endonuclease and guide by the sgRNA would be repair by homologous recombination, specifically inserting the exogenous DNA flanked by the homologous sequences to the cleavage site. The introduction of this enzymatic system to the cell could occur by several manners, using plasmids, through direct or indirect transformation, or using carriers like proteins and other chemical agents. The expression of the system components would occur in a transient or stable manner, using the cellular machinery of the receptor organism or being realized in a exogeneous way, in vitro, delivering to the target cell or tissue all the components ready to use (endonucleases + sgRNA, in vitro transcribed and combined before cell delivery). The description presented herein is not exhaustive and should not limit the use of different variations, systems and methods of genome editing on scope of the present invention, known in the State of the Art and even the ones not yet discovered.

Following transformation, transgenic plants are regenerated from the transformed plant tissue and the progeny that have exogenous DNA can be selected using an appropriate marker such as kanamycin or ammonium glufosinate resistance. One skilled in the art is familiar with the composition of suitable regeneration media.

Alternatively, other selection methods could be applied, without the insertion of any gene marker in the host genome (receptor organism) as described before.

In a preferred embodiment, genetic transformation is mediated through a bacterium of the genus *Agrobacterium*.

In an even more preferred embodiment, genetic transformation is mediated by *Agrobacterium tumefaciens.*

CTC75064-3 event exhibits a new genotype comprising two expression cassettes. The first expression cassette comprises a promoter suitable for plant expression operably linked to a gene encoding a Cry1Ac insecticide toxin useful in controlling lepidopteran insect pests and a suitable polyadenylation signal. The second expression cassette comprises a promoter suitable for plant expression operably linked to a gene encoding a protein used as a selective marker in obtaining the event of the present invention.

Promoters suitable for plant expression may be isolated from plants or from other organisms. Several promoters have been isolated or developed including constitutive promoters, "on and off" promoters, and promoters that are responsive to tissue-specific abiotic stresses, among others. Many of these promoters have intronic sequences described as relevant for proper gene expression. In a preferred aspect of the invention, promoters are constitutive promoters and may be selected from the non-limiting group consisting of CaMV 35s, CoYMV (Commelina yellow mottle virus), FMV 35s, Ubiquitin, Actin Rice Promoter (Act-1), Act -2, nopaline synthase promoter (NOS), octopine synthase promoter (OCS), corn alcohol dehydrogenase promoter (Adh-1), PvUbi1, among others.

Additional elements such as introns, enhancer sequences and transporters may also be incorporated into the expression cassette for the purpose of enhancing gene expression levels, for example, transcriptional or translation enhancers such as CaMV 35s enhancers, FMV 35s, Nos, supP, non-translated leader sequence from wheat major Chlorophyll a/b-Binding Polypeptide (L-Cab), kosak sequences 5' upstream from the translational start site, among others.

In one embodiment of the invention, the promoter is Cauliflower Mosaic Virus 35s (CaMV 35s). In an even more preferred embodiment, the promoter CaMV 35s is a doubled enhanced CaMV 35s promoter (2xCaMV35s).

In one embodiment of the invention Kosak sequence 5' upstream from the translational start site and the *Oryza sativa* Actin 1 intron (OsACT1) are contemplated in the CaMV 35s promoter region. Additionally, L-Cab leader sequence is also contemplated in the CaMV 35s promoter region.

In a preferred embodiment, the cry1Ac gene expression is regulated by the combination of elements selected from the group consisting of doubled enhanced promoter CaMV 35s, non-translated leader sequence from wheat major Chlorophyll a/b-Binding Polypeptide (L-Cab), OsACT1 intron and Kosak sequence 5' upstream from the translational start site. In a preferred embodiment, *cry1Ac* gene expression is regulated by a promoter region comprising doubled enhanced promoter CaMV 35s, wheat leader sequence L-Cab, OsACT1 intron and Kosak sequence 5' upstream from the translational start site.

In another embodiment the promoter is the maize Ubiquitin (pUBI) gene promoter. In an even more preferred embodiment, the maize Ubiquitin promoter contains an intron in the 5' sequence of the leader RNA.

The promoter region (UBI-1) of the present invention has 1992 base pairs which are subdivided into: promoter fragment (899 bases), first exon of the polyubiquitin-1 gene (83 bases) and first intron (1,010 bases).

In one embodiment, the *nptII* gene expression is regulated by maize Ubiquitin (pUBI) gene promoter. In a preferred embodiment, *nptII* gene expression is regulated by maize Ubiquitin promoter contains an intron in the 5' sequence of the leader RNA. In a specific embodiment, *nptII* gene expression is regulated by a maize Ubiquitin (pUBI) gene promoter region comprising 1992 base pairs which are subdivided into: promoter fragment (899 bases), first exon of the polyubiquitin-1 gene (83 bases) and first intron (1,010 bases).

Terminator sequences are also contemplated on the expression cassettes. Examples of suitable and functional plant polyadenylation signals include those from the *Agrobacterium tumefaciens* nopaline synthase gene (nos), proteinase inhibitor II gene rbcS (pea ribulose-1,5-bisphosphate carboxylase small subunit), Lhcb1 (tobacco chlorophyll a/b-binding proteins), CaMV 35s, octopine synthase, alpha-tubulin gene, among others.

In one embodiment of the present invention, the polyadenylation signal is that derived from the CaMV 35s gene (T35s). In another embodiment of the present invention, the polyadenylation signal is that derived from the *Agrobacterium tumefaciens* nopaline synthase (T Nos) gene.

Preferably, the polyadenylation signal for cry1Ac cassette is CaMV 35s terminator (T-35s) and polyadenylation signal for *nptII* gene is *Agrobacterium tumefaciens* nopaline synthase terminator - NOS.

**[0078]** The cry1Ac gene encodes a 615 amino acid toxin with an estimated molecular weight of 68 KDa, originating from *Bacillus thuringiensis* serovar kustaki (strain HD73), which confers resistance to *Diatraea saccharalis* (cane borer). The present invention contemplates gene modifications for expression of the active tryptic nucleus of native CrylAc protein only. Thus, in a preferred embodiment of the present invention, the polynucleotide encoding the CrylAc protein is truncated, encoding the 52 KDa tryptic insecticide nucleus. In a more preferred embodiment, the CrylAc protein is SEQ ID NO 34. The present invention also contemplates sequences having at least 80%, preferably 85%, 90%, 95%, 98%, 99% or 100% of identity with SEQ ID NO: 34. The tryptic nucleus is responsible for the insecticidal activity of the protein, binding to specific proteins of the insect's gut leading to disruption of the functional and anatomical integrity of this organ. Ingestion of the CrylAc protein by the target insect causes altered nutrient absorption, which leads to rapid toxicity and subsequent death of the insect.

According to the invention, the polynucleotide encoding the CrylAc protein may have optimized (or otherwise altered) codons to improve expression in plant material. Such codon optimization may be used to alter the predicted secondary structure of the RNA transcription product produced in any transformed cell or to destroy the cryptic RNA instability elements present in the unchanged transcription product, thereby enhancing the stability and/or availability of the transcription product in the transformed cell.

Preferably, the cry1Ac gene present at the event of the invention corresponds to a truncated synthetic DNA sequence optimized with preferred sugarcane codons. In an even more preferred aspect of the present invention, the cry1Ac gene has the sequence SEQ ID NO: 20. The invention also contemplates sequences having at least 80%, preferably 85%, 90%, 95%, 98%, 99% or 100% of identity with SEQ ID NO: 20.

Several marker genes for plant event selection have already been characterized, including some that confer tolerance to antibiotics and others that confer resistance to herbicides. Examples of marker genes that may be selected for use in the present invention include those that confer resistance or tolerance to hygromycin, kanamycin, gentamicin, geneticin, glyphosate, ammonium glufosinate or resistance to toxins such as eutypine. Other forms of selection are also available such as hormone-based selection systems, visual selection through expression of fluorescent proteins, mannose isomerase, xylose isomerase, among others. In one embodiment of the present invention, the event selection marker gene is one which confers tolerance to kanamycin and geneticin.

In a preferred embodiment of the invention, the marker gene used in the second expression cassette is the *nptII* gene, which encodes the 265 amino acid neomycin phosphotransferase II (*nptII*) enzyme with an estimated molecular weight of 29.2KDa. In an even more preferred aspect of the present invention, the *nptII* gene has the sequence SEQ ID NO 21. Neomycin phosphotransferase II confers resistance to antibiotics of the aminoglycoside class, such as kanamycin and geneticin. The *nptII* gene used as a selection marker in obtaining transformed events is derived from the Escherichia coli Tn5 transposon as described by BECK et al. (1982). The NptII protein is produced by several prokaryotes widely found in the environment, both in aquatic and terrestrial habitats, as well as in human and animal intestinal microflora. The NptII protein inactivates aminoglycoside antibiotics such as neomycin, gentamicin, geneticin, paromycin and kanamycin A, B and C using adenosine triphosphate (ATP) to phosphorylate them, thus preventing them from causing injury to cells when exposed to the mentioned antibiotics. This mechanism allows its use as a marker for selecting transformed plants. In a preferred aspect of the present invention, the NptII protein has the sequence SEQ ID NO 35.

The use of selection marker genes, such as the *nptII* gene, is important for selecting cells transformed in the process of genetic modification (HORSCH et al., 1985). The objective of inserting the *nptII* gene in the event of the present invention was, therefore, the selection of cells transformed with the cry1Ac gene.

In addition to the expression cassettes described, additional expression cassettes may also be used in event CTC75064-3.

The first and second expression cassettes comprised in event CTC75064-3 may be introduced into the plant on the same or on different plasmids. If the first and second expression cassettes are located on the same plasmid and are introduced into the plant by an *Agrobacterium-mediated* transformation method, they may be present within the same or different regions of T-DNA. In one embodiment of the present invention, the first and second expression cassettes are present in the same region as the T-DNA.

More particularly, the event of the present invention was obtained by *Agrobacterium tumefasciens-mediated* transformation with a genetic construct comprising a DNA fragment (T-DNA) containing the cry1Ac and *nptII* gene expression cassettes. Preferably, the genetic construct of the present invention comprises the nucleotide of sequence SEQ ID NO:1.

The event of the present invention was obtained by *Agrobacterium tumefasciens*-mediated transformation containing the T-DNA fragment as defined above (SEQ ID NO:1).

This T-DNA fragment was inserted into a binary plasmid that contains in its host spectrum the bacteria *Escherichia coli* and *Agrobacterium tumefaciens.* Specific genetic elements and the origins of the components of the original binary plasmid of the present invention are shown in Figure 4. The binary plasmid comprising the construct of the present invention is depicted in Figure 5.

In a preferred embodiment, the genetic construct of the present invention comprises the sequence of SEQ ID NO:14.

Said construct is transferred to an *Agrobacterium tumefaciens* (vector) strain by techniques known to one of ordinary skill in the art, such as electroporation or thermal shock, among others.

In an even more preferred embodiment, the vector is an *Agrobacterium tumefaciens* strain EHA105.

A method for producing the event of interest is further described. In a preferred embodiment, the said method comprising the steps of:

a) introducing a genetic construct into an *Agrobacterium* strain;
b) obtaining embryogenic callus from immature leaf rolls or top stalks of sugarcane *(Saccharum* spp.);
c) co-cultivating embryogenic callus with a culture of *Agrobacterium*;
d) selecting transformed cells containing the functional fragment in culture medium containing aminoglycoside antibiotics; and
e) regenerating transformed sugarcane plants.

In one embodiment, the step a) of the method of producing a genetically modified sugarcane *(Saccharum* spp.) plant of event CTC75064-3 comprises introducing a genetic construct comprising SEQ ID NO: 20 and SEQ ID NO: 21 into an *Agrobacterium* strain. In another embodiment, the step d) comprises selecting transformed cells containing the functional fragment in culture medium containing geneticin. Additionally, step e) of said method comprises regenerating transformed sugarcane plants, wherein the genetically modified sugarcane plants comprise SEQ ID NO: 20 and SEQ ID NO: 21. The invention also contemplates, a plant part, plant cell, plant tissue, or seed of the genetically modified sugarcane plants produced by the method described herein.

Those skilled in the art are familiar with the composition of suitable culture media for the generation of embryogenic callus (stage b), as well as the means of the co-cultivation stages (stage c: co-cultivation + rest), selection (stage d), and regeneration (stage e; regeneration + elongation). Preferably, the culture media used are based on compositions comprising ingredients such as MS salts (Murashige and Skoog, 1962), sucrose, and vitamins B5. Optionally, the following can also be added: amino acids selected from the group comprising proline and asparagine; casein hydrolysate; citric acid; mannitol; copper sulfate; glycine; gelling agent; auxins; antibiotics; acetosyringone; and selection agents. The use of auxins is especially important in embryogenic callus generation, co-cultivation and selection, as well as aminoglycoside antibiotics, preferable geneticin, in the selection medium.

The "co-cultivation" step refers to the incubation of plant tissue that has been infected or contacted with *Agrobacterium* to allow the transfer of *Agrobacterium* T-DNA to plant cells. This stage corresponds to the period from the moment immediately after inoculation (contact of *Agrobacterium* with plant tissue) until the moment the bacterium is removed or inactivated.

Inoculated tissue may be co-cultured for about 1 to 30 days, preferably from 1 to 20 days, or more preferably from 1 to 10 days.

During the co-cultivation step, the temperature may be any suitable temperature known in the art for the target plant. Illustratively, for sugarcane, the temperature may range from about 15 °C to about 30 °C and from about 16 °C to about 29 °C. In some embodiments, the co-cultivation step occurs in the absence of light.

Following co-cultivation with *Agrobacterium,* the medium is removed, and the cells are transferred to a culture medium lacking *Agrobacterium.* The cells are then incubated in the dark at a temperature between about 20 °C and about 26 °C for a period of 1 to 20 days.

The method provided herein further includes selecting cells comprising at least one copy of the genetic sequence of interest. "Select" as used herein means the situation in which a selective agent is used for transformants, wherein said selective agent will allow preferential growth of plant cells containing at least one copy of the marker gene positioned within the T-DNA. Whereas, those cells that were not transformed will not contain the marker gene that permits survival in the selective agent. As indicated above, any suitable selection marker may be used. Preferably, the selection marker gene used is the *nptII*gene, which encodes an enzyme that confers resistance to aminoglycoside antibiotics, such as geneticin.

In some embodiments, an agent that inhibits *Agrobacterium* growth is also added.

Selection may occur under light or dark conditions, depending on the plant species being transformed and on the genotype, for example. In some cases, embryogenic callus or other tissues undergoing transformation may be sub-cultured at regular or irregular intervals in the same medium. In the case of callus transformation, individual calluses can be kept separate to ensure that only one plant is regenerated by each callus (thus ensuring that all regenerated plants are derived from independent transformation events). In a preferred embodiment, the selection step occurs in the dark using geneticin as a selection agent for about 1 to 10 weeks. More preferably the selection step occurs for about 2 to 5 weeks.

After the selection period, plant tissue that has continued to grow in the presence of the selection agent, and has therefore been genetically modified, can be manipulated and regenerated by placing it in suitable culture media and growth conditions. The transgenic plants thus obtained can be tested for the presence of the DNA of interest. For the purpose of this invention, the term "regenerate" refers to the formation of a plant comprising both an aerial part and roots. Regenerated plants can be planted on suitable substrate (such as soil) and transferred to the greenhouse. As used herein, "genetically modified" or "transgenic" or "stably transformed" means a plant cell, plant part, plant tissue, or plant that comprises a DNA sequence of interest that is introduced into its genome by transformation.

In one embodiment, the bacterium is of the genus *Agrobacterium.*

In a more preferred embodiment, the bacterium is *Agrobacterium tumefaciens.*

In an even more preferred embodiment, the bacterium is an *Agrobacterium tumefaciens* strain EHA105.

The present invention also relates to the characterization of the selected event (CTC75064-3) and methods of detecting plant material derived therefrom. Analytical methods for detection and characterization of transgenic plants include indirect methods (protein-based detection methods) or direct methods (DNA-based detection methods).

The definition of the T-DNA stable integration site in the host cell genome and the characterization of its flanking sequences is necessary for the development and validation of methodologies for the unambiguous identification and characterization of the event.

To identify the flanking regions at the ends of the T-DNA insert in event CTC75064-3, several DNA amplification and sequencing experiments were performed. Inverse PCR (iPCR) assays were performed at both ends of the T-DNA to isolate and clone the flanking regions of the insert. Subsequently, the fragments obtained and isolated were sequenced using the Sanger method to validate the results obtained by iPCR. The genetic insertion map present in event CTC75064-3 resulting from the data generated by these experiments is shown in Figure 3 and SEQ ID NO: 2. The flanking sequences of event CTC75064-3 are shown in SEQ ID NO: 23 and SEQ ID NO: 24.

According to one aspect of the invention, a polynucleotide comprising at least 14 contiguous nucleotides of the 26-nucleotide sequence of SEQ ID NO: 18 is provided. In one embodiment, a polynucleotide comprising at least 15 contiguous nucleotides of the 26-nucleotide sequence of SEQ ID NO: 18 is provided. In one embodiment, a polynucleotide comprising at least 16 contiguous nucleotides of the 26-nucleotides of SEQ ID NO: 18 is provided. In one embodiment, said polynucleotide comprises at least 17 contiguous nucleotides of the 26 - nucleotides of SEQ ID NO: 18. In one embodiment, said polynucleotide comprises at least 18 contiguous nucleotides of the 26 - nucleotide sequence of SEQ ID NO: 18. In one embodiment, said polynucleotide comprises at least 19 contiguous nucleotides of the 26 - nucleotide sequence of SEQ ID NO: 18. In one embodiment, said polynucleotide comprises at least 20 contiguous nucleotides of the 26 - nucleotide sequence of SEQ ID NO: 18. In one embodiment, said polynucleotide comprises at least 21 contiguous nucleotides of the 26 - nucleotide sequence of SEQ ID NO: 18. In one embodiment, said polynucleotide comprises at least 22 contiguous nucleotides of the 26 - nucleotide sequence of SEQ ID NO: 18. In one embodiment, said polynucleotide comprises at least 23 contiguous nucleotides of the 26 - nucleotide sequence of SEQ ID NO: 18. In one embodiment, said polynucleotide comprises at least 24 contiguous nucleotides of the 26 - nucleotide sequence of SEQ ID NO: 18. In one embodiment, said polynucleotide comprises at least 25 contiguous nucleotides of the 26 - nucleotide sequence of SEQ ID NO: 18. In one embodiment, said polynucleotide comprises SEQ ID NO: 18. In one further aspect of the invention, said polynucleotide comprises SEQ ID NO: 13.

According to one aspect of the invention, a polynucleotide comprising at least 14 contiguous nucleotides of the 26 - nucleotide sequence of SEQ ID NO 19 is provided. In one embodiment, a polynucleotide comprising at least 15 contiguous nucleotides of the 26 - nucleotide sequence of SEQ ID NO 19 is provided. According to one aspect of the invention, a polynucleotide comprising at least 16 contiguous nucleotides of the 26 - nucleotide sequence of SEQ ID NO 19 is provided. In one embodiment, a polynucleotide comprising at least 17 contiguous nucleotides of the 26 - nucleotide sequence of SEQ ID NO: 19 is provided. In one embodiment, a polynucleotide comprising at least 18 contiguous nucleotides of the 26 - nucleotide sequence of SEQ ID NO: 19 is provided. In one embodiment, a polynucleotide comprising at least 19 contiguous nucleotides of the 26 - nucleotide sequence of SEQ ID NO: 19 is provided. In one embodiment, a polynucleotide comprising at least 20 contiguous nucleotides of the 26 - nucleotide sequence of SEQ ID NO: 19 is provided. In one embodiment, a polynucleotide comprising at least 21 contiguous nucleotides of the 26 - nucleotide sequence of SEQ ID NO: 19 is provided. In one embodiment, a polynucleotide comprising at least 22 contiguous nucleotides of the 26 - nucleotide sequence of SEQ ID NO: 19 is provided. In one embodiment, a polynucleotide comprising at least 23 contiguous nucleotides of the 26 - nucleotide sequence of SEQ ID NO: 19 is provided. In one embodiment, a polynucleotide comprising at least 24 contiguous nucleotides of the 26 - nucleotide sequence of SEQ ID NO: 19 is provided. According to one aspect of the invention, a polynucleotide comprising at least 25 contiguous nucleotides of the 26 - nucleotide of SEQ ID NO: 19 is provided. In one embodiment, a polynucleotide comprising SEQ ID NO: 19 is provided. In one aspect of the invention, a polynucleotide comprising SEQ ID NO: 12 is provided.

In a further aspect of the present invention a polynucleotide comprising the sequence of SEQ ID NO: 5 is provided. In still another aspect of the invention, a polynucleotide comprising the sequence SEQ ID NO: 22 is provided.

According to one aspect of the invention, there is provided a plant comprising at least 14 contiguous nucleotides of the 26 - nucleotide sequence of SEQ ID NO: 18. In one embodiment, a plant comprising at least 15 contiguous nucleotides of the 26 - nucleotide sequence of SEQ ID NO: 18 is provided. According to one aspect of the invention, there is provided a plant comprising at least 16 contiguous nucleotides of the 26 - nucleotide sequence of SEQ ID NO: 18. In one embodiment, a plant comprising at least 17 contiguous nucleotides of the 26 - nucleotide sequence of SEQ ID NO: 18 is provided. In one embodiment, a plant comprising at least 18 contiguous nucleotides of the 26 - nucleotide sequence of SEQ ID NO: 18 is provided. In one embodiment, a plant comprising at least 19 contiguous nucleotides of the 26 - nucleotide sequence of SEQ ID NO: 18 is provided. In one embodiment, a plant comprising at least 20 contiguous nucleotides of the 26 - nucleotide sequence of SEQ ID NO: 18 is provided. In one embodiment, a plant comprising at least 21 contiguous nucleotides of the 26 - nucleotide sequence of SEQ ID NO: 18 is provided. In one embodiment, a plant comprising at least 22 contiguous nucleotides of the 26 - nucleotide sequence of SEQ ID NO: 18 is provided. In one embodiment, a plant comprising at least 23 contiguous nucleotides of the 26 - nucleotide sequence of SEQ ID NO: 18 is provided. In one embodiment, a plant comprising at least 24 contiguous nucleotides of the 26 - nucleotide sequence of SEQ ID NO: 18 is provided. In one embodiment, a plant comprising at least 25 contiguous nucleotides of the 26 - nucleotide sequence of SEQ ID NO: 18 is provided. In one embodiment, a plant comprising SEQ ID NO: 18 is provided. In an additional embodiment, a plant comprising SEQ ID NO: 13 is provided.

According to one aspect of the invention, there is provided a plant comprising at least 14 contiguous nucleotides of the 26 - nucleotide sequence of SEQ ID NO: 19. In one embodiment, a plant comprising at least 15 contiguous nucleotides of the 26 - nucleotide sequence of SEQ ID NO: 19 is provided. In one embodiment, a plant comprising at least 16 contiguous nucleotides of the 26 - nucleotide sequence of SEQ ID NO: 19 is provided. In one embodiment, a plant comprising at least 17 contiguous nucleotides of the 26 - nucleotide sequence of SEQ ID NO: 19 is provided. In one embodiment, a plant comprising at least 18 contiguous nucleotides of the 26 - nucleotide sequence of SEQ ID NO: 19. In one embodiment, a plant comprising at least 19 contiguous nucleotides of the 26 - nucleotide sequence of SEQ ID NO: 19 is provided. In one embodiment, a plant comprising at least 20 contiguous nucleotides of the 26 - nucleotide sequence of SEQ ID NO: 19 is provided. In one embodiment, a plant comprising at least 21 contiguous nucleotides of the 26 - nucleotide sequence of SEQ ID NO: 19 is provided. In one embodiment, a plant comprising at least 22 contiguous nucleotides of the 26 - nucleotide sequence of SEQ ID NO: 19 is provided. In one embodiment, a plant comprising at least 23 contiguous nucleotides of the 26 - nucleotide sequence of SEQ ID NO: 19 is provided. In one embodiment, a plant comprising at least 24 contiguous nucleotides of the 26 - nucleotide of sequence SEQ ID NO: 19 is provided. In one embodiment, a plant comprising at least 24 contiguous nucleotides of the 26 - nucleotide sequence of SEQ ID NO: 19 is provided. In one embodiment, a plant comprising at least 25 contiguous nucleotides of the 26 - nucleotide sequence of SEQ ID NO: 19 is provided. In one embodiment, a plant comprising SEQ ID NO: 19 is provided. In an additional embodiment, a plant comprising SEQ ID NO: 12 is provided.

In one embodiment of the present invention, said plant is a genetically modified sugarcane *(Saccharum* spp.) plant. Additionally, said plant is insect resistant and comprises the sequence SEQ ID NO: 5. Still in a further aspect, the insect resistant plant of the present invention comprises SEQ ID NO: 22. In a further embodiment, said plant is an insect-resistant sugarcane plant of event CTC75064-3 or a plant derived therefrom.

In one aspect of the invention, event CTC75064-3 is a sugarcane *(Saccharum* spp.) plant comprising SEQ ID NO: 5. In a further aspect, event CTC75064-3 comprises SEQ ID NO: 22.

In other embodiment a specific method for detection and identification of CTC75064-3 event is provided.

According to the present invention, there is provided a method of detecting plant material derived from genetically modified sugarcane of event CTC75064-3 comprising the steps of:

a) obtaining a plant material sample for analysis;

b) extracting DNA from the sample;

c) providing primer pairs comprising at least a forward and a reverse primer;

d) amplifying a region between the primer pairs; and

e) detecting the presence of a product from amplification.

In one embodiment, primer pairs (step c) according to the detection method described are designed to bind to a polynucleotide comprising contiguous nucleotides of SEQ ID NO 2.

In another embodiment, the primer pairs in step c) are designed to bind to a polynucleotide comprising contiguous nucleotides of sequences selected from the group consisting of SEQ ID NO: 22 and SEQ ID NO: 29, wherein at least one pair of primers comprises contiguous nucleotides sequences selected from the group consisting of SEQ ID NO: 23, SEQ ID NO: 24, SEQ ID NO: 30 and SEQ ID NO: 31.

In one embodiment, the primer pairs above (step c) are designed to bind to a polynucleotide comprising at least 14 contiguous nucleotides of sequences selected from the group consisting of SEQ ID NO: 22 and SEQ ID NO: 29, wherein at least one primer pair comprises at least 3 contiguous nucleotides of sequences selected from the group consisting of SEQ ID NO: 23, SEQ ID NO: 24, SEQ ID NO: 30 and SEQ ID NO: 31. In one embodiment, the primer pairs are designed to bind to a polynucleotide comprising at least 14 contiguous nucleotides of sequences selected from the group consisting of SEQ ID NO: 22 and SEQ ID NO: 29, wherein at least one primer pair comprises at least 7 contiguous nucleotides of sequences selected from the group consisting of SEQ ID NO: 23,SEQ ID NO: 24, SEQ ID NO: 30 and SEQ ID NO: 31. In addition, the primer pairs are designed to bind to a polynucleotide comprising at least 14 contiguous nucleotides of sequences selected from the group consisting of SEQ ID NO: 22 and SEQ ID NO: 29, wherein at least one primer pair comprises at least 14 contiguous nucleotides of sequences selected from the group consisting of SEQ ID NO: 23, SEQ ID NO: 24, SEQ ID NO: 30 and SEQ ID NO: 31.

Additionally, primer pairs according to the detection method described are designed to bind to a polynucleotide comprising contiguous nucleotides of sequences selected from the group consisting of SEQ ID NO: 22 and SEQ ID NO: 29, where at least one primer pair consists of a first primer comprising contiguous nucleotides of sequences selected from the group consisting of SEQ ID NO: 23, SEQ ID NO: 24, SEQ ID NO: 30 and SEQ ID NO: 31 and a second primer comprising contiguous nucleotides of sequences selected from the group consisting of SEQ ID NO: 2 and SEQ ID NO: 36.

In one embodiment, the primer pairs according to the detection method described are designed to bind to a polynucleotide comprising at least 14 contiguous nucleotides of sequences selected from the group consisting of SEQ ID NO: 22 and SEQ ID NO: 29, wherein at least one primer pair consists of a first primer comprising at least 3 contiguous nucleotides of sequences selected from the group consisting of SEQ ID NO: 23, SEQ ID NO: 24, SEQ ID NO: 30 and SEQ ID NO: 31 and a second primer comprising at least 3 contiguous nucleotides of sequences selected from the group consisting of SEQ ID NO: 2 and SEQ ID NO: 36. In an additional embodiment, primer pairs are designed to bind to a polynucleotide comprising at least 14 contiguous nucleotides of sequences selected from the group consisting of SEQ ID NO: 22 and SEQ ID NO: 29, wherein at least one primer pair consists of a first primer comprising at least 7 contiguous nucleotides of sequences selected from the group consisting of SEQ ID NO: 23, SEQ ID NO: 24, SEQ ID NO: 30 and SEQ ID NO: 31 and a second primer comprising at least 7 contiguous nucleotides of sequences selected from the group consisting of SEQ ID NO: 2 and SEQ ID NO: 36. In addition, primer pairs are designed to bind to a polynucleotide comprising at least 14 contiguous nucleotides of sequences selected from the group consisting of SEQ ID NO: 22 and SEQ ID NO: 29, wherein at least one primer pair consists of a first primer comprising at least 14 contiguous nucleotides of sequences selected from the group consisting of SEQ ID NO: 23,SEQ ID NO: 24, SEQ ID NO: 30 and SEQ ID NO: 31 and a second primer comprising at least 14 contiguous nucleotides of sequences selected from the group consisting of SEQ ID NO: 2 and SEQ ID NO: 36.

In one embodiment, primer pairs according to the detection method described are designed to bind to a polynucleotide comprising contiguous nucleotides of sequences selected from the group consisting of SEQ ID NO: 5 and SEQ ID NO: 37, wherein at least one pair of primers comprises contiguous nucleotides of sequences selected from the group consisting of SEQ ID NO: 3, SEQ ID NO: 4, SEQ ID NO: 38 and SEQ ID NO: 39. In one embodiment, primer pairs according to the detection method described are designed to bind to a polynucleotide comprising at least 14 contiguous nucleotides of sequences selected from the group consisting of SEQ ID NO: 5 and SEQ ID NO: 37, wherein at least one primer pair comprises at least 3 contiguous nucleotides of sequences selected from the group consisting of SEQ ID NO: 3, SEQ ID NO: 4, SEQ ID NO: 38 and SEQ ID NO: 39. In one embodiment, primer pairs are designed to bind to a polynucleotide comprising at least 14 contiguous nucleotides of sequences selected from the group consisting of SEQ ID NO: 5 and SEQ ID NO: 37, wherein at least one primer pair comprises at least 7 contiguous nucleotides of sequences selected from the group consisting of SEQ ID NO: 3, SEQ ID NO: 4, SEQ ID NO: 38 and SEQ ID NO: 39. In addition, primer pairs are designed to bind to a polynucleotide comprising at least 14 contiguous nucleotides of sequences selected from the group consisting of SEQ ID NO: 5 and SEQ ID NO: 37, wherein at least one primer pair comprises at least 14 contiguous nucleotides of sequences selected from the group consisting of SEQ ID NO: 3, SEQ ID NO: 4, SEQ ID NO:

38 and SEQ ID NO: 39.

In one embodiment, primer pairs according to the detection method described are designed to bind to a polynucleotide comprising contiguous nucleotides of sequences selected from the group consisting of SEQ ID NO: 5 and SEQ ID NO: 37, wherein at least one primer pair consists of a first primer comprising contiguous nucleotides of sequences selected from the group consisting of SEQ ID NO: 3, SEQ ID NO: 4, SEQ ID NO: 38 and SEQ ID NO: 39 and a second primer comprising contiguous nucleotides of sequences selected from the group consisting of SEQ ID NO: 2 and SEQ ID NO: 36. In one embodiment, primer pairs, according to the detection method described, are designed to bind to a polynucleotide comprising at least 14 contiguous nucleotides of sequences selected from the group consisting of SEQ ID NO: 5 and SEQ ID NO: 37, wherein at least one primer pair consists of a first primer comprising at least 3 contiguous nucleotides of sequences selected from the group consisting of SEQ ID NO: 3, SEQ ID NO: 4, SEQ ID NO: 38 and SEQ ID NO: 39 and a second primer comprising at least 3 contiguous nucleotides of sequences selected from the group consisting of SEQ ID NO: 2 and SEQ ID NO: 36. In one embodiment, primer pairs are designed to bind to a polynucleotide comprising at least 14 contiguous nucleotides of sequences selected from the group consisting of SEQ ID NO: 2, SEQ ID NO: 5, SEQ ID NO: 36 and SEQ ID NO: 37, wherein at least one primer pair consists of a first primer comprising at least 7 contiguous nucleotides of sequences selected from the group consisting of SEQ ID NO: 3, SEQ ID NO: 4 SEQ ID NO: 38 and SEQ ID NO: 39 and a second primer comprising at least 7 contiguous nucleotides of sequences selected from the group consisting of SEQ ID NO: 2 and SEQ ID NO 36. Additionally, primer pairs are designed to bind to a polynucleotide comprising at least 14 contiguous nucleotides of sequences selected from the group consisting of SEQ ID NO: 5 and SEQ ID NO: 37, wherein at least one primer pair consists of a first primer comprising at least 14 contiguous nucleotides of sequences selected from the group consisting of SEQ ID NO: 3, SEQ ID NO: 4, SEQ ID NO: 38 and SEQ ID NO: 39 and a second primer comprising at least 14 contiguous nucleotides of sequences selected from the group consisting of SEQ ID NO: 2 and SEQ ID NO: 36.

It is well known, especially to those skilled in the art, that the DNA molecule (or DNA) is made up of two strands (of nucleotides) that are held together by hydrogen bridges between the nucleotide bases. Pairing occurs according to the complementarity of the bases, following the general rule of: Adenine with Thymine and Cytosine with Guanine. Thus, although representation of nucleotide sequences is performed for only one of the strands, their complementary strand or complementary sequence is included within the scope of this invention and is considered for the definition of primers and probes described herein.

Methods for obtaining samples for DNA extraction are widely known to one of ordinary skill in the art and include the collection of any plant material derived from the CTC75064-3 transgenic event such as stalks, roots, and leaves. Preferably, the samples are obtained from intact leaves. Plant DNA extraction methods include, without limitation, those based on the use of CTAB detergent (Alianabi et al., 1999), (optionally) followed by further sample purification with cesium chloride or ammonium acetate, as well as other commercially available methods.

Primer pairs suitable for use in this detection method may be designed using parameters well known to those skilled in the art of molecular biology now that SEQs ID Nos: 2, 3, 4, 5, 22, 23, 24, 29, 30, 31, 36, 37, 38 and 39 have become available. For example, one or both primers of the pair may be designed to be construct-specific, trait gene-specific, promoter-specific, sequence-specific to the junction between inserted DNA and genomic DNA, and/or flanking sequence-specific.

There are many amplification methods that can be used in accordance with this aspect of the invention. One of the most common amplification techniques known to those skilled in the art, is the polymerase chain reaction (PCR). The amplification product of a PCR reaction can be visualized by staining the nucleotide chain with a fluorescent tag such as ethidium bromide and then exciting it with UV light (typically after size separation using agarose gel electrophoresis).

One embodiment of the present invention employs variations of the PCR principle such as quantitative real-time PCR, nested PCR, inverse PCR (iPCR), digital PCR, Long PCR, Touchdown PCR, Hot Start PCR, Multiplex PCR, among others. The amplification product can also be detected by different methodologies which are contemplated in the present invention, such as the SYBR Green ™ system which emits fluorescence when this reagent binds to double stranded DNA and the Taqman® system where detection is based on the interaction of fluorescent probes. The Taqman® methodology uses a probe that is complementary to the intended PCR product segment located between the reaction primers. During the hybridization stage of the PCR cycle the probe is bound to the target DNA, and during Taq polymerase extension, through its 5'-exonuclease activity, it removes the probe, releasing the fluorochrome and emitting fluorescence. Additional embodiments of this aspect of the present invention include but are not limited to: loop-mediated isothermal amplification (LAMP), capillary gel electrophoresis (CGE), microarray technology Luminex, "DNA walking" and Next Generation Sequencing (NGS), Sanger method, Illumina, among others.

The present invention describes a specific detection methodology based on the quantitative real-time PCR (qPCR) technique known as "Plus-Minus" or "Presence - Absence," presenting two variations of the methodology: SYBR GREEN ™ and Taqman ® technology.

In one embodiment of the present invention, primer pairs are provided wherein the forward primer consists of SEQ ID NO: 6 and the reverse primer consists of SEQ ID NO: 7 and/or the forward primer is SEQ ID. NO: 8 and the reverse primer is SEQ ID NO: 9.

In an additional embodiment, the primer pairs used in step c) of the method of detecting plant material from genetically modified sugarcane of event CTC75064-3 comprise forward primer consists of SEQ ID NO: 6 and the reverse primer consists of SEQ ID NO: 7 and/or the forward primer is SEQ ID NO: 8 and the reverse primer is SEQ ID NO: 9. In addition, the amplicon (product from amplification) produced by the primers of SEQ ID NO: 6 and SEQ ID NO: 7 is viewed through a labeled probe of SEQ ID NO: 10. Alternatively, the amplicon produced by the primers of SEQ ID NO: 8 and SEQ ID NO: 9 is visualized through a labeled probe of SEQ ID NO 11. Thus, it is an aspect of the present invention that detection of the of the product from amplification obtained by the use of primers SEQ ID NO: 6 and SEQ ID NO: 7 and/or SEQ ID NO: 8 and SEQ ID NO: 9 is performed through hybridization of a probe comprising SEQ ID NO: 10 or SEQ ID NO: 11.

In one embodiment of the present invention, the region amplified by said method (the amplicon or product from amplification) is between 80 and 1000 base pairs in length. In an additional embodiment, the amplicon is between 100 and 300 base pairs in length. In one preferred embodiment, the amplicon obtained using the primers SEQ ID NO: 6 and SEQ ID NO: 7 is 107 base pairs in length, as defined by SEQ ID NO: 12. In another preferred embodiment, the amplicon obtained through the use of primers SEQ ID NO: 8 and SEQ ID NO: 9 is 135 base pairs in length, as defined by SEQ ID NO: 13.

Figures 6 (event-specific detection reaction of the invention via Taqman®) and 7 (SYBR GREEN ™ assay) represent the validation of both Methods.

Primers and probes described in the present invention may be used in combination to detect the CTC75064-3 event. Thus, a further embodiment of the present invention involves the use of multiplex PCR to identify plant material from the CTC75064-3 event.

Alternative primers and probes for the detection and characterization of the CTC75064-3 event are included in the invention. These and other variations may be used with but are not limited to any of the direct detection methods described above.

Additionally, the CTC75064-3 event can be detected from plant material by hybridizing DNA samples to the probes. Specifically, the present invention describes a method of detecting material from the genetically modified sugarcane event CTC75064-3 which comprises the steps of:

a) obtaining a plant material sample for analysis;

b) DNA or RNA extraction from the sample;

c) providing a probe or a combination of probes designed to bind to a polynucleotide comprising contiguous nucleotides of sequences selected from the group consisting of SEQ ID NO 2, SEQ ID NO 3, SEQ ID NO 4, SEQ ID NO 5, SEQ ID NO 12, SEQ ID NO 13, SEQ ID NO: 18, SEQ ID NO: 19, SEQ ID NO 22, SEQ ID NO 23, SEQ ID NO 24, SEQ ID NO 29, SEQ ID NO 30, SEQ ID NO 31, SEQ ID NO: 32, SEQ ID NO: 33, SEQ ID NO 36, SEQ ID NO 37, SEQ ID NO 38 and SEQ ID NO 39, wherein the polynucleotide is single stranded;

d) hybridizing said probe or a combination of probes with the sample, and

e) detecting the actual hybridization of the probe or a combination of probes.

In preferred embodiment, the present invention describes a method of detecting material from the genetically modified sugarcane event CTC75064-3 which comprises the steps of:

a) obtaining a plant material sample for analysis;

b) DNA or RNA extraction from the sample;

c) providing a probe designed to bind to a polynucleotide comprising 14 or more contiguous nucleotides of sequences selected from the group consisting of SEQ ID NO: 18, SEQ ID NO: 19, SEQ ID NO: 32 and SEQ ID NO: 33, wherein the polynucleotide is single stranded;

d) hybridizing said probe with the sample, and

e) detecting the actual hybridization of the probe.

According to one aspect of the invention, a probe designed to bind to a polynucleotide comprising at least 15 contiguous nucleotides of sequences selected from the group consisting of SEQ ID NO: 18, SEQ ID NO: 19, SEQ ID NO: 32 and SEQ ID NO: 33 is provided. In one embodiment, a probe designed to bind to a polynucleotide comprising at least 16 contiguous nucleotides of sequences selected from the group consisting of SEQ ID NO: 18, SEQ ID NO: 19, SEQ ID NO: 32 and SEQ ID NO: 33 is provided. According to one aspect of the invention, a probe designed to bind to a polynucleotide comprising at least 17 contiguous nucleotides of sequences selected from the group consisting of SEQ ID NO: 18, SEQ ID NO: 19, SEQ ID NO: 32 and SEQ ID NO: 33 is provided. In one embodiment, said polynucleotide comprises at least 18 contiguous nucleotides of sequences selected from the group consisting of SEQ ID NO: 18, SEQ ID NO: 19, SEQ ID NO: 32 and SEQ ID NO: 33. In one embodiment, said polynucleotide comprises at least 19 contiguous nucleotides of sequences selected from the group consisting of SEQ ID NO: 18, SEQ ID NO: 19, SEQ ID NO: 32 and SEQ ID NO: 33. In one embodiment, said polynucleotide comprises at least 19 contiguous nucleotides of sequences selected from the group consisting of SEQ ID NO: 18, SEQ ID NO: 19, SEQ ID NO : 32 and SEQ ID NO: 33. In one embodiment, said polynucleotide comprises at least 20 contiguous nucleotides of sequences selected from the group consisting of SEQ ID NO: 18, SEQ ID NO: 19, SEQ ID NO: 32 and SEQ ID NO: 33. In one embodiment, said polynucleotide comprises at least 21 contiguous nucleotides of sequences selected from the group consisting of SEQ ID NO: 18, SEQ ID NO: 19, SEQ ID NO: 32 and SEQ ID NO: 33. In one embodiment, said polynucleotide comprises at least 22 contiguous nucleotides of sequences selected from the group consisting of SEQ ID NO: 18, SEQ ID NO: 19, SEQ ID NO: 32 and SEQ ID NO: 33. In one embodiment, said polynucleotide comprises at least 23 contiguous nucleotides of sequences selected from the group consisting of SEQ ID NO: 18, SEQ ID NO: 19, SEQ ID NO: 32 and SEQ ID NO: 33. In one embodiment, said polynucleotide comprises at least 24 contiguous nucleotides of sequences selected from the group consisting of SEQ ID NO: 18, SEQ ID NO: 19, SEQ ID NO: 32 and SEQ ID NO: 33. According to one aspect of the invention, a polynucleotide comprising at least 25 contiguous nucleotides of sequences selected from the group consisting of SEQ ID NO: 18, SEQ ID NO: 19, SEQ ID NO: 32 and SEQ ID NO: 33 is provided. According to one aspect of the invention, a polynucleotide comprising at least 26 contiguous nucleotides of sequences selected from the group consisting of SEQ ID NO: 18, SEQ ID NO: 19, SEQ ID NO: 32 and SEQ ID NO: 33 is provided.

The probe may be, for example, a PCR product or a restriction digest fragment. In a further embodiment, the probe as described herein may be labeled with a fluorescent, radioactive, enzymatic, or other label suitable to enable hybridization to be detected. The person skilled in the art will now know how to design suitable probes given the advantage of the present disclosure.

In an additional embodiment, a probe hybridization method is provided to the sample under stringent conditions (high specificity). Stringent hybridization conditions are well known to those skilled in the art. Examples of stringent conditions include: hybridization at a temperature of approximately 65 ° C in a solution containing 6x SSC, 0.01% SDS and 0.25% skimmed milk powder followed by washing at the same temperature in a solution containing 0.2 x SSC and 0.1% SDS.

Suitable techniques for detecting plant material derived from event CTC75064-3 based on the hybridization principle include, but are not limited to, Southern Blots and *in situ* hybridization. One of skill in the art is familiar with such techniques.

Typically, these techniques involve incubating a probe with a sample, washing to remove the unbound probe, and detecting whether the probe has hybridized. Said detection method is dependent upon the type of label attached to the probe. For example, a radio-labelled probe can be detected by exposure to and development of X-ray film. Alternatively, an enzymatically labeled probe may be detected by converting a substrate to effect a color change.

Additionally, another aspect of the invention contemplates a method for detecting plant material derived from event CTC75064-3, which comprises: obtaining a sample for analysis; providing an antibody designed to bind to a Cry or NptII protein contained within a plant comprising at least 14 contiguous nucleotides of SEQ ID NO: 18 and/or SEQ ID NO: 19; incubating said antibody with the sample; and detecting whether the antibody bound. In one embodiment of the present invention, said Cry protein is encoded by nucleotide sequence SEQ ID NO: 20 and said NptII protein is encoded by nucleotide sequence SEQ ID NO: 21. In an additional embodiment, said Cry protein comprises SEQ ID NO: 34 and said NptII protein comprises SEQ ID NO: 35.

Suitable methods for detecting plant material derived from the CTC75064-3 event based on said antibody binding include (but are not limited to): western blots, ELISA (Enzyme-Linked ImmunoSorbent Assays), and mass spectrometry (e.g. surface-enhanced laser desorption/ionization (SELDI)). One of skill in the art is familiar with these immunological techniques. Typical steps include incubating a sample with an antibody that binds to the Cry or NptII protein, washing for removal of unbound antibody, and detecting whether the antibody has bound. Many such detection methods are based on enzymatic reactions: for example, the antibody may be linked with an enzyme such as peroxidase and upon application of a suitable substrate, a color change is detected. Such antibodies may be monoclonal or polyclonal.

Another aspect the invention contemplates a method for detecting plant material derived from event CTC75064-3, which comprises: obtaining a sample for analysis; providing a protein extract from the sample; providing test strips designed to detect the presence of a Cry or NptII protein in a plant comprising at least 14 contiguous nucleotides of SEQ ID NO: 18 and/or SEQ ID NO: 19; incubating the test strips with the sample; and detecting. In one embodiment of the present invention, said Cry protein is encoded by nucleotide sequence SEQ ID NO: 20 and the NptII protein is encoded by nucleotide sequence SEQ ID NO: 21. In an additional embodiment, said Cry protein comprises SEQ ID NO: 34 and said NptII protein comprises SEQ ID NO: 35.

In one embodiment of the invention there is provided a method for detecting plant material derived from the CTC75064-3 event, said method comprising: obtaining a sample derived from the CTC75064-3 event and a sample from a non-transgenic sugarcane variety for analysis (control); subjecting one or more insects of the species *Diatraea saccharallis* (susceptible to Cry1Ac) to the samples; detecting an insecticidal effect on the insects. In this aspect of the invention, "insecticide" refers to any inhibitory effect on the insect (including but not limited to): reduced feeding, retarded growth, reduced fecundity, paralysis, and death.

The method of detecting plant material from event CTC75064-3 includes, but is not limited to, biological leaf feeding assays where a leaf or other suitable part of the plant of event CTC75064-3, or any plant material derived from event CTC75064-3, is infested with one or more insect pests. Measurement of said detection can include assessing leaf or plant damage after adjusted time periods, assessing mortality or assessing other insecticidal effects. Such biological assays may be performed in the field or greenhouses and may entail either natural or artificial insect infestation.

In another aspect of the invention, a kit for detecting in a plant sample the presence of event CTC75064-3 is provided, said kit comprising: a means for detecting the presence of a polynucleotide comprising at least 14 contiguous nucleotides of the sequence of SEQ ID NO: 18 and/or SEQ ID NO: 19 and/or a pesticidal crystal protein (Cry). In one embodiment of the present invention, said kit may comprise DNA amplification detection technology such as PCR, qPCR, or Taqman®. In a further embodiment of the present invention, said kit may comprise probe hybridization detection technology such as Southern Blots or *in situ* hybridization. In one aspect, the means to detect material from transgenic sugarcane comprising a Cry1Ac protein (event CTC75064-3) comprises primer pairs designed to bind to a polynucleotide comprising contiguous nucleotides of sequences selected from the group consisting of SEQ ID NO: 22 and SEQ ID NO: 29, wherein at least one pair of primers comprises contiguous nucleotides sequences selected from the group consisting of SEQ ID NO: 23, SEQ ID NO: 24, SEQ ID NO: 30 and SEQ ID NO: 31. Additionally, the means comprise primer pairs, wherein the forward primer comprises SEQ ID NO: 6 and the reverse primer comprises SEQ ID NO: 7, or the forward primer comprises SEQ ID NO: 8 and the reverse primer comprises SEQ ID NO: 9. In a further embodiment, the means to detect material from transgenic sugarcane comprising a CrylAc protein (event CTC75064-3) comprises a probe comprising SEQ ID NO: 10 or SEQ ID NO: 11. In another embodiment of the present invention, said kit may comprise antibody binding detection technology such as western blots, ELISAs, mass spectrometry (SELDI) or test strips. In a further embodiment of the present invention, said kit may comprise detection technology by biological insect testing such as leaf feeding biological assays or biological mortality assays. In a further embodiment of the present invention, said kit may comprise any combination of the detection technologies mentioned above.

The transgenic event as described in the present invention affect insects of one or more species of the group comprising insects of the order Lepidoptera. As a result, a reduced number of insecticidal sprays is required during cultivation of said plant compared with a non-transgenic sugarcane plant of the same variety.

The present invention is not itself bound to event CTC75064-3; rather, it is further extended to include any plant material derived therefrom, including seed, provided they contain at least one of the polynucleotides sequences of the present invention. In one embodiment, the present invention comprises a plant part, plant cell, plant tissue, or seed from the genetically modified sugarcane *(Saccharum* spp.) plant, wherein said plant part, plant cell, plant tissue, or seed comprises SEQ ID NO: 18 or SEQ ID NO: 19. In other embodiment, the invention contemplates plant part, plant cell, plant tissue, or seed comprising SEQ ID NO: 12 or SEQ ID NO: 13. Additionally, the invention includes a plant part, plant cell, plant tissue, or seed comprising SEQ ID NO: 5 or SEQ ID NO: 22. The present invention also includes, but is not limited to, plants that are derived from crossbred lineages with the CTC75064-3 event or a derivative thereof by conventional or other crossbreeding methods; thus, one embodiment of the present invention relates to the use of a plant, plant cell, plant part, or seed from the genetically modified sugarcane *(Saccharum* spp.) plant as described herein, which is used for regenerating a plant, planting, cultivating a field of plants, or producing a plant product. The present invention also contemplates a tissue culture of a genetically modified sugarcane *(Saccharum* spp.) plant comprising SEQ ID NO: 18 or SEQ ID NO: 19. In other embodiment, the invention includes a tissue culture of a genetically modified sugarcane *(Saccharum* spp.) plant comprising SEQ ID NO: 12 or SEQ ID NO: 13. Also contemplate in the present invention is a tissue culture of a genetically modified sugarcane *(Saccharum* spp.) plant comprising SEQ ID NO: 5 or SEQ ID NO: 22. Additionally, a genetic modified sugarcane *(Saccharum* spp.) plant regenerated from the tissue culture describe above is also included in the present invention, wherein the regenerate plant comprises SEQ ID NO: 18 or SEQ ID NO: 19. Examples of plant cells and plant parts include but are not limited to: suspension cells, callus, somatic embryos, meristematic tissue, top stalks, stalks, leaf, leaf discs, tiller, shoots. Another aspect contemplates a method for producing an insect-resistant sugarcane *(Saccharum* spp.) plant, comprising crossing a first sugarcane plant with a second sugar cane plant, wherein the second sugar cane plant is a plant comprising event CTC75064-3, and producing offspring sugarcane plants therefrom. The plant comprising event CTC75064-3 is a genetically modified sugarcane *(Saccharum* spp.) plant comprising SEQ ID NO: 18 or SEQ ID NO: 19. The present invention also contemplates a sugarcane *(Saccharum* spp.) plant and plant parts, plant cells, plant tissues, or seeds therefrom produced by the method for producing an insect-resistant sugarcane described above.

In a further embodiment, the present invention provides a commodity product, produced from a sugarcane plant comprising event CTC75064-3. Thus, the invention includes a commodity product, produced from a genetically modified sugarcane *(Saccharum* spp.) plant comprising SEQ ID NO: 18 or SEQ ID NO: 19. In one embodiment, the invention contemplates a commodity product, produced from a genetically modified sugarcane *(Saccharum* spp.) plant comprising SEQ ID NO: 12 or SEQ ID NO: 13. Additionally, the invention includes a commodity product, produced from a genetically modified sugarcane *(Saccharum* spp.) plant comprising SEQ ID NO: 5 or SEQ ID NO: 22. Examples of commodity products include but are not limited to: bagasse, sugarcane juice, syrup, first generation ethanol (produced from sugarcane juice), second generation ethanol (cellulosic ethanol; produced from biomass), biomass, sugar, raw sugar, refined sugar, molasses, vinasse, and fiber.

The present invention further provides a plant material from CTC75064-3 event comprising additional polynucleotide sequences, modified or smaller than CTC75064-3, or exhibit other phenotypic characteristics. For example, the plant material CTC75064-3 event could be transformed to produce a new event comprising additional characteristics, such as, a second insect resistance gene. This process is known as gene stacking. Such second insect resistance gene codes, for example, insecticidal lectins, insecticidal protease inhibitors and other insecticidal proteins derived from *Bacillus thuringiensis.*

The present invention further provides an insect control method comprising providing plant material derived from the CTC75064-3 event at a location where said insects feed. The invention further provides an insect control method comprising providing the CTC75064-3 derived plant material at the site where said insects feed and applying other agrochemical reagents to said plant material (e.g. herbicides, fungicides, and the like).

In other embodiment, the invention describes, a method of making a genetically modified sugarcane *(Saccharum* spp.) plant of event CTC75064-3, comprising introducing a genetic modification to a sugarcane *(Saccharum* spp.) plant comprising SEQ ID NO: 5 or SEQ ID NO: 22 to produce a genetically modified sugarcane *(Saccharum* spp.) plant of event CTC75064-3, wherein the genetically modified sugarcane *(Saccharum* spp.) plant has improved insect resistance as compared to a sugarcane *(Saccharum* spp.) plant without the genetic modification. In one additional embodiment, the invention provides a method of cultivating a genetically modified sugarcane *(Saccharum* spp.) plant of event CTC75064-3, comprising growing a genetically modified sugarcane *(Saccharum* spp.) plant of event CTC75064-3 comprising SEQ ID NO: 5 or SEQ ID NO: 22 under conditions comprising insect infestation, wherein the genetically modified sugarcane *(Saccharum* spp.) plant has an increase in insect resistance as compared to a sugarcane *(Saccharum* spp.) plant without the genetic modification grown under the same conditions. The invention also provides, a genetically modified sugarcane *(Saccharum* spp.) plant of event CTC75064-3 comprising SEQ ID NO: 5 or SEQ ID NO: 22.

## **Description of Transgenic Sugarcane** ’CTC75064-3’

Transgenic hybrid sugarcane ’CTC75064-3’ plants are genetically substantially equal and phenotypically similar to the recipient (host) of the recombinant molecule, the parental plant variety’ RB 867515, a commercial Brazilian sugarcane variety (Plant Variety Protection/PVP - protocol number - 21806.000439/2000-45; PVP Certificate № 271), but with a new and particular feature (Cry1Ac expression), which guarantees the insect resistance to sugarcane borer *D. saccharalis* (Lepidoptera). As its parental variety ’RB 867515, ’CTC75064-3’ is a modern sugarcane hybrid that holds several desirable agronomic characteristics such as, genetic potential for high ratoon cane sprouting vigor, high cane yield, excellent ratooning, medium-late maturity, high sucrose content and is tolerant to leaf scald, smut, rust diseases and sugarcane mosaic.

Briefly, ’CTC75064-3’ plants are characterized by purple stalks with purple hues when exposed to the sun and by purple-green stalks where not exposed to sun. ’CTC75064-3’ plants exhibit medium-long size curved internodes and medium width yellow greenish growth rings. Smooth appearance of internodes and absent of growth crack, with a medium waxiness. ’CTC75064-3’ plants exhibit an obovate bud shape with an apical position of the pubescence on the bud. Leaf architecture is erect with curved tips curvature. The average auricle shape is lanceolate, presenting crescent-shaped ligule.

The average mature stalk height (measured at 330 Harvest Day after Planting - DAP, except for Juazeiro, measured at 210 DAP; measured from the crown until the insertion of leaf +1), stalk diameter (measured at 330 Harvest Day after Planting - DAP, expect for Juazeiro, measured at 210 DAP), number of tillers (at 30, 60, 90, 120, 150, 180, 210, 240, 270, 300 and 330 DAP; not measured in Juazeiro-BA), weight (measured at 330 DAP, expect for Juazeiro, measured at 210 DAP), sugar content (BRIX %; measured at 330 DAP, expect for Juazeiro, measured at 210 DAP; extracted juice), flowering (observations during all developmental cycle until 330 DAP, expect for Juazeiro, data collected until 210 DAP) and tons of pol% (% of sucrose in the sugarcane juice) per hectare (TPH; measured at 330 Harvest Day after Planting - DAP, expect for Juazeiro, measured at 210 DAP) were evaluate in six different locations in comparison with the parental variety 'RB867515'. TPH is calculated according to the formulae:

$$TPH = (Pol\,\%\,Cana\;x\;TCH)/100$$

For each data set, data across all sites were combined for statistical analysis. Combined site analysis was done using the following statistical model:

$$y_{ijk} = \mu + G_k + (S)_i + B(S)_{ij} + (SG)_{ik} + \varepsilon_{ijk},$$

,wherein $y_{ijk}$ is the measurement of replicate *j* on site *i* for treatment *k*; $\mu$ is the overall mean; $S_i$ is the effect of site *i* (*i* = 1 to 6); $B_j$ is the effect of replicate *j* (j = 1 to 4); $B(S)_{ij}$ is the effect of replicate *j* on site *i* (j = 1 to 4); $G_k$ is the effect of the treatment *k* (k = 1 to 7); $(SG)_{ik}$ is the interaction between site *i* and treatment *k*; $\varepsilon_{ijk}$ is the experimental residual error on site j and treatment k.

The main effects analysis and model interaction were performed as described by Kuznetsova et al. (2017). All data were analyzed using mixed linear model by package lme4 (Bates et al., 2015).

The results of the agronomic and phenotypic characteristics analysis are shown in the table below and corroborate the conclusion that 'CTC75064-3' is similar to its parental variety ('RB867515').

Table 01. Average of agronomic and phenotypic characteristics for 'CTC75064-3' and 'RB867515' parental variety. Combined analysis for the Barrinha-SP, Piracicaba-SP, Valparaíso-SP, Quirinópolis-GO, Mandaguaçu-PR and Juazeiro-BA locations. Data from Juazeiro-BA collected at 210 DAP and other locations at 330 DAP.

| | Parameter | Mean | | SE | Range** | |
|---|---|---|---|---|---|---|
| | | CTC75064-3 | RB867515 | | Min | Max |
| Combined analysis | Height (m) | 2.38* | 2.63 | 0.18 | 2.07 | 3.52 |
| | Diameter (cm) | 23.94* | 27.97 | 0.57 | 23.02 | 32.59 |
| | Tillers (330 DAP) † | 64.08 | 60.75 | 12.07 | 30 | 88.5 |
| | Weight (Kg) | 10.24* | 15.32 | 0.87 | 8.71 | 21.88 |
| | BRIX (%) | 17.53* | 15.66 | 0.66 | 13.03 | 20.4 |
| | TPH | 6.01 | 6.05 | 0.98 | 6.61 | 8.69 |
| | Flowering (Total No. of Panicles) | 0 | 0 | - | 0 | 0 |

* Significant difference between the transgenic event and the parental variety by the t test at the level of 5% ($p \leq 0.05$). SE: Standard Error; **Range: Average minimum and maximum values observed in 3 commercial reference varieties. † Not evaluated in Juazeiro (BA).

Other compositional studies have been made and also demonstrated that 'CTC75064-3' is highly similar to its parental variety ('RB 867515') [compositional parameters related to nutrition and the use of sugarcane in the diet, as defined by the OECD Guidance Document (OECD, 2011)]. Based on the results of combined data analysis (six representative locations in the Brazilian sugarcane growing regions), the data suggest that the presence of proteins Cry1Ac and NPTII contained in samples of whole plant and stalk does not significantly interfere in the bromatological composition of the event CTC75064-3 compared to the parental variety RB 867515, and that the compositional equivalence observed for the 11 parameters evaluated corroborates with the premise of a high similarity between the event CTC75064-3, and the parental variety RB 867515.

Table 02. Mean values of compositional parameters measured in the genetically-modified event 'CTC75064-3' and its conventional counterpart 'RB 867515'.

| | Analyte | Mean | | SE | Range** | |
|---|---|---|---|---|---|---|
| | | CTC75064-3 | RB867515 | | Min | Max |
| Combined analysis | Dry matter | 22.65* | 20.44 | 0.92 | 16.15 | 24.61 |
| | Moisture | 75.05 | 77.51 | 1.36 | 65.69 | 82.26 |
| | Crude fiber[1] | 27.19* | 29.85 | 1.11 | 22.25 | 33.23 |
| | NDF[1] | 49.29* | 52.35 | 1.61 | 41.36 | 56.65 |
| | ADF[1] | 31.69* | 35.25 | 1.16 | 26.29 | 38.41 |
| | Crude protein[1] | 3.26 | 3.19 | 0.21 | 2.19 | 3.91 |
| | Crude fat[1] | 0.8 | 1.1 | 0.12 | 0.66 | 1.48 |
| | Ash[1] | 3.38 | 3.73 | 0.36 | 1.83 | 4.99 |
| | Sucrose[2] | 10.04* | 8.49 | 0.57 | 4.99 | 13.49 |
| | Glucose[2] | 0.89* | 1.03 | 0.12 | 0.53 | 1.82 |
| | Fructose[2] | 0.71 | 0.8 | 0.1 | 0.44 | 1.33 |

[1]Results are expressed on dry weight basis; [2]Values expressed sugarcane stalk basis; *Significant difference between the transgenic event and the parental variety by the t test at the 5% level (p 0.05). EP: Standard Error; **Range: Minimum and maximum average values observed in 3 commercial reference varieties observed in the individual analyzes.

## Examples

Example 1. CTC75064-3 event Generation - *Agrobacterium* transformation

Event CTC75064-3 was obtained by *Agrobacterium tumefasciens*-mediated genetic transformation of the RB 867515 cultivar.

The RB 867515 cultivar is a commercial hybrid and is the donor genotype of the CTC75064-3 event (genetic background); that is, it represents the untransformed counterpart of the CTC75064-3 event. This cultivar has medium-late maturation and has been planting specially on Brazilian Center-South region. As with other commercial sugarcane hybrids, it is high-ploidy material with numerous chromosomes derived from its two parental varieties: S. *officinarum* and *S. spontaneum* (DANIELS and ROACH, 1987; SREENIVASAN et al., 1987).

The CTC75064-3 event has the *cry1Ac* gene, which confers resistance to *Diatraea saccharalis,* and the *nptII* gene, used as a selection marker. The purpose of the development of the CTC75064-3 event is to provide a proactive control of the sugarcane borer. It is expected that, after the hatching of the pest eggs on the leaves of the CTC75064-3 event, the young larvae begin to feed and, when ingesting the CrylAc protein, are controlled before they penetrate the culms of the CTC75064-3 event, avoiding if so the economic damage of the pest to the crop. The expression of the *cry1Ac* gene is regulated by a promoter region comprising doubled enhanced promoter CaMV 35s (2xCaMV35s), wheat leader sequence L-Cab, OsACT1 intron and Kosak sequence 5' upstream from the translational start site. The expression of *nptII* gene is regulated by the maize ubiquitin gene promoter UBI-1, which has an endogenous intron. CrylAc expression cassette uses CaMV 35S terminator and the *nptII* cassette uses the *Agrobacterium tumefaciens* nopaline synthase (nos) terminator.

### 1.1 Construct development using *cry1Ac* and *nptII* genes (Figure 5; SEQ ID NO 14).

Conventional gene cloning techniques using commercial bacterial plasmids, restriction enzyme digestion, and fragment ligation (with ligases) were used to develop the construct of the present invention (Figure 5).

The construct of the present invention was developed by joining the 2xCaMV35s-cry1Ac-T35s and UBI-*nptII*-TNOS cassettes. T-DNA containing both cassettes was transferred from a cloning plasmid to the base plasmid (Figure 4: binary plasmid vector, which contains in its host spectrum the bacteria *Escherichia coli* and *Agrobacterium tumefaciens*) using restriction enzymes, generating the construct of the present invention (Figure 5; SEQ ID NO: 14).

After the final cloning step, the construct (SEQ ID NO: 14) was inserted into *Escherichia coli* strain TOP10 using heat shock. An isolated colony containing the construct was inoculated into liquid LB medium supplemented with

150 $\mu g$/ml spectinomycin and incubated at 37°C while shaking at 250 rpm for a period of 16 hours. Stocks were then prepared containing bacterial suspension and 10% (v/v) glycerol, which were stored in an ultrafreezer at -80°C.

The construct of the present invention was then transferred from *E. coli* to *Agrobacterium tumefaciens* strain EHA105 by isolation and purification of plasmid DNA and transformation of *Agrobacterium* by electroporation. As with the E. *coli* strain, stocks containing the bacterial suspension of Agrobacterium and 10% (v/v) glycerol were stored in an ultrafreezer at -80°C.

1.2 *Agrobacterium*-mediated plant transformation

To obtain embryogenic callus, young RB 867515 sugarcane leaf rolls, grown in the field or greenhouse for up to 12 months, were collected for isolation of the initial explants.

After surface disinfection, transverse sections about 0.05-5mm thick were cut from above the meristem under aseptic conditions. The sections were placed on the surface of the callus induction culture medium [MS - Murashige and Skoog, 1962; sucrose, vitamins B5, amino acids selected from the group comprising proline, casein hydrolyzate, citric acid, mannitol, copper sulfate, glycine, gelling agent, 2,4D]. The cultures were kept in the dark at 26 $\pm$ 2 °C and sub-cultured every 15 days for three to five cycles of 7-28 days each. One week before transformation, calli were again selected for embryogenic characteristics (nodular, compact, opaque and slightly yellowish).

*Agrobacterium* culture, comprising strain EHA105 transformed with the construct of the present invention, was started from a glycerol stock and kept in the dark at 28°C for two to three days. The *Agrobacterium* suspension was prepared by resuspending the culture in MS liquid medium plus acetosyringone, adjusting to a final $OD_{600}$ of 0.1-1.0 (MS salts, sucrose, and vitamins B5) for infection of calli.

The calli with embryogenic characteristics were visually selected and directly transferred to the *Agrobacterium* suspension, where they remained for 30 minutes in the dark with constant agitation at 50 rpm.

After this period, calli were separated from the *Agrobacterium* suspension and excess suspension was removed. After, calli were cultured for 1-5 days in semi-solid medium (MS salts, sucrose, vitamins B5, citric acid, gelling agent, 2,4D and acetosyringone) at 22 °C in the dark.

After co-cultivation, callus was transferred to DT rest medium (MS salts; sucrose, B5 vitamins, amino acids selected from the group comprising proline and asparagine, casein hydrolyzate, citric acid, copper sulfate, glycine, gelling agent, 2,4D, timentin) and kept for 5-14 days at 26 °C in the dark.

Transformed cells were selected by successive sub-cultures in selection culture medium containing phytoregulators and the selective agent geneticin. (Selection medium with geneticin: MS salts, sucrose, vitamins B5, amino acids selected from the group comprising proline and asparagine, casein hydrolyzate, copper sulfate, glycine, gelling agent, 2,4D, timentin). The calli remained in this condition for 21 days at 26 °C in the dark, then the calli were transferred to the regeneration medium (equivalent to selection medium without 2,4D) and then to elongation medium (MS salts, sucrose, B5 vitamins, casein hydrolyzate, gelling agent, timentin). The calli were exposed to 16-hour photoperiod at 4,000 lux in the presence of the selective agent, then they were multiplied, rooted, and acclimatized before transfer to the greenhouse. This process was used to generate the clone that eventually created the event CTC75064-3.

Example 2. Molecular Characterization of Event CTC75064-3

2.1 DNA extraction.

Approximately 10 mg of leaf tissue from event CTC75064-3 was used. Genomic DNA extraction was performed on the BioSprint 96 Nucleic Acid Extractor (Quiagen, GER) with the BioSprint 96 DNA Plant Kit Extraction Kit (Quiagen, GER) according to the manufacturer's instructions. The DNA was normalized to a concentration of 10 ng/$\mu$L in a Multiskan GO spectrometer (Thermo Scientific, USA).

2.2 Determination of the number of transgene copies inserted into the host plant germplasm.

The copy number of *cry1Ac* and *nptII* genes inserted into CTC75064-3 event was initially evaluated by quantitative Taqman® PCR (qPCR/Taqman®), and the results were confirmed via Southern blot and/or sequencing.

The Taqman® real time PCR reactions were realized with 7500 Real-Time PCR System (Applied Biosystems, EUA) in the Fast mode. The primer pairs and probes used are shown at Table 03. As endogenous control of the *cry1Ac* and *nptII* reactions to confirm the presence and quality of the used DNA, as well as, effectiveness of the reaction, it was used the sugarcane polyubiquitin gene (forward primer: 5 'ACCATTACCCTGGAGGTTGAGA 3 ' (SEQ ID NO: 15); reverse primer: 5 'GTCCTGGATCTTCGCCTTCA 3' (SEQ ID NO: 16); probe: VIC -5 'CTCTGACACCATCGAC 3'-MGB (SEQ ID NO:17) in multiplex mode.

Table 03: Primers and probes (Taqman®) used to determine copy number via qPCR.

| Assay | ***Primers* / probes** | **Sequence 5' - 3'** | ***Amplicon* (pb)** |
|---|---|---|---|
| cry1Ac - 0 | pCTC001_Cry1Ac.F | GAGGTGCCAATCCACTTCCC<br>SEQ ID NO 40 | 82 |
| | pCTC001_Cry1Ac.R | GAGTTACCCCAGTTCACGTTGAG<br>SEQ ID NO 41 | |
| | Cry1Ac_probe | FAM-ACCTCCACCAGGTACAG-MGB<br>SEQ ID NO 42 | |
| cry1Ac - 1 | cry1AcF.1_SB | CATCCCATACAACTGCCTGAG<br>SEQ ID NO 43 | 103 |
| | cry1AcR.1_SB | CTGGGTCAAGGACAGGGAGAT<br>SEQ ID NO 44 | |
| | sondal cry1Ac_SB | CCGGTTACACCCCC<br>SEQ ID NO 45 | |
| cry1Ac - 2 | cry1AcF.2_SB | CTCTACCAAATCTACGCCGAGA<br>SEQ ID NO 46 | 96 |
| | cry1AcR.2_SB | CATGTCGTTGAATTGAATGCG<br>SEQ ID NO 47 | |
| | sonda2_cry1Ac_SB | TCTCCGCGAGGAAA<br>SEQ ID NO 48 | |
| cry1Ac - 3 | cry1AcF.3_SB | CACCGTGCTGGACATTGTGT<br>SEQ ID NO 49 | 77 |
| | cry1AcR.3_SB | TGAGTTGGGACACGGTGC<br>SEQ ID NO 50 | |
| | sonda3_cry1Ac_SB | CTCTTCCCGAACTACGACT<br>SEQ ID NO 51 | |
| cry 1Ac-4 | cry1AcF.4_SB | ACCCTGTCCTCCACCCTGTA<br>SEQ ID NO 52 | 107 |
| | cry1AcR.4_SB | AGGTTGGAGGAGGTGCCGTA<br>SEQ ID NO 53 | |
| | sonda4_cry1Ac_SB | CCTTCAACATCGGTATCA<br>SEQ ID NO 54 | |
| nptII - 0 | nptII.F | TGCCGAATATCATGGTGGAA<br>SEQ ID NO 55 | 55 |
| | nptII.R | CGGCCACAGTCGATGAATC<br>SEQ ID NO 56 | |
| | nptII.probe | FAM-TGGCCGCTTTTCT-MGB<br>SEQ ID NO 57 | |
| nptII - 1 | nptIIF.1_SB | ATGACTGGGCACAACAGACAATC<br>SEQ ID NO 58 | 99 |
| | nptIIR.1-SB | CGGACAGGTCGGTCTTGA<br>SEQ ID NO 59 | |
| | sondal_nptII_SB | CTGCTCTGATGCCGC<br>SEQ ID NO 60 | |

(continued)

| Assay | *Primers* / probes | Sequence 5' - 3' | *Amplicon* (pb) |
|---|---|---|---|
| nptII - 2 | nptIIF.2_SB | CTGCCGAGAAAGTATCCATCATG SEQ ID NO 61 | 93 |
| | nptIIR.2_SB | GATGTTTCGCTTGGTGGTCG SEQ ID NO 62 | |
| | sanda2_nptII_SB | CTGATGCAATGCGGCGGC SEQ ID NO 63 | |
| c-StaA (*backbone*) | c-StaA.F | GCCGCTTGTAGCCTTCCA SEQ ID NO 64 | 64 |
| | c-StaA.R | CCGCCGACCTGGTGGA SEQ ID NO 65 | |
| | c-StaA_BB_probe | FAM-CGTGACCTCAATGCG-MGB SEQ ID NO 66 | |
| aad-A (*backbone*) | aad-A_BB_F | CACAATCGTCACCTCAACCG SEQ ID NO 67 | 75 |
| | aad-A_BB_R | ATCAACGACCTTCTGGAAACG SEQ ID NO 68 | |
| | aad-A_BB_probe | NED-CGCAGGATTTCGCTCTCG-MGB SEQ ID NO 69 | |

The qPCR reactions used IX TaqMan® Fast PCR Master Mix II (Applied Biosystems, USA), 300 nM from each primer and 200 nM from the corresponding probes. The cycling used was: a 50 °C cycle for 2 minutes for uracil N-glycosylase activation, a 95 °C cycle for 20 seconds for DNA polymerase activation, 40 cycles of 95 °C for 3 seconds (denaturation), and 60 °C for 30 seconds (annealing and extension).

Data analysis was performed by manually entering the threshold at the exponential phase of the amplification curve. For *cry1Ac* and *nptII* genes, the copy number was inferred from DeltaCt (dCt) analysis, in which the Ct (cycle at which the fluorescence signal emitted by the amplification product reaches the threshold) of the endogenous gene is subtracted from the Ct of the target gene. In this type of analysis, the number of copies is assumed to double every Ct and the reference number of control copies of the same variety whose value is known is taken as a reference.

As a result, both assays pointed to the presence of 1 copy for the *cry1Ac* gene in the CTC75064-3 event genome. The same copy number (i.e., 1 copy) was detected for the *nptII* gene, the assay of which is based on detection of the gene promoter.

For the Southern blot, 10 μg of genomic DNA from the CTC75064-3 event were digested separately with distinct restriction enzymes (*Eco*RV, *Hind*III *and Shp*I)*,* following the cold probe tagging procedure (Digoxigenin - DIG). Briefly, genomic DNAs of the event CTC75064-3 and the parental variety RB 867515 were extracted using the *NucleoSpin® Plant II* kit in tubes, following the recommendations of the manufacturer (*Macherey-Nagel GmbH & Co. KG,* Germany). The quality of the extracted DNA was checked on a 1% agarose gel in TAE IX buffer (Tris-Acetate EDTA) and 10 μg of DNA were digested using 100 units of restriction enzyme (10 U/μg of gDNA) per sample, in a final volume of 400 μl of reaction.

The enzymatic reaction was carried out following the manufacturer's recommendations (Thermo Fisher, USA). The digestion product was precipitated with 2 volumes of ethanol and 10% 7.5 M ammonium acetate, and incubated for 48 h at -20 °C. The precipitated DNA was centrifuged at 14,000 x g for 30 min and the pellet formed was resuspended in 35 μl of milli-Q water until complete dissolution. The quality of digestion was visualized on a 1% agarose gel with IX TAE buffer (Tris-Acetate - EDTA). The *EcoRV* and *Hind*III enzymes were used to detect the cassettes containing the *cry1Ac* and *nptII*genes (Figure 23 A). *Sph*I enzyme was used to detect vector backbone (Figure 23 B).

Probes were design to detect *cry1Ac* gene (probe CrylAc - 1,079 bp), *nptII*gene (*nptII* probe 579 bp), and CaMV 35s promoter region (CaMV 35s promoter and the OsACT1 intron; 35S probe - 904 bp), presented in the CTC75064-3 event. The probes for detecting fragments of the vector (backbone) were designed throughout the vector, covering ~ 98% of the backbone (BB1 probe - 1,875 bp; BB2 probe -2,305 bp; BB3 probe -2,282 bp; Figure 23 B). The probes were prepared and used to detect targets using the *PCR DIG Probe Synthesis Kit* reagents (Roche, cat # 11636090910, Switzerland). About 100 pg of the linearized vector were used as a template for the amplification of the regions of interest and incorporation of the digoxigenin molecule (DIG) by PCR and its use as a probe. The primers used for the production of the probes, the hybridization temperatures (Thyb) and the expected size of each amplicon are listed in Table 04.

DNA from RB 867515 variety was used as a negative control. As a positive control, plasmid DNA containing the construction used to obtain event CTC75064-3was added to RB 867515 genomic DNA. Plasmid DNA was previously linearized with the restriction enzyme used in each assay.

Table 04. List of primers used in the synthesis of probes marked with DIG.

| Probes | Enzyme | Sequences 5'→ 3' | Target | Tm | Amplicon | Thyb |
|---|---|---|---|---|---|---|
| Cry | *Eco*RV/*Hind*III | CAACAACCCAAACATCAACG SEQ ID NO 105 | T-DNA | 58 °C | 1.079 bp | 50 °C |
| | | GGGTCCTGTAGACACCCTGA SEQ ID NO 106 | | | | |
| nptII | *Eco*RV/*Hind*III | GCTCACCCTGTTGTTTGGTGTT SEQ ID NO 107 | T-DNA | 58 °C | 679 bp | 50°C |
| | | CGGCCACAGTCGATGAATC SEQ ID NO 108 | | | | |
| 35S | *Eco*RV/*Hind*III | TGAAAAGAAAAACTACCGATGAA SEQ ID NO 109 | T-DNA | 58 °C | 904 bp | 50 °C |
| | | TGATGTGATGGTCCGATTGA SEQ ID NO 110 | | | | |
| *Backbone* 1 | *Sph*I | TCCCCTCGGGATCAAAGTA SEQ ID NO 111 | Vector | 57 °C | 1.875 bp | 50 °C |
| | | TAGCGTGTTTGTGCTTTTGC SEQ ID NO 112 | | | | |
| *Backbone* 2 | *Sph*I | GGCCGCTGAAATTAAATCAA SEQ ID NO 113 | Vector | 58 °C | 2.305 bp | 50 °C |
| | | GAGTCAGTGAGCGAGGAAGC SEQ ID NO 114 | | | | |
| *Backbone* 3 | *Sph*I | CGGTGAAAACCTCTGACACA SEQ ID NO 115 | Vector | 57 °C | 2.282bp | 50 °C |
| | | ATACAGGCAGCCCATCAGTC SEQ ID NO 116 | | | | |

The transfer of the digested DNA to the *nylon* membrane was carried out according to well-established protocols. Briefly, electrophoresis of the digested DNA in 1% agarose gel with TAE IX buffer was performed at 40 V for approximately 20 hours, using *SYBR Safe DNA gel stain* (Invitrogen # S33102, USA) as an intercalant agent. Then, the agarose gel was treated with four different solutions in the following order:

- Depurination solution (1.1% HCL); 10-15 min;
- Denaturation solution (0.5 N NaOH; 1.5 M NaCl); 30-40 min;
- Neutralization solution (1.5 M NaCl; 0.5 M Tris); 30-40 min;
- SSC 20x (Saline Sodium Citrate); 20 min

The DNA transfer to the *nylon* membrane was performed using *TurboBlotter Transfer System* 20 x 25 (Sigma # WHA10416324, USA) according manufacturer instructions. The membrane with the transferred DNA samples was placed in the *UV Crosslinker* equipment (UVP- Analytik-Jena, Germany) for DNA fixation (2 cycles of 700 x 100 (μJ/$cm^2$).

Prehybridization and hybridization were then performed.

The Prehybridization treatment consists of incubation of the membrane with *DIG Easy Hyb* solution (Roche # 11603558001, Switzerland) at40 °C for 3 hours, with denatured salmon sperm DNA in the final concentration of 100 $\mu g.mL^{-1}$ and constant rotation (0.5 x g). After, Hybridization was performed with the same Pre-hybridization solution *(DIG Easy Hyb* with salmon sperm DNA) added to the probe marked with DIG and denatured. The concentration of the probes used was 65 $\mu g.mL^{-1}$. The hybridization temperature was 50 °C.

Hybridization was carried out in a hybridization oven for approximately 16 hours with constant rotation (0.5 x g). The hybridized membranes were washed and blocked. After the blocking solution was discarded and the membrane was covered with a new blocking solution with the *Anti-Digoxigenin AP fragments* (Roche # 11093274910, Switzerland) diluted in a 1:20,000 ratio. The membrane was then incubated for 30 min at room temperature and slightly shaken. The antibody solution was discarded, and the membrane was washed and incubated with the *Detection buffer* (Roche # 11585762001, Switzerland) plus *CDP-Star ready to use* (Roche # 12041677001, Switzerland) for a final concentration of IX according to manufacturer instructions. A photographic film was added to detect chemiluminescence (Roche # 11666657001, Switzerland) in contact with the membrane for at least 16 hours and then immersed 1x Developing Solution (Kodak # 1900943, USA) for a visualization of the bands. The 1x Fixing Solution (Kodak # 1901875, USA) was used to fix the band images detected.

In hybridization with the Cry1Ac and 35S probe, for the materials digested with *Hind*III, a single band with about 8.5 kb was observed for all samples from CTC75064-3 event (Figure 24 left; Figure 25 right). For the same probes, the samples digested with *Eco*RV present a fragment with about 2.79 kb in all samples CTC75064-3 event (Figure 24, right; Figure 25 left). For hybridization with the *nptII* probe, in the materials digested with *Hind*III a fragment with the expected size of ~ 3.5 kb was observed for all samples from CTC75064-3 event (Figure 26 left). For the *Eco*RV enzyme, a fragment> 12 kb was observed (Figure 26 right).

The results of the Southern blot, using the enzymes *Eco*RV and *Hind*III in combination with the probes that recognize the *cry1Ac, nptII* genes and 35s promoter, confirm the results found using qPCR, which indicate that the CTC75064-3 event has only one integration of T-DNA with one copy of the *cry1Ac* gene and one copy of the *nptII* gene in the genome. In addition, the presence of the same fragments in all generations of the CTC75064-3 event from samples collected in consecutive vegetative generations (T0 - T3; Figures 24, 25 and 26), confirm the genetic stability of the insertion in the event over 4 generations of vegetative multiplication. Sequences of the vector backbone was not detected in the event CTC75064-3 (Figure 27).

2.3 Definition of flanking sequences.

To isolate the flanking regions at the ends of the T-DNA insert present in event CTC75064-3, several DNA sequencing experiments were performed. The map of the genetic insertion of event CTC75064-3 generated from the data of these experiments is shown in FIGURE 3.

Inverse PCR (iPCR) assays were performed for both ends of the T-DNA to isolate and clone the flanking regions of the insert. The iPCR methodology is based on genomic DNA digestion using enzymes that cleave the T-DNA sequence and a random event genome sequence. The cleavage products are circularized and subjected to multiple nested PCR cycles using primers for known T-DNA regions (Table 05). The isolated fragments were then isolated, cloned and sequenced by Sanger methodology. Finally, a consensus sequence of the flanking regions was assembled (SEQ ID NO: 23 and SEQ ID NO: 24).

Table 05. Restriction enzyme and primer nucleotide sequences used to perform iPCR (inverse PCR) to identify flanking regions.

| T-DNA Border | Restriction Enzyme | Reaction | Oligonucleotide sequence |
|---|---|---|---|
| Left border | *PaeI* | Nested PCR1 | 5' - ACGGAACCGTTGAAGAGGAA - 3' - - SEQ ID NO 70<br>5' - ATGTGTGAGTAGTTCCCAGATAAG - 3' - SEQ ID NO 71 |
| | | Nested PCR2 | 5' - GATCCAGGAGAACATCGGAG - 3' - SEQ ID NO 72<br>5' - CTATAGGGTTTCGCTCATGTGTTG - 3' - SEQ ID NO 73 |

(continued)

| T-DNA Border | Restriction Enzyme | Reaction | Oligonucleotide sequence |
|---|---|---|---|
| Right border | *Bgl II* | Nested PCR1 | 5' - AATTATACATTTAATACGCG - 3' - SEQ ID NO 74<br>5' - CGCGGTGTCATCTATGTTAC - 3' - SEQ ID NO 75 |
| | | Nested PCR2 | 5' - CGCGGTGTCATCTATGTTAC - 3' - SEQ ID NO75<br>5' - TCAAGAAGGCGATAGAAGGCGATG - 3' - SEQ ID NO 76 |
| Right border | *pstI* | Nested PCR1 | 5' - AATTATACATTTAATACGCG - 3' - SEQ ID NO 74<br>5' - AATAACGTCATGCATTACATG - 3' - SEQ ID NO 77 |
| | | Nested PCR2 | 5' - CGCGGTGTCATCTATGTTAC - 3' - SEQ ID NO 75<br>5' - GATAATCATCGCAAGACCGG - 3' - SEQ ID NO 78 |
| | | Nested PCR3 | 5' - TCGTCGACTCTAGACTCGAG - 3'- SEQ ID NO 79<br>5' - GATAATCATCGCAAGACCGG - 3' - SEQ ID NO 78 |
| Right border | *EcoRV* | Nested PCR1 | 5' - AATTATACATTTAATACGCG - 3' - SEQ ID NO 74<br>5' - AATAACGTCATGCATTACATG - 3' - SEQ ID NO 77 |
| | | Nested PCR2 | 5' - CGCGGTGTCATCTATGTTAC - 3' - SEQ ID NO 75<br>5' - GATAATCATCGCAAGACCGG - 3' - SEQ ID NO 78 |

| Cycle Number | Denaturation | Ringing | Extension |
|---|---|---|---|
| **1st** | 94 °C, 5 min | - | - |
| **2-36th** | 94 °C, 30 sec | 50 °C 45 sec | 72 °C, 3 min |
| **37th** | - | - | 72 °C, 7 min |

In parallel, as there is currently no fully sequenced genome that could be used as a reference for RB 867515 germplasm, a capture sequencing methodology was adopted as an additional effort to isolate the T-DNA inserted into the event CTC75064-3 and its flanking regions. In this strategy, small overlapping polynucleotide fragments (probes) were developed to cover the entire T-DNA sequence. These probes were hybridized to the fractionated genomic DNA of both RB 867515 and CTC75064-3 cultivars, and hybrid sequences were isolated. Isolated fragments were then sequenced using Illumina® technology according to standard protocol. The data obtained were aligned with the T-DNA sequence present in the transformation vector and, together with the iPCR data mentioned above, the complete T-DNA consensus sequence (SEQ ID NO: 2) of the CTC75064-3 event and its flanking sequences (SEQ ID NO: 22, SEQ ID NO: 5, SEQ ID NO: 23, SEQ ID NO: 24) were obtained.

2.4 Method for the detection and characterization of event CTC75064-3 (event-specific assay)

For the validation of the methodology, both non-GMO (WT) plants and other genetically-modified events having the same construct were used as experimental controls. DNA extraction occurred as described above.

Real-time PCR assays for identification of the CTC75064-3 event were designed and validated based upon the molecular characterization of the T-DNA insertion genomic flanking sequences. For the development of specific detection methodology, the real-time PCR (qPCR) technique known as "Plus-Minus" or "Presence - Absence" was chosen, validating the two variations of the methodology: via SYBR GREEN™ and via Taqman® technology. Specific primer pairs have been designed to generate information about the insertion of T-DNA in both methodologies, such that one primer binds in the construct and the second primer binds in the host genome. For the use of Taqman® technology, specific probes were designed between the primers.

In a preferred embodiment, the primers designed are the primers as defined on the SEQ ID Nos: 6 to 9, wherein the primer of SEQ ID NO: 6 is a forward primer of the right border, the primer of SEQ ID NO: 7 is a reverse primer of the right border, the primer of SEQ ID NO: 8 is a forward primer of the left border and the primer of SEQ ID NO: 9 is a reverse primer of the left border (Table 06).

In a preferred embodiment, the probe employed in Taqman® PCR technology consists of SEQ ID NO: 10 in the RB border and/or SEQ ID NO: 11 in the LB border.

Taqman® real-time PCR reactions were performed using the 7500 Real-Time PCR System (Applied Biosystems, USA) in its Fast mode.

The sugarcane poly-ubiquitin gene (endogenous gene) was used as an internal reaction control to confirm the presence and quality of the DNA used. The following reagents were multiplexed with the assay developed for the event: forward primer (SEQ ID NO: 15); reverse primer (SEQ ID NO: 16); probe (SEQ ID NO: 17).

Table 06. List of primers and probes used in Real-time PCR assays.

| | Primer/Probe | Oligonucleotide sequence | Amplicon |
|---|---|---|---|
| **Detection of left board (LB)** | Forward<br>Probe LB<br>Reverse | CATGACCAAAATTGCTAATATTTCAC - SEQ ID NO 08<br>FAM-CTTAATAACACATTGCGGACGT-MGB - SEQ ID NO 11<br>AATCCAGTACTAAAATCCAGATCCC - SEQ ID NO 09 | 135 bp |
| **Detection of right board (RB)** | Forward<br>Probe RB<br>Reverse | CAAGGGCGAATTCCAGCAC - SEQ ID NO 06<br>FAM-CCGTTACTAGTGGATCGA-MGB-SEQ ID NO 10<br>GTTCTTGTCAGTTTTCTGTCCAAA - SEQ ID NO 07 | 107 bp |
| **Endogenous control (pUBI)** | Forward<br>Probe RB<br>Reverse | ACCATTACCCTGGAGGTTGAGA- SEQ ID NO 15<br>VIC - CTCTGACACCATCGAC - MGB-SEQ ID NO 17<br>GTCCTGGATCTTCGCCTTCA - SEQ ID NO 16 | |

qPCR reactions used IX TaqMan® Fast PCR Master Mix II (Applied Biosystems, USA), 150nM from each event-specific primer and 100nM from the corresponding probe, 300nM from the primers for the endogenous poly-ubiquitin gene and 200nM of its probe, 100-200 ng of DNA and enough water to complete the 20 μL volume. The following PCR program was used: a 50 °C cycle for 2 minutes for uracil N-glycosylase activation, a 95 °C cycle for 20 seconds for DNA polymerase activation, 40 cycles of 95 ° C for 3 seconds (denaturation) and 60 °C for 30 seconds (annealing and extension).

qPCR reactions using SYBR GREEN™ were also performed using the 7500 Real-Time PCR System (Applied Biosystems, USA) in its standard mode for this type of assay and QuantStudio 6 Flex da Applied biosystems. Reactions were performed using 1 X QuantiFast SYBR Green™ PCR Kit (QIAGEN ™), 150 nM forward primer and 150 nM reverse primer, 20 ng DNA and sufficient water for a final volume of 25 μL. The reactions were performed using the event of the invention, the other events transformed with the same construct as the event of the invention (negative controls), wild type sugarcane (WT; non transformed) samples, and experimental controls (extraction and reaction blank).

The following PCR program was used: a DNA denaturation cycle at 95°C for 5 minutes, 35 cycles for primer annealing cycles and amplification at 95°C for 15 seconds and 60°C for 1 minute and a dissociation cycle for generation melting peak (95°C for 15 sec, 60°C for 1 min, 95°C for 15 sec). The reaction with SYBR safe does not allow for the use of multiplex; therefore, it is necessary to prepare a separate endogenous gene amplification reaction, using the same DNA, to eliminate false negatives.

As a result, it was possible to validate the event-specific detection reaction of the invention via high-accuracy Taqman®, as illustrated in Figure 6. Taqman® technology probes specifically bind to DNA and are released during DNA amplification, thus generating the fluorescence signal captured by the equipment during this process.

Samples corresponding to the event of the invention showed specific amplification: having well-defined amplification curve formation and characteristic sigmoidal shape; whereas samples from other events, WT, and extraction and reaction blanks did not show event-specific amplification. As expected, the endogenous control presented amplification for all samples except extraction and reaction blanks, demonstrating both the quality of the DNA used in the reaction, as well as the quality of the reaction and cycling.

The SYBR GREEN™ assay showed specific amplification of the event samples at the expected melting temperature. Samples of the other events, as well as WT and blanks did not peak at this temperature (Figure 7).

Example 3. Event CTC75064-3 Generation - Genome editing (GE)

The event of the invention, CTC75064-3, is generated using a genome editing (GE) approach, thus recreating the event generated using the preferred *Agrobacterium-mediated* transformation methods described herein.

In this way, the event CTC75064-3 is recreated with the insertion of the *cry1Ac* gene into the same location of the genome as the CTC75064-3 event. The *cry1Ac* gene expression is regulated by a promoter or a promoter region and a terminator capable to drive the CrylAc protein expression at levels sufficient to control infestation of the target pest. Additionally, a marker gene or selection system is also inserted (transiently or stably) to enable event selection. Preferably, the T-DNA of the claimed invention (SEQ ID NO: 2) is inserted into the same location of the genome as the CTC75064-3 event. Thus, the event CTC75064-3 is recreated with the insertion of the *cry1Ac* gene, which expresses a toxin to control *D. saccharalis,* and the *nptII* gene. The expression of the *cry1Ac* is regulated by a promoter region comprising doubled enhanced promoter CaMV35s, wheat leader sequence L-Cab, OsACT1 intron and Kosak sequence 5' upstream from the translational start site. The expression of *nptII* genes is regulated by the maize ubiquitin gene promoter UBI-1, which has an endogenous intron. The *cry1Ac* expression cassette has CaMV35s terminator (T35s) and *nptII* expression cassette uses the *Agrobacterium tumefaciens* nopaline synthase (nos) terminator.

In the case that the aforementioned genome editing approaches to generating event CTC75064-3 result in low-efficiency integration of the T-DNA at the target site, developmental genes or other regulatory elements could be delivered in conjunction with the GE reagents in order to improve the integration efficiency.

3.1 Constructs development.

Conventional gene cloning techniques using commercial plasmids, restriction enzyme digestion, fragment ligation (with ligases) and other known methodologies are used to develop the constructs (plasmids) of the present invention.

The GE reagents can be delivered on multiple plasmids, each one comprised of an element of the enzymatic complex (endonuclease, crRNA or guide RNA, and the homologous recombination (HR) template; Figure 19).

In one embodiment, the HR template constructs comprises the T-DNA (SEQ ID NO: 2) of the claimed invention flanked by DNA homologous to the flanking sequences described for CTC75064-3 (SEQ ID Nos: 23 and 24) located on either side of the T-DNA. In a preferred embodiment, the HR template constructs comprises the SEQ ID NO: 26 (Figure 21). The invention also comprises a second construct comprising an endonuclease expression cassette. In a preferred embodiment, the endonuclease expression cassette comprises a Cas9 endonuclease sequence. In more preferred embodiment the Cas9 sequence is codon optimized for sugarcane expression. In one embodiment, the Cas 9 construct comprises additionally the guide/ crRNA sequence. Preferably, the Cas 9 construct comprises the crRNA sequence SEQ ID NO: 28. In a more specific embodiment, the Cas 9 construct comprises the SEQ ID NO: 27 (Figure 20). A third construct comprising the guide RNA expression cassette alone is also contemplated in the present invention and comprises SEQ ID NO: 28.

In one additional embodiment the genome editing construct comprises HR template, the nuclease and the guide RNA expression cassettes, delivering all the GE reagents in a single construct. In one embodiment, the single construct comprises the T-DNA (SEQ ID NO: 2) of the claimed invention flanked by DNA homologous to the flanking sequences described for CTC75064-3 (SEQ ID Nos: 23 and 24) located on either side of the T-DNA. In a preferred embodiment, the construct comprises the SEQ ID NO: 25 (Figure 22).

Optionally, the constructs also comprise fluorescent/selection markers and/or other genetic engineering systems to remove marker genes and/or nucleases cassettes, such as a Cre/loxP recombination system from the bacteriophage PI. In this case, the marker/nuclease gene cassette, which should be deleted, is flanked by the loxP regions, while Cre recombinase removes this fragment during a transient expression.

3.2 Direct Delivery

In one embodiment, the event of the invention is generated using methodologies for direct delivery of proteins, RNA or plasmids. The methodologies for direct delivery are selected from the group consisting of particle bombardment, electroporation, lipofection and protoplast transfection; however, other delivery methodologies known to those of skill in the art could also be utilized.

3.2.1 Direct delivery - RNP approach

In one embodiment, the event of the invention is generated using ribonucleoprotein (RNP) delivery. The preferred methodologies for RNP delivery are selected of the group consisting of particle bombardment of sugarcane calli and protoplast transfection. Moreover, other sugarcane cell types can also be used for transformation including (but not limited to): leaf disc, meristem, and calli-derived suspension cells.

Using the RNP approach to genome editing, the endonuclease and crRNA or guide RNA are delivered in RNP form, separate from the HR template, which is delivered via plasmid.

The guide RNA may be preliminarily produced by in vitro transcription or be chemically synthesized as ribooligonucleotide, while the corresponding nuclease may be produced in vivo with further purification (bacterial expression) or purchased from any manufacturer of such products. In a preferred embodiment, the guide RNA comprises SEQ ID NO: 28. In another embodiment the nuclease is Cas9 nuclease.

A ready ribonucleoprotein (RNP) complex consisting of the corresponding nuclease and guide RNA, and the HR template plasmid are sorbed on golden particles and direct delivery to the cells or tissue.

3.2.2 Direct delivery - Plasmid

In another embodiment of the invention, the event of the invention is generated using plasmid delivery, wherein the GE reagents will be expressed in a transient manner, thus achieving the site-directed integration of CTC75064-3 without the integration of additional transgenes associated with the GE approach.

Methodologies for plasmid delivery is selected from the group consisting of particle bombardment (biolistic) of sugarcane calli or polyethylene glycol transformation of protoplasts; however, other methodologies known to those of skill in the art could also be utilized. Moreover, other sugarcane cell types can also be used for transformation including (but not limited to): protoplast, leaf disc, meristem, and calli-derived suspension cells.

In yet another embodiment of the invention, the event of the invention is generated using plasmid delivery, where the GE reagents are stably expressed, thus requiring the excision of the integrated GE reagents and selectable marker using, for example, a Cre/Lox system. Using this approach, LoxP sites will remain in the genome of the event CTC75064-3 plant. Other DNA excision approaches known to those skilled in the art may also be used to remove the GE reagent DNA from the genome of the event CTC75064-3 plant.

3.3 Indirect Delivery

In one embodiment, the event of the invention is generated using methodologies for indirect delivery of plasmids, *as_Agrobacterium* transformation. *Agrobacterium tumefaciens* and *Agrobacterium rhizogenes* can be used. Plant viruses also can be used for indirect delivery of plasmids to plant cells and tissues. For example, genetically modified plant geminiviruses make it possible to achieve higher transformation efficiency, specially without stable insertion into a genome. Moreover, different tissue and cell types can also be used for transformation including (but not limited to): calli, protoplast, leaf disc, meristem, and calli-derived suspension cells.

In a preferred embodiment the *Agrobacterium* transformation is performed as describe at Example 1.

In another preferred embodiment, the event of the invention is generated using plasmid delivery by *Agrobacterium* transformation, wherein the GE reagents will be expressed in a transient manner, thus achieving the site-directed integration of CTC75064-3 without the integration of additional transgenes associated with the GE approach.

The GE reagents can be delivered on multiple plasmids, but preferably on a single plasmid. In a preferred embodiment, the constructs is SEQ ID NO: 25 and comprises a selectable marker, a nuclease, crRNA or guide RNA, and homologous recombination (HR) template (Figure 22). The HR template comprises T-DNA (SEQ ID NO: 2) of the claimed invention flanked by the DNA homologous to the flanking sequences described for CTC75064-3 (SEQ ID Nos: 23 and 24) located on either side of the expression cassettes.

In a preferred embodiment, the event of the invention is generated using plasmid delivery, where the GE reagents are stably expressed, thus requiring the excision of the integrated GE reagents and selectable marker using, for example, a Cre/Lox system. Using this approach, LoxP sites will remain in the genome of the event CTC75064-3 plant. Other DNA excision approaches known to those skilled in the art may also be used to remove the GE reagent DNA from the genome of the event CTC75064-3 plant.

With all the transformation processes described above, the resulting transformed cells will be regenerated to form a plant containing the event of the invention.

3.4 Molecular characterization.

The event CTC75064-3, generated using genome editing, is evaluated for accurate insertion of the T-DNA into the target site of the genome using the primers of the invention (SEQ ID NO: 6-9) as is described in the specification herein.

Additionally, pair of primers designed to amplify the outermost sequences in the flanking sequences of event CTC75064-3 are validated and could be used to evaluate the integrity of the recombination site (Table 07, Figure 28).

Table 07. Pair of primers designed for Flanking sequences of CTC75064-3.

| Position | Name | ***Primer Forward 5’→ 3***<br>***Primer Reverse* 5 → 3** | **Amplicon (bp)** |
|---|---|---|---|
| 5’ Flanking region | 75_064 FSLB 1 FW 1<br>T35S Ter RV 2 | AACTTTATTCTAGGATAAAGCTATGT -SEQ ID NO 80<br>CAGATAAGGGAATTAGGGTTCCTAT-SEQ ID NO 81 | 683 |
| | 75_064 FSLB 2 FW 1<br>T35S Ter RV 2 | ACATACAACATCACAATCAAGCAGT-SEQ ID NO 82<br>CAGATAAGGGAATTAGGGTTCCTAT-SEQ ID NO 81 | 576 |
| | 75_064 FSLB 3 FW 1<br>T35S Ter RV 2 | CTCAAAACAACGCTAGATACGGTAG-SEQ ID NO 84<br>CAGATAAGGGAATTAGGGTTCCTAT-SEQ ID NO 81 | 448 |
| 5’ Flanking region + T-DNA | 75_064 FSLB 1 FW 1<br>ZmUBI RV 2 | AACTTTATTCTAGGATAAAGCTATGT-SEQ ID NO 80<br>AGAGTCCTGTTGTCAAAATACTCAA-SEQ ID NO 83 | 4079 |
| | 75_064 FSLB 2 FW 1<br>ZmUBI RV 2 | ACATACAACATCACAATCAAGCAGT-SEQ ID NO 82<br>AGAGTCCTGTTGTCAAAATACTCAA-SEQ ID NO 83 | 3988 |
| | 75_064 FSLB 3 FW 1<br>ZmUBI RV 2 | CTCAAAACAACGCTAGATACGGTAG-SEQ ID NO 84<br>AGAGTCCTGTTGTCAAAATACTCAA-SEQ ID NO 83 | 3860 |
| 3’ Flanking region | 75_064 FSRB 1 RV 1<br>NOS FW1 | GCATCTTCATGATTGTTGCCTTATG-SEQ ID NO 85<br>ATGATTAGAGTCCCGCAATTATACA-SEQ ID NO 86 | 807 |
| | 75_064 FSRB 2 RV 2<br>NOS FW1 | CTTGAATAGGAAATGCTTGAGAAAA-SEQ ID NO 87<br>ATGATTAGAGTCCCGCAATTATACA-SEQ ID NO 86 | 674 |
| | 75_064 FSRB 3 RV 1<br>NOS FW1 | GATCTGAATGTTTGGTTTGTTTGAC-SEQ ID NO 89<br>ATGATTAGAGTCCCGCAATTATACA-SEQ ID NO 86 | 548 |
| | 75_064 FSRB 4 RV 1<br>NOS FW1 | GTTTGTCCTTGTTAGCCGTAGAAT-SEQ ID NO 90<br>ATGATTAGAGTCCCGCAATTATACA-SEQ ID NO 86 | 350 |
| 3’ Flanking region + T-DNA | 75_064 FSRB 1 RV 1<br>CAB FW1 | GCATCTTCATGATTGTTGCCTTATG-SEQ ID NO 85<br>CTTGAGTGTGTGGAAGATGGTTCTA-SEQ ID NO 88 | 4454 |
| | 75_064 FSRB 2 RV 2<br>CAB FW1 | CTTGAATAGGAAATGCTTGAGAAAA-SEQ ID NO 87<br>CTTGAGTGTGTGGAAGATGGTTCTA-SEQ ID NO 88 | 4321 |
| | 75_064 FSRB 3 RV 1<br>CAB FW1 | GATCTGAATGTTTGGTTTGTTTGAC-SEQ ID NO 89<br>CTTGAGTGTGTGGAAGATGGTTCTA-SEQ ID NO 88 | 4195 |
| | 75_064 FSRB 4 RV 1<br>CAB FW1 | GTTTGTCCTTGTTAGCCGTAGAAT-SEQ ID NO 90<br>CTTGAGTGTGTGGAAGATGGTTCTA-SEQ ID NO 88 | 3997 |

PCR reactions use 0.2 µM of primers (Table 7), 20ng of DNA, IX Dream Taq- Thermo Fisher buffer (Thermo Fisher Scientific Inc - USA), Taq polymerase (final volume 20 µL). Reaction conditions are: one cycle at 94°C for 1 min;

35 cycles at 94°C for 30 seconds, 65°C for 45 seconds and 72°C for 3 min; and one cycle of extension at 72°C for 7 minutes. Amplicons with more than 2kb, the reactions are performed with 1U de Takara LA Taq enzyme - Takara (Takara Bio Inc-USA), 1 X LA PCR Buffer (Takara Bio Inc - USA), 2.5 mM MgCl2, 2.5mM dNTP, 0.5 $\mu$M of primers and 200 ng of DNA (final volume 50 $\mu$L).

Amplicons generated by PCR reactions with a combination of "T-DNA" and "Flanking regions" primers from Table 07, could be further sequencing by Sanger, using the pair of primers described at Table 08 (pair of primers designed to anneal to the T-DNA sequence of CTC75064-3 event) to confirm integrity of the insert.

Table 08. Pair of primers designed to T-DNA of the event CTC75064-3.

| *Primers* | **Sequence (5' → 3')** | **Element** |
|---|---|---|
| CLNG P04 FW | TGATGCATATACAGAGATGCTTTTT - SEQ ID NO 91 | UBI |
| CLNG P01 FW | TTCATCCATTTTATTAGTACATCCA - SEQ ID NO 92 | UBI |
| CLNG P03 FW | ACGGATGCGACCTGTACG - SEQ ID NO 93 | UBI |
| CLNG P07 RV | TCAGTAAAACCCACATCAAC - SEQ ID NO 94 | UBI |
| CLNG P08 RV | GACCACATCATCACAACCAAG - SEQ ID NO 95 | UBI |
| CLNG P09 RV | GCTCCGAACAACACGAGGTTG - SEQ ID NO 96 | UBI |
| CLNG P10 RV | ATGAAGTATTATATAGGTGAAG - SEQ ID NO 97 | UBI |
| CLNG P19 FW | AGAGGCTATTCGGCTATGAC - SEQ ID NO 98 | nptII |
| CLNG P20 FW | CAGCCGAACTGTTCGCCAGG - SEQ ID NO 99 | nptII |
| CLNG P21 RV | AGCACGAGGAAGCGGTCAGC - SEQ ID NO 100 | nptII |
| CLNG P22 RV | TGAGATGACAGGAGATCCTG - SEQ ID NO 101 | nptII |
| CLNG P65 FW | CAACAGCTCCGTGAGCATCATC - SEQ ID NO 102 | Cry1Ac |
| CLNG P66 FW | CCAGCGACTTCGGTTACTTC - SEQ ID NO 103 | Cry1Ac |
| CLNG P70 RV | CTCTCGGCGTAGATTTGGTAG - SEQ ID NO 104 | Cry1Ac |

Example 4. Evaluation of the gene expression product inserted in the event of the invention.

The gene expression product in the event of the present invention has been characterized in detail using ELISA and Western blot to determine the concentration of CrylAc and NptII proteins and to confirm the identities of these heterologous proteins.

**(ELISA) Enzyme-Linked Immunosorbent Assay**

To evaluate *crylAc* and *nptII* gene expression via ELISA, different sugarcane tissues were studied at different stages of crop development. To produce tissue samples of the event of the invention and the parental control, the experimental tests conducted was AGRO/PHENO.

"AGRO/PHENO" trials were conducted at six representative sites of the parental control cultivation area, three in the state of São Paulo (Barrinha, Piracicaba and Valparaiso), one in the state of Goiás (Quirinópolis), one in the state of Bahia (Juazeiro), and on in the state of Paraná (Mandaguaçu) (Table 09). The experiments were conducted in a randomized complete block design with 4 replications. The plots were composed of four 8 meters rows and the spacing between rows was 1.5 meter.

Table 09. *Assay* information used for sample collection for analysis of *cry1Ac* and *nptII* gene expression produced by the event of the invention. (Number of Days After Planting (DAP) represents time of sample collection for analysis.)

| Assay Type | Location | Tissue | DAP |
|---|---|---|---|
| | Barrinha-SP | leaf | 100, 200, 300,330 |
| | | stalk | 330 |
| | | root | 330 |

(continued)

| Assay Type | Location | Tissue | DAP |
|---|---|---|---|
| AGRO/PHENO | Piracicaba -SP | leaf<br>stalk<br>root | 100, 200, 300,330<br>330<br>330 |
| | Valparaiso-SP | leaf<br>stalk<br>root | 100, 200, 300,330<br>330<br>330 |
| | Quirinópolis-GO | leaf<br>stalk<br>root | 100, 200, 300,330<br>330<br>330 |
| | Juazeiro (JZ) | leaf<br>stalk<br>root | 100, 200, 300,210<br>210<br>210 |
| | Mandaguaçu (MG) | leaf<br>stalk<br>root | 100, 200, 300,330<br>330<br>330 |

The expression analysis of CrylAc and NptII proteins produced by the event CTC75064-3 was investigated at different periods of sugarcane plant development. The conditions evaluated were:

- Expression of heterologous proteins in leaves over a cultivation cycle of the event of CTC75064-3 (100, 200 and 300 DAP);
- Expression of heterologous proteins in leaves, stalk and roots at 330 DAP of a first harvest sugarcane. In Juazeiro at 210 DAP.

Leaf samples were collected on experimental treatment plots (CTC75064-3 and parental control - RB 867515) in the AGROPHENO assays at 100, 200, 300 and 330 DAP. Stalk and root samples were collected only at 330 DAP. 330 DAP samples were collected at 210 DAP in Juazeiro. After collection, the samples were sent for ELISA analysis to determine Cry1Ac and NptII protein expression levels.

Leaf Samples: 30 cm of tissue were collected from the tip of 5 to 10 "diagnostic" leaves on zigzag lines 2 and 3 avoiding diseased leaves. After removal of the central rib, the leaves were chopped into pieces, homogenized and packed in previously identified ziplock bags.

Stalk Samples: 10 whole sugarcanes were collected. After removing the dried leaves and pointers, the canes were cut into small tails, homogenized, and packed into labelled packages.

Root Samples: A representative clump from rows 2 and 3 of the experimental plot was collected. The soil was crushed, and the roots were washed with clean water to remove excess soil. The clean roots were then minced into pieces, homogenized, and packaged into labelled plastic bags.

All samples (from leaf, stalk, and root tissues) were transferred to dry ice in a Styrofoam box within 15 min of sampling. The genetic identity of all clumps sampled was confirmed by event-specific assay as described above.

For the analysis of the Cry1Ac protein 30 $\pm$ 1 mg of leaf, 200$\pm$ 1 mg of stalks, and 20 $\pm$ 1 mg of root tissue were macerated using TissueLyser equipment. To the macerated leaf tissue was added 750 $\mu$L saline phosphate extraction buffer (PBS) supplemented with Tween 20 (0.138 M NaCl; 0.027 mM KCl; 0.05% Tween 20, pH 7.4) diluted according to manufacturer's instructions (Envirologix ™, USA). For stalk 375 $\mu$L of the same buffer was used and for root samples, 1,500 $\mu$L were added.

For the analysis of the NptII protein in leaves, 40 $\pm$ 1 mg were weighted and macerated and 200 $\pm$ 1 mg were weighted for stalk and root samples and macerated. To the macerated leaf tissue samples, 750 $\mu$L of the extraction buffer were added; while in the stalk and root samples, 1,500 $\mu$L of the same buffer were added. The extraction buffer used in this case was PEB1 1x (pH 7.0), and the dilution was made according to manufacturer's instruction (PathoScreen® Kit for NPTII,Agdia, USA).

After buffer addition, vortex homogenization was performed and then centrifugation for 20 minutes at maximum speed. The resulting supernatant was collected, and total protein was quantified using the Bradford assay (CrylAc) and BCA (NptII).

The standards used for obtaining the calibration curve were the already-diluted commercial BSA (Bovine Serum Albumin) standards supplied with the kit described above. The 2000, 1000, 500, 250, 125, and 0 μg/mL calibrators (prepared in PBST buffer) were used. 10 μL of each standard calibrator was added in triplicate to plate wells. In total, 6 curves were generated from independent dilutions. For the samples, 10μL of the 3 individual protein extractions were used in each well. Then 200μL of Coomassie Plus Reagent Solution was added to each well containing the calibrators and samples. The plates were covered and incubated for 5 minutes at room temperature. Absorbance was read at 595 nanometers (nm) using SoftmaxPro 7.0 software (Molecular Device, US).

Total proteins were obtained in triplicate for each sample studied. After the total protein quantification of each replicate, the sample with the smallest variation of the median quantification value was chosen for ELISA analysis. After quantification of total proteins, leaf samples were diluted as follows: leaf samples for CrylAc analysis were diluted 2,500x, except by 100DAP samples that were 3,500x diluted; stalk samples for CrylAc analysis were diluted 2,500x; while root samples for CrylAc analysis were diluted 200x. All samples (leaf, root and stalk) for NptII analysis were diluted 8x..

Results were obtained by 96-well plate spectrometry reading at two different wavelengths: 450 nm and 630 nm on a SpectraMax Plate reader (Molecular Devices, USA). For CrylAc the Envirologix AP003 CRBS kit is used for protein detection and quantification. For NptII the *PathoScreen® Kit for NPT II* (Agdia, USA) kit was used. In all cases, the manufacturers recommendations were followed.

The analysis was based on the association of the absorbance values of the test samples with the predicted values in an equation estimated by measuring the absorbance of a standard curve. Synthetic proteins were diluted to desired concentrations in PBST buffer. Analyzes were performed in experimental duplicate for each sample. CrylAc and NptII protein concentrations were presented based on Total Protein (μg/mg), Fresh Tissue (μg/g) and Dry Tissue (μg/g).

The results of the statistical analyzes for the expression of the proteins CrylAc and *nptII* are found in Tables 10 and 11, respectively. The protein expression data were obtained from the average of four biological replicates (experimental plots) in duplicate. Table 10 presents the results of the individual and Combined analyzes by location for CrylAc protein expression data, in sugarcane leaves collected over a year of cultivation (100, 200 and 300 DAP). Table 11 shows the results of the analyzes for the NptII protein, under the same conditions.

Table 10. Comparison of the means of expression of Cry1Ac in leaves of the event CTC75064-3 over a year of cultivation (100, 200 and 300 DAP; DAP = Days After Planting), Individual and Combined statistical analysis for the 6 tested sites. Data not available in Juazeiro-BA at 300 DAP. Means followed by the same letter do not differ by t test at the 5% level ($p \leq 0.05$). SE: standard error.

| Location | DAP | μg CrylAc / g Fresh Tissue | SE | μg CrylAc / g Dry Tissue | SE |
|---|---|---|---|---|---|
| Barrinha-SP | 100 | 90.54 a | 7.89 | 290.42 a | 26.12 |
| | 200 | 159.80 b | 7.89 | 513.01 b | 26.12 |
| | 300 | 130.45 c | 7.89 | 454.28 b | 26.12 |
| Piracicaba -SP | 100 | 86.84 a | 8.20 | 269.25 a | 26.41 |
| | 200 | 127.90 b | 8.20 | 399.67 b | 26.41 |
| | 300 | 199.40 c | 8.20 | 700.38 c | 26.41 |
| Valparaiso-SP | 100 | 79.16 a | 6.31 | 317.40 a | 23.96 |
| | 200 | 103.98 b | 6.31 | 356.70 a | 23.96 |
| | 300 | 87.20 ab | 6.31 | 351.40 a | 23.96 |
| Quirinópolis-GO | 100 | 111.23 a | 12.95 | 356.77 a | 41.03 |
| | 200 | 138.51 ab | 12.95 | 408.88 ab | 41.03 |
| | 300 | 153.10b | 12.95 | 515.85 b | 41.03 |
| Mardaguaçu-PR | 100 | 94.88 a | 8.21 | 361.46 a | 28.26 |
| | 200 | 136.83 b | 8.21 | 456.10 b | 28.26 |
| | 300 | 153.97 b | 8.21 | 545.60 b | 28.26 |

(continued)

| Location | DAP | µg CrylAc / g Fresh Tissue | SE | µg CrylAc / g Dry Tissue | SE |
|---|---|---|---|---|---|
| Juazeiro-BA | 100 | 62.67 a | 10.05 | 236.01 a | 31.70 |
| | 200 | 80.11 a | 10.05 | 249.37 a | 31.70 |
| | 300 | NA | - | NA | - |
| Combined | 100 | 87.55 a | 12.07 | 305.22 a | 37.62 |
| | 200 | 124.52 b | 12.07 | 397.29 ab | 37.62 |
| | 300 | 140.89 b | 12.97 | 509.35 b | 41.14 |

Table 11. Comparison of the averages of NptII expression in leaves of the CTC75064-3 event over a year of cultivation (100, 200 and 300 DAP). Individual and Combined statistical analysis for the 6 tested sites. Data not available in Juazeiro-BA at 300 DAP in individual and combined analyzes. Means followed by the same letter do not differ by t test at the 5% level ($p \leq 0.05$), SE: standard error.

| Location | DAP | µg NptII / g Fresh Tissue | SE | µg NptII / g Dry Tissue | SE |
|---|---|---|---|---|---|
| Barrinha-SP | 100 | 1.69 a | 0.06 | 7.22 a | 0.27 |
| | 200 | 1.24 b | 0.06 | 5.32 b | 0.27 |
| | 300 | 1.78 a | 0.06 | 8.26 c | 0.27 |
| Piracicaba-SP | 100 | 1.20 a | 0.04 | 4.96 a | 0.17 |
| | 200 | 0.86 b | 0.04 | 3.57 b | 0.17 |
| | 300 | 1.49 c | 0.04 | 6.97 c | 0.17 |
| Valparaiso-SP | 100 | 1.68 a | 0.12 | 9.01 a | 0.62 |
| | 200 | 2.00 a | 0.12 | 9.13 a | 0.62 |
| | 300 | 1.64 a | 0.12 | 8.80 a | 0.62 |
| Quirinópolis-GO | 100 | 1.32 a | 0.07 | 5.63 a | 0.28 |
| | 200 | 0.45 b | 0.07 | 1.76 b | 0.28 |
| | 300 | 1.25 a | 0.07 | 5.61 a | 0.28 |
| Mandaguaçu -PR | 100 | 1.35 a | 0.1 | 6.88 a | 0.49 |
| | 200 | 1.46 a | 0.1 | 6.51 a | 0.49 |
| | 300 | 1.55 a | 0.1 | 7.32 a | 0.49 |
| Juazeiro - SP | 100 | 1.41 a | 0.13 | 7.07 a | 0.6 |
| | 200 | 1.10 a | 0.13 | 4.57 b | 0.6 |
| | 300 | NA | - | NA | - |
| Combined Analysis | 100 | 1.44 a | 0.14 | 6.80 a | 0.74 |
| | 200 | 1.18 a | 0.14 | 5.14 b | 0.74 |
| | 300 | 1.53 a | 0.15 | 7.37 a | 0.77 |

Cry1Ac expression data (leaves; µg/g fresh and dry tissue) from the combined analysis of the 6 locations of the event of the invention over a year of cane cultivation (Table 10) are shown in Figure 8 (in µg protein/g fresh tissue) and Figure 9 (in µg protein/g dry tissue). NptII expression data (leaves; µg/g fresh and dry tissue) from the combined analysis of the 6 locations of the event of the invention over a year of cane cultivation (Table 11) are shown in Figure11 (in µg protein/g fresh tissue) and Figure 12 (in µg protein/g dry tissue).

The average expression of CrylAc protein in leaves from CTC75064-3 event over a sugarcane cycle in all locations (with exception of Juazeiro at 300DAP - no data) was 125.18 μg/g of fresh tissue and 423.75 μg/g of dry tissue. For NptII, the average expression in leaves from CTC75064-3 event over a sugarcane cycle in all locations (with exception of Juazeiro at 300DAP - no data) was 1.401 μg/g of fresh tissue e de 6.370 μg/g of dry tissue.

Cry1Ac protein expression data in leaves from the event CTC75064-3 harvested at 330 DAP (210 DAP for Juazeiro) are shown in Table 12 and in Figure 10 (in μg protein/g of fresh and dry tissue).

Table 12. Comparison of average CrylAc expression in leaves of the event of the invention harvested at 330 DAP (* 210 DAP). Means followed by the same letter do not differ by t test at the 5% level ($p \leq 0.05$). (SE: standard error).

| Location | μg Cry1Ac / g Fresh Tissue | SE | μg Cry 1Ac / g Dry Tissue | SE |
|---|---|---|---|---|
| Barrinha-SP | 205.95 b | 5.71 | 678.48 b | 19.21 |
| Piracicaba -SP | 135.37 b | 5.94 | 420.73 b | 18.69 |
| Valparaiso-SP | 113.71 b | 3.31 | 383.13 b | 11.44 |
| Quirinópolis-GO | 119.45 b | 4.64 | 421.19 b | 16.45 |
| Mardaguaçu-PR | 144.72 b | 6.59 | 491.33 b | 22.55 |
| Juazeiro-BA * | 80.11 b | 8.14 | 249.37 a | 25.67 |
| Combined Analysis | 133.22 b | 10.13 | 440.70 b | 34.84 |

NptII protein expression data in leaves from the event CTC75064-3 harvested at 330 DAP (210 DAP for Juazeiro) are shown in Table 13 and Figure 13 (in μg protein/g of fresh and dry tissue).

Table 13. Comparison of average NptII expression in leaves of the event of the invention harvested at 330 DAP (*210 DAP). Means followed by the same letter do not differ by t test at the 5% level ($p \leq 0.05$). (SE: standard error).

| Location | Fresh Tissue | SE | μg NptII / g Dry Tissue | SE |
|---|---|---|---|---|
| Barrinha-SP | 1.83 b | 0.01 | 7.83 b | 0.05 |
| Piracicaba -SP | 1.23 b | 0.03 | 5.15 b | 0.14 |
| Valparaiso-SP | 1.42 b | 0.04 | 6.48 b | 0.16 |
| Quirinópolis-GO | 1.28b | 0.03 | 5.01 b | 0.11 |
| Mardaguaçu-PR | 1.41 b | 0.05 | 6.28 b | 0.2 |
| Juazeiro - BA* | 1.10 b | 0.08 | 4.57 b | 0.33 |
| Combined Analysis | 1.38 b | 0.06 | 5.89 b | 0.3 |

CrylAc protein expression data in mature stalks from the event CTC75064-3 harvested at 330 DAP are shown in Table 14 and Figure 10 (μg/g fresh and dry tissue).

Table 14. Comparison of average CrylAc expression in mature stalk of the event of the invention harvested at 330 DAP (*210 DAP). Means followed by the same letter do not differ by t test at the 5% level ($p \leq 0.05$). (SE: standard error).

| Location | μg Cry1Ac / g Fresh Tissue | SE | μg Cry1Ac / g Dry Tissue | SE |
|---|---|---|---|---|
| Barrinha-SP | 57.81 a | 5.71 | 248.56 a | 19.21 |
| Piracicaba -SP | 42.01 a | 5.94 | 179.60 a | 18.69 |
| Valparaiso-SP | 42.82 a | 3.31 | 154.33 a | 11.44 |
| Quirinópolis-GO | 30.63 a | 4.64 | 128.95 a | 16.45 |
| Mardaguaçu-PR | 41.44 a | 6.59 | 156.26 a | 22.55 |
| Juazeiro - BA* | 41.21 a | 8.14 | 163.82 a | 25.67 |

(continued)

| Location | μg Cry1Ac / g Fresh Tissue | SE | μg Cry1Ac / g Dry Tissue | SE |
|---|---|---|---|---|
| Combined Analysis | 42.65 a | 10.13 | 171.92 a | 34.84 |

NptII protein expression data from mature stalks of the event of the invention collected at 330 DAP are shown in Table 15 and Figura 13 (μg/g fresh and dry tissue)..

Table 15. Comparison of average NptII expression from mature stalks of the event of the invention (μg/g fresh and dry tissue) harvested at 330 DAP (*210 DAP). Means followed by the same letter do not differ by t test at the 5% level ($p \leq 0.05$). (SE: standard error).

| Location | Fresh Tissue | SE | μg NptII / g Dry Tissue | SE |
|---|---|---|---|---|
| Barrinha-SP | 0.17 a | 0.01 | 0.74 a | 0.05 |
| Piracicaba -SP | 0.16 a | 0.03 | 0.68 a | 0.14 |
| Valparaiso-SP | 0.14 a | 0.04 | 0.51 a | 0.16 |
| Quirinópolis-GO | 0.05 a | 0.03 | 0.22 a | 0.11 |
| Mardaguaçu-PR | 0.17 a | 0.05 | 0.64 a | 0.2 |
| Juazeiro - BA* | 0.14 a | 0.08 | 0.56 a | 0.33 |
| Combined Analysis | 0.14 a | 0.06 | 0.56 a | 0.3 |

CrylAc protein expression data in roots from the event CTC75064-3 harvested at 330 DAP are shown in Table 16 and and Figure 10 (μg/g fresh and dry tissue).

Table 16. CrylAc protein expression values in root tissues of the event of the invention (μg/g fresh and dry tissue) harvested at 330 DAP(*210 DAP). Means followed by the same letter do not differ by t test at the 5% level ($p \leq 0.05$). (SE: standard error).

| Location | μg Cry1Ac / g Fresh Tissue | SE | μg Cry1Ac / g Dry Tissue | SE |
|---|---|---|---|---|
| Barrinha-SP | 10.85 c | 5.71 | 42.55 c | 19.21 |
| Piracicaba -SP | 5.86 c | 5.94 | 28.00 c | 18.69 |
| Valparaiso-SP | 9.92 c | 3.31 | 54.61 c | 11.44 |
| Quirinópolis-GO | 12.13 c | 4.64 | 40.76 c | 16.45 |
| Mardaguaçu-PR | 12.31 c | 6.59 | 65.56 c | 22.55 |
| Juazeiro - BA* | 6.10 c | 8.14 | 29.60 b | 25.67 |
| Combined Analysis | 9.53 c | 10.13 | 43.51 c | 34.84 |

NptII protein expression data in roots of the event of the invention collected at 330 DAP are shown in Table 17 and and Figure 13 (μg/g fresh and dry tissue).

Table 17. NptII protein expression values in root tissues of the event of the invention (μg/g fresh and dry tissue). Means followed by the same letter do not differ by t test at the 5% level ($p \leq 0.05$). (SE: standard error).

| Location | Fresh Tissue | SE | μg NptII / g Dry Tissue | SE |
|---|---|---|---|---|
| Barrinha-SP | 0.07 c | 0.01 | 0.27 c | 0.05 |
| Piracicaba -SP | 0.08 a | 0.03 | 0.37 a | 0.14 |
| Valparaiso-SP | 0.12 a | 0.04 | 0.66 a | 0.16 |
| Quirinópolis-GO | 0.10 a | 0.03 | 0.32 a | 0.11 |

(continued)

| Location | Fresh Tissue | SE | μg NptII / g Dry Tissue | SE |
|---|---|---|---|---|
| Mardaguaçu-PR | 0.09 a | 0.05 | 0.49 a | 0.2 |
| Juazeiro - BA | 0.10 a | 0.08 | 0.47 a | 0.33 |
| Combined Analysis | 0.09 a | 0.06 | 0.43 a | 0.3 |

The average expression of CrylAc protein in stalks from CTC75064-3 event in all locations was 42.94 μg/g of fresh tissue and 173.54 μg/g of dry tissue. For NptII, the average expression in stalks from CTC75064-3 event in all locations was 0.138 μg/g of fresh tissue e de 0.558 μg/g of dry tissue.

The average expression of CrylAc protein in roots from CTC75064-3 event in all locations was 10.21 μg/g of fresh tissue and 46.30 μg/g of dry tissue. For NptII, the average expression in roots from CTC75064-3 event in all locations was 0.092 μg/g of fresh tissue e de 0.422 μg/g of dry tissue.

The results obtained for leaf, stem, and roots indicate that the event of the invention has CrylAc protein expression levels much higher than NptII expression.

The average concentration of CrylAc from leaves of the event of the invention at 100 DAP is lower than the average concentration at 200 and 300 DAP. For NptII the average expression levels in leaves throughout the sugarcane cycle remained constant.

Cry1Ac and NptII protein expression levels from CTC75064-3 event at 330 DAP were much higher in leaves than in stalk and roots. Expression data of CrylAc protein also showed significant differences between stalk and roots, with roots demonstrating the lowest protein expression. For NptII, the average expression of protein in stalks and roots did not differ significantly.

It is therefore concluded that the expression levels of CrylAc and NptII proteins from the event of the invention was characterized at different times, tissues and planting sites representative of its cultivation in Brazil. CrylAc protein expression levels, especially in the leaves of the event of the invention, remain high throughout the cultivation cycle, ensuring the intended effect of resistance to *Diatraea saccharalis.*

**Western blot**

For identification of the heterologous proteins expressed by the event of the invention, 50mg (±0,5 mg) of leaf frozen in liquid nitrogen was used for CryAc and 300 (±0,5 mg) for NptII. Maceration was performed in the TissueLyser equipment for 10 minutes at 25Hz, with the addition of three steel beads (3mm - Qiagen, DEU). To the macerated tissue, 500μl of Tween 20-supplemented saline phosphate extraction buffer (PBS) was added (0.138 M NaCl; 0.027 mM KCl; 0.05% Tween 20, pH 7.4) diluted according to manufacturer's instructions (Envirologix™, USA). After buffer addition, vortex homogenization was performed, and the mixture was centrifuged for 20 minutes at 4,000 RPM at 4°C. The resulting supernatant was collected and total protein was quantified. For NptII, the protein extract was concentrated before proceeding (Amicon® Ultra-0.5 Centrifugal Filter Devices (3000 NMWL).

Quantitation adopted for analysis of CrylAc and *NptII* proteins was performed according to the recommendations of ThermoScientific™ Coomassie Plus (Bradford) Protein Assay Kit (23236) - Microplate Procedure and BCA. Thus, the standards used for obtaining the calibration curve were the already-diluted commercial BSA (Bovine Serum Albumin) standards supplied with the kit described above. The 2000, 1000, 500, 250, 125, and 0 μg/ml calibrators prepared in PBST buffer were used. 10 μL of each standard calibrator was added to the plate wells in triplicate. The plates were covered and incubated for 5 minutes at room temperature. Absorbance was read at 595 nanometers (nm) using SoftmaxPro 7.0 software (Molecular Device, USA). Once the extraction of total proteins was carried out, 3 μg of protein extract were mixed with the 2X Laemmli Sample Buffer (Bio-Rad, USA) and denatured by heating at 100°C for 5 minutes to identify the CrylAc protein present in the sample. In the case of *nptII* detection and identification, 40 μg of the total protein extract was mixed with the sample buffer (2X Laemmli Sample Buffer) and denatured.

As a negative control of the presence of heterologous proteins, 3 μg of protein extract from the conventional parental variety (WT - RB 867515) was used for CryAc experiments and 40μg for NptII assays. In addition, positive controls were prepared to detect CrylAc and *NptII* proteins. The first positive control was prepared using either 0,5ng of CrylAc (~69 kDa, GenScript, USA) or NptII (~29 kDa, Bon Opus Biosciences, USA), diluted in total protein solution extracted from conventional parental variety (WT - RB 867515) leaves. The second positive control was made by diluting 1ng of purified CrylAc protein or 5ng of *NptII* protein in PBST extraction buffer.

Samples were denatured and applied on 4-20% polyacrylamide gel (Mini-PROTEAN® TGXTM Precast Gel) submerged in Tris/glycine/SDS running buffer (Bio-Rad, USA) and separated by electrophoresis at 50V for 5 minutes and then at 120V for approximately 90 minutes. Next, the polyacrylamide gels were equilibrated in Tris/Glycine Transfer Buffer (Bio-Rad, USA), which was added with 20% methanol for 10-15 minutes. The PVDF membrane was treated with absolute methanol. The transfer system was mounted in a container filled with the cold Transfer Buffer for immersion transfer ("wet transfer") at a constant voltage of 50V for 3 hours. Upon completion of the transfer, the membrane was blocked for 16 hours at 4 °C, under constant agitation, in blocking solution [5% skimmed milk powder (Bio-Rad, USA) and TBS/T (20mM Tris, 150mM NaCl, 1% Tween20)] to prevent possible nonspecific membrane binding.

In the next step, the membrane was incubated with the primary antibody for 90 minutes to detect and confirm the presence and integrity of the CrylAc and *NptII* proteins. The polyclonal antibodies used in this assay were rabbit Anti-CrylAb (Fitzgerald, USA), which bind both CrylAc and CrylAb proteins; and rabbit Anti- *NptII*(Rhea, BRA IM0770-18088),), which reacts with *NptII*protein, diluted 1: 500 in TBS/T (v/v).

The membrane was washed 3 times per 5 minute (3x5) in TBS/T and incubated with goat-produced HRP-conjugated secondary Anti-Rabbit antibody (Sigma Aldrich, USA) at a concentration of 1: 20,000 or at a concentration of 1:5,000 - v/v (Fitzgerald, USA) for 60 minutes. After incubations, the membrane was again washed with TBS/T (3x5 minutes), and the enzyme-linked immunoassay was verified on Amersham Hyperfilm ECL X-ray films (GE Healthcare, USA) by Clarit Western ECL Substrate Kit substrate reaction (Biorad, USA) according to the manufacturer's instructions. X-ray film exposure to membrane ranged from 15 seconds to 3 minutes.

The results revealed that the expression profile of CrylAc protein appears as one immunoreactive bands of ~66 kDa (Figure 14). All samples (R1-R4) are biological replicates of the event of the invention, obtained from four experimental plots at the Piracicaba site. As expected, the negative control (WT) showed no immunoreactivity. The negative control consisted of total protein samples extracted from the parental variety. The positive control (WT+CP) where the Cry1Ac protein was added to the total protein extracted from the parental cultivar, presented two immunoreactive bands, from approximately 69 and 66kDa. In this case (WT+CP),it is commonly known as doublet and accepted as a product of intracellular proteolytic breaks of Cry proteins in plant leaves generally produced by removing of terminal amino acid residues by proteases released during plant tissue processing, as well as other bands with smaller molecular weights (~20 and 10 kDa) visualized in the samples.

The protein encoded by the *nptII*selection gene was also detected by western blot assay. Even though *NptII* is expressed at lower levels than the CrylAc protein, western blot assays demonstrated the presence of an immunoreactive band at the expected size of 29kDa in all used biological replicates of the event of the invention (Figure 15).

Two positive and one negative control were added to the membrane. The first positive control (CP) corresponds to 0.5ng of purified *NptII*protein (~29 kDa, Bon Opus Biosciences, USA), and the second positive control (WT+CP) corresponds to 0.5ng of purified *NptII* protein diluted in protein extract of the parental cultivar. Negative control correspond to protein extract of the parental cultivar. The diagnostic band corresponding to the *NptII* protein is present in both controls at the expected weight and is identical to the band present at the event of the invention, confirming its identity.

It is therefore concluded that the identity of the Cry1Ac and *NptII* proteins expressed by the event of the invention was confirmed by western blot. The proteins expressed by the event of the invention are of the expected size, and no evidence of truncated/fused proteins being expressed by said event was found.

<u>Example 5. Biological tests: Susceptibility to the sugarcane borer *(D. saccharalis)*</u>

Biological Assays (bioassays) with target pest *D. saccharalis* (cane borer) can also be used for detection and characterization of event CTC75064-3, demonstrating the efficacy on the pest control provided by the expressed insecticidal protein Cry1Ac. Different bioassays may be contemplated within the scope of the present invention: for example, Leaf Disk Assay, Screenhouse bioassays, Tissue Dilution Assays, among others.

For leaf disc assay, leaves of event CTC75064-3 plants were collected, cut into discs of 16 $mm^2$ and distributed in bioassay plates containing gelled agar. Each well from culture plates was infested with *D. saccharalis* (0-24h old) neonate and incubated at 27 ± 1°C, relative humidity 60 ± 10%, and photoperiod 12:12h (light: dark) for a period of 7 days. At the end of incubation, larval mortality and inhibition of larval development of surviving individuals was evaluated, and the relative efficacy was calculated by the following formula:

$$\text{LDA Relative efficacy} = 100 - \left(\frac{\text{\%live larvae of the event}}{\text{\%live larvae of the control}}\right) \text{x } 100$$

The surviving larvae were submitted to image analysis by Digimizer software (v 4.6.1) for assessment of larval stage based on width of cephalic capsule. The larvae that did not reach the first instar were considered dead. Non-transgenic sugarcane varieties that are genetically very similar to the evaluated transgenic event can be used as assay controls.

To characterize event efficacy in controlling target pest *D. saccharalis* in laboratory, leaf disc assays were performed with plant tissue from CTC75064-3 event (30DAP). Non-transgenic sugarcane RB 867515 was used as control (CTC75-TC). The experimental design was completely randomized with four replicates per treatment. An average of 98.2% of mortality rate was observed when comparing the conventional variety (CT75-TC) and CTC75064-3 event after 7 days feeding with leaf discs. Also, based on measurement of cephalic capsule width, it was observed that 100% of the surviving individuals did not develop beyond the first instar, evidencing high suppression in the development of *D. saccharalis* after feeding with the transgenic event (Figure 18).

For tissue dilution assays, the leaves of the CTC75064-3 event were sampled in the Agro/pheno fields at Piracicaba, Valparaiso, Barrinha (SP) and Quirinópolis (GO), with collection points in 100, 200 and 300 DAP. These samples were chopped, dehydrated in a freeze dryer and then macerated to obtain a uniform green powder. To prepare the bioassay plates, each quadrant of 16 cells received the sample diluted 25x with MS diet (Multiple Species), filling approximately 1 mL per cell. For infestation, 2 neonates larvae (0-24h of age) were transferred in each cell (32 caterpillars per quadrant). The plates were identified and incubated at a temperature of 27 ± 1 ° C, relative humidity 60 ± 10% and a 12:12 photoperiod (light: dark), for a period of 10 days. At the end of the incubation, each quadrant was evaluated for effective mortality and average mass of larvae. Effective mortality was calculated from the equation:

$$\text{Effective mortality} = \frac{(\text{n dead larvae} + \text{n larvae on 1st instar})}{\text{total number of live larvae}} \text{x } 100$$

Relative effectiveness for dilution tests were calculated using the equation:

$$\text{Relative effectiveness in dilution} = 100 - \left(\frac{\%\text{Live larvae of the event}}{\%\text{Live larvae of the control}}\right) \text{x } 100$$

Through the analysis of tissue dilution from the fields of Agro/pheno, it was possible to verify that the effectiveness, both for effective mortality and for mass reduction of larvae, is 51 and 94%, respectively.

For screenhouse bioassays, seedlings of the transgenic event are planted in a screened nursery, where the plants are planted in the soil similar to natural environmental conditions, but in controlled environment to prevent the occurrence of natural infestations. At least 5 infestations are performed, containing 20-35 *Diatraea saccharalis* eggs per tiller. The evaluation occurs when all infected stalks are harvested and cutting longitudinally to quantify the damage. Infestation Intensity is calculated dividing the number of internodes with damage by the total number of internodes, and the result was multiplied by 100 (Infestation Intensity). Percentage of Effective Damage was calculated, considering the total of internodes with damage caused by the insect divided by the total number of stalks evaluated in the plot.

Screenhouse trials were performed to characterize the efficacy of CTC75064-3 event in controlling borer attack in comparison to its parental variety RB 867515 (WT;non-transgenic) in a randomized block design, with 4 replications. Each experimental plot was composed by eight clumps of cane-plant that received 10 artificial infestations with approximately 30 eggs of *D. saccharalis* every 15 days. After eight months, the Infestation Intensity (II) and the effective Damage for both varieties were calculated. Relative efficacy was calculated according to the formula below:

$$relative\ efficacy\ (\%) = 1 - \left(\frac{\text{(average I.I.\% of the event)}}{\text{average I.I.\% of the controls}}\right)$$

Under the artificial infestation the event CTC75064-3 presented relative efficacy in controlling infestation by *D. saccharalis* of 98.8% and in controlling stalk damage (length) superior to 99.9% in relation to its non-transgenic parental variety RB 867515 (Control). The damage caused by *D. saccharalis* in CTC75064-3 stalks was visibly lower than in the conventional sugarcane RB 867515. There were statistically differences (t-test, $P < 0.05$) between CTC75064-3 and the non-transgenic variety RB 867515 in both parameters evaluated ($df = 16$; $P < 0.0001$), showing that under massive infestation with *D. Saccharalis* the event suppressed the damages caused by the pest (Figure 16).

On sugarcane conventional production *D. saccharalis* is considered controlled when the infestation intensity is lower than 3% (Gallo et al., 2002). For CTC75064-3, the intensity of infestation was lower than 0,01% reinforcing the event effectiveness to control its main target pest.

In addition to employing bioassays that use artificial infestations to observe the degree of infestation and damage caused by *D. saccharalis,* observations of infestation and damage percentage can also be made based on information collected directly from the fields where the event CTC75064-3 is grown. The II (infestation intensity), for example, can be calculated for natural infestation evaluation by defining an experimental area for stalk sampling cutting and quantification the number of internodes with and without damages) to obtain the infestation intensity (II).

In the tests performed for the event of the invention, the infestation intensity (II) was calculated in four experimental areas: Piracicaba, Barrinha, and Valparaiso (SP) and Quirinopolis (GO). The conventional variety RB 867515 (parental; non transgenic) was used as control. This assay illustrates the resistance of the event CTC75064-3 to *D. saccharalis* infestation compared to the parental variety (RB 867515): it was observed a lower intensity of infestation for CTC75064-3 plants in comparison to the parental variety (RB 867515) in all the four experimental areas (Combined analysis; Figure 17). Event CTC75064-3 presented relative efficacy in controlling infestation by *D. saccharalis* of 100% in all locations, except for Valparaiso, which showed 99.1% effectiveness, very close to 100%. Thus, it appears that the event CTC75064 was, on average in the 4 locations, 99.7% more effective than conventional materials, for controlling *Diatraea saccharalis* (Figure 17).

Having described examples of preferred embodiments, it should be understood that the scope of the present invention encompasses other possible variations and is limited only by the content of the appended claims, including the possible equivalents thereof.

**SEQUENCES**

**> SEQ ID 01 - Genetic construct comprising Cry1Ac and nptII genes**

TGGCAGGATATATTGTGGTGTAAACAAATTGACGCTTAGACAACTTAATAACACATTGCGGACGTTTTTAATGTACTGAATTA
GTACTGATATCGGTACCTTAATTCGGGGGATCTGGATTTTAGTACTGGATTTTGGTTTTAGGAATTAGAAATTTTATTGATAG
AAGTATTTTACAAATACAAATACATACTAAGGGTTTCTTATATGCTCAACACATGAGCGAAACCCTATAGGAACCCTAATTCC
CTTATCTGGGAACTACTCACACATTATTATGGAGAAACTCGAGCTTGTCGATCGATCACTCAGCCTCGAGGGTGGCGGTCACT
GGGATGAACTCGAACCTGTCGATGATCACGCCGGCGGTGCCGCTGAAGTTCCTCACGCCCACGATGTTACCGAGGGAGGAGGT
GAAAGCGTTGGCGCTCTCGAAGTAACCGAAGTCGCTGGATTGGAGGTTGTCCAGGGAGGTAGCGGTAGCTGGCACGGTGTTGG
AGAAGATGGAGGAGTTACCCCAGTTCACGTTGAGGTGGATCGGGGTCACGGAAGCGTACCTCACGCGCACCCTGTACCTGGTG
GAGGTGGATGGGAAGTGGATTGGCACCTCGATGTAGCCCCTGTTCTGGATGTTGTTGCCGCTGCTGTTGAGCCTCACGAGGTC
GCCACCGGTGAAGCCTGGGCCGGAAATGACGGAACCGTTGAAGAGGAAGTTGCCCTTCACGGCCGGGATTTGGGTGATGCTGT
CGGAGGCGATGATGTTGTTGAACTCAGCGCTGCGGTGGATCCAGGAGAACATCGGAGCCCTGATGATGCTCACGGAGCTGTTG
CTGAAGCCGGAGCGGAACATGGACACGTGGCTCAGCCTGTGGGAGAAGCCTTGCCTGGGTGGCACGTTGTTGTTCTGTGGTGG
GATCTCGTCCAGGGAGTCCACGGTGCCGCTCTTCCTGTAGACAGCGGATGGCAGGTTGGAGGAGGTGCCGTAGGCGAACTCGG
TGCCGTCGAGCACGGACAGTTGCTGGTTGTTGATACCGATGTTGAAGGGCCTCCTGTACAGGGTGGAGGACAGGGTCCTGTAG
ACACCCTGACCCAGTTGAGCCACGATGCGTTGCTGTGGAGCGGCGTTGCCCATGGTGCCGTAGAGCGGGAAGGTGAACTCGGG
GCCGCTGAAGCCCACTGGGGAGGCCATGATCTGGTGGCCGGACCAGTAGTACTCGCCCCTGTGAGCGTCGGTGTAGATGGTGA
TGCTGTTCAGGATGTCCATCAGGTGTGGGCTCCTGATGGAGCCCTCGATACCCTGGGCGGAACCGCGGAAGCTACCGTCGAAG
TTCTCCAGCACTGGGTTGGTGTAGATCTCCCTGGTGAGTTGGGACACGGTGCGGATCGGGTAGGTCCTGGAGTCGTAGTTCGG
GAAGAGGGACACAATGTCCAGCACGGTGAGGGTCAACTCCCTCCTGAACTGGTTGTACCTGATCCAGTCCCTGGAGTCCGGAC
CCCAGACGCGCTCCAGGCCGGTGTTGTACCAGCGCACAGCGTGGTCGGTGTAGTTGCCAATCAGCCTGGTCAGGTCGTTGTAG
CGGCTGTTGATGGTGGCAGCATCGAAGCCCCACCTTTGGCCGAACACGCTCACGTCGCGCAGCACGCTGAGGTGCAGGTTAGC
GGCTTGCACGTACACGGACAGGAGCGGCACTTGGTAGTTCTGGACGGCGAACAGTGGGATAGCGGTGGTCAGGGCGCTGTTCA
TGTCGTTGAATTGAATGCGCATTTCCTCGCGGAGAGCTGGGTTGGTCGGGTCGGCCTCCCACTCCCTGAAGCTCTCGGCGTAG
ATTTGGTAGAGGTTGCTCAGGCCCTCCAGCCTGGAGATGGCCTGGTTCCTGGCGAACTCTTCGATCCTCTGGTTGATCAGCTG
CTCGATTTGCACCAGGAAGGCGTCCCATTGGGATGGACCGAAGATACCCCAGATGATGTCCACCAGGCCGAGCACGAAGCCAG
CACCTGGCACGAACTCGCTGAGCAGGAACTGGGTCAAGGACAGGGAGATGTCGATGGGGGTGTAACCGGTCTCGATGCGCTCG
CCACCCAGCACCTCCACCTCTGGGTTGCTCAGGCAGTTGTATGGGATGCACTCGTTGATGTTTGGGTTGTTGTCCATGGCGGG
GTTGATCAGGTTGATCACTTCTACCTACAAAAAAGCTCCGCACGAGGCTGCATTTGTCACAAATCATGAAAAGAAAAACTACC
GATGAACAATGCTGAGGGATTCAAATTCTACCCACAAAAAGAAGAAAGAAAGATCTAGCACATCTAAGCCTGACGAAGCAGCA
GAAATATATAAAAATATAAACCATAGTGCCCTTTTCCCCTCTTCCTGATCTTGTTTAGCACGGCGGAAATTTTAAACCCCCCA
TCATCTCCCCCAACAACGGCGGATCGCAGATCTACATCCGAGAGCCCCATTCCCCGCGAGATCCGGGCCGGATCCACGCCGGC
GAGAGCCCCAGCCGCGAGATCCCGCCCCTCCCGCGCACCGATCTGGGCGCGCACGAAGCCGCCTCTCGCCCACCCAAACTACC
AAGGCCAAAGATCGAGACCGAGACGGAAAAAAAAACGGAGAAAGAAAGAGGAGAGGGGCGGGGTGGTTACCGGCGGCGGCGGA
GGCCTCCCTTGGATCTTATGGTGTGTTGTCCCTGTGTGTTCTCCAATAGTGTGGCTTGAGTGTGTGGAAGATGGTTCTAGAGG
ATCTGCTAGAGTCAGCTTGTCAGCGTGTCCTCTCCAAATGAAATGAACTTCCTTATATAGAGGAAGGGTCTTGCGAAGGATAG
TGGGATTGTGCGTCATCCCTTACGTCAGTGGAGATATCACATCAATCCACTTGCTTTGAAGACGTGGTTGGAACGTCTTCTTT
TTCCACGATGCTCCTCGTGGGTGGGGGTCCATCTTTGGGACCACTGTCGGCAGAGGCATCTTCAACGATGGCCTTTCCTTTAT
CGCAATGATGGCATTTGTAGGAGCCACCTTCCTTTTCCACTATCTTCACAATAAAGTGACAGATAGCTGGGCAATGGAATCCG
AGGAGGTTTCCGGATATTACCCTTTGTTGAAAAGTCTCAATCGGACCATCACATCAATCCACTTGCTTTGAAGACGTGGTTGG
AACGTCTTCTTTTTCCACGATGCTCCTCGTGGGTGGGGGTCCATCTTTGGGACCACTGTCGGCAGAGGCATCTTCAACGATGG
CCTTTCCTTTATCGCAATGATGGCATTTGTAGGAGCCACCTTCCTTTTCCACTATCTTCACAATAAAGTGACAGATAGCTGGG
CAATGGAATCCGAGGAGGTTTCCGGATATTACCCTTTGTTGAAAAGTCTCAATCGGACCTGTTTAAACCCTGAAGCTTACGCG
TATGCCTGCAGTGCAGCGTGACCCGGTCGTGCCCCTCTCTAGAGATAATGAGCATTGCATGTCTAAGTTATAAAAAATTACCA
CATATTTTTTTTGTCACACTTGTTTGAAGTGCAGTTTATCTATCTTTATACATATATTTAAACTTTACTCTACGAATAATATA
ATCTATAGTACTACAATAATATCAGTGTTTTAGAGAATCATATAAATGAACAGTTAGACATGGTCTAAAGGACAATTGAGTAT
TTTGACAACAGGACTCTACAGTTTTATCTTTTTAGTGTGCATGTGTTCTCCTTTTTTTTTGCAAATAGCTTCACCTATATAAT
ACTTCATCCATTTTATTAGTACATCCATTTAGGGTTTAGGGTTAATGGTTTTTATAGACTAATTTTTTTAGTACATCTATTTT
ATTCTATTTTAGCCTCTAAATTAAGAAAACTAAAACTCTATTTTAGTTTTTTTATTTAATAATTTAGATATAAAATAGAATAA
AATAAAGTGACTAAAAATTAAACAAATACCCTTTAAGAAATTAAAAAAACTAAGGAAACATTTTTCTTGTTTCGAGTAGATAA
TGCCAGCCTGTTAAACGCCGTCGACGAGTCTAACGGACACCAACCAGCGAACCAGCAGCGTCGCGTCGGGCCAAGCGAAGCAG
ACGGCACGGCATCTCTGTCGCTGCCTCTGGACCCCTCTCGAGAGTTCCGCTCCACCGTTGGACTTGCTCCGCTGTCGGCATCC
AGAAATTGCGTGGCGGAGCGGCAGACGTGAGCCGGCACGGCAGGCGGCCTCCTCCTCCTCTCACGGCACGGCAGCTACGGGGG
ATTCCTTTCCCACCGCTCCTTCGCTTTCCCTTCCTCGCCCGCCGTAATAAATAGACACCCCCTCCACACCCTCTTTCCCCAAC
CTCGTGTTGTTCGGAGCGCACACACACACAACCAGATCTCCCCCAAATCCACCCGTCGGCACCTCCGCTTCAAGGTACGCCGC
TCGTCCTCCCCCCCCCCCCCTCTCTACCTTCTCTAGATCGGCGTTCCGGTCCATGGTTAGGGCCCGGTAGTTCTACTTCTGTT

CATGTTTGTGTTAGATCCGTGTTTGTGTTAGATCCGTGCTGCTAGCGTTCGTACACGGATGCGACCTGTACGTCAGACACGTT
CTGATTGCTAACTTGCCAGTGTTTCTCTTTGGGGAATCCTGGGATGGCTCTAGCCGTTCCGCAGACGGGATCGATTTCATGAT
TTTTTTTGTTTCGTTGCATAGGGTTTGGTTTGCCCTTTTCCTTTATTTCAATATATGCCGTGCACTTGTTTGTCGGGTCATCT
TTTCATGCTTTTTTTTGTCTTGGTTGTGATGATGTGGTCTGGTTGGGCGGTCGTTCTAGATCGGAGTAGAATTCTGTTTCAAA
CTACCTGGTGGATTTATTAATTTTGGATCTGTATGTGTGTGCCATACATATTCATAGTTACGAATTGAAGATGATGGATGGAA
ATATCGATCTAGGATAGGTATACATGTTGATGCGGGTTTTACTGATGCATATACAGAGATGCTTTTTGTTCGCTTGGTTGTGA
TGATGTGGTGTGGTTGGGCGGTCGTTCATTCGTTCTAGATCGGAGTAGAATACTGTTTCAAACTACCTGGTGTATTTATTAAT
TTTGGAACTGTATGTGTGTGTCATACATCTTCATAGTTACGAGTTTAAGATGGATGGAAATATCGATCTAGGATAGGTATACA
TGTTGATGTGGGTTTTACTGATGCATATACATGATGGCATATGCAGCATCTATTCATATGCTCTAACCTTGAGTACCTATCTA
TTATAATAAACAAGTATGTTTTATAATTATTTTGATCTTGATATACTTGGATGATGGCATATGCAGCAGCTATATGTGGATTT
TTTTAGCCCTGCCTTCATACGCTATTTATTTGCTTGGTACTGTTTCTTTTGTCGATGCTCACCCTGTTGTTTGGTGTTACTTC
TGCAGGTCGACTCTAGAATGTGGATTGAACAAGATGGATTGCACGCAGGTTCTCCGGCCGCTTGGGTGGAGAGGCTATTCGGC
TATGACTGGGCACAACAGACAATCGGCTGCTCTGATGCCGCCGTGTTCCGGCTGTCAGCGCAGGGGCGCCCGGTTCTTTTTGT
CAAGACCGACCTGTCCGGTGCCCTGAATGAACTGCAGGACGAGGCAGCGCGGCTATCGTGGCTGGCCACGACGGGCGTTCCTT
GCGCAGCTGTGCTCGACGTTGTCACTGAAGCGGGAAGGGACTGGCTGCTATTGGGCGAAGTGCCGGGGCAGGATCTCCTGTCA
TCTCACCTTGCTCCTGCCGAGAAAGTATCCATCATGGCTGATGCAATGCGGCGGCTGCATACGCTTGATCCGGCTACCTGCCC
ATTCGACCACCAAGCGAAACATCGCATCGAGCGAGCACGTACTCGGATGGAAGCCGGTCTTGTCGATCAGGATGATCTGGACG
AAGAGCATCAGGGGCTCGCGCCAGCCGAACTGTTCGCCAGGCTCAAGGCGCGCATGCCCGACGGCGAGGATCTCGTCGTGACC
CATGGCGATGCCTGCTTGCCGAATATCATGGTGGAAAATGGCCGCTTTTCTGGATTCATCGACTGTGGCCGGCTGGGTGTGGC
GGACCGCTATCAGGACATAGCGTTGGCTACCCGTGATATTGCTGAAGAGCTTGGCGGCGAATGGGCTGACCGCTTCCTCGTGC
TTTACGGTATCGCCGCTCCCGATTCGCAGCGCATCGCCTTCTATCGCCTTCTTGACGAGTTCTTCTGAGATCGTTCAAACATT
TGGCAATAAAGTTTCTTAAGATTGAATCCTGTTGCCGGTCTTGCGATGATTATCATATAATTTCTGTTGAATTACGTTAAGCA
TGTAATAATTAACATGTAATGCATGACGTTATTTATGAGATGGGTTTTTATGATTAGAGTCCCGCAATTATACATTTAATACG
CGATAGAAAACAAAATATAGCGCGCAAACTAGGATAAATTATCGCGCGCGGTGTCATCTATGTTACTAGATCGGCGCGCCAAG
GGCGAATTCCAGCACACTGGCGGCCGTTACTAGTGGATCGAGCTCGTCGACTCTAGACTCGAGGGCGCGCCGACAGGATATAT
TGGCGGGTAAACC

**> SEQ ID 02 (T-DNA fragment event CTC75064)**

ACAAATTGACGCTTAGACAACTTAATAACACATTGCGGACGTTTTTAATGTACTGAATTAGTACTGATATCGGTACCTTAATT
CGGGGGATCTGGATTTTAGTACTGGATTTTGGTTTTAGGAATTAGAAATTTTATTGATAGAAGTATTTTACAAATACAAATAC
ATACTAAGGGTTTCTTATATGCTCAACACATGAGCGAAACCCTATAGGAACCCTAATTCCCTTATCTGGGAACTACTCACACA
TTATTATGGAGAAACTCGAGCTTGTCGATCGATCACTCAGCCTCGAGGGTGGCGGTCACTGGGATGAACTCGAACCTGTCGAT
GATCACGCCGGCGGTGCCGCTGAAGTTCCTCACGCCCACGATGTTACCGAGGGAGGAGGTGAAAGCGTTGGCGCTCTCGAAGT
AACCGAAGTCGCTGGATTGGAGGTTGTCCAGGGAGGTAGCGGTAGCTGGCACGGTGTTGGAGAAGATGGAGGAGTTACCCCAG
TTCACGTTGAGGTGGATCGGGGTCACGGAAGCGTACCTCACGCGCACCCTGTACCTGGTGGAGGTGGATGGGAAGTGGATTGG
CACCTCGATGTAGCCCCTGTTCTGGATGTTGTTGCCGCTGCTGTTGAGCCTCACGAGGTCGCCACCGGTGAAGCCTGGGCCGG
AAATGACGGAACCGTTGAAGAGGAAGTTGCCCTTCACGGCCGGGATTTGGGTGATGCTGTCGGAGGCGATGATGTTGTTGAAC
TCAGCGCTGCGGTGGATCCAGGAGAACATCGGAGCCCTGATGATGCTCACGGAGCTGTTGCTGAAGCCGGAGCGGAACATGGA
CACGTGGCTCAGCCTGTGGGAGAAGCCTTGCCTGGGTGGCACGTTGTTGTTCTGTGGTGGGATCTCGTCCAGGGAGTCCACGG
TGCCGCTCTTCCTGTAGACAGCGGATGGCAGGTTGGAGGAGGTGCCGTAGGCGAACTCGGTGCCGTCGAGCACGGACAGTTGC
TGGTTGTTGATACCGATGTTGAAGGGCCTCCTGTACAGGGTGGAGGACAGGGTCCTGTAGACACCCTGACCCAGTTGAGCCAC
GATGCGTTGCTGTGGAGCGGCGTTGCCCATGGTGCCGTAGAGCGGGAAGGTGAACTCGGGGCCGCTGAAGCCCACTGGGGAGG
CCATGATCTGGTGGCCGGACCAGTAGTACTCGCCCCTGTGAGCGTCGGTGTAGATGGTGATGCTGTTCAGGATGTCCATCAGG
TGTGGGCTCCTGATGGAGCCCTCGATACCCTGGGCGGAACCGCGGAAGCTACCGTCGAAGTTCTCCAGCACTGGGTTGGTGTA
GATCTCCCTGGTGAGTTGGGACACGGTGCGGATCGGGTAGGTCCTGGAGTCGTAGTTCGGGAAGAGGGACACAATGTCCAGCA
CGGTGAGGGTCAACTCCCTCCTGAACTGGTTGTACCTGATCCAGTCCCTGGAGTCCGGACCCCAGACGCGCTCCAGGCCGGTG
TTGTACCAGCGCACAGCGTGGTCGGTGTAGTTGCCAATCAGCCTGGTCAGGTCGTTGTAGCGGCTGTTGATGGTGGCAGCATC
GAAGCCCCACCTTTGGCCGAACACGCTCACGTCGCGCAGCACGCTGAGGTGCAGGTTAGCGGCTTGCACGTACACGGACAGGA
GCGGCACTTGGTAGTTCTGGACGGCGAACAGTGGGATAGCGGTGGTCAGGGCGCTGTTCATGTCGTTGAATTGAATGCGCATT
TCCTCGCGGAGAGCTGGGTTGGTCGGGTCGGCCTCCCACTCCCTGAAGCTCTCGGCGTAGATTTGGTAGAGGTTGCTCAGGCC
CTCCAGCCTGGAGATGGCCTGGTTCCTGGCGAACTCTTCGATCCTCTGGTTGATCAGCTGCTCGATTTGCACCAGGAAGGCGT
CCCATTGGGATGGACCGAAGATACCCCAGATGATGTCCACCAGGCCGAGCACGAAGCCAGCACCTGGCACGAACTCGCTGAGC
AGGAACTGGGTCAAGGACAGGGAGATGTCGATGGGGGTGTAACCGGTCTCGATGCGCTCGCCACCCAGCACCTCCACCTCTGG
GTTGCTCAGGCAGTTGTATGGGATGCACTCGTTGATGTTTGGGTTGTTGTCCATGGCGGGGTTGATCAGGTTGATCACTTCTA
CCTACAAAAAAGCTCCGCACGAGGCTGCATTTGTCACAAATCATGAAAAGAAAAACTACCGATGAACAATGCTGAGGGATTCA
AATTCTACCCACAAAAAGAAGAAAGAAAGATCTAGCACATCTAAGCCTGACGAAGCAGCAGAAATATATAAAAATATAAACCA
TAGTGCCCTTTTCCCCTCTTCCTGATCTTGTTTAGCACGGCGGAAATTTTAAACCCCCCATCATCTCCCCCAACAACGGCGGA
TCGCAGATCTACATCCGAGAGCCCCATTCCCCGCGAGATCCGGGCCGGATCCACGCCGGCGAGAGCCCCAGCCGCGAGATCCC
GCCCCTCCCGCGCACCGATCTGGGCGCGCACGAAGCCGCCTCTCGCCCACCCAAACTACCAAGGCCAAAGATCGAGACCGAGA
CGGAAAAAAAAACGGAGAAAGAAAGAGGAGAGGGGCGGGGTGGTTACCGGCGGCGGCGGAGGCCTCCCTTGGATCTTATGGTG
TGTTGTCCCTGTGTGTTCTCCAATAGTGTGGCTTGAGTGTGTGGAAGATGGTTCTAGAGGATCTGCTAGAGTCAGCTTGTCAG
CGTGTCCTCTCCAAATGAAATGAACTTCCTTATATAGAGGAAGGGTCTTGCGAAGGATAGTGGGATTGTGCGTCATCCCTTAC

GTCAGTGGAGATATCACATCAATCCACTTGCTTTGAAGACGTGGTTGGAACGTCTTCTTTTTCCACGATGCTCCTCGTGGGTG
GGGGTCCATCTTTGGGACCACTGTCGGCAGAGGCATCTTCAACGATGGCCTTTCCTTTATCGCAATGATGGCATTTGTAGGAG
CCACCTTCCTTTTCCACTATCTTCACAATAAAGTGACAGATAGCTGGGCAATGGAATCCGAGGAGGTTTCCGGATATTACCCT
TTGTTGAAAAGTCTCAATCGGACCATCACATCAATCCACTTGCTTTGAAGACGTGGTTGGAACGTCTTCTTTTTCCACGATGC
TCCTCGTGGGTGGGGGTCCATCTTTGGGACCACTGTCGGCAGAGGCATCTTCAACGATGGCCTTTCCTTTATCGCAATGATGG
CATTTGTAGGAGCCACCTTCCTTTTCCACTATCTTCACAATAAAGTGACAGATAGCTGGGCAATGGAATCCGAGGAGGTTTCC
GGATATTACCCTTTGTTGAAAAGTCTCAATCGGACCTGTTTAAACCCTGAAGCTTACGCGTATGCCTGCAGTGCAGCGTGACC
CGGTCGTGCCCCTCTCTAGAGATAATGAGCATTGCATGTCTAAGTTATAAAAAATTACCACATATTTTTTTTGTCACACTTGT
TTGAAGTGCAGTTTATCTATCTTTATACATATATTTAAACTTTACTCTACGAATAATATAATCTATAGTACTACAATAATATC
AGTGTTTTAGAGAATCATATAAATGAACAGTTAGACATGGTCTAAAGGACAATTGAGTATTTTGACAACAGGACTCTACAGTT
TTATCTTTTTAGTGTGCATGTGTTCTCCTTTTTTTTTGCAAATAGCTTCACCTATATAATACTTCATCCATTTTATTAGTACA
TCCATTTAGGGTTTAGGGTTAATGGTTTTTATAGACTAATTTTTTTAGTACATCTATTTTATTCTATTTTAGCCTCTAAATTA
AGAAAACTAAAACTCTATTTTAGTTTTTTTATTTAATAATTTAGATATAAAATAGAATAAAATAAAGTGACTAAAAATTAAAC
AAATACCCTTTAAGAAATTAAAAAAACTAAGGAAACATTTTTCTTGTTTCGAGTAGATAATGCCAGCCTGTTAAACGCCGTCG
ACGAGTCTAACGGACACCAACCAGCGAACCAGCAGCGTCGCGTCGGGCCAAGCGAAGCAGACGGCACGGCATCTCTGTCGCTG
CCTCTGGACCCCTCTCGAGAGTTCCGCTCCACCGTTGGACTTGCTCCGCTGTCGGCATCCAGAAATTGCGTGGCGGAGCGGCA
GACGTGAGCCGGCACGGCAGGCGGCCTCCTCCTCCTCTCACGGCACGGCAGCTACGGGGGATTCCTTTCCCACCGCTCCTTCG
CTTTCCCTTCCTCGCCCGCCGTAATAAATAGACACCCCCTCCACACCCTCTTTCCCCAACCTCGTGTTGTTCGGAGCGCACAC
ACACACAACCAGATCTCCCCCAAATCCACCCGTCGGCACCTCCGCTTCAAGGTACGCCGCTCGTCCTCCCCCCCCCCCCCTCT
CTACCTTCTCTAGATCGGCGTTCCGGTCCATGGTTAGGGCCCGGTAGTTCTACTTCTGTTCATGTTTGTGTTAGATCCGTGTT
TGTGTTAGATCCGTGCTGCTAGCGTTCGTACACGGATGCGACCTGTACGTCAGACACGTTCTGATTGCTAACTTGCCAGTGTT
TCTCTTTGGGGAATCCTGGGATGGCTCTAGCCGTTCCGCAGACGGGATCGATTTCATGATTTTTTTTGTTTCGTTGCATAGGG
TTTGGTTTGCCCTTTTCCTTTATTTCAATATATGCCGTGCACTTGTTTGTCGGGTCATCTTTTCATGCTTTTTTTTGTCTTGG
TTGTGATGATGTGGTCTGGTTGGGCGGTCGTTCTAGATCGGAGTAGAATTCTGTTTCAAACTACCTGGTGGATTTATTAATTT
TGGATCTGTATGTGTGTGCCATACATATTCATAGTTACGAATTGAAGATGATGGATGGAAATATCGATCTAGGATAGGTATAC
ATGTTGATGCGGGTTTTACTGATGCATATACAGAGATGCTTTTTGTTCGCTTGGTTGTGATGATGTGGTGTGGTTGGGCGGTC
GTTCATTCGTTCTAGATCGGAGTAGAATACTGTTTCAAACTACCTGGTGTATTTATTAATTTTGGAACTGTATGTGTGTGTCA
TACATCTTCATAGTTACGAGTTTAAGATGGATGGAAATATCGATCTAGGATAGGTATACATGTTGATGTGGGTTTTACTGATG
CATATACATGATGGCATATGCAGCATCTATTCATATGCTCTAACCTTGAGTACCTATCTATTATAATAAACAAGTATGTTTTA
TAATTATTTTGATCTTGATATACTTGGATGATGGCATATGCAGCAGCTATATGTGGATTTTTTTAGCCCTGCCTTCATACGCT
ATTTATTTGCTTGGTACTGTTTCTTTTGTCGATGCTCACCCTGTTGTTTGGTGTTACTTCTGCAGGTCGACTCTAGAATGTGG
ATTGAACAAGATGGATTGCACGCAGGTTCTCCGGCCGCTTGGGTGGAGAGGCTATTCGGCTATGACTGGGCACAACAGACAAT
CGGCTGCTCTGATGCCGCCGTGTTCCGGCTGTCAGCGCAGGGGCGCCCGGTTCTTTTTGTCAAGACCGACCTGTCCGGTGCCC
TGAATGAACTGCAGGACGAGGCAGCGCGGCTATCGTGGCTGGCCACGACGGGCGTTCCTTGCGCAGCTGTGCTCGACGTTGTC
ACTGAAGCGGGAAGGGACTGGCTGCTATTGGGCGAAGTGCCGGGGCAGGATCTCCTGTCATCTCACCTTGCTCCTGCCGAGAA
AGTATCCATCATGGCTGATGCAATGCGGCGGCTGCATACGCTTGATCCGGCTACCTGCCCATTCGACCACCAAGCGAAACATC
GCATCGAGCGAGCACGTACTCGGATGGAAGCCGGTCTTGTCGATCAGGATGATCTGGACGAAGAGCATCAGGGGCTCGCGCCA
GCCGAACTGTTCGCCAGGCTCAAGGCGCGCATGCCCGACGGCGAGGATCTCGTCGTGACCCATGGCGATGCCTGCTTGCCGAA
TATCATGGTGGAAAATGGCCGCTTTTCTGGATTCATCGACTGTGGCCGGCTGGGTGTGGCGGACCGCTATCAGGACATAGCGT
TGGCTACCCGTGATATTGCTGAAGAGCTTGGCGGCGAATGGGCTGACCGCTTCCTCGTGCTTTACGGTATCGCCGCTCCCGAT
TCGCAGCGCATCGCCTTCTATCGCCTTCTTGACGAGTTCTTCTGAGATCGTTCAAACATTTGGCAATAAAGTTTCTTAAGATT
GAATCCTGTTGCCGGTCTTGCGATGATTATCATATAATTTCTGTTGAATTACGTTAAGCATGTAATAATTAACATGTAATGCA
TGACGTTATTTATGAGATGGGTTTTTATGATTAGAGTCCCGCAATTATACATTTAATACGCGATAGAAAACAAAATATAGCGC
GCAAACTAGGATAAATTATCGCGCGCGGTGTCATCTATGTTACTAGATCGGCGCGCCAAGGGCGAATTCCAGCACACTGGCGG
CCGTTACTAGTGGATCGAGCTCGTCGACTCTAGACTCGAGGGCGCGCCGA

**>SEQ ID NO 3 – Junction nucleotide sequence between 5' region of insert and sugarcane genome of event CTC75064.**

GTGATCCTAGACTTTCTAGAAATCTTAGGAAGATTACTACACTTTAGTTATTCATACCAAAAACTAATTCATAGCTTAACATG
ACCAAAATTGCTAATATTTCACAAATTGACGCTTAGACAACTTAATAACACATTGCGGACGTTTTTAATGTACTGAATTAGTA
CTGATATCGGTACCTTAATTCGGGGGATCTGGATTTTAGT

**>SEQ ID NO 4 – Junction nucleotide sequence between 3' region of insert and sugarcane genome of event CTC75064**

TCATCTATGTTACTAGATCGGCGCGCCAAGGGCGAATTCCAGCACACTGGCGGCCGTTACTAGTGGATCGAGCTCGTCGACTC
TAGACTCGAGGGCGCGCCGATACCAATTTGGACAGAAAACTGACAAGAACTTCGAAAATACATAATTGGAGTTCTAGAGACAC
AACAAAAGTGATCCTTAACTTTATGTAAAGCTTATTAAGT

**>SEQ ID NO 5 - Event CTC75064: flanking sequences and T-DNA**

GTGATCCTAGACTTTCTAGAAATCTTAGGAAGATTACTACACTTTAGTTATTCATACCAAAAACTAATTCATAGCTTAACATG
ACCAAAATTGCTAATATTTCACAAATTGACGCTTAGACAACTTAATAACACATTGCGGACGTTTTTAATGTACTGAATTAGTA
CTGATATCGGTACCTTAATTCGGGGGATCTGGATTTTAGTACTGGATTTTGGTTTTAGGAATTAGAAATTTTATTGATAGAAG

TATTTTACAAATACAAATACATACTAAGGGTTTCTTATATGCTCAACACATGAGCGAAACCCTATAGGAACCCTAATTCCCTT
ATCTGGGAACTACTCACACATTATTATGGAGAAACTCGAGCTTGTCGATCGATCACTCAGCCTCGAGGGTGGCGGTCACTGGG
ATGAACTCGAACCTGTCGATGATCACGCCGGCGGTGCCGCTGAAGTTCCTCACGCCCACGATGTTACCGAGGGAGGAGGTGAA
AGCGTTGGCGCTCTCGAAGTAACCGAAGTCGCTGGATTGGAGGTTGTCCAGGGAGGTAGCGGTAGCTGGCACGGTGTTGGAGA
AGATGGAGGAGTTACCCCAGTTCACGTTGAGGTGGATCGGGGTCACGGAAGCGTACCTCACGCGCACCCTGTACCTGGTGGAG
GTGGATGGGAAGTGGATTGGCACTCGATGTAGCCCCTGTTCTGGATGTTGTTGCCGCTGCTGTTGAGCCTCACGAGGTCGCCA
CCGGTGAAGCCTGGGCCGGAAATGACGGAACCGTTGAAGAGGAAGTTGCCCTTCACGGCCGGGATTTGGGTGATGCTGTCGGA
GGCGATGATGTTGTTGAACTCAGCGCTGCGGTGGATCCAGGAGAACATCGGAGCCCTGATGATGCTCACGGAGCTGTTGCTGA
AGCCGGAGCGGAACATGGACACGTGGCTCAGCCTGTGGGAGAAGCCTTGCCTGGGTGGCACGTTGTTGTTCTGTGGTGGGATC
TCGTCCAGGGAGTCCACGGTGCCGCTCTTCCTGTAGACAGCGGATGGCAGGTTGGAGGAGGTGCCGTAGGCGAACTCGGTGCC
GTCGAGCACGGACAGTTGCTGGTTGTTGATACCGATGTTGAAGGGCCTCCTGTACAGGGTGGAGGACAGGGTCCTGTAGACAC
CCTGACCCAGTTGAGCCACGATGCGTTGCTGTGGAGCGGCGTTGCCCATGGTGCCGTAGAGCGGGAAGGTGAACTCGGGGCCG
CTGAAGCCCACTGGGGAGGCCATGATCTGGTGGCCGGACCAGTAGTACTCGCCCCTGTGAGCGTCGGTGTAGATGGTGATGCT
GTTCAGGATGTCCATCAGGTGTGGGCTCCTGATGGAGCCCTCGATACCCTGGGCGGAACCGCGGAAGCTACCGTCGAAGTTCT
CCAGCACTGGGTTGGTGTAGATCTCCCTGGTGAGTTGGGACACGGTGCGGATCGGGTAGGTCCTGGAGTCGTAGTTCGGGAAG
AGGGACACAATGTCCAGCACGGTGAGGGTCAACTCCCTCCTGAACTGGTTGTACCTGATCCAGTCCCTGGAGTCCGGACCCCA
GACGCGCTCCAGGCCGGTGTTGTACCAGCGCACAGCGTGGTCGGTGTAGTTGCCAATCAGCCTGGTCAGGTCGTTGTAGCGGC
TGTTGATGGTGGCAGCATCGAAGCCCCACCTTTGGCCGAACACGCTCACGTCGCGCAGCACGCTGAGGTGCAGGTTAGCGGCT
TGCACGTACACGGACAGGAGCGGCACTTGGTAGTTCTGGACGGCGAACAGTGGGATAGCGGTGGTCAGGGCGCTGTTCATGTC
GTTGAATTGAATGCGCATTTCCTCGCGGAGAGCTGGGTTGGTCGGGTCGGCCTCCCACTCCCTGAAGCTCTCGGCGTAGATTT
GGTAGAGGTTGCTCAGGCCCTCCAGCCTGGAGATGGCCTGGTTCCTGGCGAACTCTTCGATCCTCTGGTTGATCAGCTGCTCG
ATTTGCACCAGGAAGGCGTCCCATTGGGATGGACCGAAGATACCCCAGATGATGTCCACCAGGCCGAGCACGAAGCCAGCACC
TGGCACGAACTCGCTGAGCAGGAACTGGGTCAAGGACAGGGAGATGTCGATGGGGGTGTAACCGGTCTCGATGCGCTCGCCAC
CCAGCACCTCCACCTCTGGGTTGCTCAGGCAGTTGTATGGGATGCACTCGTTGATGTTTGGGTTGTTGTCCATGGCGGGGTTG
ATCAGGTTGATCACTTCTACCTACAAAAAAGCTCCGCACGAGGCTGCATTTGTCACAAATCATGAAAAGAAAAACTACCGATG
AACAATGCTGAGGGATTCAAATTCTACCCACAAAAAGAAGAAAGAAAGATCTAGCACATCTAAGCCTGACGAAGCAGCAGAAA
TATATAAAAATATAAACCATAGTGCCCTTTTCCCCTCTTCCTGATCTTGTTTAGCACGGCGGAAATTTTAAACCCCCCATCAT
CTCCCCCAACAACGGCGGATCGCAGATCTACATCCGAGAGCCCCATTCCCCGCGAGATCCGGGCCGGATCCACGCCGGCGAGA
GCCCCAGCCGCGAGATCCCGCCCCTCCCGCGCACCGATCTGGGCGCGCACGAAGCCGCCTCTCGCCCACCCAAACTACCAAGG
CCAAAGATCGAGACCGAGACGGAAAAAAAAACGGAGAAAGAAAGAGGAGAGGGGCGGGGTGGTTACCGGCGGCGGCGGAGGCC
TCCCTTGGATCTTATGGTGTGTTGTCCCTGTGTGTTCTCCAATAGTGTGGCTTGAGTGTGTGGAAGATGGTTCTAGAGGATCT
GCTAGAGTCAGCTTGTCAGCGTGTCCTCTCCAAATGAAATGAACTTCCTTATATAGAGGAAGGGTCTTGCGAAGGATAGTGGG
ATTGTGCGTCATCCCTTACGTCAGTGGAGATATCACATCAATCCACTTGCTTTGAAGACGTGGTTGGAACGTCTTCTTTTTCC
ACGATGCTCCTCGTGGGTGGGGGTCCATCTTTGGGACCACTGTCGGCAGAGGCATCTTCAACGATGGCCTTTCCTTTATCGCA
ATGATGGCATTTGTAGGAGCCACCTTCCTTTTCCACTATCTTCACAATAAAGTGACAGATAGCTGGGCAATGGAATCCGAGGA
GGTTTCCGGATATTACCCTTTGTTGAAAAGTCTCAATCGGACCATCACATCAATCCACTTGCTTTGAAGACGTGGTTGGAACG
TCTTCTTTTTCCACGATGCTCCTCGTGGGTGGGGGTCCATCTTTGGGACCACTGTCGGCAGAGGCATCTTCAACGATGGCCTT
TCCTTTATCGCAATGATGGCATTTGTAGGAGCCACCTTCCTTTTCCACTATCTTCACAATAAAGTGACAGATAGCTGGGCAAT
GGAATCCGAGGAGGTTTCCGGATATTACCCTTTGTTGAAAAGTCTCAATCGGACCTGTTTAAACCCTGAAGCTTACGCGTATG
CCTGCAGTGCAGCGTGACCCGGTCGTGCCCCTCTCTAGAGATAATGAGCATTGCATGTCTAAGTTATAAAAAATTACCACATA
TTTTTTTTGTCACACTTGTTTGAAGTGCAGTTTATCTATCTTTATACATATATTTAAACTTTACTCTACGAATAATATAATCT
ATAGTACTACAATAATATCAGTGTTTTAGAGAATCATATAAATGAACAGTTAGACATGGTCTAAAGGACAATTGAGTATTTTG
ACAACAGGACTCTACAGTTTTATCTTTTTAGTGTGCATGTGTTCTCCTTTTTTTTTGCAAATAGCTTCACCTATATAATACTT
CATCCATTTTATTAGTACATCCATTTAGGGTTTAGGGTTAATGGTTTTTATAGACTAATTTTTTTAGTACATCTATTTTATTC
TATTTTAGCCTCTAAATTAAGAAAACTAAAACTCTATTTTAGTTTTTTTATTTAATAATTTAGATATAAAATAGAATAAAATA
AAGTGACTAAAAATTAAACAAATACCCTTTAAGAAATTAAAAAAACTAAGGAAACATTTTTCTTGTTTCGAGTAGATAATGCC
AGCCTGTTAAACGCCGTCGACGAGTCTAACGGACACCAACCAGCGAACCAGCAGCGTCGCGTCGGGCCAAGCGAAGCAGACGG
CACGGCATCTCTGTCGCTGCCTCTGGACCCCTCTCGAGAGTTCCGCTCCACCGTTGGACTTGCTCCGCTGTCGGCATCCAGAA
ATTGCGTGGCGGAGCGGCAGACGTGAGCCGGCACGGCAGGCGGCCTCCTCCTCCTCTCACGGCACGGCAGCTACGGGGGATTC
CTTTCCCACCGCTCCTTCGCTTTCCCTTCCTCGCCCGCCGTAATAAATAGACACCCCCTCCACACCCTCTTTCCCCAACCTCG
TGTTGTTCGGAGCGCACACACACACAACCAGATCTCCCCCAAATCCACCCGTCGGCACCTCCGCTTCAAGGTACGCCGCTCGT
CCTCCCCCCCCCCCCCTCTCTACCTTCTCTAGATCGGCGTTCCGGTCCATGGTTAGGGCCCGGTAGTTCTACTTCTGTTCATG
TTTGTGTTAGATCCGTGTTTGTGTTAGATCCGTGCTGCTAGCGTTCGTACACGGATGCGACCTGTACGTCAGACACGTTCTGA
TTGCTAACTTGCCAGTGTTTCTCTTTGGGGAATCCTGGGATGGCTCTAGCCGTTCCGCAGACGGGATCGATTTCATGATTTTT
TTTGTTTCGTTGCATAGGGTTTGGTTTGCCCTTTTCCTTTATTTCAATATATGCCGTGCACTTGTTTGTCGGGTCATCTTTTC
ATGCTTTTTTTTGTCTTGGTTGTGATGATGTGGTCTGGTTGGGCGGTCGTTCTAGATCGGAGTAGAATTCTGTTTCAAACTAC
CTGGTGGATTTATTAATTTTGGATCTGTATGTGTGTGCCATACATATTCATAGTTACGAATTGAAGATGATGGATGGAAATAT
CGATCTAGGATAGGTATACATGTTGATGCGGGTTTTACTGATGCATATACAGAGATGCTTTTTGTTCGCTTGGTTGTGATGAT

GTGGTGTGGTTGGGCGGTCGTTCATTCGTTCTAGATCGGAGTAGAATACTGTTTCAAACTACCTGGTGTATTTATTAATTTTG
GAACTGTATGTGTGTGTCATACATCTTCATAGTTACGAGTTTAAGATGGATGGAAATATCGATCTAGGATAGGTATACATGTT
GATGTGGGTTTTACTGATGCATATACATGATGGCATATGCAGCATCTATTCATATGCTCTAACCTTGAGTACCTATCTATTAT
AATAAACAAGTATGTTTTATAATTATTTTGATCTTGATATACTTGGATGATGGCATATGCAGCAGCTATATGTGGATTTTTTT
AGCCCTGCCTTCATACGCTATTTATTTGCTTGGTACTGTTTCTTTTGTCGATGCTCACCCTGTTGTTTGGTGTTACTTCTGCA
GGTCGACTCTAGAATGTGGATTGAACAAGATGGATTGCACGCAGGTTCTCCGGCCGCTTGGGTGGAGAGGCTATTCGGCTATG
ACTGGGCACAACAGACAATCGGCTGCTCTGATGCCGCCGTGTTCCGGCTGTCAGCGCAGGGGCGCCCGGTTCTTTTTGTCAAG
ACCGACCTGTCCGGTGCCCTGAATGAACTGCAGGACGAGGCAGCGCGGCTATCGTGGCTGGCCACGACGGGCGTTCCTTGCGC
AGCTGTGCTCGACGTTGTCACTGAAGCGGGAAGGGACTGGCTGCTATTGGGCGAAGTGCCGGGGCAGGATCTCCTGTCATCTC
ACCTTGCTCCTGCCGAGAAAGTATCCATCATGGCTGATGCAATGCGGCGGCTGCATACGCTTGATCCGGCTACCTGCCCATTC
GACCACCAAGCGAAACATCGCATCGAGCGAGCACGTACTCGGATGGAAGCCGGTCTTGTCGATCAGGATGATCTGGACGAAGA
GCATCAGGGGCTCGCGCCAGCCGAACTGTTCGCCAGGCTCAAGGCGCGCATGCCCGACGGCGAGGATCTCGTCGTGACCCATG
GCGATGCCTGCTTGCCGAATATCATGGTGGAAAATGGCCGCTTTTCTGGATTCATCGACTGTGGCCGGCTGGGTGTGGCGGAC
CGCTATCAGGACATAGCGTTGGCTACCCGTGATATTGCTGAAGAGCTTGGCGGCGAATGGGCTGACCGCTTCCTCGTGCTTTA
CGGTATCGCCGCTCCCGATTCGCAGCGCATCGCCTTCTATCGCCTTCTTGACGAGTTCTTCTGAGATCGTTCAAACATTTGGC
AATAAAGTTTCTTAAGATTGAATCCTGTTGCCGGTCTTGCGATGATTATCATATAATTTCTGTTGAATTACGTTAAGCATGTA
ATAATTAACATGTAATGCATGACGTTATTTATGAGATGGGTTTTTATGATTAGAGTCCCGCAATTATACATTTAATACGCGAT
AGAAAACAAAATATAGCGCGCAAACTAGGATAAATTATCGCGCGCGGTGTCATCTATGTTACTAGATCGGCGCGCCAAGGGCG
AATTCCAGCACACTGGCGGCCGTTACTAGTGGATCGAGCTCGTCGACTCTAGACTCGAGGGCGCGCCGATACCAATTTGGACA
GAAAACTGACAAGAACTTCGAAAATACATAATTGGAGTTCTAGAGACACAACAAAAGTGATCCTTAACTTTATGTAAAGCTTA
TTAAGT

**>SEQ** ID **NO 6** - **primer forward (Right Border)**
CAAGGGCGAATTCCAGCAC
**> SEQ** ID **NO 07** - primer reverse (Right border)
GTTCTTGTCAGTTTTCTGTCCAAA
**> SEQ** ID **NO 08** - - primer forward (Left Border)
CATGACCAAAATTGCTAATATTTCAC
**> SEQ** ID **NO 09** - - primer reverse (Left Border)
AATCCAGTACTAAAATCCAGATCCC
**> SEQ ID NO 10** - probe (Right border)
CCGTTACTAGTGGATCGA
**> SEQ ID NO 11** - - probe (Left border)
CTTAATAACACATTGCGGACGT

**> SEQ ID NO 12** - Amplicon Event CTC75064 (Right Border)

CAAGGGCGAATTCCAGCACACTGGCGGCCGTTACTAGTGGATCGAGCTCGTCGACTCTAGACTCGAGGGCGCGCCGATACCAA
TTTGGACAGAAAACTGACAAGAAC

**> SEQ ID NO 13 –** Amplicon Event CTC75064 (Left Border)

CATGACCAAAATTGCTAATATTTCACAAATTGACGCTTAGACAACTTAATAACACATTGCGGACGTTTTTAATGTACTGAATT
AGTACTGATATCGGTACCTTAATTCGGGGGATCTGGATTTTAGTACTGGATT

> **SEQ ID 14 - Binary plasmid comprising cry1Ac and nptII genes**

TGGCAGGATATATTGTGGTGTAAACAAATTGACGCTTAGACAACTTAATAACACATTGCGGACGTTTTTAATGTACTGAATTA
GTACTGATATCGGTACCTTAATTCGGGGGATCTGGATTTTAGTACTGGATTTTGGTTTTAGGAATTAGAAATTTTATTGATAG
AAGTATTTTACAAATACAAATACATACTAAGGGTTTCTTATATGCTCAACACATGAGCGAAACCCTATAGGAACCCTAATTCC

CTTATCTGGGAACTACTCACACATTATTATGGAGAAACTCGAGCTTGTCGATCGATCACTCAGCCTCGAGGGTGGCGGTCACT
GGGATGAACTCGAACCTGTCGATGATCACGCCGGCGGTGCCGCTGAAGTTCCTCACGCCCACGATGTTACCGAGGGAGGAGGT
GAAAGCGTTGGCGCTCTCGAAGTAACCGAAGTCGCTGGATTGGAGGTTGTCCAGGGAGGTAGCGGTAGCTGGCACGGTGTTGG
AGAAGATGGAGGAGTTACCCCAGTTCACGTTGAGGTGGATCGGGGTCACGGAAGCGTACCTCACGCGCACCCTGTACCTGGTG
GAGGTGGATGGGAAGTGGATTGGCACCTCGATGTAGCCCCTGTTCTGGATGTTGTTGCCGCTGCTGTTGAGCCTCACGAGGTC
GCCACCGGTGAAGCCTGGGCCGGAAATGACGGAACCGTTGAAGAGGAAGTTGCCCTTCACGGCCGGGATTTGGGTGATGCTGT
CGGAGGCGATGATGTTGTTGAACTCAGCGCTGCGGTGGATCCAGGAGAACATCGGAGCCCTGATGATGCTCACGGAGCTGTTG
CTGAAGCCGGAGCGGAACATGGACACGTGGCTCAGCCTGTGGGAGAAGCCTTGCCTGGGTGGCACGTTGTTGTTCTGTGGTGG
GATCTCGTCCAGGGAGTCCACGGTGCCGCTCTTCCTGTAGACAGCGGATGGCAGGTTGGAGGAGGTGCCGTAGGCGAACTCGG
TGCCGTCGAGCACGGACAGTTGCTGGTTGTTGATACCGATGTTGAAGGGCCTCCTGTACAGGGTGGAGGACAGGGTCCTGTAG
ACACCCTGACCCAGTTGAGCCACGATGCGTTGCTGTGGAGCGGCGTTGCCCATGGTGCCGTAGAGCGGGAAGGTGAACTCGGG
GCCGCTGAAGCCCACTGGGGAGGCCATGATCTGGTGGCCGGACCAGTAGTACTCGCCCCTGTGAGCGTCGGTGTAGATGGTGA
TGCTGTTCAGGATGTCCATCAGGTGTGGGCTCCTGATGGAGCCCTCGATACCCTGGGCGGAACCGCGGAAGCTACCGTCGAAG
TTCTCCAGCACTGGGTTGGTGTAGATCTCCCTGGTGAGTTGGGACACGGTGCGGATCGGGTAGGTCCTGGAGTCGTAGTTCGG
GAAGAGGGACACAATGTCCAGCACGGTGAGGGTCAACTCCCTCCTGAACTGGTTGTACCTGATCCAGTCCCTGGAGTCCGGAC
CCCAGACGCGCTCCAGGCCGGTGTTGTACCAGCGCACAGCGTGGTCGGTGTAGTTGCCAATCAGCCTGGTCAGGTCGTTGTAG
CGGCTGTTGATGGTGGCAGCATCGAAGCCCCACCTTTGGCCGAACACGCTCACGTCGCGCAGCACGCTGAGGTGCAGGTTAGC
GGCTTGCACGTACACGGACAGGAGCGGCACTTGGTAGTTCTGGACGGCGAACAGTGGGATAGCGGTGGTCAGGGCGCTGTTCA
TGTCGTTGAATTGAATGCGCATTTCCTCGCGGAGAGCTGGGTTGGTCGGGTCGGCCTCCCACTCCCTGAAGCTCTCGGCGTAG
ATTTGGTAGAGGTTGCTCAGGCCCTCCAGCCTGGAGATGGCCTGGTTCCTGGCGAACTCTTCGATCCTCTGGTTGATCAGCTG
CTCGATTTGCACCAGGAAGGCGTCCCATTGGGATGGACCGAAGATACCCCAGATGATGTCCACCAGGCCGAGCACGAAGCCAG
CACCTGGCACGAACTCGCTGAGCAGGAACTGGGTCAAGGACAGGGAGATGTCGATGGGGGTGTAACCGGTCTCGATGCGCTCG
CCACCCAGCACCTCCACCTCTGGGTTGCTCAGGCAGTTGTATGGGATGCACTCGTTGATGTTTGGGTTGTTGTCCATGGCGGG
GTTGATCAGGTTGATCACTTCTACCTACAAAAAAGCTCCGCACGAGGCTGCATTTGTCACAAATCATGAAAAGAAAAACTACC
GATGAACAATGCTGAGGGATTCAAATTCTACCCACAAAAAGAAGAAAGAAAGATCTAGCACATCTAAGCCTGACGAAGCAGCA
GAAATATATAAAAATATAAACCATAGTGCCCTTTTCCCCTCTTCCTGATCTTGTTTAGCACGGCGGAAATTTTAAACCCCCCA
TCATCTCCCCCAACAACGGCGGATCGCAGATCTACATCCGAGAGCCCCATTCCCCGCGAGATCCGGGCCGGATCCACGCCGGC
GAGAGCCCCAGCCGCGAGATCCCGCCCCTCCCGCGCACCGATCTGGGCGCGCACGAAGCCGCCTCTCGCCCACCCAAACTACC
AAGGCCAAAGATCGAGACCGAGACGGAAAAAAAAACGGAGAAAGAAAGAGGAGAGGGGCGGGGTGGTTACCGGCGGCGGCGGA
GGCCTCCCTTGGATCTTATGGTGTGTTGTCCCTGTGTGTTCTCCAATAGTGTGGCTTGAGTGTGTGGAAGATGGTTCTAGAGG
ATCTGCTAGAGTCAGCTTGTCAGCGTGTCCTCTCCAAATGAAATGAACTTCCTTATATAGAGGAAGGGTCTTGCGAAGGATAG
TGGGATTGTGCGTCATCCCTTACGTCAGTGGAGATATCACATCAATCCACTTGCTTTGAAGACGTGGTTGGAACGTCTTCTTT
TTCCACGATGCTCCTCGTGGGTGGGGGTCCATCTTTGGGACCACTGTCGGCAGAGGCATCTTCAACGATGGCCTTTCCTTTAT
CGCAATGATGGCATTTGTAGGAGCCACCTTCCTTTTCCACTATCTTCACAATAAAGTGACAGATAGCTGGGCAATGGAATCCG
AGGAGGTTTCCGGATATTACCCTTTGTTGAAAAGTCTCAATCGGACCATCACATCAATCCACTTGCTTTGAAGACGTGGTTGG
AACGTCTTCTTTTTCCACGATGCTCCTCGTGGGTGGGGGTCCATCTTTGGGACCACTGTCGGCAGAGGCATCTTCAACGATGG
CCTTTCCTTTATCGCAATGATGGCATTTGTAGGAGCCACCTTCCTTTTCCACTATCTTCACAATAAAGTGACAGATAGCTGGG
CAATGGAATCCGAGGAGGTTTCCGGATATTACCCTTTGTTGAAAAGTCTCAATCGGACCTGTTTAAACCCTGAAGCTTACGCG
TATGCCTGCAGTGCAGCGTGACCCGGTCGTGCCCCTCTCTAGAGATAATGAGCATTGCATGTCTAAGTTATAAAAAATTACCA
CATATTTTTTTTGTCACACTTGTTTGAAGTGCAGTTTATCTATCTTTATACATATATTTAAACTTTACTCTACGAATAATATA
ATCTATAGTACTACAATAATATCAGTGTTTTAGAGAATCATATAAATGAACAGTTAGACATGGTCTAAAGGACAATTGAGTAT
TTTGACAACAGGACTCTACAGTTTTATCTTTTTAGTGTGCATGTGTTCTCCTTTTTTTTTGCAAATAGCTTCACCTATATAAT
ACTTCATCCATTTTATTAGTACATCCATTTAGGGTTTAGGGTTAATGGTTTTTATAGACTAATTTTTTTAGTACATCTATTTT
ATTCTATTTTAGCCTCTAAATTAAGAAAACTAAAACTCTATTTTAGTTTTTTTATTTAATAATTTAGATATAAAATAGAATAA
AATAAAGTGACTAAAAATTAAACAAATACCCTTTAAGAAATTAAAAAAACTAAGGAAACATTTTTCTTGTTTCGAGTAGATAA
TGCCAGCCTGTTAAACGCCGTCGACGAGTCTAACGGACACCAACCAGCGAACCAGCAGCGTCGCGTCGGGCCAAGCGAAGCAG
ACGGCACGGCATCTCTGTCGCTGCCTCTGGACCCCTCTCGAGAGTTCCGCTCCACCGTTGGACTTGCTCCGCTGTCGGCATCC
AGAAATTGCGTGGCGGAGCGGCAGACGTGAGCCGGCACGGCAGGCGGCCTCCTCCTCCTCTCACGGCACGGCAGCTACGGGGG
ATTCCTTTCCCACCGCTCCTTCGCTTTCCCTTCCTCGCCCGCCGTAATAAATAGACACCCCCTCCACACCCTCTTTCCCCAAC
CTCGTGTTGTTCGGAGCGCACACACACACAACCAGATCTCCCCCAAATCCACCCGTCGGCACCTCCGCTTCAAGGTACGCCGC
TCGTCCTCCCCCCCCCCCCCTCTCTACCTTCTCTAGATCGGCGTTCCGGTCCATGGTTAGGGCCCGGTAGTTCTACTTCTGTT
CATGTTTGTGTTAGATCCGTGTTTGTGTTAGATCCGTGCTGCTAGCGTTCGTACACGGATGCGACCTGTACGTCAGACACGTT
CTGATTGCTAACTTGCCAGTGTTTCTCTTTGGGGAATCCTGGGATGGCTCTAGCCGTTCCGCAGACGGGATCGATTTCATGAT
TTTTTTTGTTTCGTTGCATAGGGTTTGGTTTGCCCTTTTCCTTTATTTCAATATATGCCGTGCACTTGTTTGTCGGGTCATCT
TTTCATGCTTTTTTTTGTCTTGGTTGTGATGATGTGGTCTGGTTGGGCGGTCGTTCTAGATCGGAGTAGAATTCTGTTTCAAA
CTACCTGGTGGATTTATTAATTTTGGATCTGTATGTGTGTGCCATACATATTCATAGTTACGAATTGAAGATGATGGATGGAA
ATATCGATCTAGGATAGGTATACATGTTGATGCGGGTTTTACTGATGCATATACAGAGATGCTTTTTGTTCGCTTGGTTGTGA
TGATGTGGTGTGGTTGGGCGGTCGTTCATTCGTTCTAGATCGGAGTAGAATACTGTTTCAAACTACCTGGTGTATTTATTAAT

TTTGGAACTGTATGTGTGTGTCATACATCTTCATAGTTACGAGTTTAAGATGGATGGAAATATCGATCTAGGATAGGTATACA
TGTTGATGTGGGTTTTACTGATGCATATACATGATGGCATATGCAGCATCTATTCATATGCTCTAACCTTGAGTACCTATCTA
TTATAATAAACAAGTATGTTTTATAATTATTTTGATCTTGATATACTTGGATGATGGCATATGCAGCAGCTATATGTGGATTT
TTTTAGCCCTGCCTTCATACGCTATTTATTTGCTTGGTACTGTTTCTTTTGTCGATGCTCACCCTGTTGTTTGGTGTTACTTC
TGCAGGTCGACTCTAGAATGTGGATTGAACAAGATGGATTGCACGCAGGTTCTCCGGCCGCTTGGGTGGAGAGGCTATTCGGC
TATGACTGGGCACAACAGACAATCGGCTGCTCTGATGCCGCCGTGTTCCGGCTGTCAGCGCAGGGGCGCCCGGTTCTTTTTGT
CAAGACCGACCTGTCCGGTGCCCTGAATGAACTGCAGGACGAGGCAGCGCGGCTATCGTGGCTGGCCACGACGGGCGTTCCTT
GCGCAGCTGTGCTCGACGTTGTCACTGAAGCGGGAAGGGACTGGCTGCTATTGGGCGAAGTGCCGGGGCAGGATCTCCTGTCA
TCTCACCTTGCTCCTGCCGAGAAAGTATCCATCATGGCTGATGCAATGCGGCGGCTGCATACGCTTGATCCGGCTACCTGCCC
ATTCGACCACCAAGCGAAACATCGCATCGAGCGAGCACGTACTCGGATGGAAGCCGGTCTTGTCGATCAGGATGATCTGGACG
AAGAGCATCAGGGGCTCGCGCCAGCCGAACTGTTCGCCAGGCTCAAGGCGCGCATGCCCGACGGCGAGGATCTCGTCGTGACC
CATGGCGATGCCTGCTTGCCGAATATCATGGTGGAAAATGGCCGCTTTTCTGGATTCATCGACTGTGGCCGGCTGGGTGTGGC
GGACCGCTATCAGGACATAGCGTTGGCTACCCGTGATATTGCTGAAGAGCTTGGCGGCGAATGGGCTGACCGCTTCCTCGTGC
TTTACGGTATCGCCGCTCCCGATTCGCAGCGCATCGCCTTCTATCGCCTTCTTGACGAGTTCTTCTGAGATCGTTCAAACATT
TGGCAATAAAGTTTCTTAAGATTGAATCCTGTTGCCGGTCTTGCGATGATTATCATATAATTTCTGTTGAATTACGTTAAGCA
TGTAATAATTAACATGTAATGCATGACGTTATTTATGAGATGGGTTTTTATGATTAGAGTCCCGCAATTATACATTTAATACG
CGATAGAAAACAAAATATAGCGCGCAAACTAGGATAAATTATCGCGCGCGGTGTCATCTATGTTACTAGATCGGCGCGCCAAG
GGCGAATTCCAGCACACTGGCGGCCGTTACTAGTGGATCGAGCTCGTCGACTCTAGACTCGAGGGCGCGCCGACAGGATATAT
TGGCGGGTAAACCTTAGAATAACGGATATTTAAAAGGGCGTGAAAAGGTTTATCCGTTCGTCCATTTGTATGTGCATGCCAAC
CACAGGGTTCCCCTCGGGATCAAAGTACTTTGATCCAACCCCTCCGCTGCTATAGTGCAGTCGGCTTCTGACGTTCAGTGCAG
CCGTCTTCTGAAAACGACATGTCGCACAAGTCCTAAGTTACGCGACAGGCTGCCGCCCTGCCCTTTTCCTGGCGTTTTCTTGT
CGCGTGTTTTAGTCGCATAAAGTAGAATACTTGCGACTAGAACCGGAGACATTACGCCATGAACAAGAGCGCCGCCGCTGGCC
TGCTGGGCTATGCCCGCGTCAGCACCGACGACCAGGACTTGACCAACCAACGGGCCGAACTGCACGCGGCCGGCTGCACCAAG
CTGTTTTCCGAGAAGATCACCGGCACCAGGCGCGACCGCCCGGAGCTGGCCAGGATGCTTGACCACCTACGCCCTGGCGACGT
TGTGACAGTGACCAGGCTAGACCGCCTGGCCCGCAGCACCCGCGACCTACTGGACATTGCCGAGCGCATCCAGGAGGCCGGCG
CGGGCCTGCGTAGCCTGGCAGAGCCGTGGGCCGACACCACCACGCCGGCCGGCCGCATGGTGTTGACCGTGTTCGCCGGCATT
GCCGAGTTCGAGCGTTCCCTAATCATCGACCGCACCCGGAGCGGGCGCGAGGCCGCCAAGGCCCGAGGCGTGAAGTTTGGCCC
CCGCCCTACCCTCACCCCGGCACAGATCGCGCACGCCCGCGAGCTGATCGACCAGGAAGGCCGCACCGTGAAAGAGGCGGCTG
CACTGCTTGGCGTGCATCGCTCGACCCTGTACCGCGCACTTGAGCGCAGCGAGGAAGTGACGCCCACCGAGGCCAGGCGGCGC
GGTGCCTTCCGTGAGGACGCATTGACCGAGGCCGACGCCCTGGCGGCCGCCGAGAATGAACGCCAAGAGGAACAAGCATGAAA
CCGCACCAGGACGGCCAGGACGAACCGTTTTTCATTACCGAAGAGATCGAGGCGGAGATGATCGCGGCCGGGTACGTGTTCGA
GCCGCCCGCGCACGTCTCAACCGTGCGGCTGCATGAAATCCTGGCCGGTTTGTCTGATGCCAAGCTGGCGGCCTGGCCGGCCA
GCTTGGCCGCTGAAGAAACCGAGCGCCGCCGTCTAAAAAGGTGATGTGTATTTGAGTAAAACAGCTTGCGTCATGCGGTCGCT
GCGTATATGATGCGATGAGTAAATAAACAAATACGCAAGGGGAACGCATGAAGGTTATCGCTGTACTTAACCAGAAAGGCGGG
TCAGGCAAGACGACCATCGCAACCCATCTAGCCCGCGCCCTGCAACTCGCCGGGGCCGATGTTCTGTTAGTCGATTCCGATCC
CCAGGGCAGTGCCCGCGATTGGGCGGCCGTGCGGGAAGATCAACCGCTAACCGTTGTCGGCATCGACCGCCCGACGATTGACC
GCGACGTGAAGGCCATCGGCCGGCGCGACTTCGTAGTGATCGACGGAGCGCCCCAGGCGGCGGACTTGGCTGTGTCCGCGATC
AAGGCAGCCGACTTCGTGCTGATTCCGGTGCAGCCAAGCCCTTACGACATATGGGCCACCGCCGACCTGGTGGAGCTGGTTAA
GCAGCGCATTGAGGTCACGGATGGAAGGCTACAAGCGGCCTTTGTCGTGTCGCGGGCGATCAAAGGCACGCGCATCGGCGGTG
AGGTTGCCGAGGCGCTGGCCGGGTACGAGCTGCCCATTCTTGAGTCCCGTATCACGCAGCGCGTGAGCTACCCAGGCACTGCC
GCCGCCGGCACAACCGTTCTTGAATCAGAACCCGAGGGCGACGCTGCCCGCGAGGTCCAGGCGCTGGCCGCTGAAATTAAATC
AAAACTCATTTGAGTTAATGAGGTAAAGAGAAAATGAGCAAAAGCACAAACACGCTAAGTGCCGGCCGTCCGAGCGCACGCAG
CAGCAAGGCTGCAACGTTGGCCAGCCTGGCAGACACGCCAGCCATGAAGCGGGTCAACTTTCAGTTGCCGGCGGAGGATCACA
CCAAGCTGAAGATGTACGCGGTACGCCAAGGCAAGACCATTACCGAGCTGCTATCTGAATACATCGCGCAGCTACCAGAGTAA
ATGAGCAAATGAATAAATGAGTAGATGAATTTTAGCGGCTAAAGGAGGCGGCATGGAAAATCAAGAACAACCAGGCACCGACG
CCGTGGAATGCCCCATGTGTGGAGGAACGGGCGGTTGGCCAGGCGTAAGCGGCTGGGTTGTCTGCCGGCCCTGCAATGGCACT
GGAACCCCCAAGCCCGAGGAATCGGCGTGACGGTCGCAAACCATCCGGCCCGGTACAAATCGGCGCGGCGCTGGGTGATGACC
TGGTGGAGAAGTTGAAGGCCGCGCAGGCCGCCCAGCGGCAACGCATCGAGGCAGAAGCACGCCCCGGTGAATCGTGGCAAGCG
GCCGCTGATCGAATCCGCAAAGAATCCCGGCAACCGCCGGCAGCCGGTGCGCCGTCGATTAGGAAGCCGCCCAAGGGCGACGA
GCAACCAGATTTTTTCGTTCCGATGCTCTATGACGTGGGCACCCGCGATAGTCGCAGCATCATGGACGTGGCCGTTTTCCGTC
TGTCGAAGCGTGACCGACGAGCTGGCGAGGTGATCCGCTACGAGCTTCCAGACGGGCACGTAGAGGTTTCCGCAGGGCCGGCC
GGCATGGCCAGTGTGTGGGATTACGACCTGGTACTGATGGCGGTTTCCCATCTAACCGAATCCATGAACCGATACCGGGAAGG
GAAGGGAGACAAGCCCGGCCGCGTGTTCCGTCCACACGTTGCGGACGTACTCAAGTTCTGCCGGCGAGCCGATGGCGGAAAGC
AGAAAGACGACCTGGTAGAAACCTGCATTCGGTTAAACACCACGCACGTTGCCATGCAGCGTACGAAGAAGGCCAAGAACGGC
CGCCTGGTGACGGTATCCGAGGGTGAAGCCTTGATTAGCCGCTACAAGATCGTAAAGAGCGAAACCGGGCGGCCGGAGTACAT
CGAGATCGAGCTAGCTGATTGGATGTACCGCGAGATCACAGAAGGCAAGAACCCGGACGTGCTGACGGTTCACCCCGATTACT
TTTTGATCGATCCCGGCATCGGCCGTTTTCTCTACCGCCTGGCACGCCGCGCCGCAGGCAAGGCAGAAGCCAGATGGTTGTTC
AAGACGATCTACGAACGCAGTGGCAGCGCCGGAGAGTTCAAGAAGTTCTGTTTCACCGTGCGCAAGCTGATCGGGTCAAATGA

CCTGCCGGAGTACGATTTGAAGGAGGAGGCGGGGCAGGCTGGCCCGATCCTAGTCATGCGCTACCGCAACCTGATCGAGGGCG
AAGCATCCGCCGGTTCCTAATGTACGGAGCAGATGCTAGGGCAAATTGCCCTAGCAGGGGAAAAAGGTCGAAAAGGTCTCTTT
CCTGTGGATAGCACGTACATTGGGAACCCAAAGCCGTACATTGGGAACCGGAACCCGTACATTGGGAACCCAAAGCCGTACAT
TGGGAACCGGTCACACATGTAAGTGACTGATATAAAAGAGAAAAAAGGCGATTTTTCCGCCTAAAACTCTTTAAAACTTATTA
AAACTCTTAAAACCCGCCTGGCCTGTGCATAACTGTCTGGCCAGCGCACAGCCGAAGAGCTGCAAAAAGCGCCTACCCTTCGG
TCGCTGCGCTCCCTACGCCCCGCCGCTTCGCGTCGGCCTATCGCGGCCGCTGGCCGCTCAAAAATGGCTGGCCTACGGCCAGG
CAATCTACCAGGGCGCGGACAAGCCGCGCCGTCGCCACTCGACCGCCGGCGCCCACATCAAGGCACCCTGCCTCGCGCGTTTC
GGTGATGACGGTGAAAACCTCTGACACATGCAGCTCCCGGAGACGGTCACAGCTTGTCTGTAAGCGGATGCCGGGAGCAGACA
AGCCCGTCAGGGCGCGTCAGCGGGTGTTGGCGGGTGTCGGGGCGCAGCCATGACCCAGTCACGTAGCGATAGCGGAGTGTATA
CTGGCTTAACTATGCGGCATCAGAGCAGATTGTACTGAGAGTGCACCATATGCGGTGTGAAATACCGCACAGATGCGTAAGGA
GAAAATACCGCATCAGGCGCTCTTCCGCTTCCTCGCTCACTGACTCGCTGCGCTCGGTCGTTCGGCTGCGGCGAGCGGTATCA
GCTCACTCAAAGGCGGTAATACGGTTATCCACAGAATCAGGGGATAACGCAGGAAAGAACATGTGAGCAAAAGGCCAGCAAAA
GGCCAGGAACCGTAAAAAGGCCGCGTTGCTGGCGTTTTTCCATAGGCTCCGCCCCCCTGACGAGCATCACAAAAATCGACGCT
CAAGTCAGAGGTGGCGAAACCCGACAGGACTATAAAGATACCAGGCGTTTCCCCCTGGAAGCTCCCTCGTGCGCTCTCCTGTT
CCGACCCTGCCGCTTACCGGATACCTGTCCGCCTTTCTCCCTTCGGGAAGCGTGGCGCTTTCTCATAGCTCACGCTGTAGGTA
TCTCAGTTCGGTGTAGGTCGTTCGCTCCAAGCTGGGCTGTGTGCACGAACCCCCCGTTCAGCCCGACCGCTGCGCCTTATCCG
GTAACTATCGTCTTGAGTCCAACCCGGTAAGACACGACTTATCGCCACTGGCAGCAGCCACTGGTAACAGGATTAGCAGAGCG
AGGTATGTAGGCGGTGCTACAGAGTTCTTGAAGTGGTGGCCTAACTACGGCTACACTAGAAGGACAGTATTTGGTATCTGCGC
TCTGCTGAAGCCAGTTACCTTCGGAAAAAGAGTTGGTAGCTCTTGATCCGGCAAACAAACCACCGCTGGTAGCGGTGGTTTTT
TTGTTTGCAAGCAGCAGATTACGCGCAGAAAAAAAGGATCTCAAGAAGATCCTTTGATCTTTTCTACGGGGTCTGACGCTCAG
TGGAACGAAAACTCACGTTAAGGGATTTTGGTCATGCATTCTAGGTATTATTTGCCAACGACCTTCGTGATCTCGCCCTTGAC
ATAGTGGACAAATTCTTCGAGCTGGTCGGCCCGGGACGCGAGACGGTCTTCTTCTTGGCCCAGATAGGCTTGGCGCGCTTCGA
GGATCACGGGCTGGTATTGCGCCGGAAGGCGCTCCATCGCCCAGTCGGCGGCGACATCCTTCGGCGCGATCTTGCCGGTAACC
GCCGAGTACCAAATCCGGCTCAGCGTAAGGACCACATTGCGCTCATCGCCCGCCCAATCCGGCGGGGAGTTCCACAGGGTCAG
CGTCTCGTTCAGTGCTTCGAACAGATCCTGTTCCGGCACCGGGTCGAAAAGTTCCTCGGCCGCGGGGCCGACGAGGGCCACGC
TATGCTCCCGGGCCTTGGTGAGCAGGATCGCCAGATCAATGTCGATGGTGGCCGGTTCAAAGATACCCGCCAGAATATCATTA
CGCTGCCATTCGCCGAACTGGAGTTCGCGTTTGGCCGGATAGCGCCAGGGGATGATGTCATCGTGCACCACAATCGTCACCTC
AACCGCGCGCAGGATTTCGCTCTCGCCGGGGGAGGCGGACGTTTCCAGAAGGTCGTTGATAAGCGCGCGGCGCGTGGTCTCGT
CGAGACGGACGGTAACGGTGACAAGCAGGTCGATGTCCGAATGGGGCTTAAGGCCGCCGTCAACGGCGCTACCATACAGATGC
ACGGCGAGGAGGGTCGGTTCGAGGTGGCGCTCGATGACACCCACGACTTCCGACAGCTGGGTGGACACCTCGGCGATGACCGC
TTCACCCATTTATTATTTCCTTCCTCTTTTCTACAGTATTTAAAGATACCCCAAGAAGCTAATTATAACAAGACGAACTCCAA
TTCACTGTTCCTTGCATTCTAAAACCTTAAATACCAGAAAACAGCTTTTTCAAAGTTGTTTTCAAAGTTGGCGTATAACATAG
TATCGACGGAGCCGATTTTGAAACCGCGGTGATCACAGGCAGCAACGCTCTGTCATCGTTACAATCAACATGCTACCCTCCGC
GAGATCATCCGTGTTTCAAACCCGGCAGCTTAGTTGCCGTTCTTCCGAATAGCATCGGTAACATGAGCAAAGTCTGCCGCCTT
ACAACGGCTCTCCCGCTGACGCCGTCCCGGACTGATGGGCTGCCTGTATCGAGTGGTGATTTTGTGCCGAGCTGCCGGTCGGG
GAGCTGTTGGCTGGCTGG

**>SEQ ID NO 15 - primer forward: sugarcane poly-ubiquitin gene**
ACCATTACCCTGGAGGTTGAGA
**>SEQ ID NO 16 - primer reverse: sugarcane poly-ubiquitin gene**
GTCCTGGATCTTCGCCTTCA
**>SEQ ID NO 17 - probe sugarcane poly-ubiquitin gene**
CTCTGACACCATCGAC
**>SEQ ID NO 18 - Junction sequence between 5'region of insert and sugarcane genome of event CTC75064**
TTGCTAATATTTCACAAATTGACGCT
**>SEQ ID NO 19 - Junction sequence between 3'region of insert and sugarcane genome of event CTC75064**
GAGGGCGCGCCGATACCAATTTGGAC

**>SEQ ID 20 - Sugarcane optimized truncate cry1Ac gene (B. thurigiensis).**

ATGGACAACAACCCAAACATCAACGAGTGCATCCCATACAACTGCCTGAGCAACCCAGAGGTGGAGGTGCTGGGTGGCGAGCG
CATCGAGACCGGTTACACCCCCATCGACATCTCCCTGTCCTTGACCCAGTTCCTGCTCAGCGAGTTCGTGCCAGGTGCTGGCT
TCGTGCTCGGCCTGGTGGACATCATCTGGGGTATCTTCGGTCCATCCCAATGGGACGCCTTCCTGGTGCAAATCGAGCAGCTG
ATCAACCAGAGGATCGAAGAGTTCGCCAGGAACCAGGCCATCTCCAGGCTGGAGGGCCTGAGCAACCTCTACCAAATCTACGC
CGAGAGCTTCAGGGAGTGGGAGGCCGACCCGACCAACCCAGCTCTCCGCGAGGAAATGCGCATTCAATTCAACGACATGAACA
GCGCCCTGACCACCGCTATCCCACTGTTCGCCGTCCAGAACTACCAAGTGCCGCTCCTGTCCGTGTACGTGCAAGCCGCTAAC
CTGCACCTCAGCGTGCTGCGCGACGTGAGCGTGTTCGGCCAAAGGTGGGGCTTCGATGCTGCCACCATCAACAGCCGCTACAA
CGACCTGACCAGGCTGATTGGCAACTACACCGACCACGCTGTGCGCTGGTACAACACCGGCCTGGAGCGCGTCTGGGGTCCGG
ACTCCAGGGACTGGATCAGGTACAACCAGTTCAGGAGGGAGTTGACCCTCACCGTGCTGGACATTGTGTCCCTCTTCCCGAAC
TACGACTCCAGGACCTACCCGATCCGCACCGTGTCCCAACTCACCAGGGAGATCTACACCAACCCAGTGCTGGAGAACTTCGA
CGGTAGCTTCCGCGGTTCCGCCCAGGGTATCGAGGGCTCCATCAGGAGCCCACACCTGATGGACATCCTGAACAGCATCACCA
TCTACACCGACGCTCACAGGGGCGAGTACTACTGGTCCGGCCACCAGATCATGGCCTCCCCAGTGGGCTTCAGCGGCCCCGAG
TTCACCTTCCCGCTCTACGGCACCATGGGCAACGCCGCTCCACAGCAACGCATCGTGGCTCAACTGGGTCAGGGTGTCTACAG
GACCCTGTCCTCCACCCTGTACAGGAGGCCCTTCAACATCGGTATCAACAACCAGCAACTGTCCGTGCTCGACGGCACCGAGT
TCGCCTACGGCACCTCCTCCAACCTGCCATCCGCTGTCTACAGGAAGAGCGGCACCGTGGACTCCCTGGACGAGATCCCACCA
CAGAACAACAACGTGCCACCCAGGCAAGGCTTCTCCCACAGGCTGAGCCACGTGTCCATGTTCCGCTCCGGCTTCAGCAACAG
CTCCGTGAGCATCATCAGGGCTCCGATGTTCTCCTGGATCCACCGCAGCGCTGAGTTCAACAACATCATCGCCTCCGACAGCA
TCACCCAAATCCCGGCCGTGAAGGGCAACTTCCTCTTCAACGGTTCCGTCATTTCCGGCCCAGGCTTCACCGGTGGCGACCTC
GTGAGGCTCAACAGCAGCGGCAACAACATCCAGAACAGGGGCTACATCGAGGTGCCAATCCACTTCCCATCCACCTCCACCAG
GTACAGGGTGCGCGTGAGGTACGCTTCCGTGACCCCGATCCACCTCAACGTGAACTGGGGTAACTCCTCCATCTTCTCCAACA
CCGTGCCAGCTACCGCTACCTCCCTGGACAACCTCCAATCCAGCGACTTCGGTTACTTCGAGAGCGCCAACGCTTTCACCTCC
TCCCTCGGTAACATCGTGGGCGTGAGGAACTTCAGCGGCACCGCCGGCGTGATCATCGACAGGTTCGAGTTCATCCCAGTGAC
CGCCACCCTCGAGGCTGAGTGA

**> SEQ ID NO 21- Sugarcane optimized nptII gene (B. thurigiensis)**

TCAGAAGAACTCGTCAAGAAGGCGATAGAAGGCGATGCGCTGCGAATCGGGAGCGGCGATACCGTAAAGCACGAGGAAGCGGT
CAGCCCATTCGCCGCCAAGCTCTTCAGCAATATCACGGGTAGCCAACGCTATGTCCTGATAGCGGTCCGCCACACCCAGCCGG
CCACAGTCGATGAATCCAGAAAAGCGGCCATTTTCCACCATGATATTCGGCAAGCAGGCATCGCCATGGGTCACGACGAGATC
CTCGCCGTCGGGCATGCGCGCCTTGAGCCTGGCGAACAGTTCGGCTGGCGCGAGCCCCTGATGCTCTTCGTCCAGATCATCCT
GATCGACAAGACCGGCTTCCATCCGAGTACGTGCTCGCTCGATGCGATGTTTCGCTTGGTGGTCGAATGGGCAGGTAGCCGGA
TCAAGCGTATGCAGCCGCCGCATTGCATCAGCCATGATGGATACTTTCTCGGCAGGAGCAAGGTGAGATGACAGGAGATCCTG
CCCCGGCACTTCGCCCAATAGCAGCCAGTCCCTTCCCGCTTCAGTGACAACGTCGAGCACAGCTGCGCAAGGAACGCCCGTCG
TGGCCAGCCACGATAGCCGCGCTGCCTCGTCCTGCAGTTCATTCAGGGCACCGGACAGGTCGGTCTTGACAAAAAGAACCGGG
CGCCCCTGCGCTGACAGCCGGAACACGGCGGCATCAGAGCAGCCGATTGTCTGTTGTGCCCAGTCATAGCCGAATAGCCTCTC
CACCCAAGCGGCCGGAGAACCTGCGTGCAATCCATCTTGTTCAATCCACAT

**>SEQ ID NO 22 - CTC75064 event: Flanking sequences and T DNA fragment**

AACGAGGTGACAACTAGGGCACTACCTAGATAACAAACACACATACAACATCACAATCAAGCAGTCATTTGAGAAAATTCAAA
CAAATGGGCTTAAGCAACCAACACAAGACACAAGGCTCACCCAAAATAAGCATTTATCTCAAGCATGAACTAACTACAAGTTT
AGCTCAAAACAACGCTAGATACGGTAGCCAACTTAGCATGTGCCTAGAATCTGGACAGCAACGCAGTAGTTACTTGTTTCAAC
CATAACTGAGGTTATAGACATCCCACAAAGGTGATCCTAGACTTTCTAGAAATCTTAGGAAGATTACTACACTTTAGTTATTC
ATACCAAAAACTAATTCATAGCTTAACATGACCAAAATTGCTAATATTTCACAAATTGACGCTTAGACAACTTAATAACACAT
TGCGGACGTTTTTAATGTACTGAATTAGTACTGATATCGGTACCTTAATTCGGGGGATCTGGATTTTAGTACTGGATTTTGGT
TTTAGGAATTAGAAATTTTATTGATAGAAGTATTTTACAAATACAAATACATACTAAGGGTTTCTTATATGCTCAACACATGA
GCGAAACCCTATAGGAACCCTAATTCCCTTATCTGGGAACTACTCACACATTATTATGGAGAAACTCGAGCTTGTCGATCGAT
CACTCAGCCTCGAGGGTGGCGGTCACTGGGATGAACTCGAACCTGTCGATGATCACGCCGGCGGTGCCGCTGAAGTTCCTCAC
GCCCACGATGTTACCGAGGGAGGAGGTGAAAGCGTTGGCGCTCTCGAAGTAACCGAAGTCGCTGGATTGGAGGTTGTCCAGGG
AGGTAGCGGTAGCTGGCACGGTGTTGGAGAAGATGGAGGAGTTACCCCAGTTCACGTTGAGGTGGATCGGGGTCACGGAAGCG
TACCTCACGCGCACCCTGTACCTGGTGGAGGTGGATGGGAAGTGGATTGGCACTCGATGTAGCCCCTGTTCTGGATGTTGTTG
CCGCTGCTGTTGAGCCTCACGAGGTCGCCACCGGTGAAGCCTGGGCCGGAAATGACGGAACCGTTGAAGAGGAAGTTGCCCTT
CACGGCCGGGATTTGGGTGATGCTGTCGGAGGCGATGATGTTGTTGAACTCAGCGCTGCGGTGGATCCAGGAGAACATCGGAG
CCCTGATGATGCTCACGGAGCTGTTGCTGAAGCCGGAGCGGAACATGGACACGTGGCTCAGCCTGTGGGAGAAGCCTTGCCTG
GGTGGCACGTTGTTGTTCTGTGGTGGGATCTCGTCCAGGGAGTCCACGGTGCCGCTCTTCCTGTAGACAGCGGATGGCAGGTT
GGAGGAGGTGCCGTAGGCGAACTCGGTGCCGTCGAGCACGGACAGTTGCTGGTTGTTGATACCGATGTTGAAGGGCCTCCTGT
ACAGGGTGGAGGACAGGGTCCTGTAGACACCCTGACCCAGTTGAGCCACGATGCGTTGCTGTGGAGCGGCGTTGCCCATGGTG
CCGTAGAGCGGGAAGGTGAACTCGGGGCCGCTGAAGCCCACTGGGGAGGCCATGATCTGGTGGCCGGACCAGTAGTACTCGCC
CCTGTGAGCGTCGGTGTAGATGGTGATGCTGTTCAGGATGTCCATCAGGTGTGGGCTCCTGATGGAGCCCTCGATACCCTGGG
CGGAACCGCGGAAGCTACCGTCGAAGTTCTCCAGCACTGGGTTGGTGTAGATCTCCCTGGTGAGTTGGGACACGGTGCGGATC
GGGTAGGTCCTGGAGTCGTAGTTCGGGAAGAGGGACACAATGTCCAGCACGGTGAGGGTCAACTCCCTCCTGAACTGGTTGTA
CCTGATCCAGTCCCTGGAGTCCGGACCCCAGACGCGCTCCAGGCCGGTGTTGTACCAGCGCACAGCGTGGTCGGTGTAGTTGC

CAATCAGCCTGGTCAGGTCGTTGTAGCGGCTGTTGATGGTGGCAGCATCGAAGCCCCACCTTTGGCCGAACACGCTCACGTCG
CGCAGCACGCTGAGGTGCAGGTTAGCGGCTTGCACGTACACGGACAGGAGCGGCACTTGGTAGTTCTGGACGGCGAACAGTGG
GATAGCGGTGGTCAGGGCGCTGTTCATGTCGTTGAATTGAATGCGCATTTCCTCGCGGAGAGCTGGGTTGGTCGGGTCGGCCT
CCCACTCCCTGAAGCTCTCGGCGTAGATTTGGTAGAGGTTGCTCAGGCCCTCCAGCCTGGAGATGGCCTGGTTCCTGGCGAAC
TCTTCGATCCTCTGGTTGATCAGCTGCTCGATTTGCACCAGGAAGGCGTCCCATTGGGATGGACCGAAGATACCCCAGATGAT
GTCCACCAGGCCGAGCACGAAGCCAGCACCTGGCACGAACTCGCTGAGCAGGAACTGGGTCAAGGACAGGGAGATGTCGATGG
GGGTGTAACCGGTCTCGATGCGCTCGCCACCCAGCACCTCCACCTCTGGGTTGCTCAGGCAGTTGTATGGGATGCACTCGTTG
ATGTTTGGGTTGTTGTCCATGGCGGGGTTGATCAGGTTGATCACTTCTACCTACAAAAAAGCTCCGCACGAGGCTGCATTTGT
CACAAATCATGAAAAGAAAAACTACCGATGAACAATGCTGAGGGATTCAAATTCTACCCACAAAAAGAAGAAAGAAAGATCTA
GCACATCTAAGCCTGACGAAGCAGCAGAAATATATAAAAATATAAACCATAGTGCCCTTTTCCCCTCTTCCTGATCTTGTTTA
GCACGGCGGAAATTTTAAACCCCCCATCATCTCCCCCAACAACGGCGGATCGCAGATCTACATCCGAGAGCCCCATTCCCCGC
GAGATCCGGGCCGGATCCACGCCGGCGAGAGCCCCAGCCGCGAGATCCCGCCCCTCCCGCGCACCGATCTGGGCGCGCACGAA
GCCGCCTCTCGCCCACCCAAACTACCAAGGCCAAAGATCGAGACCGAGACGGAAAAAAAAACGGAGAAAGAAAGAGGAGAGGG
GCGGGGTGGTTACCGGCGGCGGCGGAGGCCTCCCTTGGATCTTATGGTGTGTTGTCCCTGTGTGTTCTCCAATAGTGTGGCTT
GAGTGTGTGGAAGATGGTTCTAGAGGATCTGCTAGAGTCAGCTTGTCAGCGTGTCCTCTCCAAATGAAATGAACTTCCTTATA
TAGAGGAAGGGTCTTGCGAAGGATAGTGGGATTGTGCGTCATCCCTTACGTCAGTGGAGATATCACATCAATCCACTTGCTTT
GAAGACGTGGTTGGAACGTCTTCTTTTTCCACGATGCTCCTCGTGGGTGGGGGTCCATCTTTGGGACCACTGTCGGCAGAGGC
ATCTTCAACGATGGCCTTTCCTTTATCGCAATGATGGCATTTGTAGGAGCCACCTTCCTTTTCCACTATCTTCACAATAAAGT
GACAGATAGCTGGGCAATGGAATCCGAGGAGGTTTCCGGATATTACCCTTTGTTGAAAAGTCTCAATCGGACCATCACATCAA
TCCACTTGCTTTGAAGACGTGGTTGGAACGTCTTCTTTTTCCACGATGCTCCTCGTGGGTGGGGGTCCATCTTTGGGACCACT
GTCGGCAGAGGCATCTTCAACGATGGCCTTTCCTTTATCGCAATGATGGCATTTGTAGGAGCCACCTTCCTTTTCCACTATCT
TCACAATAAAGTGACAGATAGCTGGGCAATGGAATCCGAGGAGGTTTCCGGATATTACCCTTTGTTGAAAAGTCTCAATCGGA
CCTGTTTAAACCCTGAAGCTTACGCGTATGCCTGCAGTGCAGCGTGACCCGGTCGTGCCCCTCTCTAGAGATAATGAGCATTG
CATGTCTAAGTTATAAAAAATTACCACATATTTTTTTTGTCACACTTGTTTGAAGTGCAGTTTATCTATCTTTATACATATAT
TTAAACTTTACTCTACGAATAATATAATCTATAGTACTACAATAATATCAGTGTTTTAGAGAATCATATAAATGAACAGTTAG
ACATGGTCTAAAGGACAATTGAGTATTTTGACAACAGGACTCTACAGTTTTATCTTTTTAGTGTGCATGTGTTCTCCTTTTTT
TTTGCAAATAGCTTCACCTATATAATACTTCATCCATTTTATTAGTACATCCATTTAGGGTTTAGGGTTAATGGTTTTTATAG
ACTAATTTTTTTAGTACATCTATTTTATTCTATTTTAGCCTCTAAATTAAGAAAACTAAAACTCTATTTTAGTTTTTTTATTT
AATAATTTAGATATAAAATAGAATAAAATAAAGTGACTAAAAATTAAACAAATACCCTTTAAGAAATTAAAAAAACTAAGGAA
ACATTTTTCTTGTTTCGAGTAGATAATGCCAGCCTGTTAAACGCCGTCGACGAGTCTAACGGACACCAACCAGCGAACCAGCA
GCGTCGCGTCGGGCCAAGCGAAGCAGACGGCACGGCATCTCTGTCGCTGCCTCTGGACCCCTCTCGAGAGTTCCGCTCCACCG
TTGGACTTGCTCCGCTGTCGGCATCCAGAAATTGCGTGGCGGAGCGGCAGACGTGAGCCGGCACGGCAGGCGGCCTCCTCCTC
CTCTCACGGCACGGCAGCTACGGGGGATTCCTTTCCCACCGCTCCTTCGCTTTCCCTTCCTCGCCCGCCGTAATAAATAGACA
CCCCCTCCACACCCTCTTTCCCCAACCTCGTGTTGTTCGGAGCGCACACACACACAACCAGATCTCCCCCAAATCCACCCGTC
GGCACCTCCGCTTCAAGGTACGCCGCTCGTCCTCCCCCCCCCCCCCTCTCTACCTTCTCTAGATCGGCGTTCCGGTCCATGGT
TAGGGCCCGGTAGTTCTACTTCTGTTCATGTTTGTGTTAGATCCGTGTTTGTGTTAGATCCGTGCTGCTAGCGTTCGTACACG
GATGCGACCTGTACGTCAGACACGTTCTGATTGCTAACTTGCCAGTGTTTCTCTTTGGGGAATCCTGGGATGGCTCTAGCCGT
TCCGCAGACGGGATCGATTTCATGATTTTTTTTGTTTCGTTGCATAGGGTTTGGTTTGCCCTTTTCCTTTATTTCAATATATG
CCGTGCACTTGTTTGTCGGGTCATCTTTTCATGCTTTTTTTTGTCTTGGTTGTGATGATGTGGTCTGGTTGGGCGGTCGTTCT
AGATCGGAGTAGAATTCTGTTTCAAACTACCTGGTGGATTTATTAATTTTGGATCTGTATGTGTGTGCCATACATATTCATAG
TTACGAATTGAAGATGATGGATGGAAATATCGATCTAGGATAGGTATACATGTTGATGCGGGTTTTACTGATGCATATACAGA
GATGCTTTTTGTTCGCTTGGTTGTGATGATGTGGTGTGGTTGGGCGGTCGTTCATTCGTTCTAGATCGGAGTAGAATACTGTT
TCAAACTACCTGGTGTATTTATTAATTTTGGAACTGTATGTGTGTGTCATACATCTTCATAGTTACGAGTTTAAGATGGATGG
AAATATCGATCTAGGATAGGTATACATGTTGATGTGGGTTTTACTGATGCATATACATGATGGCATATGCAGCATCTATTCAT
ATGCTCTAACCTTGAGTACCTATCTATTATAATAAACAAGTATGTTTTATAATTATTTTGATCTTGATATACTTGGATGATGG
CATATGCAGCAGCTATATGTGGATTTTTTTAGCCCTGCCTTCATACGCTATTTATTTGCTTGGTACTGTTTCTTTTGTCGATG
CTCACCCTGTTGTTTGGTGTTACTTCTGCAGGTCGACTCTAGAATGTGGATTGAACAAGATGGATTGCACGCAGGTTCTCCGG
CCGCTTGGGTGGAGAGGCTATTCGGCTATGACTGGGCACAACAGACAATCGGCTGCTCTGATGCCGCCGTGTTCCGGCTGTCA
GCGCAGGGGCGCCCGGTTCTTTTTGTCAAGACCGACCTGTCCGGTGCCCTGAATGAACTGCAGGACGAGGCAGCGCGGCTATC
GTGGCTGGCCACGACGGGCGTTCCTTGCGCAGCTGTGCTCGACGTTGTCACTGAAGCGGGAAGGGACTGGCTGCTATTGGGCG
AAGTGCCGGGGCAGGATCTCCTGTCATCTCACCTTGCTCCTGCCGAGAAAGTATCCATCATGGCTGATGCAATGCGGCGGCTG
CATACGCTTGATCCGGCTACCTGCCCATTCGACCACCAAGCGAAACATCGCATCGAGCGAGCACGTACTCGGATGGAAGCCGG
TCTTGTCGATCAGGATGATCTGGACGAAGAGCATCAGGGGCTCGCGCCAGCCGAACTGTTCGCCAGGCTCAAGGCGCGCATGC
CCGACGGCGAGGATCTCGTCGTGACCCATGGCGATGCCTGCTTGCCGAATATCATGGTGGAAAATGGCCGCTTTTCTGGATTC
ATCGACTGTGGCCGGCTGGGTGTGGCGGACCGCTATCAGGACATAGCGTTGGCTACCCGTGATATTGCTGAAGAGCTTGGCGG
CGAATGGGCTGACCGCTTCCTCGTGCTTTACGGTATCGCCGCTCCCGATTCGCAGCGCATCGCCTTCTATCGCCTTCTTGACG
AGTTCTTCTGAGATCGTTCAAACATTTGGCAATAAAGTTTCTTAAGATTGAATCCTGTTGCCGGTCTTGCGATGATTATCATA
TAATTTCTGTTGAATTACGTTAAGCATGTAATAATTAACATGTAATGCATGACGTTATTTATGAGATGGGTTTTTATGATTAG
AGTCCCGCAATTATACATTTAATACGCGATAGAAAACAAAATATAGCGCGCAAACTAGGATAAATTATCGCGCGCGGTGTCAT
CTATGTTACTAGATCGGCGCGCCAAGGGCGAATTCCAGCACACTGGCGGCCGTTACTAGTGGATCGAGCTCGTCGACTCTAGA
CTCGAGGGCGCGCCGATACCAATTTGGACAGAAAACTGACAAGAACTTCGAAAATACATAATTGGAGTTCTAGAGACACAACA
AAAGTGATCCTTAACTTTATGTAAAGCTTATTAAGTTGTCTATAACTTTCTTATTATCATATTAAGATGATTCTACGGCTAAC
AAGGACAAACATAGCAAAGAAAACATCTCTGCCGGATTTGGACAGATTCTGTCATTCTACTTTGCAAGCTCATAACTAGAGAT
TTAGACCATCACAATTAGTGCTATAGGACATTTTGGAAAGCTTAGAAAAAGTACTACACTTCTTCTATTATCTCTAAGACACG
ATTCCATATGCACACAGGTCAAACAAACCAAACATTCAGATCTATTCAGAACATTGACAAAACCTGACCGTAAACTTTTAAAA
TTCATAACTCTAAGCTCAAGCATGTAGTCACACTTATTTCTAGTAAAGCTAAGCACACATTTTTCTCAAGCATTTCCTATTCA

AGCAACATGGAACAAAGCATTCTTGTTTGACTCCACACAAGGAAGATAGGATACCACATCACTCACAAATTACTCATCATTTG
AACAAGGGTGAGAGGAGCATAGCTATGCATAAGGCAACAATCATGAAGATGC

**>SEQ ID NO 23 - CTC75064 event: Flanking sequence - Left border (5')**

AACGAGGTGACAACTAGGGCACTACCTAGATAACAAACACACATACAACATCACAATCAAGCAGTCATTTGAGAAAATTCAAAC
AAATGGGCTTAAGCAACCAACACAAGACACAAGGCTCACCCAAAATAAGCATTTATCTCAAGCATGAACTAACTACAAGTTTA
GCTCAAAACAACGCTAGATACGGTAGCCAACTTAGCATGTGCCTAGAATCTGGACAGCAACGCAGTAGTTACTTGTTTCAACC
ATAACTGAGGTTATAGACATCCCACAAAGGTGATCCTAGACTTTCTAGAAATCTTAGGAAGATTACTACACTTTAGTTATTCA
TACCAAAAACTAATTCATAGCTTAACATGACCAAAATTGCTAATATTTC

**> SEQ ID NO 24 - CTC75064 event: Flanking sequence - Right border (3')**

TACCAATTTGGACAGAAAACTGACAAGAACTTCGAAAATACATAATTGGAGTTCTAGAGACACAACAAAAGTGATCCTTAACT
TTATGTAAAGCTTATTAAGTTGTCTATAACTTTCTTATTATCATATTAAGATGATTCTACGGCTAACAAGGACAAACATAGCA
AAGAAAACATCTCTGCCGGATTTGGACAGATTCTGTCATTCTACTTTGCAAGCTCATAACTAGAGATTTAGACCATCACAATT
AGTGCTATAGGACATTTTGGAAAGCTTAGAAAAAGTACTACACTTCTTCTATTATCTCTAAGACACGATTCCATATGCACACA
GGTCAAACAAACCAAACATTCAGATCTATTCAGAACATTGACAAAACCTGACCGTAAACTTTTAAAATTCATAACTCTAAGCT
CAAGCATGTAGTCACACTTATTTCTAGTAAAGCTAAGCACACATTTTTCTCAAGCATTTCCTATTCAAGCAACATGGAACAAA
GCATTCTTGTTTGACTCCACACAAGGAAGATAGGATACCACATCACTCACAAATTACTCATCATTTGAACAAGGGTGAGAGGA
GCATAGCTATGCATAAGGCAACAATCATGAAGATGC

**>SEQ ID 25 Cas9/crRNA/HR template construction**

CTTAGAATAACGGATATTTAAAAGGGCGTGAAAAGGTTTATCCGTTCGTCCATTTGTATGTGCATGCCAACCACAGGGTTCCC
CTCGGGATCAAAGTACTTTGATCCAACCCCTCCGCTGCTATAGTGCAGTCGGCTTCTGACGTTCAGTGCAGCCGTCTTCTGAA
AACGACATGTCGCACAAGTCCTAAGTTACGCGACAGGCTGCCGCCCTGCCCTTTTCCTGGCGTTTTCTTGTCGCGTGTTTTAG
TCGCATAAAGTAGAATACTTGCGACTAGAACCGGAGACATTACGCCATGAACAAGAGCGCCGCCGCTGGCCTGCTGGGCTATG
CCCGCGTCAGCACCGACGACCAGGACTTGACCAACCAACGGGCCGAACTGCACGCGGCCGGCTGCACCAAGCTGTTTTCCGAG
AAGATCACCGGCACCAGGCGCGACCGCCCGGAGCTGGCCAGGATGCTTGACCACCTACGCCCTGGCGACGTTGTGACAGTGAC
CAGGCTAGACCGCCTGGCCCGCAGCACCCGCGACCTACTGGACATTGCCGAGCGCATCCAGGAGGCCGGCGCGGGCCTGCGTA
GCCTGGCAGAGCCGTGGGCCGACACCACCACGCCGGCCGGCCGCATGGTGTTGACCGTGTTCGCCGGCATTGCCGAGTTCGAG
CGTTCCCTAATCATCGACCGCACCCGGAGCGGGCGCGAGGCCGCCAAGGCCCGAGGCGTGAAGTTTGGCCCCCGCCCTACCCT
CACCCCGGCACAGATCGCGCACGCCCGCGAGCTGATCGACCAGGAAGGCCGCACCGTGAAAGAGGCGGCTGCACTGCTTGGCG
TGCATCGCTCGACCCTGTACCGCGCACTTGAGCGCAGCGAGGAAGTGACGCCCACCGAGGCCAGGCGGCGCGGTGCCTTCCGT
GAGGACGCATTGACCGAGGCCGACGCCCTGGCGGCCGCCGAGAATGAACGCCAAGAGGAACAAGCATGAAACCGCACCAGGAC
GGCCAGGACGAACCGTTTTTCATTACCGAAGAGATCGAGGCGGAGATGATCGCGGCCGGGTACGTGTTCGAGCCGCCCGCGCA
CGTCTCAACCGTGCGGCTGCATGAAATCCTGGCCGGTTTGTCTGATGCCAAGCTGGCGGCCTGGCCGGCCAGCTTGGCCGCTG
AAGAAACCGAGCGCCGCCGTCTAAAAAGGTGATGTGTATTTGAGTAAAACAGCTTGCGTCATGCGGTCGCTGCGTATATGATG
CGATGAGTAAATAAACAAATACGCAAGGGGAACGCATGAAGGTTATCGCTGTACTTAACCAGAAAGGCGGGTCAGGCAAGACG
ACCATCGCAACCCATCTAGCCCGCGCCCTGCAACTCGCCGGGGCCGATGTTCTGTTAGTCGATTCCGATCCCCAGGGCAGTGC
CCGCGATTGGGCGGCCGTGCGGGAAGATCAACCGCTAACCGTTGTCGGCATCGACCGCCCGACGATTGACCGCGACGTGAAGG
CCATCGGCCGGCGCGACTTCGTAGTGATCGACGGAGCGCCCCAGGCGGCGGACTTGGCTGTGTCCGCGATCAAGGCAGCCGAC
TTCGTGCTGATTCCGGTGCAGCCAAGCCCTTACGACATATGGGCCACCGCCGACCTGGTGGAGCTGGTTAAGCAGCGCATTGA
GGTCACGGATGGAAGGCTACAAGCGGCCTTTGTCGTGTCGCGGGCGATCAAAGGCACGCGCATCGGCGGTGAGGTTGCCGAGG
CGCTGGCCGGGTACGAGCTGCCCATTCTTGAGTCCCGTATCACGCAGCGCGTGAGCTACCCAGGCACTGCCGCCGCCGGCACA
ACCGTTCTTGAATCAGAACCCGAGGGCGACGCTGCCCGCGAGGTCCAGGCGCTGGCCGCTGAAATTAAATCAAAACTCATTTG
AGTTAATGAGGTAAAGAGAAAATGAGCAAAAGCACAAACACGCTAAGTGCCGGCCGTCCGAGCGCACGCAGCAGCAAGGCTGC
AACGTTGGCCAGCCTGGCAGACACGCCAGCCATGAAGCGGGTCAACTTTCAGTTGCCGGCGGAGGATCACACCAAGCTGAAGA
TGTACGCGGTACGCCAAGGCAAGACCATTACCGAGCTGCTATCTGAATACATCGCGCAGCTACCAGAGTAAATGAGCAAATGA
ATAAATGAGTAGATGAATTTTAGCGGCTAAAGGAGGCGGCATGGAAAATCAAGAACAACCAGGCACCGACGCCGTGGAATGCC
CCATGTGTGGAGGAACGGGCGGTTGGCCAGGCGTAAGCGGCTGGGTTGTCTGCCGGCCCTGCAATGGCACTGGAACCCCCAAG
CCCGAGGAATCGGCGTGACGGTCGCAAACCATCCGGCCCGGTACAAATCGGCGCGGCGCTGGGTGATGACCTGGTGGAGAAGT
TGAAGGCCGCGCAGGCCGCCCAGCGGCAACGCATCGAGGCAGAAGCACGCCCCGGTGAATCGTGGCAAGCGGCCGCTGATCGA
ATCCGCAAAGAATCCCGGCAACCGCCGGCAGCCGGTGCGCCGTCGATTAGGAAGCCGCCCAAGGGCGACGAGCAACCAGATTT
TTTCGTTCCGATGCTCTATGACGTGGGCACCCGCGATAGTCGCAGCATCATGGACGTGGCCGTTTTCCGTCTGTCGAAGCGTG
ACCGACGAGCTGGCGAGGTGATCCGCTACGAGCTTCCAGACGGGCACGTAGAGGTTTCCGCAGGGCCGGCCGGCATGGCCAGT
GTGTGGGATTACGACCTGGTACTGATGGCGGTTTCCCATCTAACCGAATCCATGAACCGATACCGGGAAGGGAAGGGAGACAA
GCCCGGCCGCGTGTTCCGTCCACACGTTGCGGACGTACTCAAGTTCTGCCGGCGAGCCGATGGCGGAAAGCAGAAAGACGACC

TGGTAGAAACCTGCATTCGGTTAAACACCACGCACGTTGCCATGCAGCGTACGAAGAAGGCCAAGAACGGCCGCCTGGTGACG
GTATCCGAGGGTGAAGCCTTGATTAGCCGCTACAAGATCGTAAAGAGCGAAACCGGGCGGCCGGAGTACATCGAGATCGAGCT
AGCTGATTGGATGTACCGCGAGATCACAGAAGGCAAGAACCCGGACGTGCTGACGGTTCACCCCGATTACTTTTTGATCGATC
CCGGCATCGGCCGTTTTCTCTACCGCCTGGCACGCCGCGCCGCAGGCAAGGCAGAAGCCAGATGGTTGTTCAAGACGATCTAC
GAACGCAGTGGCAGCGCCGGAGAGTTCAAGAAGTTCTGTTTCACCGTGCGCAAGCTGATCGGGTCAAATGACCTGCCGGAGTA
CGATTTGAAGGAGGAGGCGGGGCAGGCTGGCCCGATCCTAGTCATGCGCTACCGCAACCTGATCGAGGGCGAAGCATCCGCCG
GTTCCTAATGTACGGAGCAGATGCTAGGGCAAATTGCCCTAGCAGGGGAAAAAGGTCGAAAAGGTCTCTTTCCTGTGGATAGC
ACGTACATTGGGAACCCAAAGCCGTACATTGGGAACCGGAACCCGTACATTGGGAACCCAAAGCCGTACATTGGGAACCGGTC
ACACATGTAAGTGACTGATATAAAAGAGAAAAAAGGCGATTTTTCCGCCTAAAACTCTTTAAAACTTATTAAAACTCTTAAAA
CCCGCCTGGCCTGTGCATAACTGTCTGGCCAGCGCACAGCCGAAGAGCTGCAAAAAGCGCCTACCCTTCGGTCGCTGCGCTCC
CTACGCCCCGCCGCTTCGCGTCGGCCTATCGCGGCCGCTGGCCGCTCAAAAATGGCTGGCCTACGGCCAGGCAATCTACCAGG
GCGCGGACAAGCCGCGCCGTCGCCACTCGACCGCCGGCGCCCACATCAAGGCACCCTGCCTCGCGCGTTTCGGTGATGACGGT
GAAAACCTCTGACACATGCAGCTCCCGGAGACGGTCACAGCTTGTCTGTAAGCGGATGCCGGGAGCAGACAAGCCCGTCAGGG
CGCGTCAGCGGGTGTTGGCGGGTGTCGGGGCGCAGCCATGACCCAGTCACGTAGCGATAGCGGAGTGTATACTGGCTTAACTA
TGCGGCATCAGAGCAGATTGTACTGAGAGTGCACCATATGCGGTGTGAAATACCGCACAGATGCGTAAGGAGAAAATACCGCA
TCAGGCGCTCTTCCGCTTCCTCGCTCACTGACTCGCTGCGCTCGGTCGTTCGGCTGCGGCGAGCGGTATCAGCTCACTCAAAG
GCGGTAATACGGTTATCCACAGAATCAGGGGATAACGCAGGAAAGAACATGTGAGCAAAAGGCCAGCAAAAGGCCAGGAACCG
TAAAAAGGCCGCGTTGCTGGCGTTTTTCCATAGGCTCCGCCCCCCTGACGAGCATCACAAAAATCGACGCTCAAGTCAGAGGT
GGCGAAACCCGACAGGACTATAAAGATACCAGGCGTTTCCCCCTGGAAGCTCCCTCGTGCGCTCTCCTGTTCCGACCCTGCCG
CTTACCGGATACCTGTCCGCCTTTCTCCCTTCGGGAAGCGTGGCGCTTTCTCATAGCTCACGCTGTAGGTATCTCAGTTCGGT
GTAGGTCGTTCGCTCCAAGCTGGGCTGTGTGCACGAACCCCCCGTTCAGCCCGACCGCTGCGCCTTATCCGGTAACTATCGTC
TTGAGTCCAACCCGGTAAGACACGACTTATCGCCACTGGCAGCAGCCACTGGTAACAGGATTAGCAGAGCGAGGTATGTAGGC
GGTGCTACAGAGTTCTTGAAGTGGTGGCCTAACTACGGCTACACTAGAAGGACAGTATTTGGTATCTGCGCTCTGCTGAAGCC
AGTTACCTTCGGAAAAAGAGTTGGTAGCTCTTGATCCGGCAAACAAACCACCGCTGGTAGCGGTGGTTTTTTTGTTTGCAAGC
AGCAGATTACGCGCAGAAAAAAAGGATCTCAAGAAGATCCTTTGATCTTTTCTACGGGGTCTGACGCTCAGTGGAACGAAAAC
TCACGTTAAGGGATTTTGGTCATGCATTCTAGGTATTATTTGCCAACGACCTTCGTGATCTCGCCCTTGACATAGTGGACAAA
TTCTTCGAGCTGGTCGGCCCGGGACGCGAGACGGTCTTCTTCTTGGCCCAGATAGGCTTGGCGCGCTTCGAGGATCACGGGCT
GGTATTGCGCCGGAAGGCGCTCCATCGCCCAGTCGGCGGCGACATCCTTCGGCGCGATCTTGCCGGTAACCGCCGAGTACCAA
ATCCGGCTCAGCGTAAGGACCACATTGCGCTCATCGCCCGCCCAATCCGGCGGGGAGTTCCACAGGGTCAGCGTCTCGTTCAG
TGCTTCGAACAGATCCTGTTCCGGCACCGGGTCGAAAAGTTCCTCGGCCGCGGGGCCGACGAGGGCCACGCTATGCTCCCGGG
CCTTGGTGAGCAGGATCGCCAGATCAATGTCGATGGTGGCCGGTTCAAAGATACCCGCCAGAATATCATTACGCTGCCATTCG
CCGAACTGGAGTTCGCGTTTGGCCGGATAGCGCCAGGGGATGATGTCATCGTGCACCACAATCGTCACCTCAACCGCGCGCAG
GATTTCGCTCTCGCCGGGGGAGGCGGACGTTTCCAGAAGGTCGTTGATAAGCGCGCGGCGCGTGGTCTCGTCGAGACGGACGG
TAACGGTGACAAGCAGGTCGATGTCCGAATGGGGCTTAAGGCCGCCGTCAACGGCGCTACCATACAGATGCACGGCGAGGAGG
GTCGGTTCGAGGTGGCGCTCGATGACACCCACGACTTCCGACAGCTGGGTGGACACCTCGGCGATGACCGCTTCACCCATTTA
TTATTTCCTTCCTCTTTTCTACAGTATTTAAAGATACCCCAAGAAGCTAATTATAACAAGACGAACTCCAATTCACTGTTCCT
TGCATTCTAAAACCTTAAATACCAGAAAACAGCTTTTTCAAAGTTGTTTTCAAAGTTGGCGTATAACATAGTATCGACGGAGC
CGATTTTGAAACCGCGGTGATCACAGGCAGCAACGCTCTGTCATCGTTACAATCAACATGCTACCCTCCGCGAGATCATCCGT
GTTTCAAACCCGGCAGCTTAGTTGCCGTTCTTCCGAATAGCATCGGTAACATGAGCAAAGTCTGCCGCCTTACAACGGCTCTC
CCGCTGACGCCGTCCCGGACTGATGGGCTGCCTGTATCGAGTGGTGATTTTGTGCCGAGCTGCCGGTCGGGGAGCTGTTGGCT
GGCTGGTGGCAGGATATATTGTGGTGTAAACAAATTGACGCTTAGACAACTTAATAACACATTGCGGACGTTTTTAATGTACT
GAATTAGTACTGATAAATGGCGCGCCAAGCTTTGGCAAACAGCTATTATGGGTATTATGGGTGGTACCACGCGTCGATCCACT
AGTAACGGCCGCCAGTGTGCTGGAATTCGCCCTTGGCGCGCCGATCTAGTAACATAGATGACACCGCGCGCGATAATTTATCC
TAGTTTGCGCGCTATATTTTGTTTTCTATCGCGTATTAAATGTATAATTGCGGGACTCTAATCATAAAAACCCATCTCATAAA
TAACGTCATGCATTACATGTTAATTATTACATGCTTAACGTAATTCAACAGAAATTATATGATAATCATCGCAAGACCGGCAA
CAGGATTCAATCTTAAGAAACTTTATTGCCAAATGTTTGAACGATCTCAGAAGAACTCGTCAAGAAGGCGATAGAAGGCGATG
CGCTGCGAATCGGGAGCGGCGATACCGTAAAGCACGAGGAAGCGGTCAGCCCATTCGCCGCCAAGCTCTTCAGCAATATCACG
GGTAGCCAACGCTATGTCCTGATAGCGGTCCGCCACACCCAGCCGGCCACAGTCGATGAATCCAGAAAAGCGGCCATTTTCCA
CCATGATATTCGGCAAGCAGGCATCGCCATGGGTCACGACGAGATCCTCGCCGTCGGGCATGCGCGCCTTGAGCCTGGCGAAC
AGTTCGGCTGGCGCGAGCCCCTGATGCTCTTCGTCCAGATCATCCTGATCGACAAGACCGGCTTCCATCCGAGTACGTGCTCG
CTCGATGCGATGTTTCGCTTGGTGGTCGAATGGGCAGGTAGCCGGATCAAGCGTATGCAGCCGCCGCATTGCATCAGCCATGA
TGGATACTTTCTCGGCAGGAGCAAGGTGAGATGACAGGAGATCCTGCCCCGGCACTTCGCCCAATAGCAGCCAGTCCCTTCCC
GCTTCAGTGACAACGTCGAGCACAGCTGCGCAAGGAACGCCCGTCGTGGCCAGCCACGATAGCCGCGCTGCCTCGTCCTGCAG
TTCATTCAGGGCACCGGACAGGTCGGTCTTGACAAAAAGAACCGGGCGCCCCTGCGCTGACAGCCGGAACACGGCGGCATCAG
AGCAGCCGATTGTCTGTTGTGCCCAGTCATAGCCGAATAGCCTCTCCACCCAAGCGGCCGGAGAACCTGCGTGCAATCCATCT
TGTTCAATCCACATGGTGGTGTGACCTGCAGAAGTAACACCAAACAACAGGGTGAGCATCGACAAAAGAAACAGTACCAAGCA
AATAAATAGCGTATGAAGGCAGGGCTAAAAAAATCCACATATAGCTGCTGCATATGCCATCATCCAAGTATATCAAGATCAAA
ATAATTATAAAACATACTTGTTTATTATAATAGATAGGTACTCAAGGTTAGAGCATATGAATAGATGCTGCATATGCCATCAT

GTATATGCATCAGTAAAACCCACATCAACATGTATACCTATCCTAGATCGATATTTCCATCCATCTTAAACTCGTAACTATGA
AGATGTATGACACACACATACAGTTCCAAAATTAATAAATACACCAGGTAGTTTGAAACAGTATTCTACTCCGATCTAGAACG
AATGAACGACCGCCCAACCACACCACATCATCACAACCAAGCGAACAAAAAGCATCTCTGTATATGCATCAGTAAAACCCGCA
TCAACATGTATACCTATCCTAGATCGATATTTCCATCCATCATCTTCAATTCGTAACTATGAATATGTATGGCACACACATAC
AGATCCAAAATTAATAAATCCACCAGGTAGTTTGAAACAGAATTCTACTCCGATCTAGAACGACCGCCCAACCAGACCACATC
ATCACAACCAAGACAAAAAAAAGCATGAAAAGATGACCCGACAAACAAGTGCACGGCATATATTGAAATAAAGGAAAAGGGCA
AACCAAACCCTATGCAACGAAACAAAAAAAATCATGAAATCGATCCCGTCTGCGGAACGGCTAGAGCCATCCCAGGATTCCCC
AAAGAGAAACACTGGCAAGTTAGCAATCAGAACGTGTCTGACGTACAGGTCGCATCCGTGTACGAACGCTAGCAGCACGGATC
TAACACAAACACGGATCTAACACAAACATGAACAGAAGTAGAACTACCGGGCCCTAACCATGGACCGGAACGCCGATCTAGAG
AAGGTAGAGAGGGGGGGGGGGGGAGGACGAGCGGCGTACCTTGAAGCGGAGGTGCCGACGGGTGGATTTGGGGGAGATCTGGT
TGTGTGTGTGTGCGCTCCGAACAACACGAGGTTGGGGAAAGAGGGTGTGGAGGGGGTGTCTATTTATTACGGCGGGCGAGGAA
GGGAAAGCGAAGGAGCGGTGGGAAAGGAATCCCCCGTAGCTGCCGTGCCGTGAGAGGAGGAGGAGGCCGCCTGCCGTGCCGGC
TCACGTCTGCCGCTCCGCCACGCAATTTCTGGATGCCGACAGCGGAGCAAGTCCAACGGTGGAGCGGAACTCTCGAGAGGGGT
CCAGAGGCAGCGACAGAGATGCCGTGCCGTCTGCTTCGCTTGGCCCGACGCGACGCTGCTGGTTCGCTGGTTGGTGTCCGTTA
GACTCGTCGACGGCGTTTAACAGGCTGGCATTATCTACTCGAAACAAGAAAAATGTTTCCTTAGTTTTTTTAATTTCTTAAAG
GGTATTTGTTTAATTTTTAGTCACTTTATTTTATTCTATTTTATATCTAAATTATTAAATAAAAAAACTAAAATAGAGTTTTA
GTTTTCTTAATTTAGAGGCTAAAATAGAATAAAATAGATGTACTAAAAAAATTAGTCTATAAAAACCATTAACCCTAAACCCT
AAATGGATGTACTAATAAAATGGATGAAGTATTATATAGGTGAAGCTATTTGCAAAAAAAAAGGAGAACACATGCACACTAAA
AAGATAAAACTGTAGAGTCCTGTTGTCAAAATACTCAATTGTCCTTTAGACCATGTCTAACTGTTCATTTATATGATTCTCTA
AAACACTGATATTATTGTAGTACTATAGATTATATTATTCGTAGAGTAAAGTTTAAATATATGTATAAAGATAGATAAACTGC
ACTTCAAACAAGTGTGACAAAAAAAATATGTGGTAATTTTTTATAACTTAGACATGCAATGCTCATTATCTCTAGAGAGGGGC
ACGACCGGGTCACGCTGCACTGCAGGGATCCGATCTAGTAACATAGATGACACCGCGCGCGATAATTTATCCTAGTTTGCGCG
CTATATTTTGTTTTCTATCGCGTATTAAATGTATAATTGCGGGACTCTAATCATAAAAACCCATCTCATAAATAACGTCATGC
ATTACATGTTAATTATTACATGCTTAACGTAATTCAACAGAAATTATATGATAATCATCGCAAGACCGGCAACAGGATTCAAT
CTTAAGAAACTTTATTGCCAAATGTTTGAACGATCCCTAGGACGATCTCACTTGTACAGCTCGTCCATGCCGTGGGTGATGCC
AGCTGCGGTGACGAACTCCAGCAGGACCATGTGGTCGCGCTTCTCGTTGGGGTCCTTGCTCAGAGCGGACTGGGTGCTCAGGT
AGTGGTTGTCGGGCAGCAGCACGGGACCGTCGCCGATGGGCGTGTTCTGCTGGTAGTGGTCGGCGAGCTGGACGCTGCCGTCC
TCGATGTTGTGGCGGATCTTGAAGTTGACCTTGATGCCGTTCTTCTGCTTGTCAGCCATGATGTAGACGTTGTGGCTGTTGTA
GTTGTACTCCAGCTTGTGCCCCAGGATGTTGCCGTCCTCCTTGAAGTCGATGCCCTTCAGCTCGATGCGGTTCACCAGGGTGT
CGCCCTCGAACTTCACCTCGGCTCGGGTCTTGTAGTTGCCGTCGTCCTTGAAGAAGATGGTGCGCTCCTGGACGTAGCCTTCG
GGCATGGCGGACTTGAAGAAGTCGTGCTGCTTCATGTGGTCGGGGTAGCGGCTGAAGCACTGCACGCCGTAGGTGAAGGTGGT
CACGAGGGTGGGCCAGGGCACGGGCAGCTTGCCGGTGGTGCAGATGAACTTCAGGGTCAGCTTGCCGTAGGTGGCGTCGCCCT
CGCCCTCGCCGCTGACGCTGAACTTGTGGCCGTTCACGTCGCCGTCCAGCTCGACCAGGATGGGCACCACCCCAGTGAACAGC
TCCTCGCCCTTGCTCACTACAAAAAAGCTCCGCACGAGGCTGCATTTGTCACAAATCATGAAAAGAAAAACTACCGATGAACA
ATGCTGAGGGATTCAAATTCTACCCACAAAAAGAAGAAAGAAAGATCTAGCACATCTAAGCCTGACGAAGCAGCAGAAATATA
TAAAAATATAAACCATAGTGCCCTTTTCCCCTCTTCCTGATCTTGTTTAGCACGGCGGAAATTTTAAACCCCCCATCATCTCC
CCCAACAACGGCGGATCGCAGATCTACATCCGAGAGCCCCATTCCCCGCGAGATCCGGGCCGGATCCACGCCGGCGAGAGCCC
CAGCCGCGAGATCCCGCCCCTCCCGCGCACCGATCTGGGCGCGCACGAAGCCGCCTCTCGCCCACCCAAACTACCAAGGCCAA
AGATCGAGACCGAGACGGAAAAAAAAAACGGAGAAAGAAAGAGGAGAGGGGCGGGGTGGTTACCGGCGGCGGCGGAGGCCTCCC
TTGGATCTTATGGTGTGTTGTCCCTGTGTGTTCTCCAATAGTGTGGCTTGAGTGTGTGGAAGATGGTTCTAGAGGATCTGCTA
GAGTCAGCTTGTCAGCGTGTCCTCTCCAAATGAAATGAACTTCCTTATATAGAGGAAGGGTCTTGCGAAGGATAGTGGGATTG
TGCGTCATCCCTTACGTCAGTGGAGATATCACATCAATCCACTTGCTTTGAAGACGTGGTTGGAACGTCTTCTTTTTCCACGA
TGCTCCTCGTGGGTGGGGGTCCATCTTTGGGACCACTGTCGGCAGAGGCATCTTCAACGATGGCCTTTCCTTTATCGCAATGA
TGGCATTTGTAGGAGCCACCTTCCTTTTCCACTATCTTCACAATAAAGTGACAGATAGCTGGGCAATGGAATCCGAGGAGGTT
TCCGGATATTACCCTTTGTTGAAAAGTCTCAATCGGACCATCACATCAATCCACTTGCTTTGAAGACGTGGTTGGAACGTCTT
CTTTTTCCACGATGCTCCTCGTGGGTGGGGGTCCATCTTTGGGACCACTGTCGGCAGAGGCATCTTCAACGATGGCCTTTCCT
TTATCGCAATGATGGCATTTGTAGGAGCCACCTTCCTTTTCCACTATCTTCACAATAAAGTGACAGATAGCTGGGCAATGGAA
TCCGAGGAGGTTTCCGGATATTACCCTTTGTTGAAAAGTCTCAATCGGACCCCCTCAGCCTGCAGTGCAGCGTGACCCGGTCG
TGCCCCTCTCTAGAGATAATGAGCATTGCATGTCTAAGTTATAAAAAATTACCACATATTTTTTTTGTCACACTTGTTTGAAG
TGCAGTTTATCTATCTTTATACATATATTTAAACTTTACTCTACGAATAATATAATCTATAGTACTACAATAATATCAGTGTT
TTAGAGAATCATATAAATGAACAGTTAGACATGGTCTAAAGGACAATTGAGTATTTTGACAACAGGACTCTACAGTTTTATCT
TTTTAGTGTGCATGTGTTCTCCTTTTTTTTTGCAAATAGCTTCACCTATATAATACTTCATCCATTTTATTAGTACATCCATT
TAGGGTTTAGGGTTAATGGTTTTTATAGACTAATTTTTTTAGTACATCTATTTTATTCTATTTTAGCCTCTAAATTAAGAAAA
CTAAAACTCTATTTTAGTTTTTTTATTTAATAATTTAGATATAAAATAGAATAAAATAAAGTGACTAAAAATTAAACAAATAC
CCTTTAAGAAATTAAAAAAACTAAGGAAACATTTTTCTTGTTTCGAGTAGATAATGCCAGCCTGTTAAACGCCGTCGACGAGT
CTAACGGACACCAACCAGCGAACCAGCAGCGTCGCGTCGGGCCAAGCGAAGCAGACGGCACGGCATCTCTGTCGCTGCCTCTG
GACCCCTCTCGAGAGTTCCGCTCCACCGTTGGACTTGCTCCGCTGTCGGCATCCAGAAATTGCGTGGCGGAGCGGCAGACGTG
AGCCGGCACGGCAGGCGGCCTCCTCCTCCTCTCACGGCACGGCAGCTACGGGGGATTCCTTTCCCACCGCTCCTTCGCTTTCC

CTTCCTCGCCCGCCGTAATAAATAGACACCCCCTCCACACCCTCTTTCCCCAACCTCGTGTTGTTCGGAGCGCACACACACAC
AACCAGATCTCCCCCAAATCCACCCGTCGGCACCTCCGCTTCAAGGTACGCCGCTCGTCCTCCCCCCCCCCCCCTCTCTACCT
TCTCTAGATCGGCGTTCCGGTCCATGGTTAGGGCCCGGTAGTTCTACTTCTGTTCATGTTTGTGTTAGATCCGTGTTTGTGTT
AGATCCGTGCTGCTAGCGTTCGTACACGGATGCGACCTGTACGTCAGACACGTTCTGATTGCTAACTTGCCAGTGTTTCTCTT
TGGGGAATCCTGGGATGGCTCTAGCCGTTCCGCAGACGGGATCGATTTCATGATTTTTTTTGTTTCGTTGCATAGGGTTTGGT
TTGCCCTTTTCCTTTATTTCAATATATGCCGTGCACTTGTTTGTCGGGTCATCTTTTCATGCTTTTTTTTGTCTTGGTTGTGA
TGATGTGGTCTGGTTGGGCGGTCGTTCTAGATCGGAGTAGAATTCTGTTTCAAACTACCTGGTGGATTTATTAATTTTGGATC
TGTATGTGTGTGCCATACATATTCATAGTTACGAATTGAAGATGATGGATGGAAATATCGATCTAGGATAGGTATACATGTTG
ATGCGGGTTTTACTGATGCATATACAGAGATGCTTTTTGTTCGCTTGGTTGTGATGATGTGGTGTGGTTGGGCGGTCGTTCAT
TCGTTCTAGATCGGAGTAGAATACTGTTTCAAACTACCTGGTGTATTTATTAATTTTGGAACTGTATGTGTGTGTCATACATC
TTCATAGTTACGAGTTTAAGATGGATGGAAATATCGATCTAGGATAGGTATACATGTTGATGTGGGTTTTACTGATGCATATA
CATGATGGCATATGCAGCATCTATTCATATGCTCTAACCTTGAGTACCTATCTATTATAATAAACAAGTATGTTTTATAATTA
TTTTGATCTTGATATACTTGGATGATGGCATATGCAGCAGCTATATGTGGATTTTTTTAGCCCTGCCTTCATACGCTATTTAT
TTGCTTGGTACTGTTTCTTTTGTCGATGCTCACCCTGTTGTTTGGTGTTACTTCTGCAGGCGATCGCCACACCACCATGCCGA
AGAAGAAGCGCAAGGTCATGGACAAGAAGTACTCCATCGGCCTGGACATCGGCACCAACAGCGTGGGCTGGGCCGTCATCACC
GACGAGTACAAGGTGCCCTCCAAGAAGTTCAAGGTCCTCGGCAACACCGACAGGCACAGCATCAAGAAGAACCTGATCGGCGC
CCTGCTGTTCGACTCCGGCGAGACTGCGGAGGCTACCAGGCTGAAGCGCACTGCTCGCAGGCGCTACACCAGGCGCAAGAACC
GCATCTGCTACCTCCAGGAGATTTTCTCCAACGAGATGGCCAAGGTGGACGACTCCTTCTTCCACCGCCTGGAGGAGAGCTTC
CTGGTCGAGGAAGACAAGAAGCACGAGCGCCACCCTATCTTCGGCAACATCGTGGACGAGGTCGCCTACCACGAGAAGTACCC
AACCATCTACCACCTCCGCAAGAAGCTGGTGGACTCCACCGACAAGGCCGACCTGAGGCTCATCTACCTGGCCCTCGCCCACA
TGATCAAGTTCCGCGGCCACTTCCTCATCGAGGGCGACCTGAACCCGGACAACAGCGACGTGGACAAGCTCTTCATCCAGCTG
GTCCAGACCTACAACCAGCTGTTCGAGGAGAACCCCATCAACGCCTCCGGCGTGGACGCTAAGGCTATCCTCAGCGCTAGGCT
GTCCAAGAGCAGGCGCCTGGAGAACCTCATCGCCCAGCTCCCGGGCGAGAAGAAGAACGGCCTCTTCGGCAACCTGATCGCTC
TGTCCCTCGGCCTGACCCCCAACTTCAAGAGCAACTTCGACCTGGCCGAGGACGCCAAGCTCCAGCTGTCCAAGGACACCTAC
GACGACGACCTCGACAACCTGCTCGCCCAGATCGGCGACCAGTACGCCGACCTCTTCCTGGCCGCCAAGAACCTCTCCGACGC
CATCCTGCTCAGCGACATCCTGAGGGTGAACACCGAGATCACCAAGGCCCCGCTGTCCGCCAGCATGATCAAGCGCTACGACG
AGCACCACCAGGACCTCACTCTCCTGAAGGCCCTCGTCCGCCAGCAGCTGCCCGAGAAGTACAAGGAGATTTTCTTCGACCAG
AGCAAGAACGGCTACGCGGGCTACATCGATGGCGGCGCCTCCCAGGAAGAGTTCTACAAGTTCATCAAGCCTATCCTGGAGAA
GATGGACGGCACCGAGGAGCTGCTCGTGAAGCTGAACCGCGAGGACCTGCTCCGCAAGCAGAGGACCTTCGACAACGGCAGCA
TCCCTCACCAGATCCACCTGGGCGAGCTGCACGCTATCCTCCGCCGCCAGGAAGACTTCTACCCATTCCTGAAGGACAACCGC
GAGAAGATCGAGAAGATCCTCACCTTCCGCATCCCGTACTACGTGGGCCCCCTGGCCCGCGGCAACTCCAGGTTCGCCTGGAT
GACCAGGAAGAGCGAGGAGACCATCACCCCGTGGAACTTCGAGGAAGTGGTGGACAAGGGCGCCTCCGCTCAGAGCTTCATCG
AGCGCATGACCAACTTCGACAAGAACCTCCCTAACGAGAAGGTGCTGCCAAAGCACTCCCTGCTCTACGAGTACTTCACCGTC
TACAACGAGCTGACCAAGGTGAAGTATGTGACCGAGGGCATGAGGAAGCCCGCCTTCCTCAGCGGCGAGCAGAAGAAGGCCAT
CGTGGACCTGCTCTTCAAGACCAACCGCAAGGTGACCGTCAAGCAGCTGAAGGAAGACTACTTCAAGAAGATCGAGTGCTTCG
ACTCCGTGGAGATCAGCGGCGTGGAGGACCGCTTCAACGCCTCCCTCGGCACCTACCACGACCTGCTCAAGATCATCAAGGAC
AAGGACTTCCTCGACAACGAGGAGAACGAGGACATCCTGGAGGACATCGTGCTCACCCTGACCCTCTTCGAGGACCGCGAGAT
GATCGAGGAGAGGCTCAAGACCTACGCCCACCTGTTCGACGACAAGGTCATGAAGCAGCTGAAGAGGCGCAGGTACACTGGCT
GGGGCCGCCTCAGCAGGAAGCTGATCAACGGCATCAGGGACAAGCAGTCCGGCAAGACCATCCTGGACTTCCTCAAGAGCGAC
GGCTTCGCCAACCGCAACTTCATGCAGCTCATCCACGACGACTCCCTGACCTTCAAGGAAGACATCCAGAAGGCTCAGGTGTC
CGGCCAGGGCGACAGCCTCCACGAGCACATCGCTAACCTGGCGGGCAGCCCTGCCATCAAGAAGGGCATCCTCCAGACCGTGA
AGGTGGTGGACGAGCTGGTGAAGGTCATGGGCCGCCACAAGCCAGAGAACATCGTCATCGAGATGGCCAGGGAGAACCAGACC
ACCCAGAAGGGTCAGAAGAACTCCCGCGAGAGGATGAAGAGGATCGAGGAAGGCATCAAGGAGCTGGGCAGCCAGATCCTGAA
GGAGCACCCGGTGGAGAACACCCAGCTCCAGAACGAGAAGCTGTACCTCTACTACCTGCAGAACGGCCGCGACATGTATGTGG
ACCAGGAGCTGGACATCAACAGGCTGTCCGACTACGACGTGGACCACATCGTCCCTCAGTCCTTCCTCAAGGACGACAGCATC
GACAACAAGGTGCTGACCCGCAGCGACAAGAACAGGGGCAAGTCCGACAACGTCCCAAGCGAGGAAGTGGTCAAGAAGATGAA
GAACTACTGGCGCCAGCTGCTCAACGCCAAGCTCATCACCCAGCGCAAGTTCGACAACCTGACTAAGGCGGAGAGGGGCGGCC
TGTCCGAGCTGGACAAGGCTGGCTTCATCAAGCGCCAGCTCGTGGAGACCAGGCAGATCACCAAGCACGTCGCCCAGATCCTG
GACAGCAGGATGAACACCAAGTACGACGAGAACGACAAGCTCATCCGCGAGGTGAAGGTCATCACCCTCAAGTCCAAGCTGGT
GAGCGACTTCCGCAAGGACTTCCAGTTCTACAAGGTCAGGGAGATCAACAACTACCACCACGCCCACGATGCTTACCTCAACG
CGGTGGTGGGCACCGCCCTCATCAAGAAGTACCCTAAGCTGGAGAGCGAGTTCGTGTACGGCGACTACAAGGTGTACGACGTC
CGCAAGATGATCGCCAAGTCCGAGCAGGAGATCGGCAAGGCCACCGCCAAGTACTTCTTCTACAGCAACATCATGAACTTCTT
CAAGACCGAGATCACCCTCGCCAACGGCGAGATCCGCAAGAGGCCACTGATCGAGACCAACGGCGAGACTGGCGAGATCGTGT
GGGACAAGGGCAGGGACTTCGCCACCGTGAGGAAGGTCCTGTCCATGCCTCAGGTGAACATCGTCAAGAAGACCGAGGTCCAG
ACCGGCGGCTTCTCCAAGGAGAGCATCCTCCCAAAGCGCAACAGCGACAAGCTGATCGCCAGGAAGAAGGACTGGGACCCGAA
GAAGTACGGTGGCTTCGACTCCCCTACTGTGGCTTACAGCGTCCTGGTGGTCGCCAAGGTGGAGAAGGGCAAGTCCAAGAAGC
TGAAGAGCGTCAAGGAGCTGCTCGGCATCACCATCATGGAGAGGTCCAGCTTCGAGAAGAACCCGATCGACTTCCTGGAGGCC
AAGGGCTACAAGGAAGTGAAGAAGGACCTGATCATCAAGCTGCCCAAGTACAGCCTGTTCGAGCTGGAGAACGGCCGCAAGAG

GATGCTCGCCTCCGCTGGCGAGCTGCAGAAGGGCAACGAGCTGGCCCTCCCGTCCAAGTATGTGAACTTCCTGTACCTCGCCT
CCCACTACGAGAAGCTGAAGGGCAGCCCCGAGGACAACGAGCAGAAGCAGCTCTTCGTCGAGCAGCACAAGCACTACCTGGAC
GAGATCATCGAGCAGATCAGCGAGTTCAGCAAGCGCGTGATCCTCGCCGACGCCAACCTCGACAAGGTCCTGTCCGCCTACAA
CAAGCACCGCGACAAGCCTATCAGGGAGCAGGCCGAGAACATCATCCACCTGTTCACCCTCACCAACCTGGGCGCCCCAGCTG
CCTTCAAGTACTTCGACACCACCATCGACCGCAAGAGGTACACCAGCACCAAGGAAGTGCTGGACGCCACCCTGATCCACCAG
TCCATCACCGGCCTGTACGAGACTCGCATCGACCTCAGCCAGCTGGGCGGCGACCCGAAGAAGAAGCGCAAAGTCTGAGGGAC
CCTCGATCGACAAGCTCGAGTTTCTCCATAATAATGTGTGAGTAGTTCCCAGATAAGGGAATTAGGGTTCCTATAGGGTTTCG
CTCATGTGTTGAGCATATAAGAAACCCTTAGTATGTATTTGTATTTGTAAAATACTTCTATCAATAAAATTTCTAATTCCTAA
AACCAAAATCCAGTACTAAAATCCAGATCCCCCGAATTAACCTGCAGGGGCGGCAGGGAGAGTTTTAACATTGACTAGCGTGC
TGATAATTTGTGAGAAATAATAATTGACAAGTAGATACTGACATTTGAGAAGAGCTTCTGAACTGTTATTAGTAACAAAAATG
GAAAGCTGATGCACGGAAAAAGGAAAGAAAAAGCCATACTTTTTTTTAGGTAGGAAAAGAAAAAGCCATACGAGACTGATGTC
TCTCAGATGGGCCGGGATCTGTCTATCTAGCAGGCAGCAGCCCTACCAACCTCACGGGCCAGCAATTACGAGTCCTTCTAAAA
CGTCCCGCCGAGGGCGCGTGGCCGTGCTGTGCAGCAGCACGTCTAACATTAGTCCCACCTCGCCAGTTTACAGGGAGCAGAAC
CAGCTTATAAGCGGAGGCGCGGCACCAAGAAGCAAAGTCTAGGATCACCTTTGTGTTTTAGAGCTAGAAATAGCAAGTTAAAA
TAAGGCTAGTCCGTTATCAACTTGAAAAAGTGGCACCGAGTCGGTGCTTTTTTTTCCTCGAGGAAGTCTAGGATCACCTTTGT
GGGAACGAGGTGACAACTAGGGCACTACCTAGATAACAAACACACATACAACATCACAATCAAGCAGTCATTTGAGAAAATTC
AAACAAATGGGCTTAAGCAACCAACACAAGACACAAGGCTCACCCAAAATAAGCATTTATCTCAAGCATGAACTAACTACAAG
TTTAGCTCAAAACAACGCTAGATACGGTAGCCAACTTAGCATGTGCCTAGAATCTGGACAGCAACGCAGTAGTTACTTGTTTC
AACCATAACTGAGGTTATAGACATGCCACAAAGGTGATCCTAGACTTTCTAGAAATCTTAGGAAGATTACTACACTTTAGTTA
TTCATACCAAAAACTAATTCATAGCTTAACATGACCAAAATTGCTAATATTTCACAAATTGACGCTTAGACAACTTAATAACA
CATTGCGGACGTTTTTAATGTACTGAATTAGTACTGATATCGGTACCTTAATTCGGGGGATCTGGATTTTAGTACTGGATTTT
GGTTTTAGGAATTAGAAATTTTATTGATAGAAGTATTTTACAAATACAAATACATACTAAGGGTTTCTTATATGCTCAACACA
TGAGCGAAACCCTATAGGAACCCTAATTCCCTTATCTGGGAACTACTCACACATTATTATGGAGAAACTCGAGCTTGTCGATC
GATCACTCAGCCTCGAGGGTGGCGGTCACTGGGATGAACTCGAACCTGTCGATGATCACGCCGGCGGTGCCGCTGAAGTTCCT
CACGCCCACGATGTTACCGAGGGAGGAGGTGAAAGCGTTGGCGCTCTCGAAGTAACCGAAGTCGCTGGATTGGAGGTTGTCCA
GGGAGGTAGCGGTAGCTGGCACGGTGTTGGAGAAGATGGAGGAGTTACCCCAGTTCACGTTGAGGTGGATCGGGGTCACGGAA
GCGTACCTCACGCGCACCCTGTACCTGGTGGAGGTGGATGGGAAGTGGATTGGCACTCGATGTAGCCCCTGTTCTGGATGTTG
TTGCCGCTGCTGTTGAGCCTCACGAGGTCGCCACCGGTGAAGCCTGGGCCGGAAATGACGGAACCGTTGAAGAGGAAGTTGCC
CTTCACGGCCGGGATTTGGGTGATGCTGTCGGAGGCGATGATGTTGTTGAACTCAGCGCTGCGGTGGATCCAGGAGAACATCG
GAGCCCTGATGATGCTCACGGAGCTGTTGCTGAAGCCGGAGCGGAACATGGACACGTGGCTCAGCCTGTGGGAGAAGCCTTGC
CTGGGTGGCACGTTGTTGTTCTGTGGTGGGATCTCGTCCAGGGAGTCCACGGTGCCGCTCTTCCTGTAGACAGCGGATGGCAG
GTTGGAGGAGGTGCCGTAGGCGAACTCGGTGCCGTCGAGCACGGACAGTTGCTGGTTGTTGATACCGATGTTGAAGGGCCTCC
TGTACAGGGTGGAGGACAGGGTCCTGTAGACACCCTGACCCAGTTGAGCCACGATGCGTTGCTGTGGAGCGGCGTTGCCCATG
GTGCCGTAGAGCGGGAAGGTGAACTCGGGGCCGCTGAAGCCCACTGGGGAGGCCATGATCTGGTGGCCGGACCAGTAGTACTC
GCCCCTGTGAGCGTCGGTGTAGATGGTGATGCTGTTCAGGATGTCCATCAGGTGTGGGCTCCTGATGGAGCCCTCGATACCCT
GGGCGGAACCGCGGAAGCTACCGTCGAAGTTCTCCAGCACTGGGTTGGTGTAGATCTCCCTGGTGAGTTGGGACACGGTGCGG
ATCGGGTAGGTCCTGGAGTCGTAGTTCGGGAAGAGGGACACAATGTCCAGCACGGTGAGGGTCAACTCCCTCCTGAACTGGTT
GTACCTGATCCAGTCCCTGGAGTCCGGACCCCAGACGCGCTCCAGGCCGGTGTTGTACCAGCGCACAGCGTGGTCGGTGTAGT
TGCCAATCAGCCTGGTCAGGTCGTTGTAGCGGCTGTTGATGGTGGCAGCATCGAAGCCCCACCTTTGGCCGAACACGCTCACG
TCGCGCAGCACGCTGAGGTGCAGGTTAGCGGCTTGCACGTACACGGACAGGAGCGGCACTTGGTAGTTCTGGACGGCGAACAG
TGGGATAGCGGTGGTCAGGGCGCTGTTCATGTCGTTGAATTGAATGCGCATTTCCTCGCGGAGAGCTGGGTTGGTCGGGTCGG
CCTCCCACTCCCTGAAGCTCTCGGCGTAGATTTGGTAGAGGTTGCTCAGGCCCTCCAGCCTGGAGATGGCCTGGTTCCTGGCG
AACTCTTCGATCCTCTGGTTGATCAGCTGCTCGATTTGCACCAGGAAGGCGTCCCATTGGGATGGACCGAAGATACCCCAGAT
GATGTCCACCAGGCCGAGCACGAAGCCAGCACCTGGCACGAACTCGCTGAGCAGGAACTGGGTCAAGGACAGGGAGATGTCGA
TGGGGGTGTAACCGGTCTCGATGCGCTCGCCACCCAGCACCTCCACCTCTGGGTTGCTCAGGCAGTTGTATGGGATGCACTCG
TTGATGTTTGGGTTGTTGTCCATGGCGGGGTTGATCAGGTTGATCACTTCTACCTACAAAAAAGCTCCGCACGAGGCTGCATT
TGTCACAAATCATGAAAAGAAAAACTACCGATGAACAATGCTGAGGGATTCAAATTCTACCCACAAAAAGAAGAAAGAAAGAT
CTAGCACATCTAAGCCTGACGAAGCAGCAGAAATATATAAAAATATAAACCATAGTGCCCTTTTCCCCTCTTCCTGATCTTGT
TTAGCACGGCGGAAATTTTAAACCCCCCATCATCTCCCCCAACAACGGCGGATCGCAGATCTACATCCGAGAGCCCCATTCCC
CGCGAGATCCGGGCCGGATCCACGCCGGCGAGAGCCCCAGCCGCGAGATCCCGCCCCTCCCGCGCACCGATCTGGGCGCGCAC
GAAGCCGCCTCTCGCCCACCCAAACTACCAAGGCCAAAGATCGAGACCGAGACGGAAAAAAAAACGGAGAAAGAAAGAGGAGA
GGGGCGGGGTGGTTACCGGCGGCGGCGGAGGCCTCCCTTGGATCTTATGGTGTGTTGTCCCTGTGTGTTCTCCAATAGTGTGG
CTTGAGTGTGTGGAAGATGGTTCTAGAGGATCTGCTAGAGTCAGCTTGTCAGCGTGTCCTCTCCAAATGAAATGAACTTCCTT
ATATAGAGGAAGGGTCTTGCGAAGGATAGTGGGATTGTGCGTCATCCCTTACGTCAGTGGAGATATCACATCAATCCACTTGC
TTTGAAGACGTGGTTGGAACGTCTTCTTTTTCCACGATGCTCCTCGTGGGTGGGGGTCCATCTTTGGGACCACTGTCGGCAGA
GGCATCTTCAACGATGGCCTTTCCTTTATCGCAATGATGGCATTTGTAGGAGCCACCTTCCTTTTCCACTATCTTCACAATAA
AGTGACAGATAGCTGGGCAATGGAATCCGAGGAGGTTTCCGGATATTACCCTTTGTTGAAAAGTCTCAATCGGACCATCACAT
CAATCCACTTGCTTTGAAGACGTGGTTGGAACGTCTTCTTTTTCCACGATGCTCCTCGTGGGTGGGGGTCCATCTTTGGGACC

ACTGTCGGCAGAGGCATCTTCAACGATGGCCTTTCCTTTATCGCAATGATGGCATTTGTAGGAGCCACCTTCCTTTTCCACTA
TCTTCACAATAAAGTGACAGATAGCTGGGCAATGGAATCCGAGGAGGTTTCCGGATATTACCCTTTGTTGAAAAGTCTCAATC
GGACCTGTTTAAACCCTGAAGCTTACGCGTATGCCTGCAGTGCAGCGTGACCCGGTCGTGCCCCTCTCTAGAGATAATGAGCA
TTGCATGTCTAAGTTATAAAAAATTACCACATATTTTTTTTGTCACACTTGTTTGAAGTGCAGTTTATCTATCTTTATACATA
TATTTAAACTTTACTCTACGAATAATATAATCTATAGTACTACAATAATATCAGTGTTTTAGAGAATCATATAAATGAACAGT
TAGACATGGTCTAAAGGACAATTGAGTATTTTGACAACAGGACTCTACAGTTTTATCTTTTTAGTGTGCATGTGTTCTCCTTT
TTTTTTGCAAATAGCTTCACCTATATAATACTTCATCCATTTTATTAGTACATCCATTTAGGGTTTAGGGTTAATGGTTTTTA
TAGACTAATTTTTTTAGTACATCTATTTTATTCTATTTTAGCCTCTAAATTAAGAAAACTAAAACTCTATTTTAGTTTTTTTA
TTTAATAATTTAGATATAAAATAGAATAAAATAAAGTGACTAAAAATTAAACAAATACCCTTTAAGAAATTAAAAAAACTAAG
GAAACATTTTTCTTGTTTCGAGTAGATAATGCCAGCCTGTTAAACGCCGTCGACGAGTCTAACGGACACCAACCAGCGAACCA
GCAGCGTCGCGTCGGGCCAAGCGAAGCAGACGGCACGGCATCTCTGTCGCTGCCTCTGGACCCCTCTCGAGAGTTCCGCTCCA
CCGTTGGACTTGCTCCGCTGTCGGCATCCAGAAATTGCGTGGCGGAGCGGCAGACGTGAGCCGGCACGGCAGGCGGCCTCCTC
CTCCTCTCACGGCACGGCAGCTACGGGGGATTCCTTTCCCACCGCTCCTTCGCTTTCCCTTCCTCGCCCGCCGTAATAAATAG
ACACCCCCTCCACACCCTCTTTCCCCAACCTCGTGTTGTTCGGAGCGCACACACACACAACCAGATCTCCCCCAAATCCACCC
GTCGGCACCTCCGCTTCAAGGTACGCCGCTCGTCCTCCCCCCCCCCCCCTCTCTACCTTCTCTAGATCGGCGTTCCGGTCCAT
GGTTAGGGCCCGGTAGTTCTACTTCTGTTCATGTTTGTGTTAGATCCGTGTTTGTGTTAGATCCGTGCTGCTAGCGTTCGTAC
ACGGATGCGACCTGTACGTCAGACACGTTCTGATTGCTAACTTGCCAGTGTTTCTCTTTGGGGAATCCTGGGATGGCTCTAGC
CGTTCCGCAGACGGGATCGATTTCATGATTTTTTTTGTTTCGTTGCATAGGGTTTGGTTTGCCCTTTTCCTTTATTTCAATAT
ATGCCGTGCACTTGTTTGTCGGGTCATCTTTTCATGCTTTTTTTTGTCTTGGTTGTGATGATGTGGTCTGGTTGGGCGGTCGT
TCTAGATCGGAGTAGAATTCTGTTTCAAACTACCTGGTGGATTTATTAATTTTGGATCTGTATGTGTGTGCCATACATATTCA
TAGTTACGAATTGAAGATGATGGATGGAAATATCGATCTAGGATAGGTATACATGTTGATGCGGGTTTTACTGATGCATATAC
AGAGATGCTTTTTGTTCGCTTGGTTGTGATGATGTGGTGTGGTTGGGCGGTCGTTCATTCGTTCTAGATCGGAGTAGAATACT
GTTTCAAACTACCTGGTGTATTTATTAATTTTGGAACTGTATGTGTGTGTCATACATCTTCATAGTTACGAGTTTAAGATGGA
TGGAAATATCGATCTAGGATAGGTATACATGTTGATGTGGGTTTTACTGATGCATATACATGATGGCATATGCAGCATCTATT
CATATGCTCTAACCTTGAGTACCTATCTATTATAATAAACAAGTATGTTTTATAATTATTTTGATCTTGATATACTTGGATGA
TGGCATATGCAGCAGCTATATGTGGATTTTTTTAGCCCTGCCTTCATACGCTATTTATTTGCTTGGTACTGTTTCTTTTGTCG
ATGCTCACCCTGTTGTTTGGTGTTACTTCTGCAGGTCGACTCTAGAATGTGGATTGAACAAGATGGATTGCACGCAGGTTCTC
CGGCCGCTTGGGTGGAGAGGCTATTCGGCTATGACTGGGCACAACAGACAATCGGCTGCTCTGATGCCGCCGTGTTCCGGCTG
TCAGCGCAGGGGCGCCCGGTTCTTTTTGTCAAGACCGACCTGTCCGGTGCCCTGAATGAACTGCAGGACGAGGCAGCGCGGCT
ATCGTGGCTGGCCACGACGGGCGTTCCTTGCGCAGCTGTGCTCGACGTTGTCACTGAAGCGGGAAGGGACTGGCTGCTATTGG
GCGAAGTGCCGGGGCAGGATCTCCTGTCATCTCACCTTGCTCCTGCCGAGAAAGTATCCATCATGGCTGATGCAATGCGGCGG
CTGCATACGCTTGATCCGGCTACCTGCCCATTCGACCACCAAGCGAAACATCGCATCGAGCGAGCACGTACTCGGATGGAAGC
CGGTCTTGTCGATCAGGATGATCTGGACGAAGAGCATCAGGGGCTCGCGCCAGCCGAACTGTTCGCCAGGCTCAAGGCGCGCA
TGCCCGACGGCGAGGATCTCGTCGTGACCCATGGCGATGCCTGCTTGCCGAATATCATGGTGGAAAATGGCCGCTTTTCTGGA
TTCATCGACTGTGGCCGGCTGGGTGTGGCGGACCGCTATCAGGACATAGCGTTGGCTACCCGTGATATTGCTGAAGAGCTTGG
CGGCGAATGGGCTGACCGCTTCCTCGTGCTTTACGGTATCGCCGCTCCCGATTCGCAGCGCATCGCCTTCTATCGCCTTCTTG
ACGAGTTCTTCTGAGATCGTTCAAACATTTGGCAATAAAGTTTCTTAAGATTGAATCCTGTTGCCGGTCTTGCGATGATTATC
ATATAATTTCTGTTGAATTACGTTAAGCATGTAATAATTAACATGTAATGCATGACGTTATTTATGAGATGGGTTTTTATGAT
TAGAGTCCCGCAATTATACATTTAATACGCGATAGAAAACAAAATATAGCGCGCAAACTAGGATAAATTATCGCGCGCGGTGT
CATCTATGTTACTAGATCGGCGCGCCAAGGGCGAATTCCAGCACACTGGCGGCCGTTACTAGTGGATCGAGCTCGTCGACTCT
AGACTCGAGGGCGCGCCGATACCAATTTGGACAGAAAACTGACAAGAACTTCGAAAATACATAATTGGAGTTCTAGAGACACA
ACAAAAGTGATCCTTAACTTTATGTAAAGCTTATTAAGTTGTCTATAACTTTCTTATTATCATATTAAGATGATTCTACGGCT
AACAAGGACAAACATAGCAAAGAAAACATCTCTGCCGGATTTGGACAGATTCTGTCATTCTACTTTGCAAGCTCATAACTAGA
GATTTAGACCATCACAATTAGTGCTATAGGACATTTTGGAAAGCTTAGAAAAAGTACTACACTTCTTCTATTATCTCTAAGAC
ACGATTCCATATGCACACAGGTCAAACAAACCAAACATTCAGATCTATTCAGAACATTGACAAAACCTGCCCACAAAGGTGAT
CCTAGACTTGGTACCACTAGTATTAATTAAGTTTAAACGGCGCGCCAAGGGCGAATTCCAGCACACTGGCGGCCGTTACTAGT
GGATCGAGCTCGTCGACTCTAGACTCGAGGGCGCGCCTGACAGGATATATTGGCGGGTAAAC

**>SEQ ID 26 HR_template construct**

CTTAGAATAACGGATATTTAAAAGGGCGTGAAAAGGTTTATCCGTTCGTCCATTTGTATGTGCATGCCAACCACAGGGTTCCC
CTCGGGATCAAAGTACTTTGATCCAACCCCTCCGCTGCTATAGTGCAGTCGGCTTCTGACGTTCAGTGCAGCCGTCTTCTGAA
AACGACATGTCGCACAAGTCCTAAGTTACGCGACAGGCTGCCGCCCTGCCCTTTTCCTGGCGTTTTCTTGTCGCGTGTTTTAG
TCGCATAAAGTAGAATACTTGCGACTAGAACCGGAGACATTACGCCATGAACAAGAGCGCCGCCGCTGGCCTGCTGGGCTATG
CCCGCGTCAGCACCGACGACCAGGACTTGACCAACCAACGGGCCGAACTGCACGCGGCCGGCTGCACCAAGCTGTTTTCCGAG
AAGATCACCGGCACCAGGCGCGACCGCCCGGAGCTGGCCAGGATGCTTGACCACCTACGCCCTGGCGACGTTGTGACAGTGAC
CAGGCTAGACCGCCTGGCCCGCAGCACCCGCGACCTACTGGACATTGCCGAGCGCATCCAGGAGGCCGGCGCGGGCCTGCGTA
GCCTGGCAGAGCCGTGGGCCGACACCACCACGCCGGCCGGCCGCATGGTGTTGACCGTGTTCGCCGGCATTGCCGAGTTCGAG

CGTTCCCTAATCATCGACCGCACCCGGAGCGGGCGCGAGGCCGCCAAGGCCCGAGGCGTGAAGTTTGGCCCCCGCCCTACCCT
CACCCCGGCACAGATCGCGCACGCCCGCGAGCTGATCGACCAGGAAGGCCGCACCGTGAAAGAGGCGGCTGCACTGCTTGGCG
TGCATCGCTCGACCCTGTACCGCGCACTTGAGCGCAGCGAGGAAGTGACGCCCACCGAGGCCAGGCGGCGCGGTGCCTTCCGT
GAGGACGCATTGACCGAGGCCGACGCCCTGGCGGCCGCCGAGAATGAACGCCAAGAGGAACAAGCATGAAACCGCACCAGGAC
GGCCAGGACGAACCGTTTTTCATTACCGAAGAGATCGAGGCGGAGATGATCGCGGCCGGGTACGTGTTCGAGCCGCCCGCGCA
CGTCTCAACCGTGCGGCTGCATGAAATCCTGGCCGGTTTGTCTGATGCCAAGCTGGCGGCCTGGCCGGCCAGCTTGGCCGCTG
AAGAAACCGAGCGCCGCCGTCTAAAAAGGTGATGTGTATTTGAGTAAAACAGCTTGCGTCATGCGGTCGCTGCGTATATGATG
CGATGAGTAAATAAACAAATACGCAAGGGGAACGCATGAAGGTTATCGCTGTACTTAACCAGAAAGGCGGGTCAGGCAAGACG
ACCATCGCAACCCATCTAGCCCGCGCCCTGCAACTCGCCGGGGCCGATGTTCTGTTAGTCGATTCCGATCCCCAGGGCAGTGC
CCGCGATTGGGCGGCCGTGCGGGAAGATCAACCGCTAACCGTTGTCGGCATCGACCGCCCGACGATTGACCGCGACGTGAAGG
CCATCGGCCGGCGCGACTTCGTAGTGATCGACGGAGCGCCCCAGGCGGCGGACTTGGCTGTGTCCGCGATCAAGGCAGCCGAC
TTCGTGCTGATTCCGGTGCAGCCAAGCCCTTACGACATATGGGCCACCGCCGACCTGGTGGAGCTGGTTAAGCAGCGCATTGA
GGTCACGGATGGAAGGCTACAAGCGGCCTTTGTCGTGTCGCGGGCGATCAAAGGCACGCGCATCGGCGGTGAGGTTGCCGAGG
CGCTGGCCGGGTACGAGCTGCCCATTCTTGAGTCCCGTATCACGCAGCGCGTGAGCTACCCAGGCACTGCCGCCGCCGGCACA
ACCGTTCTTGAATCAGAACCCGAGGGCGACGCTGCCCGCGAGGTCCAGGCGCTGGCCGCTGAAATTAAATCAAAACTCATTTG
AGTTAATGAGGTAAAGAGAAAATGAGCAAAAGCACAAACACGCTAAGTGCCGGCCGTCCGAGCGCACGCAGCAGCAAGGCTGC
AACGTTGGCCAGCCTGGCAGACACGCCAGCCATGAAGCGGGTCAACTTTCAGTTGCCGGCGGAGGATCACACCAAGCTGAAGA
TGTACGCGGTACGCCAAGGCAAGACCATTACCGAGCTGCTATCTGAATACATCGCGCAGCTACCAGAGTAAATGAGCAAATGA
ATAAATGAGTAGATGAATTTTAGCGGCTAAAGGAGGCGGCATGGAAAATCAAGAACAACCAGGCACCGACGCCGTGGAATGCC
CCATGTGTGGAGGAACGGGCGGTTGGCCAGGCGTAAGCGGCTGGGTTGTCTGCCGGCCCTGCAATGGCACTGGAACCCCCAAG
CCCGAGGAATCGGCGTGACGGTCGCAAACCATCCGGCCCGGTACAAATCGGCGCGGCGCTGGGTGATGACCTGGTGGAGAAGT
TGAAGGCCGCGCAGGCCGCCCAGCGGCAACGCATCGAGGCAGAAGCACGCCCCGGTGAATCGTGGCAAGCGGCCGCTGATCGA
ATCCGCAAAGAATCCCGGCAACCGCCGGCAGCCGGTGCGCCGTCGATTAGGAAGCCGCCCAAGGGCGACGAGCAACCAGATTT
TTTCGTTCCGATGCTCTATGACGTGGGCACCCGCGATAGTCGCAGCATCATGGACGTGGCCGTTTTCCGTCTGTCGAAGCGTG
ACCGACGAGCTGGCGAGGTGATCCGCTACGAGCTTCCAGACGGGCACGTAGAGGTTTCCGCAGGGCCGGCCGGCATGGCCAGT
GTGTGGGATTACGACCTGGTACTGATGGCGGTTTCCCATCTAACCGAATCCATGAACCGATACCGGGAAGGGAAGGGAGACAA
GCCCGGCCGCGTGTTCCGTCCACACGTTGCGGACGTACTCAAGTTCTGCCGGCGAGCCGATGGCGGAAAGCAGAAAGACGACC
TGGTAGAAACCTGCATTCGGTTAAACACCACGCACGTTGCCATGCAGCGTACGAAGAAGGCCAAGAACGGCCGCCTGGTGACG
GTATCCGAGGGTGAAGCCTTGATTAGCCGCTACAAGATCGTAAAGAGCGAAACCGGGCGGCCGGAGTACATCGAGATCGAGCT
AGCTGATTGGATGTACCGCGAGATCACAGAAGGCAAGAACCCGGACGTGCTGACGGTTCACCCCGATTACTTTTTGATCGATC
CCGGCATCGGCCGTTTTCTCTACCGCCTGGCACGCCGCGCCGCAGGCAAGGCAGAAGCCAGATGGTTGTTCAAGACGATCTAC
GAACGCAGTGGCAGCGCCGGAGAGTTCAAGAAGTTCTGTTTCACCGTGCGCAAGCTGATCGGGTCAAATGACCTGCCGGAGTA
CGATTTGAAGGAGGAGGCGGGGCAGGCTGGCCCGATCCTAGTCATGCGCTACCGCAACCTGATCGAGGGCGAAGCATCCGCCG
GTTCCTAATGTACGGAGCAGATGCTAGGGCAAATTGCCCTAGCAGGGGAAAAAGGTCGAAAAGGTCTCTTTCCTGTGGATAGC
ACGTACATTGGGAACCCAAAGCCGTACATTGGGAACCGGAACCCGTACATTGGGAACCCAAAGCCGTACATTGGGAACCGGTC
ACACATGTAAGTGACTGATATAAAAGAGAAAAAAGGCGATTTTTCCGCCTAAAACTCTTTAAAACTTATTAAAACTCTTAAAA
CCCGCCTGGCCTGTGCATAACTGTCTGGCCAGCGCACAGCCGAAGAGCTGCAAAAAGCGCCTACCCTTCGGTCGCTGCGCTCC
CTACGCCCCGCCGCTTCGCGTCGGCCTATCGCGGCCGCTGGCCGCTCAAAAATGGCTGGCCTACGGCCAGGCAATCTACCAGG
GCGCGGACAAGCCGCGCCGTCGCCACTCGACCGCCGGCGCCCACATCAAGGCACCCTGCCTCGCGCGTTTCGGTGATGACGGT
GAAAACCTCTGACACATGCAGCTCCCGGAGACGGTCACAGCTTGTCTGTAAGCGGATGCCGGGAGCAGACAAGCCCGTCAGGG
CGCGTCAGCGGGTGTTGGCGGGTGTCGGGGCGCAGCCATGACCCAGTCACGTAGCGATAGCGGAGTGTATACTGGCTTAACTA
TGCGGCATCAGAGCAGATTGTACTGAGAGTGCACCATATGCGGTGTGAAATACCGCACAGATGCGTAAGGAGAAAATACCGCA
TCAGGCGCTCTTCCGCTTCCTCGCTCACTGACTCGCTGCGCTCGGTCGTTCGGCTGCGGCGAGCGGTATCAGCTCACTCAAAG
GCGGTAATACGGTTATCCACAGAATCAGGGGATAACGCAGGAAAGAACATGTGAGCAAAAGGCCAGCAAAAGGCCAGGAACCG
TAAAAAGGCCGCGTTGCTGGCGTTTTTCCATAGGCTCCGCCCCCCTGACGAGCATCACAAAAATCGACGCTCAAGTCAGAGGT
GGCGAAACCCGACAGGACTATAAAGATACCAGGCGTTTCCCCCTGGAAGCTCCCTCGTGCGCTCTCCTGTTCCGACCCTGCCG
CTTACCGGATACCTGTCCGCCTTTCTCCCTTCGGGAAGCGTGGCGCTTTCTCATAGCTCACGCTGTAGGTATCTCAGTTCGGT
GTAGGTCGTTCGCTCCAAGCTGGGCTGTGTGCACGAACCCCCCGTTCAGCCCGACCGCTGCGCCTTATCCGGTAACTATCGTC
TTGAGTCCAACCCGGTAAGACACGACTTATCGCCACTGGCAGCAGCCACTGGTAACAGGATTAGCAGAGCGAGGTATGTAGGC
GGTGCTACAGAGTTCTTGAAGTGGTGGCCTAACTACGGCTACACTAGAAGGACAGTATTTGGTATCTGCGCTCTGCTGAAGCC
AGTTACCTTCGGAAAAAGAGTTGGTAGCTCTTGATCCGGCAAACAAACCACCGCTGGTAGCGGTGGTTTTTTTGTTTGCAAGC
AGCAGATTACGCGCAGAAAAAAAGGATCTCAAGAAGATCCTTTGATCTTTTCTACGGGGTCTGACGCTCAGTGGAACGAAAAC
TCACGTTAAGGGATTTTGGTCATGCATTCTAGGTATTATTTGCCAACGACCTTCGTGATCTCGCCCTTGACATAGTGGACAAA
TTCTTCGAGCTGGTCGGCCCGGGACGCGAGACGGTCTTCTTCTTGGCCCAGATAGGCTTGGCGCGCTTCGAGGATCACGGGCT
GGTATTGCGCCGGAAGGCGCTCCATCGCCCAGTCGGCGGCGACATCCTTCGGCGCGATCTTGCCGGTAACCGCCGAGTACCAA
ATCCGGCTCAGCGTAAGGACCACATTGCGCTCATCGCCCGCCCAATCCGGCGGGGAGTTCCACAGGGTCAGCGTCTCGTTCAG
TGCTTCGAACAGATCCTGTTCCGGCACCGGGTCGAAAAGTTCCTCGGCCGCGGGGCCGACGAGGGCCACGCTATGCTCCCGGG
CCTTGGTGAGCAGGATCGCCAGATCAATGTCGATGGTGGCCGGTTCAAAGATACCCGCCAGAATATCATTACGCTGCCATTCG

CCGAACTGGAGTTCGCGTTTGGCCGGATAGCGCCAGGGGATGATGTCATCGTGCACCACAATCGTCACCTCAACCGCGCGCAG
GATTTCGCTCTCGCCGGGGGAGGCGGACGTTTCCAGAAGGTCGTTGATAAGCGCGCGGCGCGTGGTCTCGTCGAGACGGACGG
TAACGGTGACAAGCAGGTCGATGTCCGAATGGGGCTTAAGGCCGCCGTCAACGGCGCTACCATACAGATGCACGGCGAGGAGG
GTCGGTTCGAGGTGGCGCTCGATGACACCCACGACTTCCGACAGCTGGGTGGACACCTCGGCGATGACCGCTTCACCCATTTA
TTATTTCCTTCCTCTTTTCTACAGTATTTAAAGATACCCCAAGAAGCTAATTATAACAAGACGAACTCCAATTCACTGTTCCT
TGCATTCTAAAACCTTAAATACCAGAAAACAGCTTTTTCAAAGTTGTTTTCAAAGTTGGCGTATAACATAGTATCGACGGAGC
CGATTTTGAAACCGCGGTGATCACAGGCAGCAACGCTCTGTCATCGTTACAATCAACATGCTACCCTCCGCGAGATCATCCGT
GTTTCAAACCCGGCAGCTTAGTTGCCGTTCTTCCGAATAGCATCGGTAACATGAGCAAAGTCTGCCGCCTTACAACGGCTCTC
CCGCTGACGCCGTCCCGGACTGATGGGCTGCCTGTATCGAGTGGTGATTTTGTGCCGAGCTGCCGGTCGGGGAGCTGTTGGCT
GGCTGGTGGCAGGATATATTGTGGTGTAAACAAATTGACGCTTAGACAACTTAATAACACATTGCGGACGTTTTTAATGTACT
GAATTAGTACTGATAAATGGCGCGCCAAGCTTTGGCAAACAGCTATTATGGGTATTATGGGTGGTACCACGCGTCGGATCCGA
TCTAGTAACATAGATGACACCGCGCGCGATAATTTATCCTAGTTTGCGCGCTATATTTTGTTTTCTATCGCGTATTAAATGTA
TAATTGCGGGACTCTAATCATAAAAACCCATCTCATAAATAACGTCATGCATTACATGTTAATTATTACATGCTTAACGTAAT
TCAACAGAAATTATATGATAATCATCGCAAGACCGGCAACAGGATTCAATCTTAAGAAACTTTATTGCCAAATGTTTGAACGA
TCCCTAGGACGATCTCACTTGTAGAGCTCGTCCATGCCGTAGAGGAACAGGTGATGGCGGCCCTCGGACCTCTCGTACTGCTC
AACAATAGTGTAATCCTCGTTATGGCTAGTAATATCCAGCTTAGTATCCACGTAGTAGTAGCCAGGGAGCTGAACCGGCTTCT
TGGCCATGTAGATAGTCTTGAACTCCACCAGGTAATGGCCACCATCCTTCAGCTTGAGAGCCTGATGAATCTCGCCCTTCAGA
ACGCCATCCCTAGGGTAGAGCCTCTCAGTGGAGGCCTCCCACCCCATGGTCTTCTTCTGCATGACAGGGCCGTCTGGAGGGAA
GTTGGTGCCGCGCATCTTCACCTTGTAGATGAGGGTGCCATCTTGGAGGGAGCTATCCTGAGTCACAGTAACCAGGCCACCAT
CTTCAAAGTTCATGACGCGTTCCCACTTGAAGCCTTCTGGGAAGGAGAGCTTCTTGTAATCAGGAATATCAGCAGGGTGCTTC
ACGTAGGCTTTGGAGCCGTACATGAACTGAGGGGAGAGGATATCCCAGGCGAAAGGGAGAGGGCCGCCCTTAGTCACTTTGAG
CTTAGCAGTCTGGGTGCCTTCATAAGGGCGGCCCTCGCCCTCACCTTCAATCTCGAACTCATGGCCGTTCATGGAGCCCTCCA
TCCTGACCTTGAAGCGCATGAACTCCTTGATCACGGCCATGTTGTTGTCCTCGCTGGAGGCGGTGCCGCTGGAGCCGCTGCCA
GTGGAGCCAGTGCCGTGGCCGAGGAACAGGTGGTGCCTGCCCTCGCTGCGCTCGTACTGCTCCACGATGGTGTAGTCCTCGTT
GTGGGAGGTGATGTCGAGCTTGGTGTCGACGTAGTAGTAGCCTGGCAGCTGCACAGGCTTCTTGGCCATGTAGATGGTCTTGA
ACTCGACCAGGTAGTGGCCACCGTCCTTGAGCTTCAGGGCCTGGTGGATCTCGCCCTTGAGCACGCCGTCCCTGGGGTACAGC
CTCTCAGTGCTAGCCTCCCAGCCCATGGTCTTCTTCTGCATGACCGGGCCATCGGGCGGGAAGTTAGTGCCCCTCATCTTCAC
CTTGTAGATGAGGGTGCCATCCTGGAGGCTGGAGTCCTGAGTGACGGTCACGAGGCCACCGTCCTCGAAGTTCATGACGCGCT
CCCACTTGAAGCCCTCGGGGAAGGACAGCTTCTTGTAGTCGGGGATGTCGGCCGGGTGCTTCACGTAGGCCTTGCTGCCGTAC
ATGAACTGCGGGGAGAGGATGTCCCAAGCGAATGGCAGCGGGCCGCCCTTAGTGACCTTGAGCTTGGCGGTCTGGGTGCCCTC
GTAAGGCCTGCCCTCGCCCTCGCCCTCGATCTCGAACTCGTGGCCGTTCATGCTGCCCTCCATCCTCACCTTGAAGCGCATGA
ACTCCTTGATCACTTCCTCGCCCTTGGACACCACTACAAAAAAGCTCCGCACGAGGCTGCATTTGTCACAAATCATGAAAAGA
AAAACTACCGATGAACAATGCTGAGGGATTCAAATTCTACCCACAAAAAGAAGAAAGAAAGATCTAGCACATCTAAGCCTGAC
GAAGCAGCAGAAATATATAAAAATATAAACCATAGTGCCCTTTTCCCCTCTTCCTGATCTTGTTTAGCACGGCGGAAATTTTA
AACCCCCCATCATCTCCCCCAACAACGGCGGATCGCAGATCTACATCCGAGAGCCCCATTCCCCGCGAGATCCGGGCCGGATC
CACGCCGGCGAGAGCCCCAGCCGCGAGATCCCGCCCCTCCCGCGCACCGATCTGGGCGCGCACGAAGCCGCCTCTCGCCCACC
CAAACTACCAAGGCCAAAGATCGAGACCGAGACGGAAAAAAAAAACGGAGAAAGAAAGAGGAGAGGGGCGGGGTGGTTACCGGC
GGCGGCGGAGGCCTCCCTTGGATCTTATGGTGTGTTGTCCCTGTGTGTTCTCCAATAGTGTGGCTTGAGTGTGTGGAAGATGG
TTCTAGAGGATCTGCTAGAGTCAGCTTGTCAGCGTGTCCTCTCCAAATGAAATGAACTTCCTTATATAGAGGAAGGGTCTTGC
GAAGGATAGTGGGATTGTGCGTCATCCCTTACGTCAGTGGAGATATCACATCAATCCACTTGCTTTGAAGACGTGGTTGGAAC
GTCTTCTTTTTCCACGATGCTCCTCGTGGGTGGGGGTCCATCTTTGGGACCACTGTCGGCAGAGGCATCTTCAACGATGGCCT
TTCCTTTATCGCAATGATGGCATTTGTAGGAGCCACCTTCCTTTTCCACTATCTTCACAATAAAGTGACAGATAGCTGGGCAA
TGGAATCCGAGGAGGTTTCCGGATATTACCCTTTGTTGAAAAGTCTCAATCGGACCATCACATCAATCCACTTGCTTTGAAGA
CGTGGTTGGAACGTCTTCTTTTTCCACGATGCTCCTCGTGGGTGGGGGTCCATCTTTGGGACCACTGTCGGCAGAGGCATCTT
CAACGATGGCCTTTCCTTTATCGCAATGATGGCATTTGTAGGAGCCACCTTCCTTTTCCACTATCTTCACAATAAAGTGACAG
ATAGCTGGGCAATGGAATCCGAGGAGGTTTCCGGATATTACCCTTTGTTGAAAAGTCTCAATCGGACCCCCTCAGCCTGCACC
TCGAGGAAGTCTAGGATCACCTTTGTGGGAACGAGGTGACAACTAGGGCACTACCTAGATAACAAACACACATACAACATCAC
AATCAAGCAGTCATTTGAGAAAATTCAAACAAATGGGCTTAAGCAACCAACACAAGACACAAGGCTCACCCAAAATAAGCATT
TATCTCAAGCATGAACTAACTACAAGTTTAGCTCAAAACAACGCTAGATACGGTAGCCAACTTAGCATGTGCCTAGAATCTGG
ACAGCAACGCAGTAGTTACTTGTTTCAACCATAACTGAGGTTATAGACATACCACAAAGGTGATCCTAGACTTTCTAGAAATC
TTAGGAAGATTACTACACTTTAGTTATTCATACCAAAAACTAATTCATAGCTTAACATGACCAAAATTGCTAATATTTCACAA
ATTGACGCTTAGACAACTTAATAACACATTGCGGACGTTTTTAATGTACTGAATTAGTACTGATATCGGTACCTTAATTCGGG
GGATCTGGATTTTAGTACTGGATTTTGGTTTTAGGAATTAGAAATTTTATTGATAGAAGTATTTTACAAATACAAATACATAC
TAAGGGTTTCTTATATGCTCAACACATGAGCGAAACCCTATAGGAACCCTAATTCCCTTATCTGGGAACTACTCACACATTAT
TATGGAGAAACTCGAGCTTGTCGATCGATCACTCAGCCTCGAGGGTGGCGGTCACTGGGATGAACTCGAACCTGTCGATGATC
ACGCCGGCGGTGCCGCTGAAGTTCCTCACGCCCACGATGTTACCGAGGGAGGAGGTGAAAGCGTTGGCGCTCTCGAAGTAACC
GAAGTCGCTGGATTGGAGGTTGTCCAGGGAGGTAGCGGTAGCTGGCACGGTGTTGGAGAAGATGGAGGAGTTACCCCAGTTCA
CGTTGAGGTGGATCGGGGTCACGGAAGCGTACCTCACGCGCACCCTGTACCTGGTGGAGGTGGATGGGAAGTGGATTGGCACT

CGATGTAGCCCCTGTTCTGGATGTTGTTGCCGCTGCTGTTGAGCCTCACGAGGTCGCCACCGGTGAAGCCTGGGCCGGAAATG
ACGGAACCGTTGAAGAGGAAGTTGCCCTTCACGGCCGGGATTTGGGTGATGCTGTCGGAGGCGATGATGTTGTTGAACTCAGC
GCTGCGGTGGATCCAGGAGAACATCGGAGCCCTGATGATGCTCACGGAGCTGTTGCTGAAGCCGGAGCGGAACATGGACACGT
GGCTCAGCCTGTGGGAGAAGCCTTGCCTGGGTGGCACGTTGTTGTTCTGTGGTGGGATCTCGTCCAGGGAGTCCACGGTGCCG
CTCTTCCTGTAGACAGCGGATGGCAGGTTGGAGGAGGTGCCGTAGGCGAACTCGGTGCCGTCGAGCACGGACAGTTGCTGGTT
GTTGATACCGATGTTGAAGGGCCTCCTGTACAGGGTGGAGGACAGGGTCCTGTAGACACCCTGACCCAGTTGAGCCACGATGC
GTTGCTGTGGAGCGGCGTTGCCCATGGTGCCGTAGAGCGGGAAGGTGAACTCGGGGCCGCTGAAGCCCACTGGGGAGGCCATG
ATCTGGTGGCCGGACCAGTAGTACTCGCCCCTGTGAGCGTCGGTGTAGATGGTGATGCTGTTCAGGATGTCCATCAGGTGTGG
GCTCCTGATGGAGCCCTCGATACCCTGGGCGGAACCGCGGAAGCTACCGTCGAAGTTCTCCAGCACTGGGTTGGTGTAGATCT
CCCTGGTGAGTTGGGACACGGTGCGGATCGGGTAGGTCCTGGAGTCGTAGTTCGGGAAGAGGGACACAATGTCCAGCACGGTG
AGGGTCAACTCCCTCCTGAACTGGTTGTACCTGATCCAGTCCCTGGAGTCCGGACCCCAGACGCGCTCCAGGCCGGTGTTGTA
CCAGCGCACAGCGTGGTCGGTGTAGTTGCCAATCAGCCTGGTCAGGTCGTTGTAGCGGCTGTTGATGGTGGCAGCATCGAAGC
CCCACCTTTGGCCGAACACGCTCACGTCGCGCAGCACGCTGAGGTGCAGGTTAGCGGCTTGCACGTACACGGACAGGAGCGGC
ACTTGGTAGTTCTGGACGGCGAACAGTGGGATAGCGGTGGTCAGGGCGCTGTTCATGTCGTTGAATTGAATGCGCATTTCCTC
GCGGAGAGCTGGGTTGGTCGGGTCGGCCTCCCACTCCCTGAAGCTCTCGGCGTAGATTTGGTAGAGGTTGCTCAGGCCCTCCA
GCCTGGAGATGGCCTGGTTCCTGGCGAACTCTTCGATCCTCTGGTTGATCAGCTGCTCGATTTGCACCAGGAAGGCGTCCCAT
TGGGATGGACCGAAGATACCCCAGATGATGTCCACCAGGCCGAGCACGAAGCCAGCACCTGGCACGAACTCGCTGAGCAGGAA
CTGGGTCAAGGACAGGGAGATGTCGATGGGGGTGTAACCGGTCTCGATGCGCTCGCCACCCAGCACCTCCACCTCTGGGTTGC
TCAGGCAGTTGTATGGGATGCACTCGTTGATGTTTGGGTTGTTGTCCATGGCGGGGTTGATCAGGTTGATCACTTCTACCTAC
AAAAAAGCTCCGCACGAGGCTGCATTTGTCACAAATCATGAAAAGAAAAACTACCGATGAACAATGCTGAGGGATTCAAATTC
TACCCACAAAAAGAAGAAAGAAAGATCTAGCACATCTAAGCCTGACGAAGCAGCAGAAATATATAAAAATATAAACCATAGTG
CCCTTTTCCCCTCTTCCTGATCTTGTTTAGCACGGCGGAAATTTAAACCCCCCATCATCTCCCCCAACAACGGCGGATCGCA
GATCTACATCCGAGAGCCCCATTCCCCGCGAGATCCGGGCCGGATCCACGCCGGCGAGAGCCCCAGCCGCGAGATCCCGCCCC
TCCCGCGCACCGATCTGGGCGCGCACGAAGCCGCCTCTCGCCCACCCAAACTACCAAGGCCAAAGATCGAGACCGAGACGGAA
AAAAAAACGGAGAAAGAAAGAGGAGAGGGGCGGGGTGGTTACCGGCGGCGGCGGAGGCCTCCCTTGGATCTTATGGTGTGTTG
TCCCTGTGTGTTCTCCAATAGTGTGGCTTGAGTGTGTGGAAGATGGTTCTAGAGGATCTGCTAGAGTCAGCTTGTCAGCGTGT
CCTCTCCAAATGAAATGAACTTCCTTATATAGAGGAAGGGTCTTGCGAAGGATAGTGGGATTGTGCGTCATCCCTTACGTCAG
TGGAGATATCACATCAATCCACTTGCTTTGAAGACGTGGTTGGAACGTCTTCTTTTTCCACGATGCTCCTCGTGGGTGGGGGT
CCATCTTTGGGACCACTGTCGGCAGAGGCATCTTCAACGATGGCCTTTCCTTTATCGCAATGATGGCATTTGTAGGAGCCACC
TTCCTTTTCCACTATCTTCACAATAAAGTGACAGATAGCTGGGCAATGGAATCCGAGGAGGTTTCCGGATATTACCCTTTGTT
GAAAAGTCTCAATCGGACCATCACATCAATCCACTTGCTTTGAAGACGTGGTTGGAACGTCTTCTTTTTCCACGATGCTCCTC
GTGGGTGGGGGTCCATCTTTGGGACCACTGTCGGCAGAGGCATCTTCAACGATGGCCTTTCCTTTATCGCAATGATGGCATTT
GTAGGAGCCACCTTCCTTTTCCACTATCTTCACAATAAAGTGACAGATAGCTGGGCAATGGAATCCGAGGAGGTTTCCGGATA
TTACCCTTTGTTGAAAAGTCTCAATCGGACCTGTTTAAACCCTGAAGCTTACGCGTATGCCTGCAGTGCAGCGTGACCCGGTC
GTGCCCCTCTCTAGAGATAATGAGCATTGCATGTCTAAGTTATAAAAAATTACCACATATTTTTTTTGTCACACTTGTTTGAA
GTGCAGTTTATCTATCTTTATACATATATTTAAACTTTACTCTACGAATAATATAATCTATAGTACTACAATAATATCAGTGT
TTTAGAGAATCATATAAATGAACAGTTAGACATGGTCTAAAGGACAATTGAGTATTTTGACAACAGGACTCTACAGTTTTATC
TTTTTAGTGTGCATGTGTTCTCCTTTTTTTTTGCAAATAGCTTCACCTATATAATACTTCATCCATTTTATTAGTACATCCAT
TTAGGGTTTAGGGTTAATGGTTTTTATAGACTAATTTTTTTAGTACATCTATTTTATTCTATTTTAGCCTCTAAATTAAGAAA
ACTAAAACTCTATTTTAGTTTTTTTATTTAATAATTTAGATATAAAATAGAATAAAATAAAGTGACTAAAAATTAAACAAATA
CCCTTTAAGAAATTAAAAAAACTAAGGAAACATTTTTCTTGTTTCGAGTAGATAATGCCAGCCTGTTAAACGCCGTCGACGAG
TCTAACGGACACCAACCAGCGAACCAGCAGCGTCGCGTCGGGCCAAGCGAAGCAGACGGCACGGCATCTCTGTCGCTGCCTCT
GGACCCCTCTCGAGAGTTCCGCTCCACCGTTGGACTTGCTCCGCTGTCGGCATCCAGAAATTGCGTGGCGGAGCGGCAGACGT
GAGCCGGCACGGCAGGCGGCCTCCTCCTCCTCTCACGGCACGGCAGCTACGGGGGATTCCTTTCCCACCGCTCCTTCGCTTTC
CCTTCCTCGCCCGCCGTAATAAATAGACACCCCCTCCACACCCTCTTTCCCCAACCTCGTGTTGTTCGGAGCGCACACACACA
CAACCAGATCTCCCCCAAATCCACCCGTCGGCACCTCCGCTTCAAGGTACGCCGCTCGTCCTCCCCCCCCCCCCCTCTCTACC
TTCTCTAGATCGGCGTTCCGGTCCATGGTTAGGGCCCGGTAGTTCTACTTCTGTTCATGTTTGTGTTAGATCCGTGTTTGTGT
TAGATCCGTGCTGCTAGCGTTCGTACACGGATGCGACCTGTACGTCAGACACGTTCTGATTGCTAACTTGCCAGTGTTTCTCT
TTGGGGAATCCTGGGATGGCTCTAGCCGTTCCGCAGACGGGATCGATTTCATGATTTTTTTTGTTTCGTTGCATAGGGTTTGG
TTTGCCCTTTTCCTTTATTTCAATATATGCCGTGCACTTGTTTGTCGGGTCATCTTTTCATGCTTTTTTTTGTCTTGGTTGTG
ATGATGTGGTCTGGTTGGGCGGTCGTTCTAGATCGGAGTAGAATTCTGTTTCAAACTACCTGGTGGATTTATTAATTTTGGAT
CTGTATGTGTGTGCCATACATATTCATAGTTACGAATTGAAGATGATGGATGGAAATATCGATCTAGGATAGGTATACATGTT
GATGCGGGTTTTACTGATGCATATACAGAGATGCTTTTTGTTCGCTTGGTTGTGATGATGTGGTGTGGTTGGGCGGTCGTTCA
TTCGTTCTAGATCGGAGTAGAATACTGTTTCAAACTACCTGGTGTATTTATTAATTTTGGAACTGTATGTGTGTGTCATACAT
CTTCATAGTTACGAGTTTAAGATGGATGGAAATATCGATCTAGGATAGGTATACATGTTGATGTGGGTTTTACTGATGCATAT
ACATGATGGCATATGCAGCATCTATTCATATGCTCTAACCTTGAGTACCTATCTATTATAATAAACAAGTATGTTTTATAATT
ATTTTGATCTTGATATACTTGGATGATGGCATATGCAGCAGCTATATGTGGATTTTTTTAGCCCTGCCTTCATACGCTATTTA
TTTGCTTGGTACTGTTTCTTTTGTCGATGCTCACCCTGTTGTTTGGTGTTACTTCTGCAGGTCGACTCTAGAATGTGGATTGA

ACAAGATGGATTGCACGCAGGTTCTCCGGCCGCTTGGGTGGAGAGGCTATTCGGCTATGACTGGGCACAACAGACAATCGGCT
GCTCTGATGCCGCCGTGTTCCGGCTGTCAGCGCAGGGGCGCCCGGTTCTTTTTGTCAAGACCGACCTGTCCGGTGCCCTGAAT
GAACTGCAGGACGAGGCAGCGCGGCTATCGTGGCTGGCCACGACGGGCGTTCCTTGCGCAGCTGTGCTCGACGTTGTCACTGA
AGCGGGAAGGGACTGGCTGCTATTGGGCGAAGTGCCGGGGCAGGATCTCCTGTCATCTCACCTTGCTCCTGCCGAGAAAGTAT
CCATCATGGCTGATGCAATGCGGCGGCTGCATACGCTTGATCCGGCTACCTGCCCATTCGACCACCAAGCGAAACATCGCATC
GAGCGAGCACGTACTCGGATGGAAGCCGGTCTTGTCGATCAGGATGATCTGGACGAAGAGCATCAGGGGCTCGCGCCAGCCGA
ACTGTTCGCCAGGCTCAAGGCGCGCATGCCCGACGGCGAGGATCTCGTCGTGACCCATGGCGATGCCTGCTTGCCGAATATCA
TGGTGGAAAATGGCCGCTTTTCTGGATTCATCGACTGTGGCCGGCTGGGTGTGGCGGACCGCTATCAGGACATAGCGTTGGCT
ACCCGTGATATTGCTGAAGAGCTTGGCGGCGAATGGGCTGACCGCTTCCTCGTGCTTTACGGTATCGCCGCTCCCGATTCGCA
GCGCATCGCCTTCTATCGCCTTCTTGACGAGTTCTTCTGAGATCGTTCAAACATTTGGCAATAAAGTTTCTTAAGATTGAATC
CTGTTGCCGGTCTTGCGATGATTATCATATAATTTCTGTTGAATTACGTTAAGCATGTAATAATTAACATGTAATGCATGACG
TTATTTATGAGATGGGTTTTTATGATTAGAGTCCCGCAATTATACATTTAATACGCGATAGAAAACAAAATATAGCGCGCAAA
CTAGGATAAATTATCGCGCGCGGTGTCATCTATGTTACTAGATCGGCGCGCCAAGGGCGAATTCCAGCACACTGGCGGCCGTT
ACTAGTGGATCGAGCTCGTCGACTCTAGACTCGAGGGCGCGCCGATACCAATTTGGACAGAAAACTGACAAGAACTTCGAAAA
TACATAATTGGAGTTCTAGAGACACAACAAAAGTGATCCTTAACTTTATGTAAAGCTTATTAAGTTGTCTATAACTTTCTTAT
TATCATATTAAGATGATTCTACGGCTAACAAGGACAAACATAGCAAAGAAAACATCTCTGCCGGATTTGGACAGATTCTGTCA
TTCTACTTTGCAAGCTCATAACTAGAGATTTAGACCATCACAATTAGTGCTATAGGACATTTTGGAAAGCTTAGAAAAAGTAC
TACACTTCTTCTATTATCTCTAAGACACGATTCCATATGCACACAGGTCAAACAAACCAAACATTCAGATCTATTCAGAACAT
TGACAAAACCTGCCCACAAAGGTGATCCTAGACTTGGTACCACTAGTATTAATTAAGTTTAAACGGCGCGCCAAGGGCGAATT
CCAGCACACTGGCGGCCGTTACTAGTGGATCGAGCTCGTCGACTCTAGACTCGAGGGCGCGCCTGACAGGATATATTGGCGGG
TAAAC

**>SEQ ID 27 Cas9/crRNA construction**

CTTAGAATAACGGATATTTAAAAGGGCGTGAAAAGGTTTATCCGTTCGTCCATTTGTATGTGCATGCCAACCACAGGGTTCCC
CTCGGGATCAAAGTACTTTGATCCAACCCCTCCGCTGCTATAGTGCAGTCGGCTTCTGACGTTCAGTGCAGCCGTCTTCTGAA
AACGACATGTCGCACAAGTCCTAAGTTACGCGACAGGCTGCCGCCCTGCCCTTTTCCTGGCGTTTTCTTGTCGCGTGTTTTAG
TCGCATAAAGTAGAATACTTGCGACTAGAACCGGAGACATTACGCCATGAACAAGAGCGCCGCCGCTGGCCTGCTGGGCTATG
CCCGCGTCAGCACCGACGACCAGGACTTGACCAACCAACGGGCCGAACTGCACGCGGCCGGCTGCACCAAGCTGTTTTCCGAG
AAGATCACCGGCACCAGGCGCGACCGCCCGGAGCTGGCCAGGATGCTTGACCACCTACGCCCTGGCGACGTTGTGACAGTGAC
CAGGCTAGACCGCCTGGCCCGCAGCACCCGCGACCTACTGGACATTGCCGAGCGCATCCAGGAGGCCGGCGCGGGCCTGCGTA
GCCTGGCAGAGCCGTGGGCCGACACCACCACGCCGGCCGGCCGCATGGTGTTGACCGTGTTCGCCGGCATTGCCGAGTTCGAG
CGTTCCCTAATCATCGACCGCACCCGGAGCGGGCGCGAGGCCGCCAAGGCCCGAGGCGTGAAGTTTGGCCCCCGCCCTACCCT
CACCCCGGCACAGATCGCGCACGCCCGCGAGCTGATCGACCAGGAAGGCCGCACCGTGAAAGAGGCGGCTGCACTGCTTGGCG
TGCATCGCTCGACCCTGTACCGCGCACTTGAGCGCAGCGAGGAAGTGACGCCCACCGAGGCCAGGCGGCGCGGTGCCTTCCGT
GAGGACGCATTGACCGAGGCCGACGCCCTGGCGGCCGCCGAGAATGAACGCCAAGAGGAACAAGCATGAAACCGCACCAGGAC
GGCCAGGACGAACCGTTTTTCATTACCGAAGAGATCGAGGCGGAGATGATCGCGGCCGGGTACGTGTTCGAGCCGCCCGCGCA
CGTCTCAACCGTGCGGCTGCATGAAATCCTGGCCGGTTTGTCTGATGCCAAGCTGGCGGCCTGGCCGGCCAGCTTGGCCGCTG
AAGAAACCGAGCGCCGCCGTCTAAAAAGGTGATGTGTATTTGAGTAAAACAGCTTGCGTCATGCGGTCGCTGCGTATATGATG
CGATGAGTAAATAAACAAATACGCAAGGGGAACGCATGAAGGTTATCGCTGTACTTAACCAGAAAGGCGGGTCAGGCAAGACG
ACCATCGCAACCCATCTAGCCCGCGCCCTGCAACTCGCCGGGGCCGATGTTCTGTTAGTCGATTCCGATCCCCAGGGCAGTGC
CCGCGATTGGGCGGCCGTGCGGGAAGATCAACCGCTAACCGTTGTCGGCATCGACCGCCCGACGATTGACCGCGACGTGAAGG
CCATCGGCCGGCGCGACTTCGTAGTGATCGACGGAGCGCCCCAGGCGGCGGACTTGGCTGTGTCCGCGATCAAGGCAGCCGAC
TTCGTGCTGATTCCGGTGCAGCCAAGCCCTTACGACATATGGGCCACCGCCGACCTGGTGGAGCTGGTTAAGCAGCGCATTGA
GGTCACGGATGGAAGGCTACAAGCGGCCTTTGTCGTGTCGCGGGCGATCAAAGGCACGCGCATCGGCGGTGAGGTTGCCGAGG
CGCTGGCCGGGTACGAGCTGCCCATTCTTGAGTCCCGTATCACGCAGCGCGTGAGCTACCCAGGCACTGCCGCCGCCGGCACA
ACCGTTCTTGAATCAGAACCCGAGGGCGACGCTGCCCGCGAGGTCCAGGCGCTGGCCGCTGAAATTAAATCAAAACTCATTTG
AGTTAATGAGGTAAAGAGAAAATGAGCAAAAGCACAAACACGCTAAGTGCCGGCCGTCCGAGCGCACGCAGCAGCAAGGCTGC
AACGTTGGCCAGCCTGGCAGACACGCCAGCCATGAAGCGGGTCAACTTTCAGTTGCCGGCGGAGGATCACACCAAGCTGAAGA
TGTACGCGGTACGCCAAGGCAAGACCATTACCGAGCTGCTATCTGAATACATCGCGCAGCTACCAGAGTAAATGAGCAAATGA
ATAAATGAGTAGATGAATTTTAGCGGCTAAAGGAGGCGGCATGGAAAATCAAGAACAACCAGGCACCGACGCCGTGGAATGCC
CCATGTGTGGAGGAACGGGCGGTTGGCCAGGCGTAAGCGGCTGGGTTGTCTGCCGGCCCTGCAATGGCACTGGAACCCCCAAG
CCCGAGGAATCGGCGTGACGGTCGCAAACCATCCGGCCCGGTACAAATCGGCGCGGCGCTGGGTGATGACCTGGTGGAGAAGT
TGAAGGCCGCGCAGGCCGCCCAGCGGCAACGCATCGAGGCAGAAGCACGCCCCGGTGAATCGTGGCAAGCGGCCGCTGATCGA
ATCCGCAAAGAATCCCGGCAACCGCCGGCAGCCGGTGCGCCGTCGATTAGGAAGCCGCCCAAGGGCGACGAGCAACCAGATTT
TTTCGTTCCGATGCTCTATGACGTGGGCACCCGCGATAGTCGCAGCATCATGGACGTGGCCGTTTTCCGTCTGTCGAAGCGTG
ACCGACGAGCTGGCGAGGTGATCCGCTACGAGCTTCCAGACGGGCACGTAGAGGTTTCCGCAGGGCCGGCCGGCATGGCCAGT
GTGTGGGATTACGACCTGGTACTGATGGCGGTTTCCCATCTAACCGAATCCATGAACCGATACCGGGAAGGGAAGGGAGACAA
GCCCGGCCGCGTGTTCCGTCCACACGTTGCGGACGTACTCAAGTTCTGCCGGCGAGCCGATGGCGGAAAGCAGAAAGACGACC

TGGTAGAAACCTGCATTCGGTTAAACACCACGCACGTTGCCATGCAGCGTACGAAGAAGGCCAAGAACGGCCGCCTGGTGACG
GTATCCGAGGGTGAAGCCTTGATTAGCCGCTACAAGATCGTAAAGAGCGAAACCGGGCGGCCGGAGTACATCGAGATCGAGCT
AGCTGATTGGATGTACCGCGAGATCACAGAAGGCAAGAACCCGGACGTGCTGACGGTTCACCCCGATTACTTTTTGATCGATC
CCGGCATCGGCCGTTTTCTCTACCGCCTGGCACGCCGCGCCGCAGGCAAGGCAGAAGCCAGATGGTTGTTCAAGACGATCTAC
GAACGCAGTGGCAGCGCCGGAGAGTTCAAGAAGTTCTGTTTCACCGTGCGCAAGCTGATCGGGTCAAATGACCTGCCGGAGTA
CGATTTGAAGGAGGAGGCGGGGCAGGCTGGCCCGATCCTAGTCATGCGCTACCGCAACCTGATCGAGGGCGAAGCATCCGCCG
GTTCCTAATGTACGGAGCAGATGCTAGGGCAAATTGCCCTAGCAGGGGAAAAAGGTCGAAAAGGTCTCTTTCCTGTGGATAGC
ACGTACATTGGGAACCCAAAGCCGTACATTGGGAACCGGAACCCGTACATTGGGAACCCAAAGCCGTACATTGGGAACCGGTC
ACACATGTAAGTGACTGATATAAAAGAGAAAAAAGGCGATTTTTCCGCCTAAAACTCTTTAAAACTTATTAAAACTCTTAAAA
CCCGCCTGGCCTGTGCATAACTGTCTGGCCAGCGCACAGCCGAAGAGCTGCAAAAAGCGCCTACCCTTCGGTCGCTGCGCTCC
CTACGCCCCGCCGCTTCGCGTCGGCCTATCGCGGCCGCTGGCCGCTCAAAAATGGCTGGCCTACGGCCAGGCAATCTACCAGG
GCGCGGACAAGCCGCGCCGTCGCCACTCGACCGCCGGCGCCCACATCAAGGCACCCTGCCTCGCGCGTTTCGGTGATGACGGT
GAAAACCTCTGACACATGCAGCTCCCGGAGACGGTCACAGCTTGTCTGTAAGCGGATGCCGGGAGCAGACAAGCCCGTCAGGG
CGCGTCAGCGGGTGTTGGCGGGTGTCGGGGCGCAGCCATGACCCAGTCACGTAGCGATAGCGGAGTGTATACTGGCTTAACTA
TGCGGCATCAGAGCAGATTGTACTGAGAGTGCACCATATGCGGTGTGAAATACCGCACAGATGCGTAAGGAGAAAATACCGCA
TCAGGCGCTCTTCCGCTTCCTCGCTCACTGACTCGCTGCGCTCGGTCGTTCGGCTGCGGCGAGCGGTATCAGCTCACTCAAAG
GCGGTAATACGGTTATCCACAGAATCAGGGGATAACGCAGGAAAGAACATGTGAGCAAAAGGCCAGCAAAAGGCCAGGAACCG
TAAAAAGGCCGCGTTGCTGGCGTTTTTCCATAGGCTCCGCCCCCCTGACGAGCATCACAAAAATCGACGCTCAAGTCAGAGGT
GGCGAAACCCGACAGGACTATAAAGATACCAGGCGTTTCCCCCTGGAAGCTCCCTCGTGCGCTCTCCTGTTCCGACCCTGCCG
CTTACCGGATACCTGTCCGCCTTTCTCCCTTCGGGAAGCGTGGCGCTTTCTCATAGCTCACGCTGTAGGTATCTCAGTTCGGT
GTAGGTCGTTCGCTCCAAGCTGGGCTGTGTGCACGAACCCCCCGTTCAGCCCGACCGCTGCGCCTTATCCGGTAACTATCGTC
TTGAGTCCAACCCGGTAAGACACGACTTATCGCCACTGGCAGCAGCCACTGGTAACAGGATTAGCAGAGCGAGGTATGTAGGC
GGTGCTACAGAGTTCTTGAAGTGGTGGCCTAACTACGGCTACACTAGAAGGACAGTATTTGGTATCTGCGCTCTGCTGAAGCC
AGTTACCTTCGGAAAAAGAGTTGGTAGCTCTTGATCCGGCAAACAAACCACCGCTGGTAGCGGTGGTTTTTTTGTTTGCAAGC
AGCAGATTACGCGCAGAAAAAAAGGATCTCAAGAAGATCCTTTGATCTTTTCTACGGGGTCTGACGCTCAGTGGAACGAAAAC
TCACGTTAAGGGATTTTGGTCATGCATTCTAGGTATTATTTGCCAACGACCTTCGTGATCTCGCCCTTGACATAGTGGACAAA
TTCTTCGAGCTGGTCGGCCCGGGACGCGAGACGGTCTTCTTCTTGGCCCAGATAGGCTTGGCGCGCTTCGAGGATCACGGGCT
GGTATTGCGCCGGAAGGCGCTCCATCGCCCAGTCGGCGGCGACATCCTTCGGCGCGATCTTGCCGGTAACCGCCGAGTACCAA
ATCCGGCTCAGCGTAAGGACCACATTGCGCTCATCGCCCGCCCAATCCGGCGGGGAGTTCCACAGGGTCAGCGTCTCGTTCAG
TGCTTCGAACAGATCCTGTTCCGGCACCGGGTCGAAAAGTTCCTCGGCCGCGGGGCCGACGAGGGCCACGCTATGCTCCCGGG
CCTTGGTGAGCAGGATCGCCAGATCAATGTCGATGGTGGCCGGTTCAAAGATACCCGCCAGAATATCATTACGCTGCCATTCG
CCGAACTGGAGTTCGCGTTTGGCCGGATAGCGCCAGGGGATGATGTCATCGTGCACCACAATCGTCACCTCAACCGCGCGCAG
GATTTCGCTCTCGCCGGGGGAGGCGGACGTTTCCAGAAGGTCGTTGATAAGCGCGCGGCGCGTGGTCTCGTCGAGACGGACGG
TAACGGTGACAAGCAGGTCGATGTCCGAATGGGGCTTAAGGCCGCCGTCAACGGCGCTACCATACAGATGCACGGCGAGGAGG
GTCGGTTCGAGGTGGCGCTCGATGACACCCACGACTTCCGACAGCTGGGTGGACACCTCGGCGATGACCGCTTCACCCATTTA
TTATTTCCTTCCTCTTTTCTACAGTATTTAAAGATACCCCAAGAAGCTAATTATAACAAGACGAACTCCAATTCACTGTTCCT
TGCATTCTAAAACCTTAAATACCAGAAAACAGCTTTTTCAAAGTTGTTTTCAAAGTTGGCGTATAACATAGTATCGACGGAGC
CGATTTTGAAACCGCGGTGATCACAGGCAGCAACGCTCTGTCATCGTTACAATCAACATGCTACCCTCCGCGAGATCATCCGT
GTTTCAAACCCGGCAGCTTAGTTGCCGTTCTTCCGAATAGCATCGGTAACATGAGCAAAGTCTGCCGCCTTACAACGGCTCTC
CCGCTGACGCCGTCCCGGACTGATGGGCTGCCTGTATCGAGTGGTGATTTTGTGCCGAGCTGCCGGTCGGGGAGCTGTTGGCT
GGCTGGTGGCAGGATATATTGTGGTGTAAACAAATTGACGCTTAGACAACTTAATAACACATTGCGGACGTTTTTAATGTACT
GAATTAGTACTGATAAATGGCGCGCCAAGCTTTGGCAAACAGCTATTATGGGTATTATGGGTGGTACCACGCGTCGATCCACT
AGTAACGGCCGCCAGTGTGCTGGAATTCGCCCTTGGCGCGCCGATCTAGTAACATAGATGACACCGCGCGCGATAATTTATCC
TAGTTTGCGCGCTATATTTTGTTTTCTATCGCGTATTAAATGTATAATTGCGGGACTCTAATCATAAAAACCCATCTCATAAA
TAACGTCATGCATTACATGTTAATTATTACATGCTTAACGTAATTCAACAGAAATTATATGATAATCATCGCAAGACCGGCAA
CAGGATTCAATCTTAAGAAACTTTATTGCCAAATGTTTGAACGATCTCAGAAGAACTCGTCAAGAAGGCGATAGAAGGCGATG
CGCTGCGAATCGGGAGCGGCGATACCGTAAAGCACGAGGAAGCGGTCAGCCCATTCGCCGCCAAGCTCTTCAGCAATATCACG
GGTAGCCAACGCTATGTCCTGATAGCGGTCCGCCACACCCAGCCGGCCACAGTCGATGAATCCAGAAAAGCGGCCATTTTCCA
CCATGATATTCGGCAAGCAGGCATCGCCATGGGTCACGACGAGATCCTCGCCGTCGGGCATGCGCGCCTTGAGCCTGGCGAAC
AGTTCGGCTGGCGCGAGCCCCTGATGCTCTTCGTCCAGATCATCCTGATCGACAAGACCGGCTTCCATCCGAGTACGTGCTCG
CTCGATGCGATGTTTCGCTTGGTGGTCGAATGGGCAGGTAGCCGGATCAAGCGTATGCAGCCGCCGCATTGCATCAGCCATGA
TGGATACTTTCTCGGCAGGAGCAAGGTGAGATGACAGGAGATCCTGCCCCGGCACTTCGCCCAATAGCAGCCAGTCCCTTCCC
GCTTCAGTGACAACGTCGAGCACAGCTGCGCAAGGAACGCCCGTCGTGGCCAGCCACGATAGCCGCGCTGCCTCGTCCTGCAG
TTCATTCAGGGCACCGGACAGGTCGGTCTTGACAAAAAGAACCGGGCGCCCCTGCGCTGACAGCCGGAACACGGCGGCATCAG
AGCAGCCGATTGTCTGTTGTGCCCAGTCATAGCCGAATAGCCTCTCCACCCAAGCGGCCGGAGAACCTGCGTGCAATCCATCT
TGTTCAATCCACATGGTGGTGTGACCTGCAGAAGTAACACCAAACAACAGGGTGAGCATCGACAAAAGAAACAGTACCAAGCA
AATAAATAGCGTATGAAGGCAGGGCTAAAAAAATCCACATATAGCTGCTGCATATGCCATCATCCAAGTATATCAAGATCAAA
ATAATTATAAAACATACTTGTTTATTATAATAGATAGGTACTCAAGGTTAGAGCATATGAATAGATGCTGCATATGCCATCAT

GTATATGCATCAGTAAAACCCACATCAACATGTATACCTATCCTAGATCGATATTTCCATCCATCTTAAACTCGTAACTATGA
AGATGTATGACACACACATACAGTTCCAAAATTAATAAATACACCAGGTAGTTTGAAACAGTATTCTACTCCGATCTAGAACG
AATGAACGACCGCCCAACCACACCACATCATCACAACCAAGCGAACAAAAAGCATCTCTGTATATGCATCAGTAAAACCCGCA
TCAACATGTATACCTATCCTAGATCGATATTTCCATCCATCATCTTCAATTCGTAACTATGAATATGTATGGCACACACATAC
AGATCCAAAATTAATAAATCCACCAGGTAGTTTGAAACAGAATTCTACTCCGATCTAGAACGACCGCCCAACCAGACCACATC
ATCACAACCAAGACAAAAAAAAGCATGAAAAGATGACCCGACAAACAAGTGCACGGCATATATTGAAATAAAGGAAAAGGGCA
AACCAAACCCTATGCAACGAAACAAAAAAAATCATGAAATCGATCCCGTCTGCGGAACGGCTAGAGCCATCCCAGGATTCCCC
AAAGAGAAACACTGGCAAGTTAGCAATCAGAACGTGTCTGACGTACAGGTCGCATCCGTGTACGAACGCTAGCAGCACGGATC
TAACACAAACACGGATCTAACACAAACATGAACAGAAGTAGAACTACCGGGCCCTAACCATGGACCGGAACGCCGATCTAGAG
AAGGTAGAGAGGGGGGGGGGGGGAGGACGAGCGGCGTACCTTGAAGCGGAGGTGCCGACGGGTGGATTTGGGGGAGATCTGGT
TGTGTGTGTGTGCGCTCCGAACAACACGAGGTTGGGGAAAGAGGGTGTGGAGGGGGTGTCTATTTATTACGGCGGGCGAGGAA
GGGAAAGCGAAGGAGCGGTGGGAAAGGAATCCCCCGTAGCTGCCGTGCCGTGAGAGGAGGAGGAGGCCGCCTGCCGTGCCGGC
TCACGTCTGCCGCTCCGCCACGCAATTTCTGGATGCCGACAGCGGAGCAAGTCCAACGGTGGAGCGGAACTCTCGAGAGGGGT
CCAGAGGCAGCGACAGAGATGCCGTGCCGTCTGCTTCGCTTGGCCCGACGCGACGCTGCTGGTTCGCTGGTTGGTGTCCGTTA
GACTCGTCGACGGCGTTTAACAGGCTGGCATTATCTACTCGAAACAAGAAAAATGTTTCCTTAGTTTTTTTAATTTCTTAAAG
GGTATTTGTTTAATTTTTAGTCACTTTATTTTATTCTATTTTATATCTAAATTATTAAATAAAAAAACTAAAATAGAGTTTTA
GTTTTCTTAATTTAGAGGCTAAAATAGAATAAAATAGATGTACTAAAAAAATTAGTCTATAAAAACCATTAACCCTAAACCCT
AAATGGATGTACTAATAAAATGGATGAAGTATTATATAGGTGAAGCTATTTGCAAAAAAAAAGGAGAACACATGCACACTAAA
AAGATAAAACTGTAGAGTCCTGTTGTCAAAATACTCAATTGTCCTTTAGACCATGTCTAACTGTTCATTTATATGATTCTCTA
AAACACTGATATTATTGTAGTACTATAGATTATATTATTCGTAGAGTAAAGTTTAAATATATGTATAAAGATAGATAAACTGC
ACTTCAAACAAGTGTGACAAAAAAAATATGTGGTAATTTTTTATAACTTAGACATGCAATGCTCATTATCTCTAGAGAGGGGC
ACGACCGGGTCACGCTGCACTGCAGGGATCCGATCTAGTAACATAGATGACACCGCGCGCGATAATTTATCCTAGTTTGCGCG
CTATATTTTGTTTTCTATCGCGTATTAAATGTATAATTGCGGGACTCTAATCATAAAAACCCATCTCATAAATAACGTCATGC
ATTACATGTTAATTATTACATGCTTAACGTAATTCAACAGAAATTATATGATAATCATCGCAAGACCGGCAACAGGATTCAAT
CTTAAGAAACTTTATTGCCAAATGTTTGAACGATCCCTAGGACGATCTCACTTGTACAGCTCGTCCATGCCGTGGGTGATGCC
AGCTGCGGTGACGAACTCCAGCAGGACCATGTGGTCGCGCTTCTCGTTGGGGTCCTTGCTCAGAGCGGACTGGGTGCTCAGGT
AGTGGTTGTCGGGCAGCAGCACGGGACCGTCGCCGATGGGCGTGTTCTGCTGGTAGTGGTCGGCGAGCTGGACGCTGCCGTCC
TCGATGTTGTGGCGGATCTTGAAGTTGACCTTGATGCCGTTCTTCTGCTTGTCAGCCATGATGTAGACGTTGTGGCTGTTGTA
GTTGTACTCCAGCTTGTGCCCCAGGATGTTGCCGTCCTCCTTGAAGTCGATGCCCTTCAGCTCGATGCGGTTCACCAGGGTGT
CGCCCTCGAACTTCACCTCGGCTCGGGTCTTGTAGTTGCCGTCGTCCTTGAAGAAGATGGTGCGCTCCTGGACGTAGCCTTCG
GGCATGGCGGACTTGAAGAAGTCGTGCTGCTTCATGTGGTCGGGGTAGCGGCTGAAGCACTGCACGCCGTAGGTGAAGGTGGT
CACGAGGGTGGGCCAGGGCACGGGCAGCTTGCCGGTGGTGCAGATGAACTTCAGGGTCAGCTTGCCGTAGGTGGCGTCGCCCT
CGCCCTCGCCGCTGACGCTGAACTTGTGGCCGTTCACGTCGCCGTCCAGCTCGACCAGGATGGGCACCACCCCAGTGAACAGC
TCCTCGCCCTTGCTCACTACAAAAAAGCTCCGCACGAGGCTGCATTTGTCACAAATCATGAAAAGAAAAACTACCGATGAACA
ATGCTGAGGGATTCAAATTCTACCCACAAAAAGAAGAAAGAAAGATCTAGCACATCTAAGCCTGACGAAGCAGCAGAAATATA
TAAAAATATAAACCATAGTGCCCTTTTCCCCTCTTCCTGATCTTGTTTAGCACGGCGGAAATTTTAAACCCCCCATCATCTCC
CCCAACAACGGCGGATCGCAGATCTACATCCGAGAGCCCCATTCCCCGCGAGATCCGGGCCGGATCCACGCCGGCGAGAGCCC
CAGCCGCGAGATCCCGCCCCTCCCGCGCACCGATCTGGGCGCGCACGAAGCCGCCTCTCGCCCACCCAAACTACCAAGGCCAA
AGATCGAGACCGAGACGGAAAAAAAAAACGGAGAAAGAAAGAGGAGAGGGGCGGGGTGGTTACCGGCGGCGGCGGAGGCCTCCC
TTGGATCTTATGGTGTGTTGTCCCTGTGTGTTCTCCAATAGTGTGGCTTGAGTGTGTGGAAGATGGTTCTAGAGGATCTGCTA
GAGTCAGCTTGTCAGCGTGTCCTCTCCAAATGAAATGAACTTCCTTATATAGAGGAAGGGTCTTGCGAAGGATAGTGGGATTG
TGCGTCATCCCTTACGTCAGTGGAGATATCACATCAATCCACTTGCTTTGAAGACGTGGTTGGAACGTCTTCTTTTTCCACGA
TGCTCCTCGTGGGTGGGGGTCCATCTTTGGGACCACTGTCGGCAGAGGCATCTTCAACGATGGCCTTTCCTTTATCGCAATGA
TGGCATTTGTAGGAGCCACCTTCCTTTTCCACTATCTTCACAATAAAGTGACAGATAGCTGGGCAATGGAATCCGAGGAGGTT
TCCGGATATTACCCTTTGTTGAAAAGTCTCAATCGGACCATCACATCAATCCACTTGCTTTGAAGACGTGGTTGGAACGTCTT
CTTTTTCCACGATGCTCCTCGTGGGTGGGGGTCCATCTTTGGGACCACTGTCGGCAGAGGCATCTTCAACGATGGCCTTTCCT
TTATCGCAATGATGGCATTTGTAGGAGCCACCTTCCTTTTCCACTATCTTCACAATAAAGTGACAGATAGCTGGGCAATGGAA
TCCGAGGAGGTTTCCGGATATTACCCTTTGTTGAAAAGTCTCAATCGGACCCCCTCAGCCTGCAGTGCAGCGTGACCCGGTCG
TGCCCCTCTCTAGAGATAATGAGCATTGCATGTCTAAGTTATAAAAAATTACCACATATTTTTTTTGTCACACTTGTTTGAAG
TGCAGTTTATCTATCTTTATACATATATTTAAACTTTACTCTACGAATAATATAATCTATAGTACTACAATAATATCAGTGTT
TTAGAGAATCATATAAATGAACAGTTAGACATGGTCTAAAGGACAATTGAGTATTTTGACAACAGGACTCTACAGTTTTATCT
TTTTAGTGTGCATGTGTTCTCCTTTTTTTTTGCAAATAGCTTCACCTATATAATACTTCATCCATTTTATTAGTACATCCATT
TAGGGTTTAGGGTTAATGGTTTTTATAGACTAATTTTTTTAGTACATCTATTTTATTCTATTTTAGCCTCTAAATTAAGAAAA
CTAAAACTCTATTTTAGTTTTTTTATTTAATAATTTAGATATAAAATAGAATAAAATAAAGTGACTAAAAATTAAACAAATAC
CCTTTAAGAAATTAAAAAAACTAAGGAAACATTTTTCTTGTTTCGAGTAGATAATGCCAGCCTGTTAAACGCCGTCGACGAGT
CTAACGGACACCAACCAGCGAACCAGCAGCGTCGCGTCGGGCCAAGCGAAGCAGACGGCACGGCATCTCTGTCGCTGCCTCTG
GACCCCTCTCGAGAGTTCCGCTCCACCGTTGGACTTGCTCCGCTGTCGGCATCCAGAAATTGCGTGGCGGAGCGGCAGACGTG
AGCCGGCACGGCAGGCGGCCTCCTCCTCCTCTCACGGCACGGCAGCTACGGGGGATTCCTTTCCCACCGCTCCTTCGCTTTCC

CTTCCTCGCCCGCCGTAATAAATAGACACCCCCTCCACACCCTCTTTCCCCAACCTCGTGTTGTTCGGAGCGCACACACACAC
AACCAGATCTCCCCCAAATCCACCCGTCGGCACCTCCGCTTCAAGGTACGCCGCTCGTCCTCCCCCCCCCCCCCTCTCTACCT
TCTCTAGATCGGCGTTCCGGTCCATGGTTAGGGCCCGGTAGTTCTACTTCTGTTCATGTTTGTGTTAGATCCGTGTTTGTGTT
AGATCCGTGCTGCTAGCGTTCGTACACGGATGCGACCTGTACGTCAGACACGTTCTGATTGCTAACTTGCCAGTGTTTCTCTT
TGGGGAATCCTGGGATGGCTCTAGCCGTTCCGCAGACGGGATCGATTTCATGATTTTTTTTGTTTCGTTGCATAGGGTTTGGT
TTGCCCTTTTCCTTTATTTCAATATATGCCGTGCACTTGTTTGTCGGGTCATCTTTTCATGCTTTTTTTTGTCTTGGTTGTGA
TGATGTGGTCTGGTTGGGCGGTCGTTCTAGATCGGAGTAGAATTCTGTTTCAAACTACCTGGTGGATTTATTAATTTTGGATC
TGTATGTGTGTGCCATACATATTCATAGTTACGAATTGAAGATGATGGATGGAAATATCGATCTAGGATAGGTATACATGTTG
ATGCGGGTTTTACTGATGCATATACAGAGATGCTTTTTGTTCGCTTGGTTGTGATGATGTGGTGTGGTTGGGCGGTCGTTCAT
TCGTTCTAGATCGGAGTAGAATACTGTTTCAAACTACCTGGTGTATTTATTAATTTTGGAACTGTATGTGTGTGTCATACATC
TTCATAGTTACGAGTTTAAGATGGATGGAAATATCGATCTAGGATAGGTATACATGTTGATGTGGGTTTTACTGATGCATATA
CATGATGGCATATGCAGCATCTATTCATATGCTCTAACCTTGAGTACCTATCTATTATAATAAACAAGTATGTTTTATAATTA
TTTTGATCTTGATATACTTGGATGATGGCATATGCAGCAGCTATATGTGGATTTTTTTAGCCCTGCCTTCATACGCTATTTAT
TTGCTTGGTACTGTTTCTTTTGTCGATGCTCACCCTGTTGTTTGGTGTTACTTCTGCAGGCGATCGCCACACCACCATGCCGA
AGAAGAAGCGCAAGGTCATGGACAAGAAGTACTCCATCGGCCTGGACATCGGCACCAACAGCGTGGGCTGGGCCGTCATCACC
GACGAGTACAAGGTGCCCTCCAAGAAGTTCAAGGTCCTCGGCAACACCGACAGGCACAGCATCAAGAAGAACCTGATCGGCGC
CCTGCTGTTCGACTCCGGCGAGACTGCGGAGGCTACCAGGCTGAAGCGCACTGCTCGCAGGCGCTACACCAGGCGCAAGAACC
GCATCTGCTACCTCCAGGAGATTTTCTCCAACGAGATGGCCAAGGTGGACGACTCCTTCTTCCACCGCCTGGAGGAGAGCTTC
CTGGTCGAGGAAGACAAGAAGCACGAGCGCCACCCTATCTTCGGCAACATCGTGGACGAGGTCGCCTACCACGAGAAGTACCC
AACCATCTACCACCTCCGCAAGAAGCTGGTGGACTCCACCGACAAGGCCGACCTGAGGCTCATCTACCTGGCCCTCGCCCACA
TGATCAAGTTCCGCGGCCACTTCCTCATCGAGGGCGACCTGAACCCGGACAACAGCGACGTGGACAAGCTCTTCATCCAGCTG
GTCCAGACCTACAACCAGCTGTTCGAGGAGAACCCCATCAACGCCTCCGGCGTGGACGCTAAGGCTATCCTCAGCGCTAGGCT
GTCCAAGAGCAGGCGCCTGGAGAACCTCATCGCCCAGCTCCCGGGCGAGAAGAAGAACGGCCTCTTCGGCAACCTGATCGCTC
TGTCCCTCGGCCTGACCCCCAACTTCAAGAGCAACTTCGACCTGGCCGAGGACGCCAAGCTCCAGCTGTCCAAGGACACCTAC
GACGACGACCTCGACAACCTGCTCGCCCAGATCGGCGACCAGTACGCCGACCTCTTCCTGGCCGCCAAGAACCTCTCCGACGC
CATCCTGCTCAGCGACATCCTGAGGGTGAACACCGAGATCACCAAGGCCCCGCTGTCCGCCAGCATGATCAAGCGCTACGACG
AGCACCACCAGGACCTCACTCTCCTGAAGGCCCTCGTCCGCCAGCAGCTGCCCGAGAAGTACAAGGAGATTTTCTTCGACCAG
AGCAAGAACGGCTACGCGGGCTACATCGATGGCGGCGCCTCCCAGGAAGAGTTCTACAAGTTCATCAAGCCTATCCTGGAGAA
GATGGACGGCACCGAGGAGCTGCTCGTGAAGCTGAACCGCGAGGACCTGCTCCGCAAGCAGAGGACCTTCGACAACGGCAGCA
TCCCTCACCAGATCCACCTGGGCGAGCTGCACGCTATCCTCCGCCGCCAGGAAGACTTCTACCCATTCCTGAAGGACAACCGC
GAGAAGATCGAGAAGATCCTCACCTTCCGCATCCCGTACTACGTGGGCCCCCTGGCCCGCGGCAACTCCAGGTTCGCCTGGAT
GACCAGGAAGAGCGAGGAGACCATCACCCCGTGGAACTTCGAGGAAGTGGTGGACAAGGGCGCCTCCGCTCAGAGCTTCATCG
AGCGCATGACCAACTTCGACAAGAACCTCCCTAACGAGAAGGTGCTGCCAAAGCACTCCCTGCTCTACGAGTACTTCACCGTC
TACAACGAGCTGACCAAGGTGAAGTATGTGACCGAGGGCATGAGGAAGCCCGCCTTCCTCAGCGGCGAGCAGAAGAAGGCCAT
CGTGGACCTGCTCTTCAAGACCAACCGCAAGGTGACCGTCAAGCAGCTGAAGGAAGACTACTTCAAGAAGATCGAGTGCTTCG
ACTCCGTGGAGATCAGCGGCGTGGAGGACCGCTTCAACGCCTCCCTCGGCACCTACCACGACCTGCTCAAGATCATCAAGGAC
AAGGACTTCCTCGACAACGAGGAGAACGAGGACATCCTGGAGGACATCGTGCTCACCCTGACCCTCTTCGAGGACCGCGAGAT
GATCGAGGAGAGGCTCAAGACCTACGCCCACCTGTTCGACGACAAGGTCATGAAGCAGCTGAAGAGGCGCAGGTACACTGGCT
GGGGCCGCCTCAGCAGGAAGCTGATCAACGGCATCAGGGACAAGCAGTCCGGCAAGACCATCCTGGACTTCCTCAAGAGCGAC
GGCTTCGCCAACCGCAACTTCATGCAGCTCATCCACGACGACTCCCTGACCTTCAAGGAAGACATCCAGAAGGCTCAGGTGTC
CGGCCAGGGCGACAGCCTCCACGAGCACATCGCTAACCTGGCGGGCAGCCCTGCCATCAAGAAGGGCATCCTCCAGACCGTGA
AGGTGGTGGACGAGCTGGTGAAGGTCATGGGCCGCCACAAGCCAGAGAACATCGTCATCGAGATGGCCAGGGAGAACCAGACC
ACCCAGAAGGGTCAGAAGAACTCCCGCGAGAGGATGAAGAGGATCGAGGAAGGCATCAAGGAGCTGGGCAGCCAGATCCTGAA
GGAGCACCCGGTGGAGAACACCCAGCTCCAGAACGAGAAGCTGTACCTCTACTACCTGCAGAACGGCCGCGACATGTATGTGG
ACCAGGAGCTGGACATCAACAGGCTGTCCGACTACGACGTGGACCACATCGTCCCTCAGTCCTTCCTCAAGGACGACAGCATC
GACAACAAGGTGCTGACCCGCAGCGACAAGAACAGGGGCAAGTCCGACAACGTCCCAAGCGAGGAAGTGGTCAAGAAGATGAA
GAACTACTGGCGCCAGCTGCTCAACGCCAAGCTCATCACCCAGCGCAAGTTCGACAACCTGACTAAGGCGGAGAGGGGCGGCC
TGTCCGAGCTGGACAAGGCTGGCTTCATCAAGCGCCAGCTCGTGGAGACCAGGCAGATCACCAAGCACGTCGCCCAGATCCTG
GACAGCAGGATGAACACCAAGTACGACGAGAACGACAAGCTCATCCGCGAGGTGAAGGTCATCACCCTCAAGTCCAAGCTGGT
GAGCGACTTCCGCAAGGACTTCCAGTTCTACAAGGTCAGGGAGATCAACAACTACCACCACGCCCACGATGCTTACCTCAACG
CGGTGGTGGGCACCGCCCTCATCAAGAAGTACCCTAAGCTGGAGAGCGAGTTCGTGTACGGCGACTACAAGGTGTACGACGTC
CGCAAGATGATCGCCAAGTCCGAGCAGGAGATCGGCAAGGCCACCGCCAAGTACTTCTTCTACAGCAACATCATGAACTTCTT
CAAGACCGAGATCACCCTCGCCAACGGCGAGATCCGCAAGAGGCCACTGATCGAGACCAACGGCGAGACTGGCGAGATCGTGT
GGGACAAGGGCAGGGACTTCGCCACCGTGAGGAAGGTCCTGTCCATGCCTCAGGTGAACATCGTCAAGAAGACCGAGGTCCAG
ACCGGCGGCTTCTCCAAGGAGAGCATCCTCCCAAAGCGCAACAGCGACAAGCTGATCGCCAGGAAGAAGGACTGGGACCCGAA
GAAGTACGGTGGCTTCGACTCCCCTACTGTGGCTTACAGCGTCCTGGTGGTCGCCAAGGTGGAGAAGGGCAAGTCCAAGAAGC
TGAAGAGCGTCAAGGAGCTGCTCGGCATCACCATCATGGAGAGGTCCAGCTTCGAGAAGAACCCGATCGACTTCCTGGAGGCC
AAGGGCTACAAGGAAGTGAAGAAGGACCTGATCATCAAGCTGCCCAAGTACAGCCTGTTCGAGCTGGAGAACGGCCGCAAGAG

GATGCTCGCCTCCGCTGGCGAGCTGCAGAAGGGCAACGAGCTGGCCCTCCCGTCCAAGTATGTGAACTTCCTGTACCTCGCCT
CCCACTACGAGAAGCTGAAGGGCAGCCCCGAGGACAACGAGCAGAAGCAGCTCTTCGTCGAGCAGCACAAGCACTACCTGGAC
GAGATCATCGAGCAGATCAGCGAGTTCAGCAAGCGCGTGATCCTCGCCGACGCCAACCTCGACAAGGTCCTGTCCGCCTACAA
CAAGCACCGCGACAAGCCTATCAGGGAGCAGGCCGAGAACATCATCCACCTGTTCACCCTCACCAACCTGGGCGCCCCAGCTG
CCTTCAAGTACTTCGACACCACCATCGACCGCAAGAGGTACACCAGCACCAAGGAAGTGCTGGACGCCACCCTGATCCACCAG
TCCATCACCGGCCTGTACGAGACTCGCATCGACCTCAGCCAGCTGGGCGGCGACCCGAAGAAGAAGCGCAAAGTCTGAGGGAC
CCTCGATCGACAAGCTCGAGTTTCTCCATAATAATGTGTGAGTAGTTCCCAGATAAGGGAATTAGGGTTCCTATAGGGTTTCG
CTCATGTGTTGAGCATATAAGAAACCCTTAGTATGTATTTGTATTTGTAAAATACTTCTATCAATAAAATTTCTAATTCCTAA
AACCAAAATCCAGTACTAAAATCCAGATCCCCCGAATTAACCTGCAGGGGCGGCAGGGAGAGTTTTAACATTGACTAGCGTGC
TGATAATTTGTGAGAAATAATAATTGACAAGTAGATACTGACATTTGAGAAGAGCTTCTGAACTGTTATTAGTAACAAAAATG
GAAAGCTGATGCACGGAAAAAGGAAAGAAAAAGCCATACTTTTTTTTAGGTAGGAAAAGAAAAAGCCATACGAGACTGATGTC
TCTCAGATGGGCCGGGATCTGTCTATCTAGCAGGCAGCAGCCCTACCAACCTCACGGGCCAGCAATTACGAGTCCTTCTAAAA
CGTCCCGCCGAGGGCGCGTGGCCGTGCTGTGCAGCAGCACGTCTAACATTAGTCCCACCTCGCCAGTTTACAGGGAGCAGAAC
CAGCTTATAAGCGGAGGCGCGGCACCAAGAAGCAAAGTCTAGGATCACCTTTGTGTTTTAGAGCTAGAAATAGCAAGTTAAAA
TAAGGCTAGTCCGTTATCAACTTGAAAAAGTGGCACCGAGTCGGTGCTTTTTTTTCCTCGAGGGGCGATAGGAACACGTACAA
CGGCCGTTGTACGTGTTCCTATCGCCGGTACCACTAGTATTAATTAAGTTTAAACGGCGCGCCAAGGGCGAATTCCAGCACAC
TGGCGGCCGTTACTAGTGGATCGAGCTCGTCGACTCTAGACTCGAGGGCGCGCCTGACAGGATATATTGGCGGGTAAAC

**>SEQ ID 28- Cas9 - crRNA**
AAGTCTAGGATCACCTTTGT

**>SEQ ID 29- Complementary sequence of SEQ ID NO 22 (reverse-complement)**

GCATCTTCATGATTGTTGCCTTATGCATAGCTATGCTCCTCTCACCCTTGTTCAAATGATGAGTAATTTGTGAGTGATGTGGT
ATCCTATCTTCCTTGTGTGGAGTCAAACAAGAATGCTTTGTTCCATGTTGCTTGAATAGGAAATGCTTGAGAAAAATGTGTGC
TTAGCTTTACTAGAAATAAGTGTGACTACATGCTTGAGCTTAGAGTTATGAATTTTAAAAGTTTACGGTCAGGTTTTGTCAAT
GTTCTGAATAGATCTGAATGTTTGGTTTGTTTGACCTGTGTGCATATGGAATCGTGTCTTAGAGATAATAGAAGAAGTGTAGT
ACTTTTTCTAAGCTTTCCAAAATGTCCTATAGCACTAATTGTGATGGTCTAAATCTCTAGTTATGAGCTTGCAAAGTAGAATG
ACAGAATCTGTCCAAATCCGGCAGAGATGTTTTCTTTGCTATGTTTGTCCTTGTTAGCCGTAGAATCATCTTAATATGATAAT
AAGAAAGTTATAGACAACTTAATAAGCTTTACATAAAGTTAAGGATCACTTTTGTTGTGTCTCTAGAACTCCAATTATGTATT
TTCGAAGTTCTTGTCAGTTTTCTGTCCAAATTGGTATCGGCGCGCCCTCGAGTCTAGAGTCGACGAGCTCGATCCACTAGTAA
CGGCCGCCAGTGTGCTGGAATTCGCCCTTGGCGCGCCGATCTAGTAACATAGATGACACCGCGCGCGATAATTTATCCTAGTT
TGCGCGCTATATTTTGTTTTCTATCGCGTATTAAATGTATAATTGCGGGACTCTAATCATAAAAACCCATCTCATAAATAACG
TCATGCATTACATGTTAATTATTACATGCTTAACGTAATTCAACAGAAATTATATGATAATCATCGCAAGACCGGCAACAGGA
TTCAATCTTAAGAAACTTTATTGCCAAATGTTTGAACGATCTCAGAAGAACTCGTCAAGAAGGCGATAGAAGGCGATGCGCTG
CGAATCGGGAGCGGCGATACCGTAAAGCACGAGGAAGCGGTCAGCCCATTCGCCGCCAAGCTCTTCAGCAATATCACGGGTAG
CCAACGCTATGTCCTGATAGCGGTCCGCCACACCCAGCCGGCCACAGTCGATGAATCCAGAAAAGCGGCCATTTTCCACCATG
ATATTCGGCAAGCAGGCATCGCCATGGGTCACGACGAGATCCTCGCCGTCGGGCATGCGCGCCTTGAGCCTGGCGAACAGTTC
GGCTGGCGCGAGCCCCTGATGCTCTTCGTCCAGATCATCCTGATCGACAAGACCGGCTTCCATCCGAGTACGTGCTCGCTCGA
TGCGATGTTTCGCTTGGTGGTCGAATGGGCAGGTAGCCGGATCAAGCGTATGCAGCCGCCGCATTGCATCAGCCATGATGGAT
ACTTTCTCGGCAGGAGCAAGGTGAGATGACAGGAGATCCTGCCCCGGCACTTCGCCCAATAGCAGCCAGTCCCTTCCCGCTTC
AGTGACAACGTCGAGCACAGCTGCGCAAGGAACGCCCGTCGTGGCCAGCCACGATAGCCGCGCTGCCTCGTCCTGCAGTTCAT
TCAGGGCACCGGACAGGTCGGTCTTGACAAAAAGAACCGGGCGCCCCTGCGCTGACAGCCGGAACACGGCGGCATCAGAGCAG
CCGATTGTCTGTTGTGCCCAGTCATAGCCGAATAGCCTCTCCACCCAAGCGGCCGGAGAACCTGCGTGCAATCCATCTTGTTC
AATCCACATTCTAGAGTCGACCTGCAGAAGTAACACCAAACAACAGGGTGAGCATCGACAAAAGAAACAGTACCAAGCAAATA
AATAGCGTATGAAGGCAGGGCTAAAAAAATCCACATATAGCTGCTGCATATGCCATCATCCAAGTATATCAAGATCAAAATAA
TTATAAAACATACTTGTTTATTATAATAGATAGGTACTCAAGGTTAGAGCATATGAATAGATGCTGCATATGCCATCATGTAT
ATGCATCAGTAAAACCCACATCAACATGTATACCTATCCTAGATCGATATTTCCATCCATCTTAAACTCGTAACTATGAAGAT
GTATGACACACACATACAGTTCCAAAATTAATAAATACACCAGGTAGTTTGAAACAGTATTCTACTCCGATCTAGAACGAATG
AACGACCGCCCAACCACACCACATCATCACAACCAAGCGAACAAAAAGCATCTCTGTATATGCATCAGTAAAACCCGCATCAA
CATGTATACCTATCCTAGATCGATATTTCCATCCATCATCTTCAATTCGTAACTATGAATATGTATGGCACACACATACAGAT
CCAAAATTAATAAATCCACCAGGTAGTTTGAAACAGAATTCTACTCCGATCTAGAACGACCGCCCAACCAGACCACATCATCA
CAACCAAGACAAAAAAAAGCATGAAAAGATGACCCGACAAACAAGTGCACGGCATATATTGAAATAAAGGAAAAGGGCAAACC
AAACCCTATGCAACGAAACAAAAAAAATCATGAAATCGATCCCGTCTGCGGAACGGCTAGAGCCATCCCAGGATTCCCCAAAG
AGAAACACTGGCAAGTTAGCAATCAGAACGTGTCTGACGTACAGGTCGCATCCGTGTACGAACGCTAGCAGCACGGATCTAAC
ACAAACACGGATCTAACACAAACATGAACAGAAGTAGAACTACCGGGCCCTAACCATGGACCGGAACGCCGATCTAGAGAAGG
TAGAGAGGGGGGGGGGGGGAGGACGAGCGGCGTACCTTGAAGCGGAGGTGCCGACGGGTGGATTTGGGGGAGATCTGGTTGTG
TGTGTGTGCGCTCCGAACAACACGAGGTTGGGGAAAGAGGGTGTGGAGGGGGTGTCTATTTATTACGGCGGGCGAGGAAGGGA

AAGCGAAGGAGCGGTGGGAAAGGAATCCCCCGTAGCTGCCGTGCCGTGAGAGGAGGAGGAGGCCGCCTGCCGTGCCGGCTCAC
GTCTGCCGCTCCGCCACGCAATTTCTGGATGCCGACAGCGGAGCAAGTCCAACGGTGGAGCGGAACTCTCGAGAGGGGTCCAG
AGGCAGCGACAGAGATGCCGTGCCGTCTGCTTCGCTTGGCCCGACGCGACGCTGCTGGTTCGCTGGTTGGTGTCCGTTAGACT
CGTCGACGGCGTTTAACAGGCTGGCATTATCTACTCGAAACAAGAAAAATGTTTCCTTAGTTTTTTTAATTTCTTAAAGGGTA
TTTGTTTAATTTTTAGTCACTTTATTTTATTCTATTTTATATCTAAATTATTAAATAAAAAAACTAAAATAGAGTTTTAGTTT
TCTTAATTTAGAGGCTAAAATAGAATAAAATAGATGTACTAAAAAAATTAGTCTATAAAAACCATTAACCCTAAACCCTAAAT
GGATGTACTAATAAAATGGATGAAGTATTATATAGGTGAAGCTATTTGCAAAAAAAAAGGAGAACACATGCACACTAAAAAGA
TAAAACTGTAGAGTCCTGTTGTCAAAATACTCAATTGTCCTTTAGACCATGTCTAACTGTTCATTTATATGATTCTCTAAAAC
ACTGATATTATTGTAGTACTATAGATTATATTATTCGTAGAGTAAAGTTTAAATATATGTATAAAGATAGATAAACTGCACTT
CAAACAAGTGTGACAAAAAAAATATGTGGTAATTTTTTATAACTTAGACATGCAATGCTCATTATCTCTAGAGAGGGGCACGA
CCGGGTCACGCTGCACTGCAGGCATACGCGTAAGCTTCAGGGTTTAAACAGGTCCGATTGAGACTTTTCAACAAAGGGTAATA
TCCGGAAACCTCCTCGGATTCCATTGCCCAGCTATCTGTCACTTTATTGTGAAGATAGTGGAAAAGGAAGGTGGCTCCTACAA
ATGCCATCATTGCGATAAAGGAAAGGCCATCGTTGAAGATGCCTCTGCCGACAGTGGTCCCAAAGATGGACCCCCACCCACGA
GGAGCATCGTGGAAAAAGAAGACGTTCCAACCACGTCTTCAAAGCAAGTGGATTGATGTGATGGTCCGATTGAGACTTTTCAA
CAAAGGGTAATATCCGGAAACCTCCTCGGATTCCATTGCCCAGCTATCTGTCACTTTATTGTGAAGATAGTGGAAAAGGAAGG
TGGCTCCTACAAATGCCATCATTGCGATAAAGGAAAGGCCATCGTTGAAGATGCCTCTGCCGACAGTGGTCCCAAAGATGGAC
CCCCACCCACGAGGAGCATCGTGGAAAAAGAAGACGTTCCAACCACGTCTTCAAAGCAAGTGGATTGATGTGATATCTCCACT
GACGTAAGGGATGACGCACAATCCCACTATCCTTCGCAAGACCCTTCCTCTATATAAGGAAGTTCATTTCATTTGGAGAGGAC
ACGCTGACAAGCTGACTCTAGCAGATCCTCTAGAACCATCTTCCACACACTCAAGCCACACTATTGGAGAACACACAGGGACA
ACACACCATAAGATCCAAGGGAGGCCTCCGCCGCCGCCGGTAACCACCCCGCCCCTCTCCTCTTTCTTTCTCCGTTTTTTTTT
CCGTCTCGGTCTCGATCTTTGGCCTTGGTAGTTTGGGTGGGCGAGAGGCGGCTTCGTGCGCGCCCAGATCGGTGCGCGGGAGG
GGCGGGATCTCGCGGCTGGGGCTCTCGCCGGCGTGGATCCGGCCCGGATCTCGCGGGGAATGGGGCTCTCGGATGTAGATCTG
CGATCCGCCGTTGTTGGGGGAGATGATGGGGGGTTTAAAATTTCCGCCGTGCTAAACAAGATCAGGAAGAGGGGAAAAGGGCA
CTATGGTTTATATTTTTATATATTTCTGCTGCTTCGTCAGGCTTAGATGTGCTAGATCTTTCTTTCTTCTTTTTGTGGGTAGA
ATTTGAATCCCTCAGCATTGTTCATCGGTAGTTTTTCTTTTCATGATTTGTGACAAATGCAGCCTCGTGCGGAGCTTTTTTGT
AGGTAGAAGTGATCAACCTGATCAACCCCGCCATGGACAACAACCCAAACATCAACGAGTGCATCCCATACAACTGCCTGAGC
AACCCAGAGGTGGAGGTGCTGGGTGGCGAGCGCATCGAGACCGGTTACACCCCCATCGACATCTCCCTGTCCTTGACCCAGTT
CCTGCTCAGCGAGTTCGTGCCAGGTGCTGGCTTCGTGCTCGGCCTGGTGGACATCATCTGGGGTATCTTCGGTCCATCCCAAT
GGGACGCCTTCCTGGTGCAAATCGAGCAGCTGATCAACCAGAGGATCGAAGAGTTCGCCAGGAACCAGGCCATCTCCAGGCTG
GAGGGCCTGAGCAACCTCTACCAAATCTACGCCGAGAGCTTCAGGGAGTGGGAGGCCGACCCGACCAACCCAGCTCTCCGCGA
GGAAATGCGCATTCAATTCAACGACATGAACAGCGCCCTGACCACCGCTATCCCACTGTTCGCCGTCCAGAACTACCAAGTGC
CGCTCCTGTCCGTGTACGTGCAAGCCGCTAACCTGCACCTCAGCGTGCTGCGCGACGTGAGCGTGTTCGGCCAAAGGTGGGGC
TTCGATGCTGCCACCATCAACAGCCGCTACAACGACCTGACCAGGCTGATTGGCAACTACACCGACCACGCTGTGCGCTGGTA
CAACACCGGCCTGGAGCGCGTCTGGGGTCCGGACTCCAGGGACTGGATCAGGTACAACCAGTTCAGGAGGGAGTTGACCCTCA
CCGTGCTGGACATTGTGTCCCTCTTCCCGAACTACGACTCCAGGACCTACCCGATCCGCACCGTGTCCCAACTCACCAGGGAG
ATCTACACCAACCCAGTGCTGGAGAACTTCGACGGTAGCTTCCGCGGTTCCGCCCAGGGTATCGAGGGCTCCATCAGGAGCCC
ACACCTGATGGACATCCTGAACAGCATCACCATCTACACCGACGCTCACAGGGGCGAGTACTACTGGTCCGGCCACCAGATCA
TGGCCTCCCCAGTGGGCTTCAGCGGCCCCGAGTTCACCTTCCCGCTCTACGGCACCATGGGCAACGCCGCTCCACAGCAACGC
ATCGTGGCTCAACTGGGTCAGGGTGTCTACAGGACCCTGTCCTCCACCCTGTACAGGAGGCCCTTCAACATCGGTATCAACAA
CCAGCAACTGTCCGTGCTCGACGGCACCGAGTTCGCCTACGGCACCTCCTCCAACCTGCCATCCGCTGTCTACAGGAAGAGCG
GCACCGTGGACTCCCTGGACGAGATCCCACCACAGAACAACAACGTGCCACCCAGGCAAGGCTTCTCCCACAGGCTGAGCCAC
GTGTCCATGTTCCGCTCCGGCTTCAGCAACAGCTCCGTGAGCATCATCAGGGCTCCGATGTTCTCCTGGATCCACCGCAGCGC
TGAGTTCAACAACATCATCGCCTCCGACAGCATCACCCAAATCCCGGCCGTGAAGGGCAACTTCCTCTTCAACGGTTCCGTCA
TTTCCGGCCCAGGCTTCACCGGTGGCGACCTCGTGAGGCTCAACAGCAGCGGCAACAACATCCAGAACAGGGGCTACATCGAG
TGCCAATCCACTTCCCATCCACCTCCACCAGGTACAGGGTGCGCGTGAGGTACGCTTCCGTGACCCCGATCCACCTCAACGTG
AACTGGGGTAACTCCTCCATCTTCTCCAACACCGTGCCAGCTACCGCTACCTCCCTGGACAACCTCCAATCCAGCGACTTCGG
TTACTTCGAGAGCGCCAACGCTTTCACCTCCTCCCTCGGTAACATCGTGGGCGTGAGGAACTTCAGCGGCACCGCCGGCGTGA
TCATCGACAGGTTCGAGTTCATCCCAGTGACCGCCACCCTCGAGGCTGAGTGATCGATCGACAAGCTCGAGTTTCTCCATAAT
AATGTGTGAGTAGTTCCCAGATAAGGGAATTAGGGTTCCTATAGGGTTTCGCTCATGTGTTGAGCATATAAGAAACCCTTAGT
ATGTATTTGTATTTGTAAAATACTTCTATCAATAAAATTTCTAATTCCTAAAACCAAAATCCAGTACTAAAATCCAGATCCCC
CGAATTAAGGTACCGATATCAGTACTAATTCAGTACATTAAAAACGTCCGCAATGTGTTATTAAGTTGTCTAAGCGTCAATTT
GTGAAATATTAGCAATTTTGGTCATGTTAAGCTATGAATTAGTTTTTGGTATGAATAACTAAAGTGTAGTAATCTTCCTAAGA
TTTCTAGAAAGTCTAGGATCACCTTTGTGGGATGTCTATAACCTCAGTTATGGTTGAAACAAGTAACTACTGCGTTGCTGTCC
AGATTCTAGGCACATGCTAAGTTGGCTACCGTATCTAGCGTTGTTTTGAGCTAAACTTGTAGTTAGTTCATGCTTGAGATAAA
TGCTTATTTTGGGTGAGCCTTGTGTCTTGTGTTGGTTGCTTAAGCCCATTTGTTTGAATTTTCTCAAATGACTGCTTGATTGT
GATGTTGTATGTGTGTTTGTTATCTAGGTAGTGCCCTAGTTGCCACCTC

**>SEQ ID 30 - Complementary sequence of SEQ ID NO 23 (reverse-complement)**

GAAATATTAGCAATTTTGGTCATGTTAAGCTATGAATTAGTTTTTGGTATGAATAACTAAAGTGTAGTAATCTTCCTAAGATT
TCTAGAAAGTCTAGGATCACCTTTGTGGGATGTCTATAACCTCAGTTATGGTTGAAACAAGTAACTACTGCGTTGCTGTCCAG
ATTCTAGGCACATGCTAAGTTGGCTACCGTATCTAGCGTTGTTTTGAGCTAAACTTGTAGTTAGTTCATGCTTGAGATAAATG
CTTATTTTGGGTGAGCCTTGTGTCTTGTGTTGGTTGCTTAAGCCCATTTGTTTGAATTTTCTCAAATGACTGCTTGATTGTGA
TGTTGTATGTGTGTTTGTTATCTAGGTAGTGCCCTAGTTGCCACCTC

**>SEQ ID 31- Complementary sequence of SEQ ID NO 24 (reverse-complement)**

GCATCTTCATGATTGTTGCCTTATGCATAGCTATGCTCCTCTCACCCTTGTTCAAATGATGAGTAATTTGTGAGTGATGTGGT
ATCCTATCTTCCTTGTGTGGAGTCAAACAAGAATGCTTTGTTCCATGTTGCTTGAATAGGAAATGCTTGAGAAAAATGTGTGC
TTAGCTTTACTAGAAATAAGTGTGACTACATGCTTGAGCTTAGAGTTATGAATTTTAAAAGTTTACGGTCAGGTTTTGTCAAT
GTTCTGAATAGATCTGAATGTTTGGTTTGTTTGACCTGTGTGCATATGGAATCGTGTCTTAGAGATAATAGAAGAAGTGTAGT
ACTTTTTCTAAGCTTTCCAAAATGTCCTATAGCACTAATTGTGATGGTCTAAATCTCTAGTTATGAGCTTGCAAAGTAGAATG
ACAGAATCTGTCCAAATCCGGCAGAGATGTTTTCTTTGCTATGTTTGTCCTTGTTAGCCGTAGAATCATCTTAATATGATAAT
AAGAAAGTTATAGACAACTTAATAAGCTTTACATAAAGTTAAGGATCACTTTTGTTGTGTCTCTAGAACTCCAATTATGTATT
TTCGAAGTTCTTGTCAGTTTTCTGTCCAAATTGGTA

**>SEQ ID 32- Complemmentary sequence of SEQ ID NO 18 (reverse-complement)**
AGCGTCAATTTGTGAAATATTAGCAA
**>SEQ ID 33- Complementary sequence of SEQ ID NO 19 (reverse-complement)**
GTCCAAATTGGTATCGGCGCGCCCTC

**>SEQ ID NO 34 (Cry1Ac protein) - Truncate cry1Ac protein (B. thurigiensis)**

Met Asp Asn Asn Pro Asn Ile Asn Glu Cys Ile Pro Tyr Asn Cys Leu Ser Asn Pro Glu Val
Glu Val Leu Gly Gly Glu Arg Ile Glu Thr Gly Tyr Thr Pro Ile Asp Ile Ser Leu Ser Leu
Thr Gln Phe Leu Leu Ser Glu Phe Val Pro Gly Ala Gly Phe Val Leu Gly Leu Val Asp Ile
Ile Trp Gly Ile Phe Gly Pro Ser Gln Trp Asp Ala Phe Leu Val Gln Ile Glu Gln Leu Ile
Asn Gln Arg Ile Glu Glu Phe Ala Arg Asn Gln Ala Ile Ser Arg Leu Glu Gly Leu Ser Asn
Leu Tyr Gln Ile Tyr Ala Glu Ser Phe Arg Glu Trp Glu Ala Asp Pro Thr Asn Pro Ala Leu
Arg Glu Glu Met Arg Ile Gln Phe Asn Asp Met Asn Ser Ala Leu Thr Thr Ala Ile Pro Leu
Phe Ala Val Gln Asn Tyr Gln Val Pro Leu Leu Ser Val Tyr Val Gln Ala Ala Asn Leu His
Leu Ser Val Leu Arg Asp Val Ser Val Phe Gly Gln Arg Trp Gly Phe Asp Ala Ala Thr Ile
Asn Ser Arg Tyr Asn Asp Leu Thr Arg Leu Ile Gly Asn Tyr Thr Asp His Ala Val Arg Trp
Tyr Asn Thr Gly Leu Glu Arg Val Trp Gly Pro Asp Ser Arg Asp Trp Ile Arg Tyr Asn Gln
Phe Arg Arg Glu Leu Thr Leu Thr Val Leu Asp Ile Val Ser Leu Phe Pro Asn Tyr Asp Ser
Arg Thr Tyr Pro Ile Arg Thr Val Ser Gln Leu Thr Arg Glu Ile Tyr Thr Asn Pro Val Leu
Glu Asn Phe Asp Gly Ser Phe Arg Gly Ser Ala Gln Gly Ile Glu Gly Ser Ile Arg Ser Pro
His Leu Met Asp Ile Leu Asn Ser Ile Thr Ile Tyr Thr Asp Ala His Arg Gly Glu Tyr Tyr
Trp Ser Gly His Gln Ile Met Ala Ser Pro Val Gly Phe Ser Gly Pro Glu Phe Thr Phe Pro
Leu Tyr Gly Thr Met Gly Asn Ala Ala Pro Gln Gln Arg Ile Val Ala Gln Leu Gly Gln Gly
Val Tyr Arg Thr Leu Ser Ser Thr Leu Tyr Arg Arg Pro Phe Asn Ile Gly Ile Asn Asn Gln
Gln Leu Ser Val Leu Asp Gly Thr Glu Phe Ala Tyr Gly Thr Ser Ser Asn Leu Pro Ser Ala
Val Tyr Arg Lys Ser Gly Thr Val Asp Ser Leu Asp Glu Ile Pro Pro Gln Asn Asn Asn Val
Pro Pro Arg Gln Gly Phe Ser His Arg Leu Ser His Val Ser Met Phe Arg Ser Gly Phe Ser
Asn Ser Ser Val Ser Ile Ile Arg Ala Pro Met Phe Ser Trp Ile His Arg Ser Ala Glu Phe
Asn Asn Ile Ile Ala Ser Asp Ser Ile Thr Gln Ile Pro Ala Val Lys Gly Asn Phe Leu Phe
Asn Gly Ser Val Ile Ser Gly Pro Gly Phe Thr Gly Gly Asp Leu Val Arg Leu Asn Ser Ser
Gly Asn Asn Ile Gln Asn Arg Gly Tyr Ile Glu Val Pro Ile His Phe Pro Ser Thr Ser Thr
Arg Tyr Arg Val Arg Val Arg Tyr Ala Ser Val Thr Pro Ile His Leu Asn Val Asn Trp Gly
Asn Ser Ser Ile Phe Ser Asn Thr Val Pro Ala Thr Ala Thr Ser Leu Asp Asn Leu Gln Ser
Ser Asp Phe Gly Tyr Phe Glu Ser Ala Asn Ala Phe Thr Ser Ser Leu Gly Asn Ile Val Gly
Val Arg Asn Phe Ser Gly Thr Ala Gly Val Ile Ile Asp Arg Phe Glu Phe Ile Pro Val Thr
Ala Thr Leu Glu Ala Glu

**> SEQ ID NO 35 (nptII protein)**

MWIEQDGLHAGSPAAWVERLFGYDWAQQTIGCSDAAVFRLSAQGRPVLFVKTDLSGALNELQDEAARLSWLATTGVPCAAVLD
VVTEAGRDWLLLGEVPGQDLLSSHLAPAEKVSIMADAMRRLHTLDPATCPFDHQAKHRIERARTRMEAGLVDQDDLDEEHQGL
APAELFARLKARMPDGEDLVVTHGDACLPNIMVENGRFSGFIDCGRLGVADRYQDIALATRDIAEELGGEWADRFLVLYGIAA
PDSQRIAFYRLLDEFF

**>SEQ ID 36- Complementary sequence of SEQ ID NO 02 (reverse-complement)**

TCGGCGCGCCCTCGAGTCTAGAGTCGACGAGCTCGATCCACTAGTAACGGCCGCCAGTGTGCTGGAATTCGCCCTTGGCGCGC
CGATCTAGTAACATAGATGACACCGCGCGATAATTTATCCTAGTTTGCGCGCTATATTTTGTTTTCTATCGCGTATTAAAT
GTATAATTGCGGGACTCTAATCATAAAAACCCATCTCATAAATAACGTCATGCATTACATGTTAATTATTACATGCTTAACGT
AATTCAACAGAAATTATATGATAATCATCGCAAGACCGGCAACAGGATTCAATCTTAAGAAACTTTATTGCCAAATGTTTGAA
CGATCTCAGAAGAACTCGTCAAGAAGGCGATAGAAGGCGATGCGCTGCGAATCGGGAGCGGCGATACCGTAAAGCACGAGGAA
GCGGTCAGCCCATTCGCCGCCAAGCTCTTCAGCAATATCACGGGTAGCCAACGCTATGTCCTGATAGCGGTCCGCCACACCCA
GCCGGCCACAGTCGATGAATCCAGAAAAGCGGCCATTTTCCACCATGATATTCGGCAAGCAGGCATCGCCATGGGTCACGACG
AGATCCTCGCCGTCGGGCATGCGCGCCTTGAGCCTGGCGAACAGTTCGGCTGGCGCGAGCCCCTGATGCTCTTCGTCCAGATC
ATCCTGATCGACAAGACCGGCTTCCATCCGAGTACGTGCTCGCTCGATGCGATGTTTCGCTTGGTGGTCGAATGGGCAGGTAG
CCGGATCAAGCGTATGCAGCCGCCGCATTGCATCAGCCATGATGGATACTTTCTCGGCAGGAGCAAGGTGAGATGACAGGAGA
TCCTGCCCCGGCACTTCGCCCAATAGCAGCCAGTCCCTTCCCGCTTCAGTGACAACGTCGAGCACAGCTGCGCAAGGAACGCC
CGTCGTGGCCAGCCACGATAGCCGCGCTGCCTCGTCCTGCAGTTCATTCAGGGCACCGGACAGGTCGGTCTTGACAAAAAGAA
CCGGGCGCCCCTGCGCTGACAGCCGGAACACGGCGGCATCAGAGCAGCCGATTGTCTGTTGTGCCCAGTCATAGCCGAATAGC
CTCTCCACCCAAGCGGCCGGAGAACCTGCGTGCAATCCATCTTGTTCAATCCACATTCTAGAGTCGACCTGCAGAAGTAACAC
CAAACAACAGGGTGAGCATCGACAAAAGAAACAGTACCAAGCAAATAAATAGCGTATGAAGGCAGGGCTAAAAAAATCCACAT
ATAGCTGCTGCATATGCCATCATCCAAGTATATCAAGATCAAAATAATTATAAAACATACTTGTTTATTATAATAGATAGGTA
CTCAAGGTTAGAGCATATGAATAGATGCTGCATATGCCATCATGTATATGCATCAGTAAAACCCACATCAACATGTATACCTA
TCCTAGATCGATATTTCCATCCATCTTAAACTCGTAACTATGAAGATGTATGACACACACATACAGTTCCAAAATTAATAAAT
ACACCAGGTAGTTTGAAACAGTATTCTACTCCGATCTAGAACGAATGAACGACCGCCCAACCACACCACATCATCACAACCAA
GCGAACAAAAAGCATCTCTGTATATGCATCAGTAAAACCCGCATCAACATGTATACCTATCCTAGATCGATATTTCCATCCAT
CATCTTCAATTCGTAACTATGAATATGTATGGCACACACATACAGATCCAAAATTAATAAATCCACCAGGTAGTTTGAAACAG
AATTCTACTCCGATCTAGAACGACCGCCCAACCAGACCACATCATCACAACCAAGACAAAAAAAAGCATGAAAAGATGACCCG
ACAAACAAGTGCACGGCATATATTGAAATAAAGGAAAAGGGCAAACCAAACCCTATGCAACGAAACAAAAAAAATCATGAAAT
CGATCCCGTCTGCGGAACGGCTAGAGCCATCCCAGGATTCCCCAAAGAGAAACACTGGCAAGTTAGCAATCAGAACGTGTCTG
ACGTACAGGTCGCATCCGTGTACGAACGCTAGCAGCACGGATCTAACACAAACACGGATCTAACACAAACATGAACAGAAGTA
GAACTACCGGGCCCTAACCATGGACCGGAACGCCGATCTAGAGAAGGTAGAGAGGGGGGGGGGGGGAGGACGAGCGGCGTACC
TTGAAGCGGAGGTGCCGACGGGTGGATTTGGGGGAGATCTGGTTGTGTGTGTGTGCGCTCCGAACAACACGAGGTTGGGGAAA
GAGGGTGTGGAGGGGGTGTCTATTTATTACGGCGGGCGAGGAAGGGAAAGCGAAGGAGCGGTGGGAAAGGAATCCCCCGTAGC
TGCCGTGCCGTGAGAGGAGGAGGAGGCCGCCTGCCGTGCCGGCTCACGTCTGCCGCTCCGCCACGCAATTTCTGGATGCCGAC
AGCGGAGCAAGTCCAACGGTGGAGCGGAACTCTCGAGAGGGGTCCAGAGGCAGCGACAGAGATGCCGTGCCGTCTGCTTCGCT
TGGCCCGACGCGACGCTGCTGGTTCGCTGGTTGGTGTCCGTTAGACTCGTCGACGGCGTTTAACAGGCTGGCATTATCTACTC
GAAACAAGAAAAATGTTTCCTTAGTTTTTTTAATTTCTTAAAGGGTATTTGTTTAATTTTTAGTCACTTTATTTTATTCTATT
TTATATCTAAATTATTAAATAAAAAAACTAAAATAGAGTTTTAGTTTTCTTAATTTAGAGGCTAAAATAGAATAAAATAGATG
TACTAAAAAAATTAGTCTATAAAAACCATTAACCCTAAACCCTAAATGGATGTACTAATAAAATGGATGAAGTATTATATAGG
TGAAGCTATTTGCAAAAAAAAAGGAGAACACATGCACACTAAAAAGATAAAACTGTAGAGTCCTGTTGTCAAAATACTCAATT
GTCCTTTAGACCATGTCTAACTGTTCATTTATATGATTCTCTAAAACACTGATATTATTGTAGTACTATAGATTATATTATTC
GTAGAGTAAAGTTTAAATATATGTATAAAGATAGATAAACTGCACTTCAAACAAGTGTGACAAAAAAAATATGTGGTAATTTT
TTATAACTTAGACATGCAATGCTCATTATCTCTAGAGAGGGGCACGACCGGGTCACGCTGCACTGCAGGCATACGCGTAAGCT
TCAGGGTTTAAACAGGTCCGATTGAGACTTTTCAACAAAGGGTAATATCCGGAAACCTCCTCGGATTCCATTGCCCAGCTATC
TGTCACTTTATTGTGAAGATAGTGGAAAAGGAAGGTGGCTCCTACAAATGCCATCATTGCGATAAAGGAAAGGCCATCGTTGA
AGATGCCTCTGCCGACAGTGGTCCCAAAGATGGACCCCCACCCACGAGGAGCATCGTGGAAAAAGAAGACGTTCCAACCACGT
CTTCAAAGCAAGTGGATTGATGTGATGGTCCGATTGAGACTTTTCAACAAAGGGTAATATCCGGAAACCTCCTCGGATTCCAT
TGCCCAGCTATCTGTCACTTTATTGTGAAGATAGTGGAAAAGGAAGGTGGCTCCTACAAATGCCATCATTGCGATAAAGGAAA
GGCCATCGTTGAAGATGCCTCTGCCGACAGTGGTCCCAAAGATGGACCCCCACCCACGAGGAGCATCGTGGAAAAAGAAGACG
TTCCAACCACGTCTTCAAAGCAAGTGGATTGATGTGATATCTCCACTGACGTAAGGGATGACGCACAATCCCACTATCCTTCG
CAAGACCCTTCCTCTATATAAGGAAGTTCATTTCATTTGGAGAGGACACGCTGACAAGCTGACTCTAGCAGATCCTCTAGAAC
CATCTTCCACACACTCAAGCCACACTATTGGAGAACACACAGGGACAACACACCATAAGATCCAAGGGAGGCCTCCGCCGCCG
CCGGTAACCACCCCGCCCCTCTCCTCTTTCTTTCTCCGTTTTTTTTTCCGTCTCGGTCTCGATCTTTGGCCTTGGTAGTTTGG
GTGGGCGAGAGGCGGCTTCGTGCGCGCCCAGATCGGTGCGCGGGAGGGGCGGGATCTCGCGGCTGGGGCTCTCGCCGGCGTGG
ATCCGGCCCGGATCTCGCGGGGAATGGGGCTCTCGGATGTAGATCTGCGATCCGCCGTTGTTGGGGGAGATGATGGGGGGTTT
AAAATTTCCGCCGTGCTAAACAAGATCAGGAAGAGGGGAAAAGGGCACTATGGTTTATATTTTTATATATTTCTGCTGCTTCG
TCAGGCTTAGATGTGCTAGATCTTTCTTTCTTCTTTTTGTGGGTAGAATTTGAATCCCTCAGCATTGTTCATCGGTAGTTTTT
CTTTTCATGATTTGTGACAAATGCAGCCTCGTGCGGAGCTTTTTTGTAGGTAGAAGTGATCAACCTGATCAACCCCGCCATGG

ACAACAACCCAAACATCAACGAGTGCATCCCATACAACTGCCTGAGCAACCCAGAGGTGGAGGTGCTGGGTGGCGAGCGCATC
GAGACCGGTTACACCCCCATCGACATCTCCCTGTCCTTGACCCAGTTCCTGCTCAGCGAGTTCGTGCCAGGTGCTGGCTTCGT
GCTCGGCCTGGTGGACATCATCTGGGGTATCTTCGGTCCATCCCAATGGGACGCCTTCCTGGTGCAAATCGAGCAGCTGATCA
ACCAGAGGATCGAAGAGTTCGCCAGGAACCAGGCCATCTCCAGGCTGGAGGGCCTGAGCAACCTCTACCAAATCTACGCCGAG
AGCTTCAGGGAGTGGGAGGCCGACCCGACCAACCCAGCTCTCCGCGAGGAAATGCGCATTCAATTCAACGACATGAACAGCGC
CCTGACCACCGCTATCCCACTGTTCGCCGTCCAGAACTACCAAGTGCCGCTCCTGTCCGTGTACGTGCAAGCCGCTAACCTGC
ACCTCAGCGTGCTGCGCGACGTGAGCGTGTTCGGCCAAAGGTGGGGCTTCGATGCTGCCACCATCAACAGCCGCTACAACGAC
CTGACCAGGCTGATTGGCAACTACACCGACCACGCTGTGCGCTGGTACAACACCGGCCTGGAGCGCGTCTGGGGTCCGGACTC
CAGGGACTGGATCAGGTACAACCAGTTCAGGAGGGAGTTGACCCTCACCGTGCTGGACATTGTGTCCCTCTTCCCGAACTACG
ACTCCAGGACCTACCCGATCCGCACCGTGTCCCAACTCACCAGGGAGATCTACACCAACCCAGTGCTGGAGAACTTCGACGGT
AGCTTCCGCGGTTCCGCCCAGGGTATCGAGGGCTCCATCAGGAGCCCACACCTGATGGACATCCTGAACAGCATCACCATCTA
CACCGACGCTCACAGGGGCGAGTACTACTGGTCCGGCCACCAGATCATGGCCTCCCCAGTGGGCTTCAGCGGCCCCGAGTTCA
CCTTCCCGCTCTACGGCACCATGGGCAACGCCGCTCCACAGCAACGCATCGTGGCTCAACTGGGTCAGGGTGTCTACAGGACC
CTGTCCTCCACCCTGTACAGGAGGCCCTTCAACATCGGTATCAACAACCAGCAACTGTCCGTGCTCGACGGCACCGAGTTCGC
CTACGGCACCTCCTCCAACCTGCCATCCGCTGTCTACAGGAAGAGCGGCACCGTGGACTCCCTGGACGAGATCCCACCACAGA
ACAACAACGTGCCACCCAGGCAAGGCTTCTCCCACAGGCTGAGCCACGTGTCCATGTTCCGCTCCGGCTTCAGCAACAGCTCC
GTGAGCATCATCAGGGCTCCGATGTTCTCCTGGATCCACCGCAGCGCTGAGTTCAACAACATCATCGCCTCCGACAGCATCAC
CCAAATCCCGGCCGTGAAGGGCAACTTCCTCTTCAACGGTTCCGTCATTTCCGGCCCAGGCTTCACCGGTGGCGACCTCGTGA
GGCTCAACAGCAGCGGCAACAACATCCAGAACAGGGGCTACATCGAGGTGCCAATCCACTTCCCATCCACCTCCACCAGGTAC
AGGGTGCGCGTGAGGTACGCTTCCGTGACCCCGATCCACCTCAACGTGAACTGGGGTAACTCCTCCATCTTCTCCAACACCGT
GCCAGCTACCGCTACCTCCCTGGACAACCTCCAATCCAGCGACTTCGGTTACTTCGAGAGCGCCAACGCTTTCACCTCCTCCC
TCGGTAACATCGTGGGCGTGAGGAACTTCAGCGGCACCGCCGGCGTGATCATCGACAGGTTCGAGTTCATCCCAGTGACCGCC
ACCCTCGAGGCTGAGTGATCGATCGACAAGCTCGAGTTTCTCCATAATAATGTGTGAGTAGTTCCCAGATAAGGGAATTAGGG
TTCCTATAGGGTTTCGCTCATGTGTTGAGCATATAAGAAACCCTTAGTATGTATTTGTATTTGTAAAATACTTCTATCAATAA
AATTTCTAATTCCTAAAACCAAAATCCAGTACTAAAATCCAGATCCCCCGAATTAAGGTACCGATATCAGTACTAATTCAGTA
CATTAAAAACGTCCGCAATGTGTTATTAAGTTGTCTAAGCGTCAATTTGT

**>SEQ ID 37- Complementary sequence of SEQ ID NO 05 (reverse-complement)**

ACTTAATAAGCTTTACATAAAGTTAAGGATCACTTTTGTTGTGTCTCTAGAACTCCAATTATGTATTTTCGAAGTTCTTGTCA
GTTTTCTGTCCAAATTGGTATCGGCGCGCCCTCGAGTCTAGAGTCGACGAGCTCGATCCACTAGTAACGGCCGCCAGTGTGCT
GGAATTCGCCCTTGGCGCGCCGATCTAGTAACATAGATGACACCGCGCGCGATAATTTATCCTAGTTTGCGCGCTATATTTTG
TTTTCTATCGCGTATTAAATGTATAATTGCGGGACTCTAATCATAAAAACCCATCTCATAAATAACGTCATGCATTACATGTT
AATTATTACATGCTTAACGTAATTCAACAGAAATTATATGATAATCATCGCAAGACCGGCAACAGGATTCAATCTTAAGAAAC
TTTATTGCCAAATGTTTGAACGATCTCAGAAGAACTCGTCAAGAAGGCGATAGAAGGCGATGCGCTGCGAATCGGGAGCGGCG
ATACCGTAAAGCACGAGGAAGCGGTCAGCCCATTCGCCGCCAAGCTCTTCAGCAATATCACGGGTAGCCAACGCTATGTCCTG
ATAGCGGTCCGCCACACCCAGCCGGCCACAGTCGATGAATCCAGAAAAGCGGCCATTTTCCACCATGATATTCGGCAAGCAGG
CATCGCCATGGGTCACGACGAGATCCTCGCCGTCGGGCATGCGCGCCTTGAGCCTGGCGAACAGTTCGGCTGGCGCGAGCCCC
TGATGCTCTTCGTCCAGATCATCCTGATCGACAAGACCGGCTTCCATCCGAGTACGTGCTCGCTCGATGCGATGTTTCGCTTG
GTGGTCGAATGGGCAGGTAGCCGGATCAAGCGTATGCAGCCGCCGCATTGCATCAGCCATGATGGATACTTTCTCGGCAGGAG
CAAGGTGAGATGACAGGAGATCCTGCCCCGGCACTTCGCCCAATAGCAGCCAGTCCCTTCCCGCTTCAGTGACAACGTCGAGC
ACAGCTGCGCAAGGAACGCCCGTCGTGGCCAGCCACGATAGCCGCGCTGCCTCGTCCTGCAGTTCATTCAGGGCACCGGACAG
GTCGGTCTTGACAAAAAGAACCGGGCGCCCCTGCGCTGACAGCCGGAACACGGCGGCATCAGAGCAGCCGATTGTCTGTTGTG
CCCAGTCATAGCCGAATAGCCTCTCCACCCAAGCGGCCGGAGAACCTGCGTGCAATCCATCTTGTTCAATCCACATTCTAGAG
TCGACCTGCAGAAGTAACACCAAACAACAGGGTGAGCATCGACAAAAGAAACAGTACCAAGCAAATAAATAGCGTATGAAGGC
AGGGCTAAAAAAATCCACATATAGCTGCTGCATATGCCATCATCCAAGTATATCAAGATCAAAATAATTATAAAACATACTTG
TTTATTATAATAGATAGGTACTCAAGGTTAGAGCATATGAATAGATGCTGCATATGCCATCATGTATATGCATCAGTAAAACC
CACATCAACATGTATACCTATCCTAGATCGATATTTCCATCCATCTTAAACTCGTAACTATGAAGATGTATGACACACACATA
CAGTTCCAAAATTAATAAATACACCAGGTAGTTTGAAACAGTATTCTACTCCGATCTAGAACGAATGAACGACCGCCCAACCA
CACCACATCATCACAACCAAGCGAACAAAAAGCATCTCTGTATATGCATCAGTAAAACCCGCATCAACATGTATACCTATCCT
AGATCGATATTTCCATCCATCATCTTCAATTCGTAACTATGAATATGTATGGCACACACATACAGATCCAAAATTAATAAATC
CACCAGGTAGTTTGAAACAGAATTCTACTCCGATCTAGAACGACCGCCCAACCAGACCACATCATCACAACCAAGACAAAAAA
AAGCATGAAAAGATGACCCGACAAACAAGTGCACGGCATATATTGAAATAAAGGAAAAGGGCAAACCAAACCCTATGCAACGA
AACAAAAAAAATCATGAAATCGATCCCGTCTGCGGAACGGCTAGAGCCATCCCAGGATTCCCCAAAGAGAAACACTGGCAAGT
TAGCAATCAGAACGTGTCTGACGTACAGGTCGCATCCGTGTACGAACGCTAGCAGCACGGATCTAACACAAACACGGATCTAA
CACAAACATGAACAGAAGTAGAACTACCGGGCCCTAACCATGGACCGGAACGCCGATCTAGAGAAGGTAGAGAGGGGGGGGGG
GGGAGGACGAGCGGCGTACCTTGAAGCGGAGGTGCCGACGGGTGGATTTGGGGGAGATCTGGTTGTGTGTGTGTGCGCTCCGA
ACAACACGAGGTTGGGGAAAGAGGGTGTGGAGGGGGTGTCTATTTATTACGGCGGGCGAGGAAGGGAAAGCGAAGGAGCGGTG

GGAAAGGAATCCCCCGTAGCTGCCGTGCCGTGAGAGGAGGAGGAGGCCGCCTGCCGTGCCGGCTCACGTCTGCCGCTCCGCCA
CGCAATTTCTGGATGCCGACAGCGGAGCAAGTCCAACGGTGGAGCGGAACTCTCGAGAGGGGTCCAGAGGCAGCGACAGAGAT
GCCGTGCCGTCTGCTTCGCTTGGCCCGACGCGACGCTGCTGGTTCGCTGGTTGGTGTCCGTTAGACTCGTCGACGGCGTTTAA
CAGGCTGGCATTATCTACTCGAAACAAGAAAAATGTTTCCTTAGTTTTTTTAATTTCTTAAAGGGTATTTGTTTAATTTTTAG
TCACTTTATTTTATTCTATTTTATATCTAAATTATTAAATAAAAAAACTAAAATAGAGTTTTAGTTTTCTTAATTTAGAGGCT
AAAATAGAATAAAATAGATGTACTAAAAAAATTAGTCTATAAAAACCATTAACCCTAAACCCTAAATGGATGTACTAATAAAA
TGGATGAAGTATTATATAGGTGAAGCTATTTGCAAAAAAAAAGGAGAACACATGCACACTAAAAAGATAAAACTGTAGAGTCC
TGTTGTCAAAATACTCAATTGTCCTTTAGACCATGTCTAACTGTTCATTTATATGATTCTCTAAAACACTGATATTATTGTAG
TACTATAGATTATATTATTCGTAGAGTAAAGTTTAAATATATGTATAAAGATAGATAAACTGCACTTCAAACAAGTGTGACAA
AAAAAATATGTGGTAATTTTTTATAACTTAGACATGCAATGCTCATTATCTCTAGAGAGGGGCACGACCGGGTCACGCTGCAC
TGCAGGCATACGCGTAAGCTTCAGGGTTTAAACAGGTCCGATTGAGACTTTTCAACAAAGGGTAATATCCGGAAACCTCCTCG
GATTCCATTGCCCAGCTATCTGTCACTTTATTGTGAAGATAGTGGAAAAGGAAGGTGGCTCCTACAAATGCCATCATTGCGAT
AAAGGAAAGGCCATCGTTGAAGATGCCTCTGCCGACAGTGGTCCCAAAGATGGACCCCCACCCACGAGGAGCATCGTGGAAAA
AGAAGACGTTCCAACCACGTCTTCAAAGCAAGTGGATTGATGTGATGGTCCGATTGAGACTTTTCAACAAAGGGTAATATCCG
GAAACCTCCTCGGATTCCATTGCCCAGCTATCTGTCACTTTATTGTGAAGATAGTGGAAAAGGAAGGTGGCTCCTACAAATGC
CATCATTGCGATAAAGGAAAGGCCATCGTTGAAGATGCCTCTGCCGACAGTGGTCCCAAAGATGGACCCCCACCCACGAGGAG
CATCGTGGAAAAAGAAGACGTTCCAACCACGTCTTCAAAGCAAGTGGATTGATGTGATATCTCCACTGACGTAAGGGATGACG
CACAATCCCACTATCCTTCGCAAGACCCTTCCTCTATATAAGGAAGTTCATTTCATTTGGAGAGGACACGCTGACAAGCTGAC
TCTAGCAGATCCTCTAGAACCATCTTCCACACACTCAAGCCACACTATTGGAGAACACACAGGGACAACACACCATAAGATCC
AAGGGAGGCCTCCGCCGCCGCCGGTAACCACCCCGCCCCTCTCCTCTTTCTTTCTCCGTTTTTTTTTCCGTCTCGGTCTCGAT
CTTTGGCCTTGGTAGTTTGGGTGGGCGAGAGGCGGCTTCGTGCGCGCCCAGATCGGTGCGCGGGAGGGGCGGGATCTCGCGGC
TGGGGCTCTCGCCGGCGTGGATCCGGCCCGGATCTCGCGGGGAATGGGGCTCTCGGATGTAGATCTGCGATCCGCCGTTGTTG
GGGGAGATGATGGGGGGTTTAAAATTTCCGCCGTGCTAAACAAGATCAGGAAGAGGGGAAAAGGGCACTATGGTTTATATTTT
TATATATTTCTGCTGCTTCGTCAGGCTTAGATGTGCTAGATCTTTCTTTCTTCTTTTTGTGGGTAGAATTTGAATCCCTCAGC
ATTGTTCATCGGTAGTTTTTCTTTTCATGATTTGTGACAAATGCAGCCTCGTGCGGAGCTTTTTTGTAGGTAGAAGTGATCAA
CCTGATCAACCCCGCCATGGACAACAACCCAAACATCAACGAGTGCATCCCATACAACTGCCTGAGCAACCCAGAGGTGGAGG
TGCTGGGTGGCGAGCGCATCGAGACCGGTTACACCCCCATCGACATCTCCCTGTCCTTGACCCAGTTCCTGCTCAGCGAGTTC
GTGCCAGGTGCTGGCTTCGTGCTCGGCCTGGTGGACATCATCTGGGGTATCTTCGGTCCATCCCAATGGGACGCCTTCCTGGT
GCAAATCGAGCAGCTGATCAACCAGAGGATCGAAGAGTTCGCCAGGAACCAGGCCATCTCCAGGCTGGAGGGCCTGAGCAACC
TCTACCAAATCTACGCCGAGAGCTTCAGGGAGTGGGAGGCCGACCCGACCAACCCAGCTCTCCGCGAGGAAATGCGCATTCAA
TTCAACGACATGAACAGCGCCCTGACCACCGCTATCCCACTGTTCGCCGTCCAGAACTACCAAGTGCCGCTCCTGTCCGTGTA
CGTGCAAGCCGCTAACCTGCACCTCAGCGTGCTGCGCGACGTGAGCGTGTTCGGCCAAAGGTGGGGCTTCGATGCTGCCACCA
TCAACAGCCGCTACAACGACCTGACCAGGCTGATTGGCAACTACACCGACCACGCTGTGCGCTGGTACAACACCGGCCTGGAG
CGCGTCTGGGGTCCGGACTCCAGGGACTGGATCAGGTACAACCAGTTCAGGAGGGAGTTGACCCTCACCGTGCTGGACATTGT
GTCCCTCTTCCCGAACTACGACTCCAGGACCTACCCGATCCGCACCGTGTCCCAACTCACCAGGGAGATCTACACCAACCCAG
TGCTGGAGAACTTCGACGGTAGCTTCCGCGGTTCCGCCCAGGGTATCGAGGGCTCCATCAGGAGCCCACACCTGATGGACATC
CTGAACAGCATCACCATCTACACCGACGCTCACAGGGGCGAGTACTACTGGTCCGGCCACCAGATCATGGCCTCCCCAGTGGG
CTTCAGCGGCCCCGAGTTCACCTTCCCGCTCTACGGCACCATGGGCAACGCCGCTCCACAGCAACGCATCGTGGCTCAACTGG
GTCAGGGTGTCTACAGGACCCTGTCCTCCACCCTGTACAGGAGGCCCTTCAACATCGGTATCAACAACCAGCAACTGTCCGTG
CTCGACGGCACCGAGTTCGCCTACGGCACCTCCTCCAACCTGCCATCCGCTGTCTACAGGAAGAGCGGCACCGTGGACTCCCT
GGACGAGATCCCACCACAGAACAACAACGTGCCACCCAGGCAAGGCTTCTCCCACAGGCTGAGCCACGTGTCCATGTTCCGCT
CCGGCTTCAGCAACAGCTCCGTGAGCATCATCAGGGCTCCGATGTTCTCCTGGATCCACCGCAGCGCTGAGTTCAACAACATC
ATCGCCTCCGACAGCATCACCCAAATCCCGGCCGTGAAGGGCAACTTCCTCTTCAACGGTTCCGTCATTTCCGGCCCAGGCTT
CACCGGTGGCGACCTCGTGAGGCTCAACAGCAGCGGCAACAACATCCAGAACAGGGGCTACATCGAGTGCCAATCCACTTCCC
ATCCACCTCCACCAGGTACAGGGTGCGCGTGAGGTACGCTTCCGTGACCCCGATCCACCTCAACGTGAACTGGGGTAACTCCT
CCATCTTCTCCAACACCGTGCCAGCTACCGCTACCTCCCTGGACAACCTCCAATCCAGCGACTTCGGTTACTTCGAGAGCGCC
AACGCTTTCACCTCCTCCCTCGGTAACATCGTGGGCGTGAGGAACTTCAGCGGCACCGCCGGCGTGATCATCGACAGGTTCGA
GTTCATCCCAGTGACCGCCACCCTCGAGGCTGAGTGATCGATCGACAAGCTCGAGTTTCTCCATAATAATGTGTGAGTAGTTC
CCAGATAAGGGAATTAGGGTTCCTATAGGGTTTCGCTCATGTGTTGAGCATATAAGAAACCCTTAGTATGTATTTGTATTTGT
AAAATACTTCTATCAATAAAATTTCTAATTCCTAAAACCAAAATCCAGTACTAAAATCCAGATCCCCCGAATTAAGGTACCGA
TATCAGTACTAATTCAGTACATTAAAAACGTCCGCAATGTGTTATTAAGTTGTCTAAGCGTCAATTTGTGAAATATTAGCAAT
TTTGGTCATGTTAAGCTATGAATTAGTTTTTGGTATGAATAACTAAAGTGTAGTAATCTTCCTAAGATTTCTAGAAAGTCTAG
GATCAC

**>SEQ ID 38- Complementary sequence of SEQ ID NO 03 (reverse-complement)**

ACTAAAATCCAGATCCCCCGAATTAAGGTACCGATATCAGTACTAATTCAGTACATTAAAAACGTCCGCAATGTGTTATTAAG
TTGTCTAAGCGTCAATTTGTGAAATATTAGCAATTTTGGTCATGTTAAGCTATGAATTAGTTTTTGGTATGAATAACTAAAGT
GTAGTAATCTTCCTAAGATTTCTAGAAAGTCTAGGATCAC

**>SEQ ID 39- Complementary sequence of SEQ ID NO 04 (reverse-complement)**

ACTTAATAAGCTTTACATAAAGTTAAGGATCACTTTTGTTGTGTCTCTAGAACTCCAATTATGTATTTTCGAAGTTCTTGTCA
GTTTTCTGTCCAAATTGGTATCGGCGCGCCCTCGAGTCTAGAGTCGACGAGCTCGATCCACTAGTAACGGCCGCCAGTGTGCT
GGAATTCGCCCTTGGCGCGCCGATCTAGTAACATAGATGA

>SEQ ID NO 40 - Artificial sequence
GAGGTGCCAATCCACTTCCC
**>SEQ ID NO 41 - Artificial sequence**
GAGTTACCCCAGTTCACGTTGAG
**>SEQ ID NO 42 - Artificial sequence**
ACCTCCACCAGGTACAG
**>SEQ ID NO 43 - Artificial sequence**
CATCCCATACAACTGCCTGAG
**>SEQ ID NO 44 - Artificial sequence**
CTGGGTCAAGGACAGGGAGAT
**>SEQ ID NO 45 - Artificial sequence**
CCGGTTACACCCCC
**>SEQ ID NO 46 - Artificial sequence**
CTCTACCAAATCTACGCCGAGA
**>SEQ ID NO 47 - Artificial sequence**
CATGTCGTTGAATTGAATGCG
**>SEQ ID NO 48 - Artificial sequence**
TCTCCGCGAGGAAA
**>SEQ ID NO 49 - Artificial sequence**
CACCGTGCTGGACATTGTGT
**>SEQ ID NO 50 - Artificial sequence**
TGAGTTGGGACACGGTGC
**>SEQ ID NO 51 - Artificial sequence**
CTCTTCCCGAACTACGACT
**>SEQ ID NO 52 - Artificial sequence**
ACCCTGTCCTCCACCCTGTA
**>SEQ ID NO 53** - **Artificial sequence**
AGGTTGGAGGAGGTGCCGTA
**>SEQ ID NO 54** - **Artificial sequence**
CCTTCAACATCGGTATCA
**>SEQ ID NO 55** - **Artificial sequence**
TGCCGAATATCATGGTGGAA
**>SEQ ID NO 56** - **Artificial sequence**
CGGCCACAGTCGATGAATC
**>SEQ ID NO 57** - **Artificial sequence**
TGGCCGCTTTTCT
**>SEQ ID NO 58** - **Artificial sequence**
ATGACTGGGCACAACAGACAATC
**>SEQ ID NO 59** - **Artificial sequence**
CGGACAGGTCGGTCTTGA
**>SEQ ID NO 60** - **Artificial sequence**
CTGCTCTGATGCCGC
**>SEQ ID NO 61** - **Artificial sequence**
CTGCCGAGAAAGTATCCATCATG
**>SEQ ID NO 62** - **Artificial sequence**
GATGTTTCGCTTGGTGGTCG
**>SEQ ID NO 63** - **Artificial sequence**
CTGATGCAATGCGGCGGC
**>SEQ ID NO 64** - **Artificial sequence**
GCCGCTTGTAGCCTTCCA
**>SEQ ID NO 65** - **Artificial sequence**
CCGCCGACCTGGTGGA

**>SEQ ID NO 66** - **Artificial sequence**
CGTGACCTCAATGCG
**>SEQ ID NO 67** - **Artificial sequence**
CACAATCGTCACCTCAACCG
**>SEQ ID NO 68** - **Artificial sequence**
ATCAACGACCTTCTGGAAACG
**>SEQ ID NO 69 - Artificial sequence**
CGCAGGATTTCGCTCTCG
**>SEQ ID NO 70 - Artificial sequence**
ACGGAACCGTTGAAGAGGAA
**>SEQ ID NO 71 - Artificial sequence**
ATGTGTGAGTAGTTCCCAGATAAG
**>SEQ ID NO 72 - Artificial sequence**
GATCCAGGAGAACATCGGAG
**>SEQ ID NO 73 - Artificial sequence**
CTATAGGGTTTCGCTCATGTGTTG
**>SEQ ID NO 74 - Artificial sequence**
aattatacatttaatacgcg
**>SEQ ID NO 75 - Artificial sequence**
cgcggtgtcatctatgttac
**>SEQ ID NO 76 - Artificial sequence**
TCAAGAAGGCGATAGAAGGCGATG
**>SEQ ID NO 77 - Artificial sequence**
aataacgtcatgcattacatg
**>SEQ ID NO 78 - Artificial sequence**
gataatcatcgcaagaccgg
**>SEQ ID NO 79 - Artificial sequence**
tcgtcgactctagactcgag
**>SEQ ID NO 80 - Artificial sequence**
AACTTTATTCTAGGATAAAGCTATGT
**>SEQ ID NO 81 - Artificial sequence**
CAGATAAGGGAATTAGGGTTCCTAT
**>SEQ ID NO 82 - Artificial sequence**
ACATACAACATCACAATCAAGCAGT
**>SEQ ID NO 83 - Artificial sequence**
AGAGTCCTGTTGTCAAAATACTCAA
**>SEQ ID NO 84 - Artificial sequence**
CTCAAAACAACGCTAGATACGGTAG
**>SEQ ID NO 85** - **Artificial sequence**
GCATCTTCATGATTGTTGCCTTATG
**>SEQ ID NO 86** - **Artificial sequence**
ATGATTAGAGTCCCGCAATTATACA
**>SEQ ID NO 87** - **Artificial sequence**
CTTGAATAGGAAATGCTTGAGAAAA
**>SEQ ID NO 88** - **Artificial sequence**
CTTGAGTGTGTGGAAGATGGTTCTA
**>SEQ ID NO 89** - **Artificial sequence**
GATCTGAATGTTTGGTTTGTTTGAC
**>SEQ ID NO 90** - **Artificial sequence**
GTTTGTCCTTGTTAGCCGTAGAAT
**>SEQ ID NO 91** - **Artificial sequence**
TGATGCATATACAGAGATGCTTTTT
**>SEQ ID NO 92** - **Artificial sequence**
TTCATCCATTTTATTAGTACATCCA
**>SEQ ID NO 93** - **Artificial sequence**
ACGGATGCGACCTGTACG
**>SEQ ID NO 94** - **Artificial sequence**
TCAGTAAAACCCACATCAAC

**>SEQ ID NO 95** - **Artificial sequence**
GACCACATCATCACAACCAAG
**>SEQ ID NO 96** - **Artificial sequence**
GCTCCGAACAACACGAGGTTG
**>SEQ ID NO 97** - **Artificial sequence**
ATGAAGTATTATATAGGTGAAG
**>SEQ ID NO 98** - **Artificial sequence**
AGAGGCTATTCGGCTATGAC
**>SEQ ID NO 99** - **Artificial sequence**
CAGCCGAACTGTTCGCCAGG
**>SEQ ID NO 100 -Artificial sequence**
AGCACGAGGAAGCGGTCAGC
**>SEQ ID NO 101 -Artificial sequence**
TGAGATGACAGGAGATCCTG
**>SEQ ID NO 102 -Artificial sequence**
CAACAGCTCCGTGAGCATCATC
**>SEQ ID NO 103 -Artificial sequence**
CCAGCGACTTCGGTTACTTC
**>SEQ ID NO 104 -Artificial sequence**
CTCTCGGCGTAGATTTGGTAG
**>SEQ ID NO 105 -Artificial sequence**
CAACAACCCAAACATCAACG
**>SEQ ID NO 106 -Artificial sequence**
GGGTCCTGTAGACACCCTGA
**>SEQ ID NO 107 -Artificial sequence**
GCTCACCCTGTTGTTTGGTGTT
**>SEQ ID NO 108 -Artificial sequence**
CGGCCACAGTCGATGAATC
**>SEQ ID NO 109 -Artificial sequence**
TGAAAAGAAAAACTACCGATGAA
**>SEQ ID NO 110 -Artificial sequence**
TGATGTGATGGTCCGATTGA
**>SEQ ID NO 111 -Artificial sequence**
TCCCCTCGGGATCAAAGTA
**>SEQ ID NO 112 -Artificial sequence**
TAGCGTGTTTGTGCTTTTGC
**>SEQ ID NO 113 -Artificial sequence**
GGCCGCTGAAATTAAATCAA
**>SEQ ID NO 114 -Artificial sequence**
GAGTCAGTGAGCGAGGAAGC
**>SEQ ID NO 115 -Artificial sequence**
CGGTGAAAACCTCTGACACA
**>SEQ ID NO 116 -Artificial sequence**
ATACAGGCAGCCCATCAGTC

## Claims

1. A Polynucleotide comprising at least 14 contiguous nucleotides of SEQ ID NO: 18.

2. The polynucleotide of claim 1, comprising at least 15 contiguous nucleotides of SEQ ID NO: 18, preferably at least 16 contiguous nucleotides of SEQ ID NO: 18, wherein preferably the polynucleotide comprises SEQ ID NO: 18, and wherein more preferably the polynucleotide comprises SEQ ID NO: 13, 5 or 22.

3. A Polynucleotide, comprising at least 14 contiguous nucleotides of SEQ ID NO: 19.

4. The polynucleotide of claim 3 comprising at least 15 contiguous nucleotides of SEQ ID NO: 19, preferably comprising at least 16 contiguous nucleotides of SEQ ID NO: 19, wherein preferably the polynucleotide comprises SEQ ID NO: 19, and more preferably wherein the polynucleotide comprises SEQ ID NO: 12, 5 or 22.

5. Primer pairs comprising forward and reverse primers wherein the forward primer comprises SEQ ID NO: 6 and the reverse primer comprises SEQ ID NO: 7, or the forward primer comprises SEQ ID NO: 8 and the reverse primer comprises SEQ ID NO: 9.

6. A method of detecting plant material from genetically modified sugarcane of event CTC75064-3, comprising the steps of:

   a) obtaining a plant material sample for analysis;
   b) extracting DNA from the sample;
   c) providing primer pairs comprising at least a forward and a reverse primer;
   d) amplifying a region between the primer pair; and
   e) detecting the presence of a product from amplification;
   or comprising the steps of

      (a) obtaining a plant material sample for analysis;
      (b) extracting DNA or RNA from the sample;
      (c) providing a probe or a combination of probes designed to bind to a polynucleotide comprising contiguous nucleotides of sequences selected from the group consisting of SEQ ID NO 2, SEQ ID NO 3, SEQ ID NO 4, SEQ ID NO 5, SEQ ID NO 12, SEQ ID NO 13, SEQ ID NO: 18, SEQ ID NO: 19, SEQ ID NO 22, SEQ ID NO 23, SEQ ID NO 24, SEQ ID NO 29, SEQ ID NO 30, SEQ ID NO 31, SEQ ID NO: 32, SEQ ID NO: 33, SEQ ID NO 36, SEQ ID NO 37, SEQ ID NO 38 and SEQ ID NO 39;
      (d) hybridizing said probe with the sample; and
      (e) detecting the actual hybridization of the probe.

7. The method of claim 6, wherein the primer pairs in step c) are designed to bind to a polynucleotide comprising contiguous nucleotides of sequences selected from the group consisting of SEQ ID NO: 22 and SEQ ID NO: 29, wherein at least one pair of primers comprises contiguous nucleotides sequences selected from the group consisting of SEQ ID NO: 23, SEQ ID NO: 24, SEQ ID NO: 30 and SEQ ID NO: 31 and optionally at least one primer pair consists of a first primer comprising contiguous nucleotides of sequences selected from the group consisting of SEQ ID NO: 23, SEQ ID NO: 24, SEQ ID NO: 30 and SEQ ID NO: 31 and a second primer comprising contiguous nucleotides of sequences selected from the group consisting of SEQ ID NO: 2 and SEQ ID NO: 36; or wherein the primer pairs in step c) are designed to bind to a polynucleotide comprising contiguous nucleotides of sequences selected from the group consisting of SEQ ID NO: 5 and SEQ ID NO: 37, wherein at least one pair of primers comprises contiguous nucleotides of sequences selected from the group consisting of SEQ ID NO: 3, SEQ ID NO: 4, SEQ ID NO: 38 and SEQ ID NO: 39; and optionally at least one primer pair consists of a first primer comprising contiguous nucleotides of sequences selected from the group consisting of SEQ ID NO: 3, SEQ ID NO: 4, SEQ ID NO: 38 and SEQ ID NO: 39 and a second primer comprising contiguous nucleotides of sequences selected from the group consisting of SEQ ID NO: 2 and SEQ ID NO: 36; wherein more preferably the forward primer comprises SEQ ID NO: 6 and the reverse primer comprises SEQ ID NO: 7, or the forward primer comprises SEQ ID NO: 8 and the reverse primer comprises SEQ ID NO: 9.; and wherein preferably the product from amplification comprises SEQ ID NO: 12 or SEQ ID NO: 13, wherein optionally detection of the product from amplification is performed through hybridization of a probe comprising SEQ ID NO: 10 or SEQ ID NO: 11.

8. A kit for detecting material from transgenic sugarcane comprising a Cry1Ac protein (event CTC75064-3) comprising a means to detect the presence of a polynucleotide comprising, at least, 14 contiguous nucleotides of SEQ ID NO: 18 and/or of SEQ ID NO: 19 and/or a pesticidal crystal protein (Cry), wherein preferably the means comprise primer pairs designed to bind to a polynucleotide comprising contiguous nucleotides of sequences selected from the group consisting of SEQ ID NO: 22 and SEQ ID NO: 29, wherein at least one pair of primers comprises contiguous nucleotides sequences selected from the group consisting of SEQ ID NO: 23, SEQ ID NO: 24, SEQ ID NO: 30 and SEQ ID NO: 31, and wherein preferably the means comprise a probe comprising SEQ ID NO: 10 or SEQ ID NO: 11.

9. Genetic construct comprising SEQ ID NO: 1 or SEQ ID NO:2, preferably comprising SEQ ID NO: 14.

10. A genetically modified sugarcane (Saccharum spp.) plant or a plant part, plant cell, plant tissue, or seed thereof comprising SEQ ID NO: 18 or SEQ ID NO: 19; or comprising SEQ ID NO: 12 or SEQ ID NO: 13; or comprising SEQ ID NO: 5 or SEQ ID NO: 22, wherein the plant is insect-resistant.

11. A tissue culture of the genetically modified sugarcane (Saccharum spp.) plant of claim 10; and optionally a genetically modified sugarcane (Saccharum spp.) plant regenerated therefrom, wherein the regenerate plant comprises SEQ ID NO: 18 or SEQ ID NO: 19.

12. A commodity product produced from the genetically modified sugarcane (Saccharum spp.) plant of claim 10.

**13.** A method of producing a genetically modified sugarcane (Saccharum spp.) plant of event CTC75064-3, comprising the steps of:

a) introducing a genetic construct comprising SEQ ID NO: 20 and SEQ ID NO: 21 into an Agrobacterium strain;
b) obtaining embryogenic callus from immature leaf rolls or top stalks of sugarcane (Saccharum spp.);
c) co-cultivating embryogenic callus with a culture of Agrobacterium;
d) selecting transformed cells containing the functional fragment in culture medium containing aminoglycoside antibiotics; and
e) regenerating transformed sugarcane plants, wherein the genetically modified sugarcane plants comprise SEQ ID NO: 20 and SEQ ID NO: 21.

**14.** A method of making a genetically modified sugarcane (Saccharum spp.) plant of event CTC75064-3, comprising introducing a genetic modification to a sugarcane (Saccharum spp.) plant comprising SEQ ID NO: 5 or SEQ ID NO: 22 to produce a genetically modified sugarcane (Saccharum spp.) plant of event CTC75064-3, wherein the genetically modified sugarcane (Saccharum spp.) plant has improved insect resistance as compared to a sugarcane (Saccharum spp.) plant without the genetic modification.

**15.** A method of cultivating a genetically modified sugarcane (Saccharum spp.) plant of event CTC75064-3, comprising growing a genetically modified sugarcane (Saccharum spp.) plant of event CTC75064-3 comprising SEQ ID NO: 5 or SEQ ID NO: 22 under conditions comprising insect infestation, wherein the genetically modified sugarcane (Saccharum spp.) plant has an increase in insect resistance as compared to a sugarcane (Saccharum spp.) plant without the genetic modification grown under the same conditions

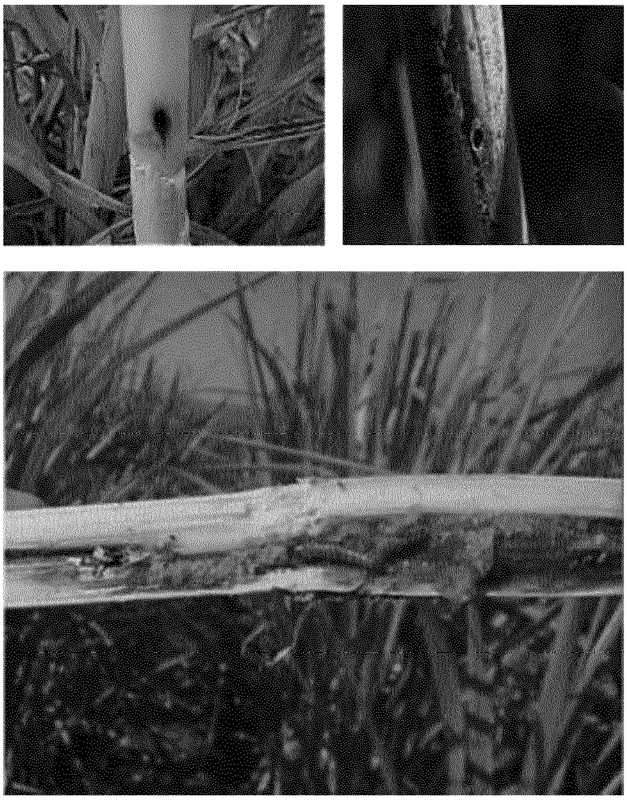

Figure 1

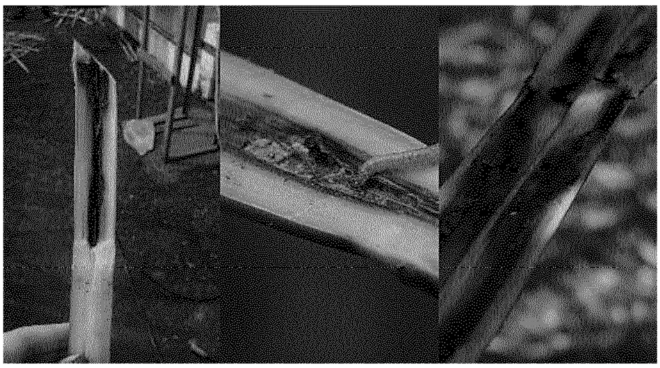

Figure 2

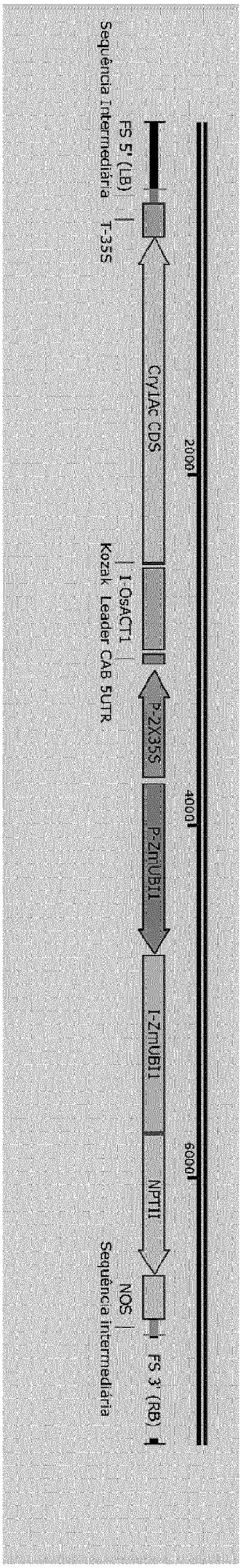
Sequência Intermediária
FS 5' (LB)
T-35S
Cry1Ac CDS
2000
I-OsACT1
Kozak
Leader CAB 5UTR
P-2X35S
4000
P-ZmUBI1
I-ZmUBI1
6000
NPTII
NOS
Sequência intermediária
FS 3' (RB)

Figure 3

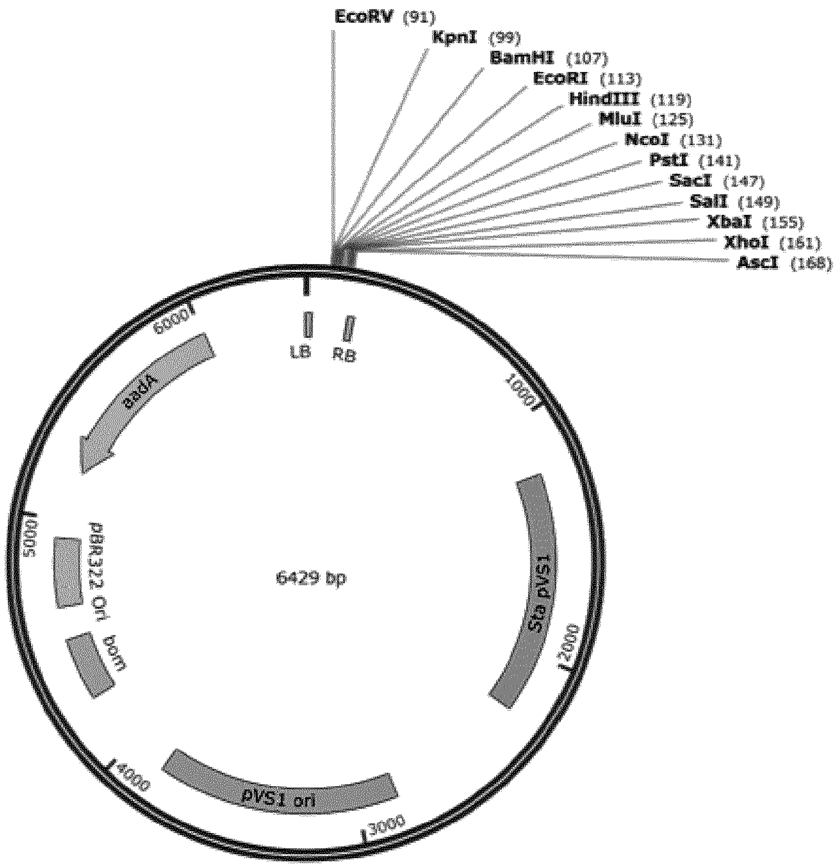


Figure 4

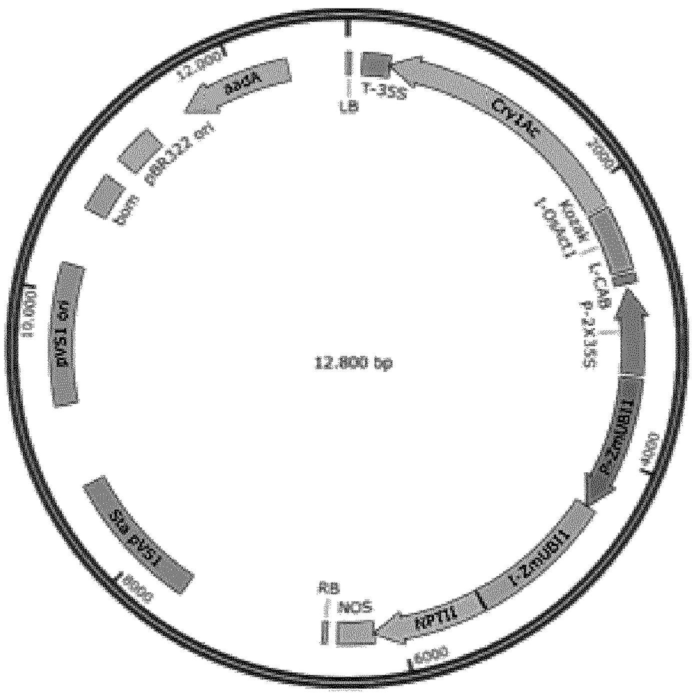


Figure 5

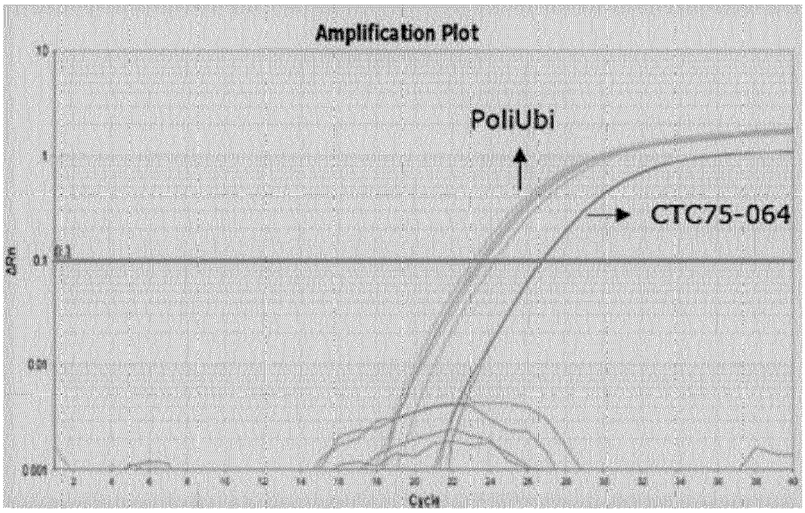


Figure 6

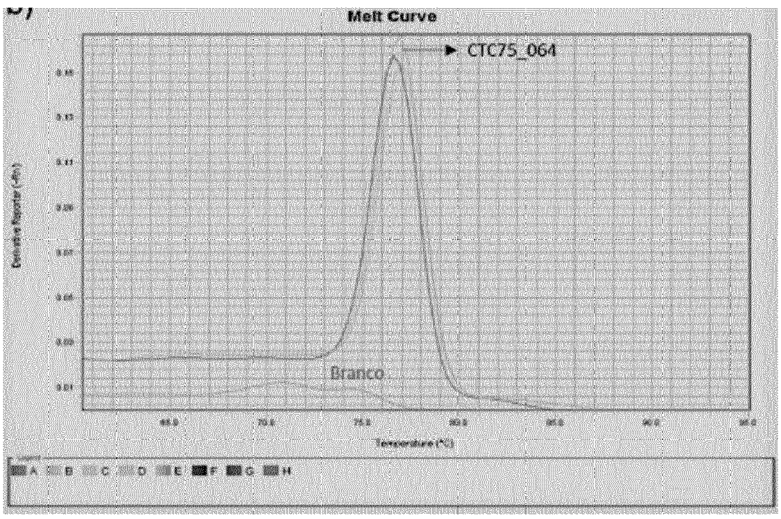


Figure 7

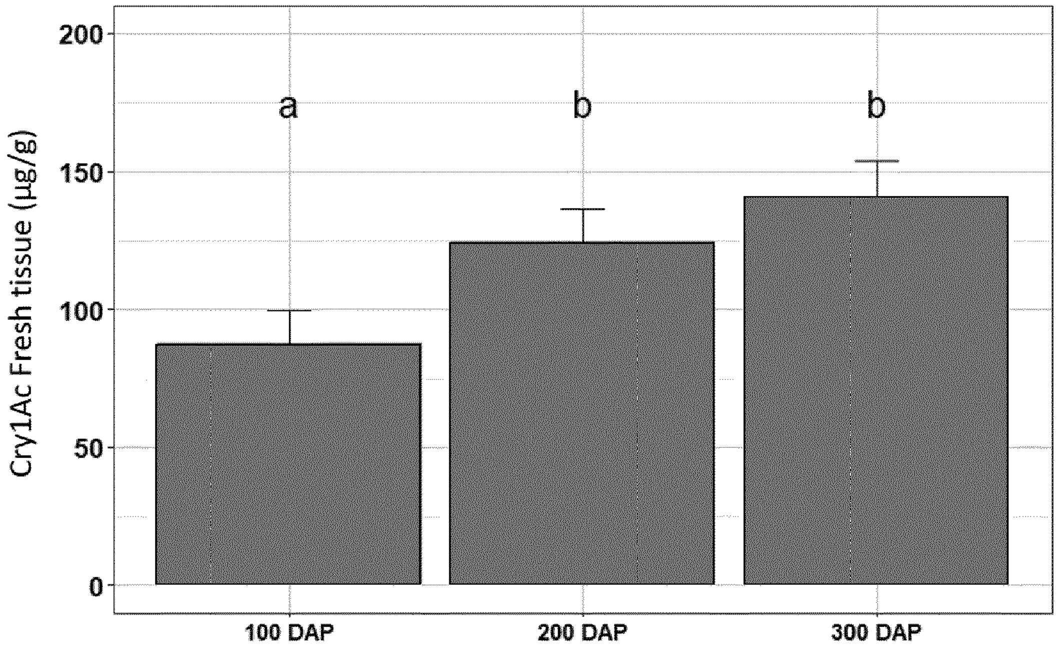


Figure 8

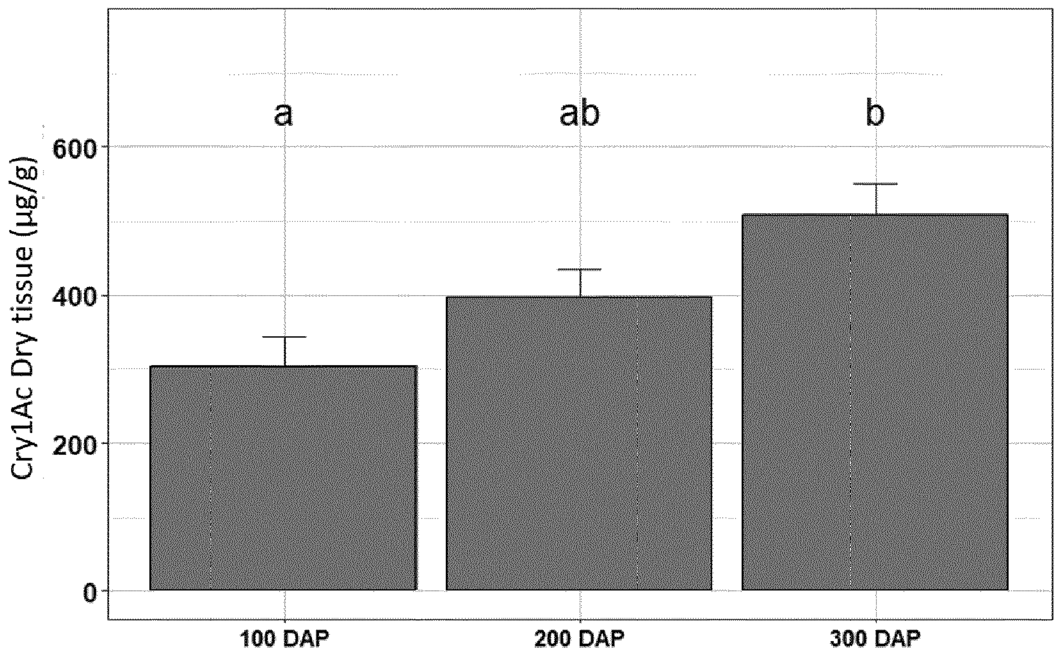


Figure 9

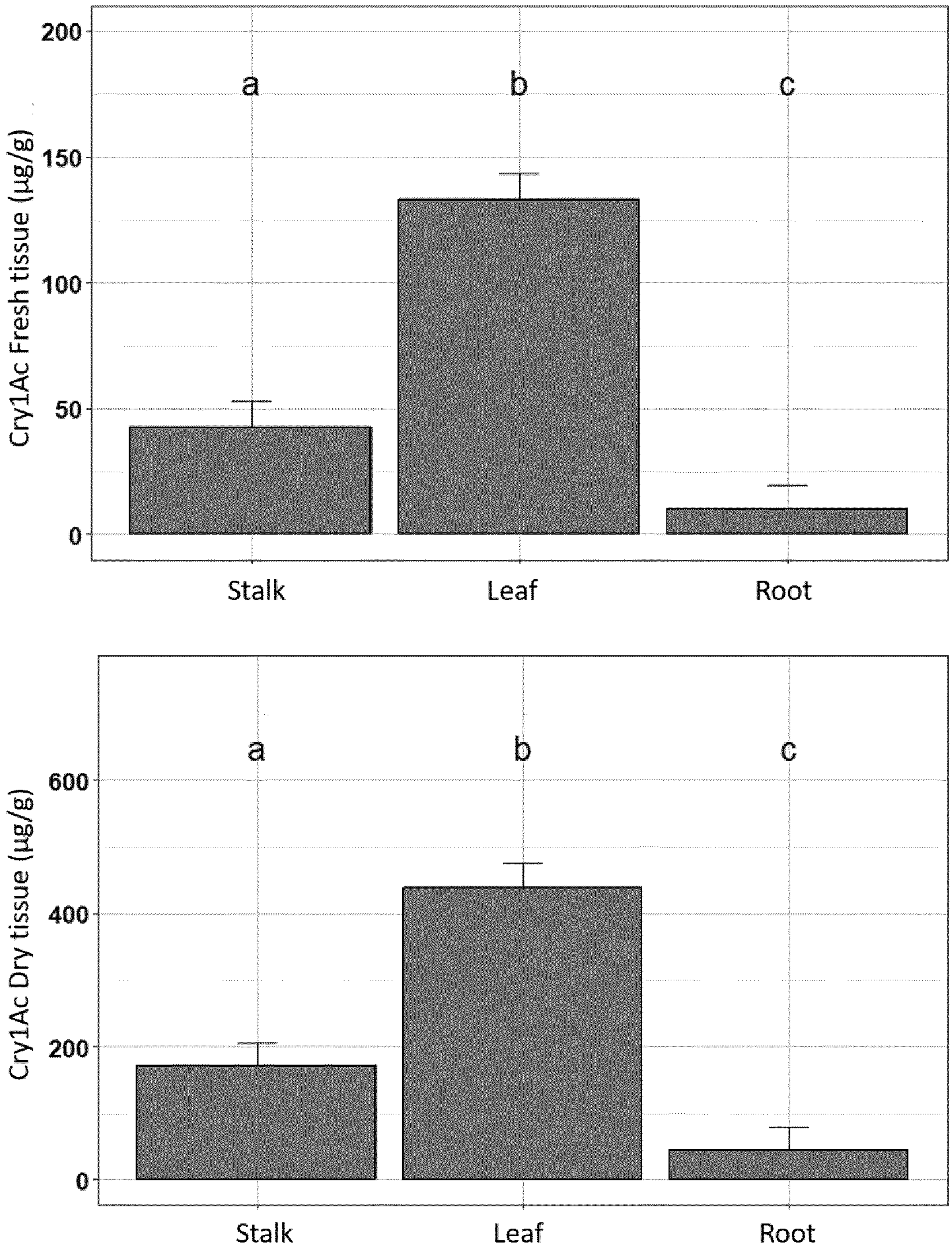
200
150
100
50
0
Cry1Ac Fresh tissue (µg/g)
a
b
c
Stalk
Leaf
Root
600
400
200
0
Cry1Ac Dry tissue (µg/g)
a
b
c
Stalk
Leaf
Root

Figure 10

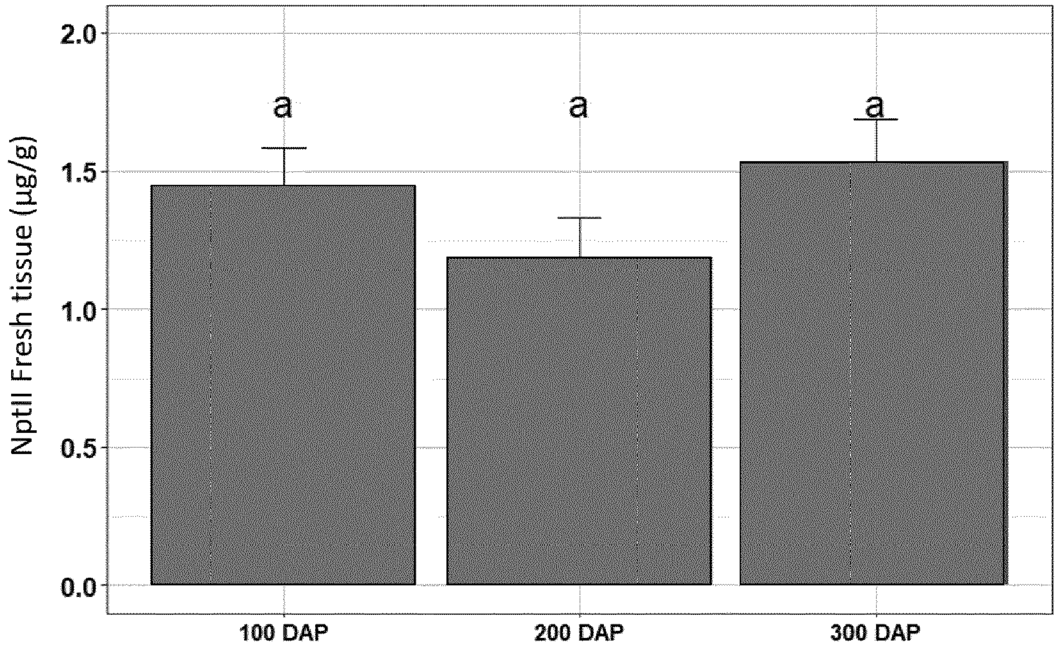


Figure 11

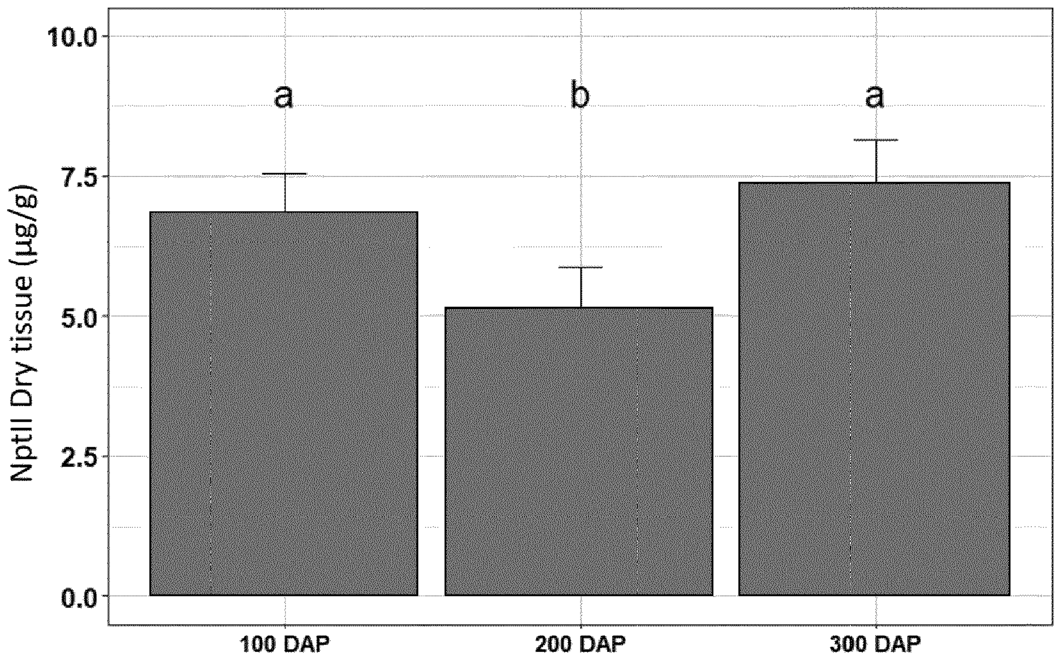


Figure 12

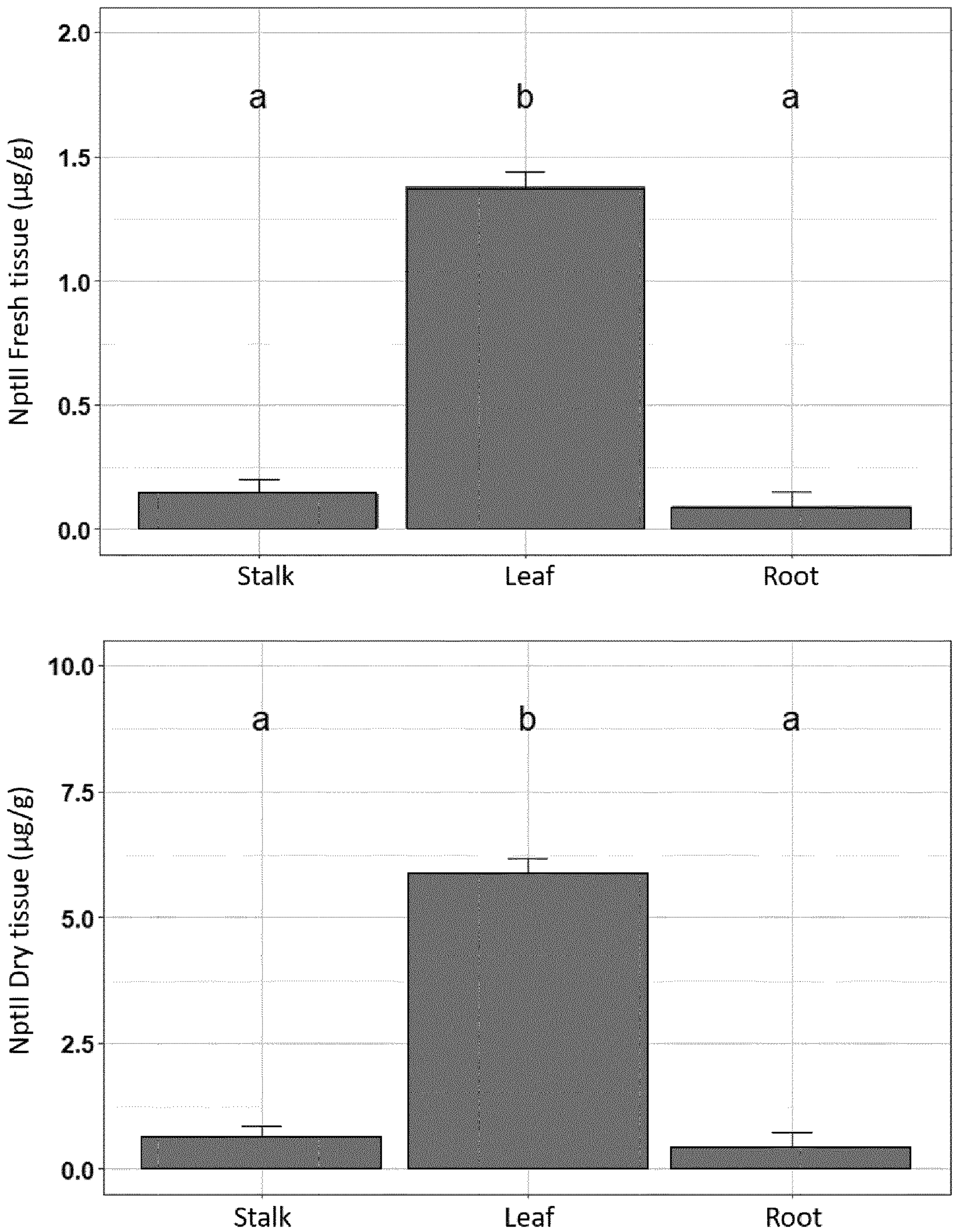
2.0
1.5
1.0
0.5
0.0
NptII Fresh tissue (µg/g)
a
b
a
Stalk
Leaf
Root
10.0
7.5
5.0
2.5
0.0
NptII Dry tissue (µg/g)
a
b
a
Stalk
Leaf
Root

Figure 13

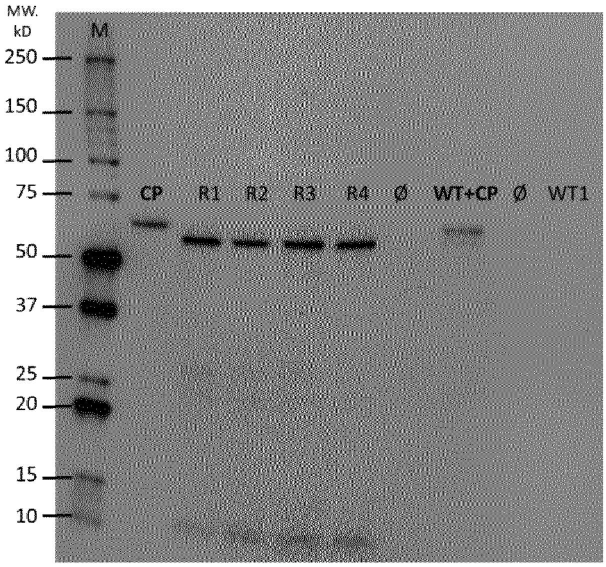


Figure 14

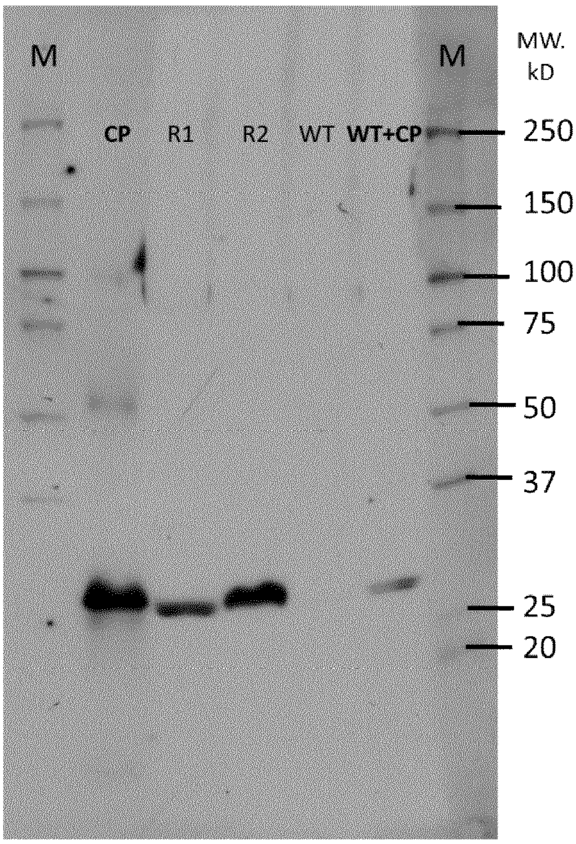


Figure 15

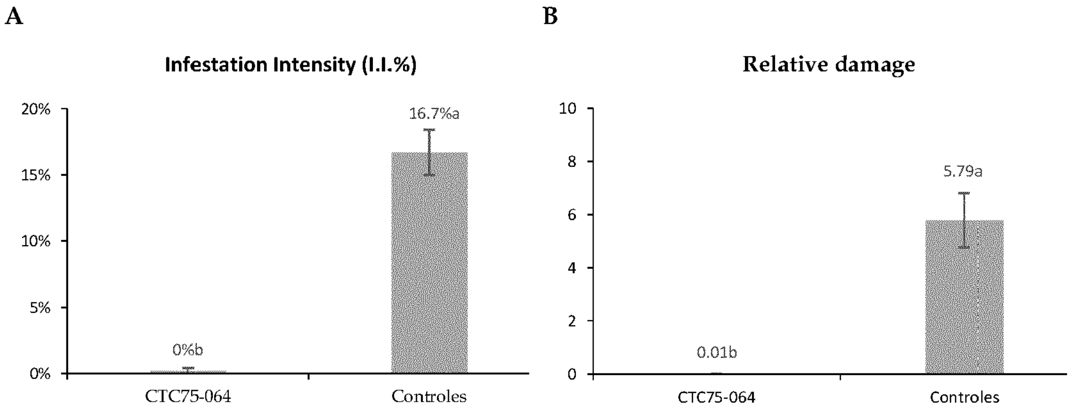


Figure 16

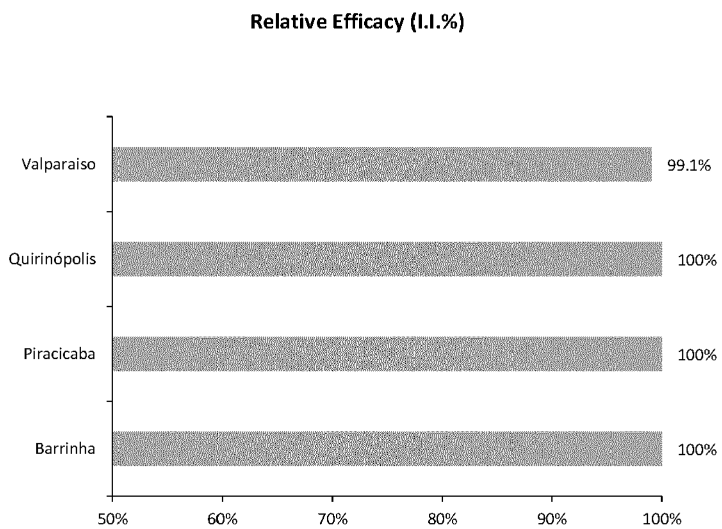


Figure 17

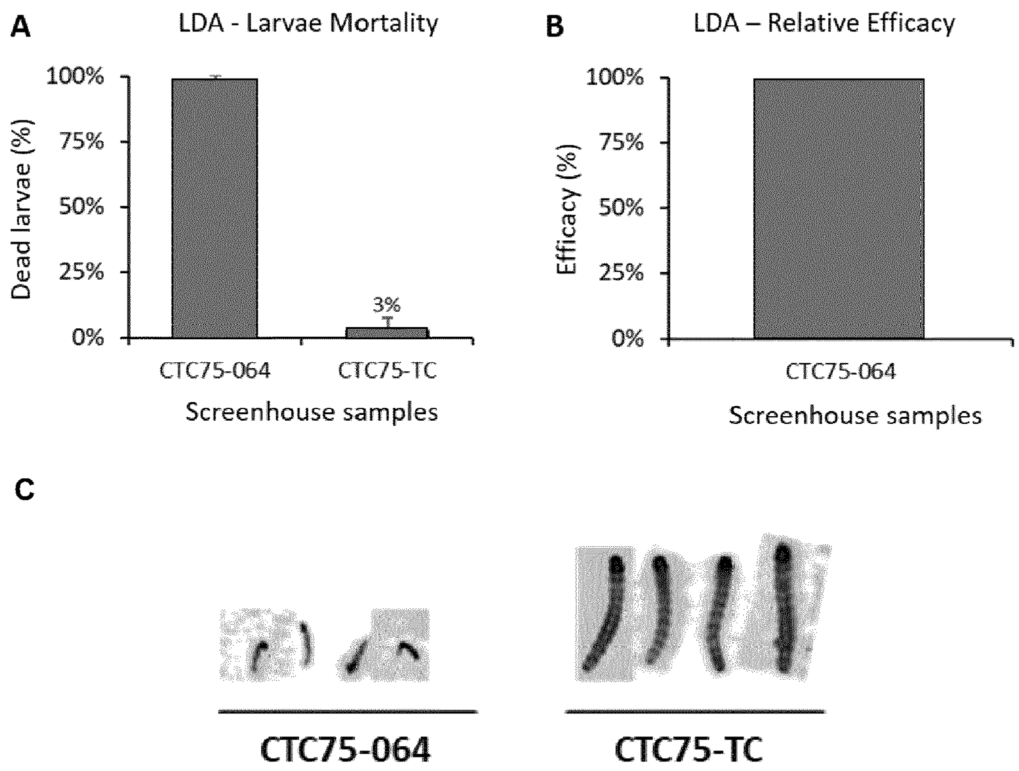


Figure 18

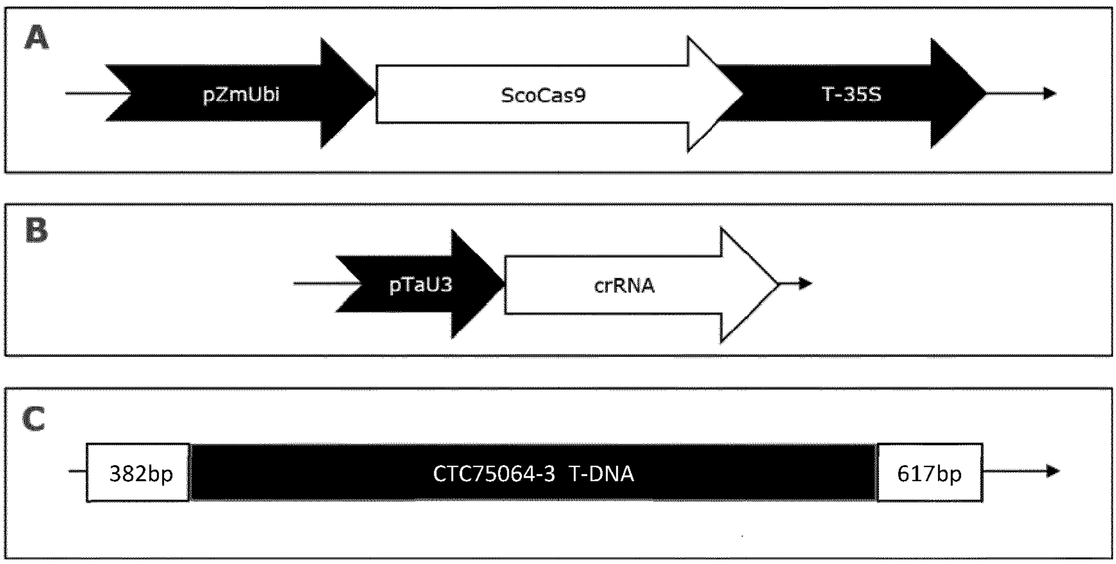


Figure 19

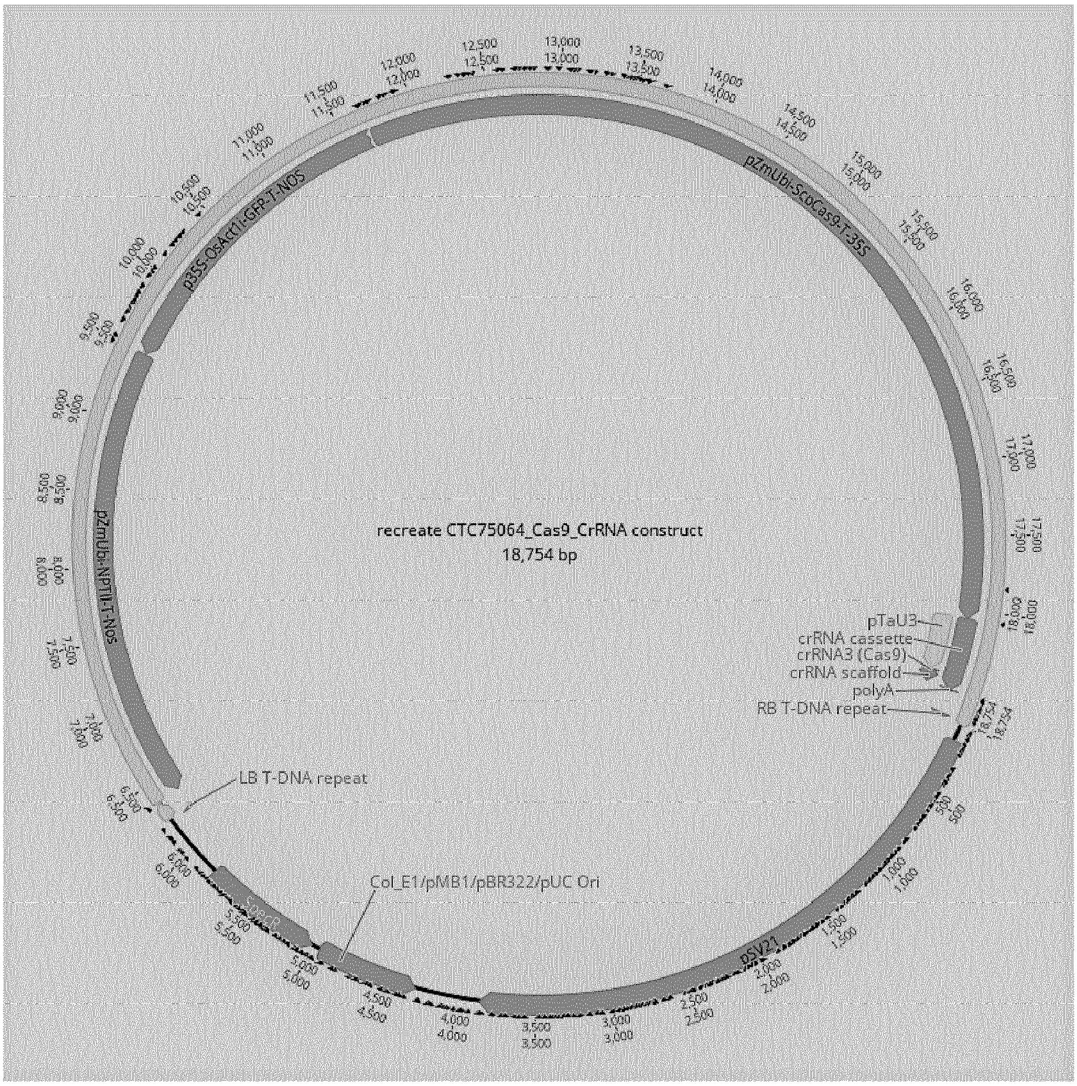
recreate CTC75064_Cas9_CrRNA construct
18,754 bp
pZmUbi-ScoCas9-T-35S
p35S-OsAct(1)-GFP-T-NOS
pZmUbi-NPTII-T-Nos
pTaU3
crRNA cassette
crRNA3 (Cas9)
crRNA scaffold
polyA
RB T-DNA repeat
LB T-DNA repeat
Col_E1/pMB1/pBR322/pUC Ori
pSV21

Figure 20

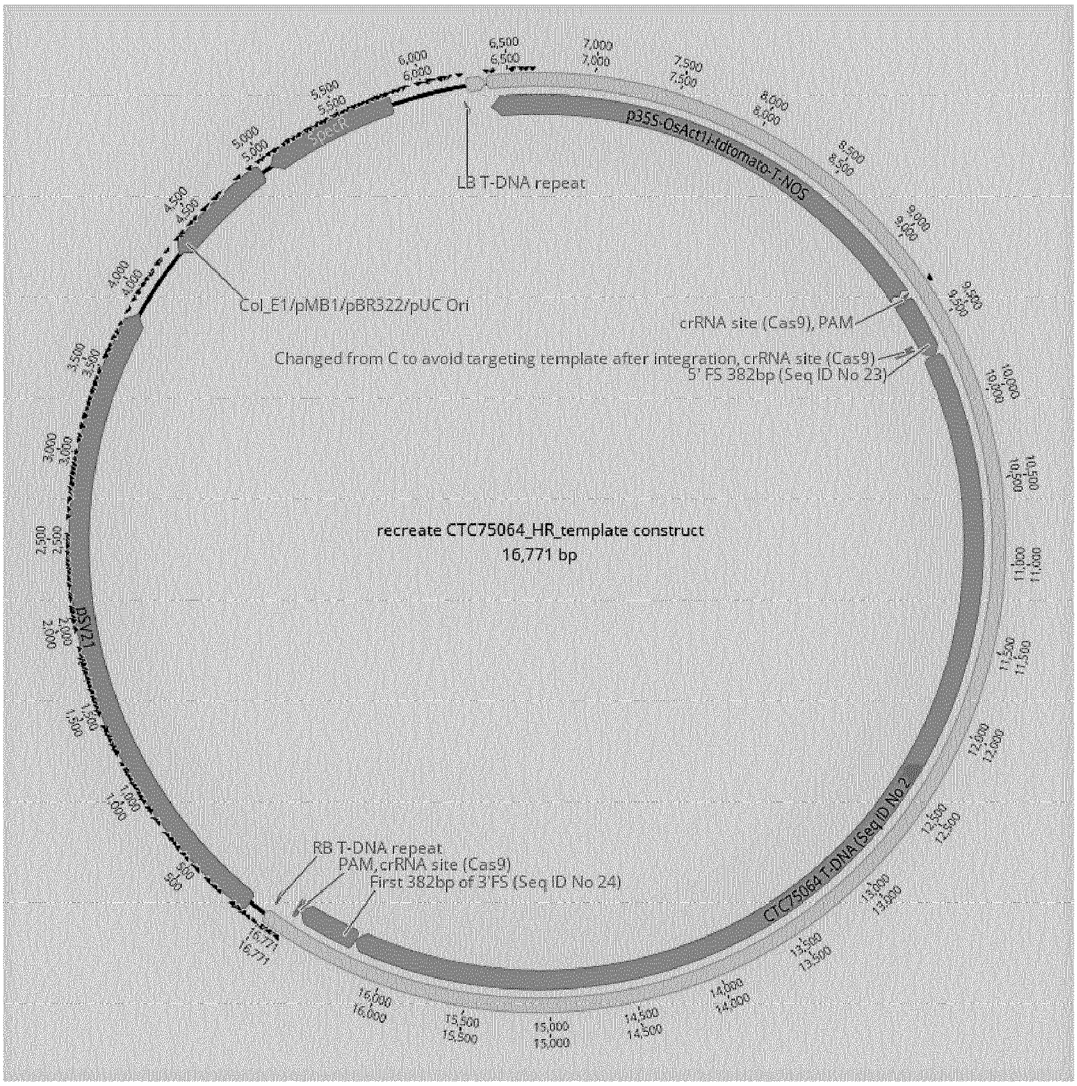
recreate CTC75064_HR_template construct
16,771 bp
p35S-OsAct1I-tdtomato-T-NOS
LB T-DNA repeat
SpecR
Col_E1/pMB1/pBR322/pUC Ori
crRNA site (Cas9), PAM
Changed from C to avoid targeting template after integration, crRNA site (Cas9)
5' FS 382bp (Seq ID No 23)
DSV21
RB T-DNA repeat
PAM, crRNA site (Cas9)
First 382bp of 3'FS (Seq ID No 24)
CTC75064 T-DNA (Seq ID No 2)

Figure 21

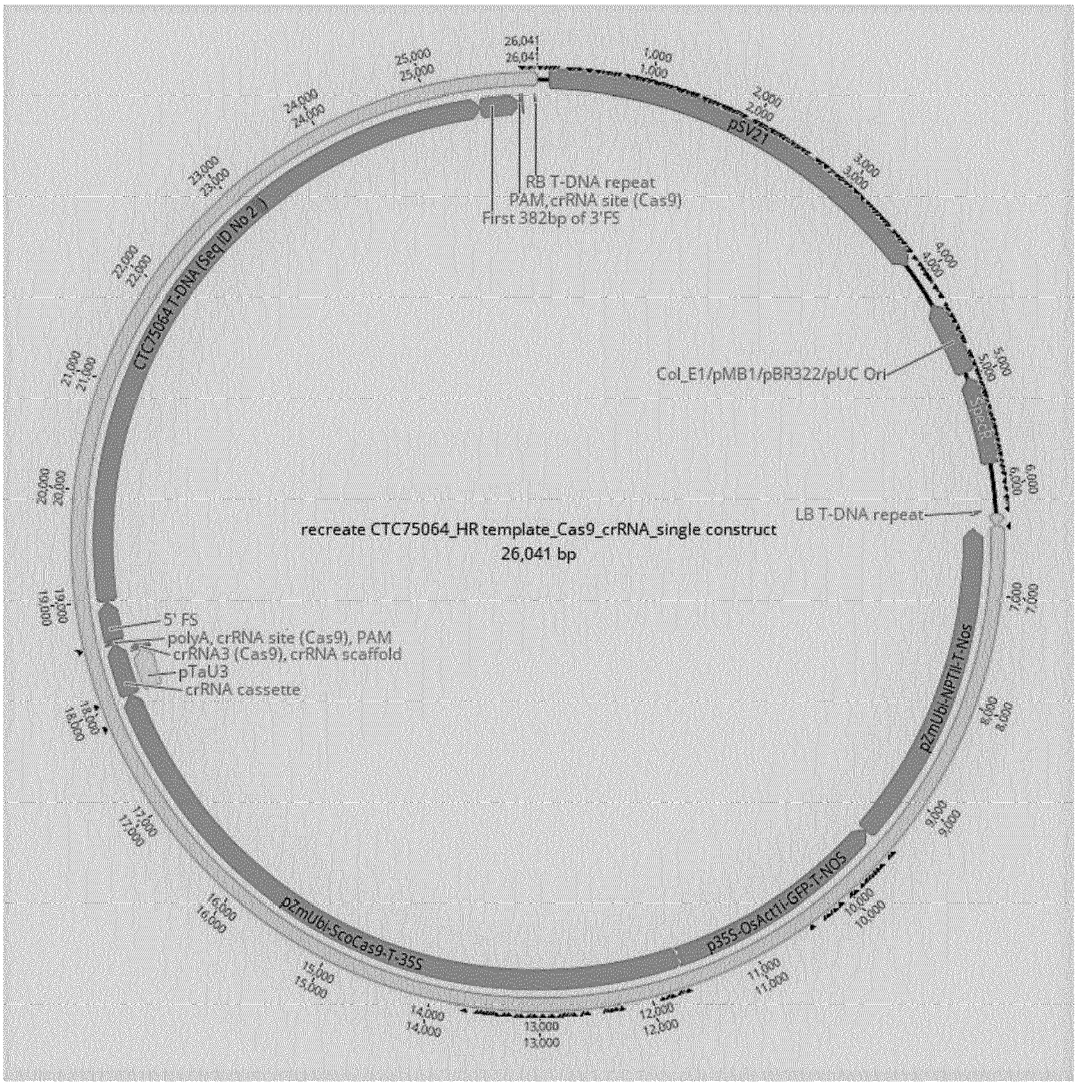
recreate CTC75064_HR template_Cas9_crRNA_single construct
26,041 bp
pSV21
Col_E1/pMB1/pBR322/pUC Ori
LB T-DNA repeat
pZmUbi-NPTII-T-Nos
p35S-OsAct1I-GFP-T-NOS
pZmUbi-ScoCas9-T-35S
crRNA cassette
pTaU3
crRNA3 (Cas9), crRNA scaffold
polyA, crRNA site (Cas9), PAM
5' FS
CTC75064 T-DNA (Seq ID No 2 )
First 382bp of 3'FS
PAM,crRNA site (Cas9)
RB T-DNA repeat

Figure 22

A)

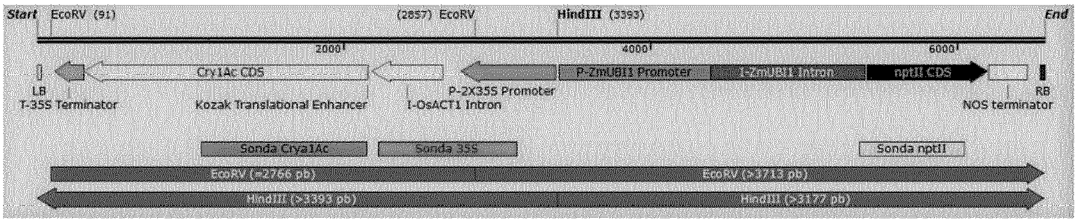


B)

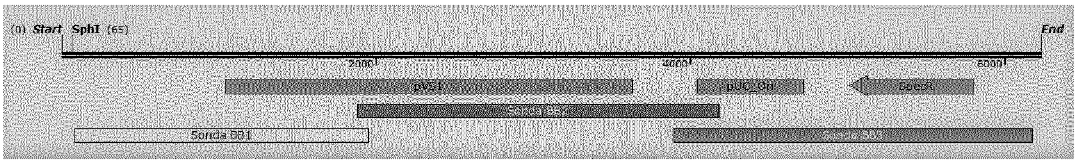


Figure 23

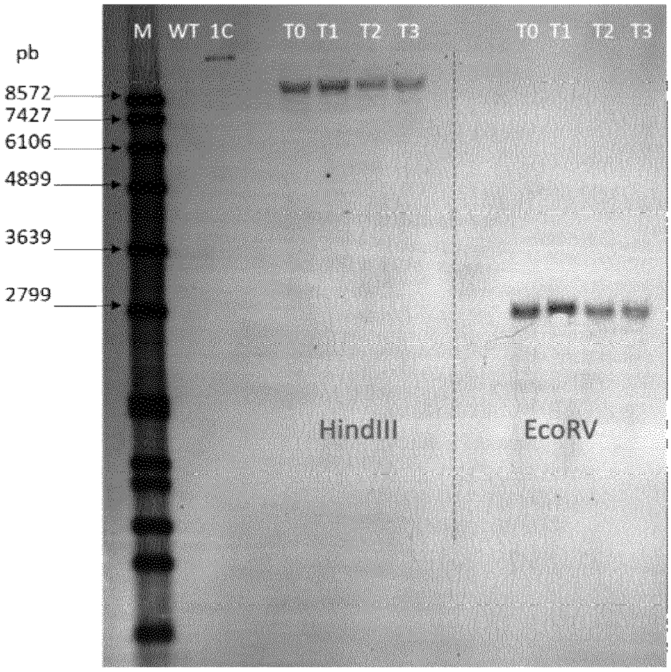


Figure 24

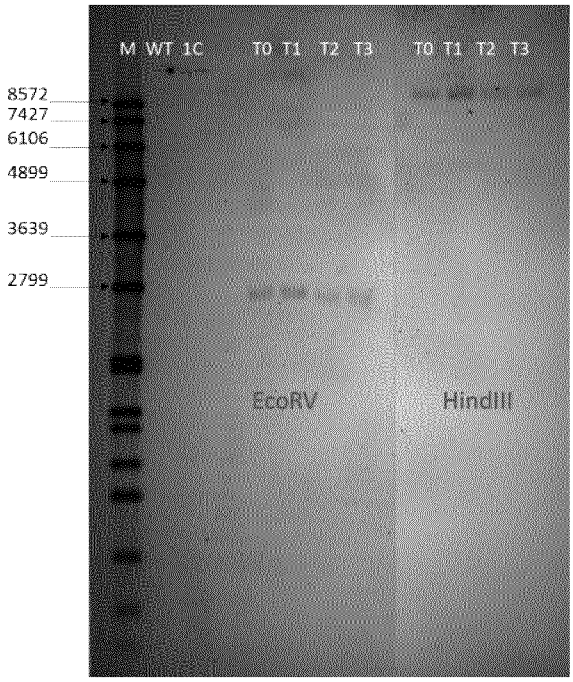


Figure 25

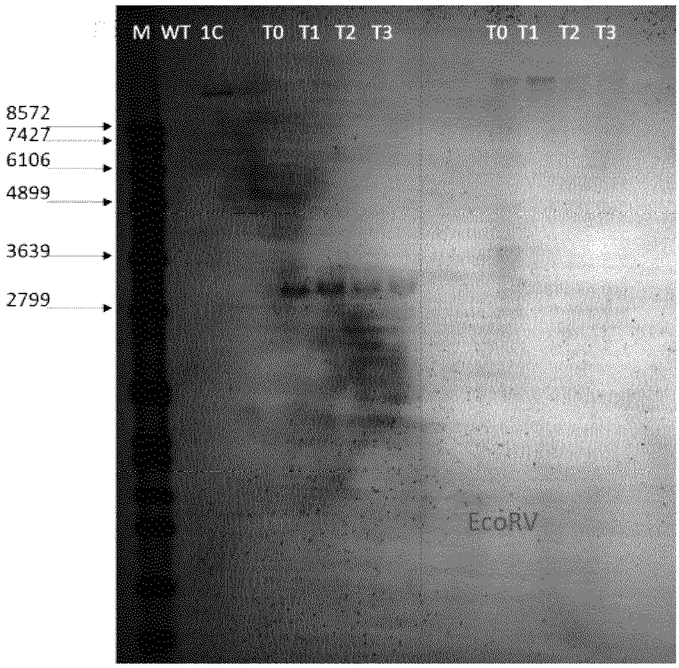


Figure 26

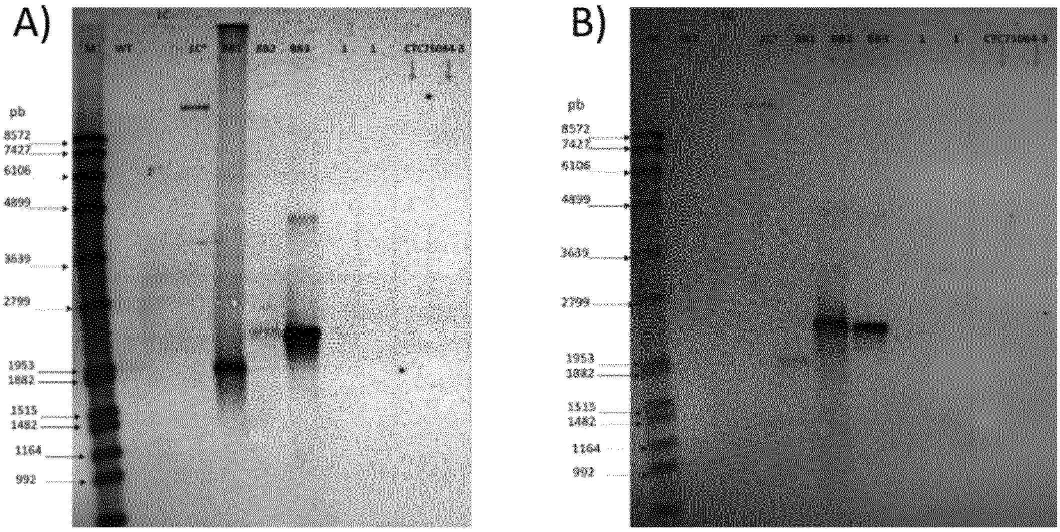


Figure 27

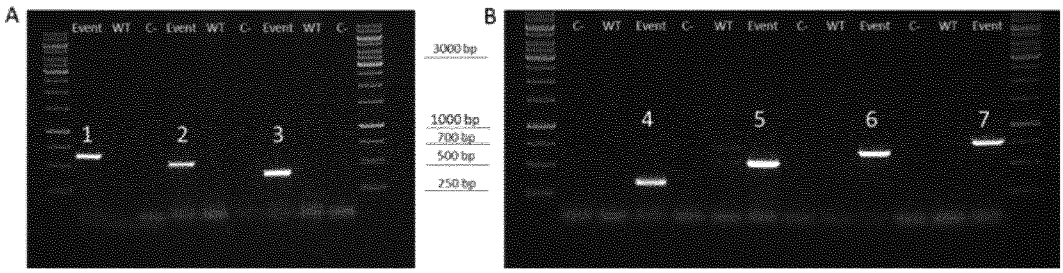


Figure 28

Europäisches Patentamt
European Patent Office
Office européen des brevets

# EUROPEAN SEARCH REPORT

Application Number
EP 20 20 6809

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br><br><br>Y | GAO SHIWU ET AL: "Transgenic Sugarcane with a cry1Ac Gene Exhibited Better Phenotypic Traits and Enhanced Resistance against Sugarcane Borer", PLOS ONE, vol. 11, no. 4, 19 April 2016 (2016-04-19), page e0153929, XP055795869, DOI: 10.1371/journal.pone.0153929<br>* abstract *<br>----- | 1-15<br><br><br><br><br><br><br><br>9,13 | INV.<br>C12N15/82<br>C07K14/325 |
| X<br><br><br><br><br><br><br><br><br><br><br>Y | ZHOU DINGGANG ET AL: "Foreign cry1Ac gene integration and endogenous borer stress-related genes synergistically improve insect resistance in sugarcane", BMC PLANT BIOLOGY, vol. 18, no. 1, 1 December 2018 (2018-12-01), XP055795875, DOI: 10.1186/s12870-018-1536-6 Retrieved from the Internet: URL:http://link.springer.com/content/pdf/10.1186/s12870-018-1536-6.pdf><br>* abstract *<br>----- | 1-15<br><br><br><br><br><br><br><br><br><br><br>9,13 | TECHNICAL FIELDS SEARCHED (IPC)<br>C12N<br>C12R<br>C07K |
| X<br><br><br><br><br><br><br><br><br><br><br><br>Y | ADRIANA C. GIANOTTO ET AL: "The insect-protected CTC91087-6 sugarcane event expresses Cry1Ac protein preferentially in leaves and presents compositional equivalence to conventional sugarcane", GM CROPS & FOOD, vol. 10, no. 4, 20 August 2019 (2019-08-20), pages 208-219, XP055766948, ISSN: 2164-5698, DOI: 10.1080/21645698.2019.1651191<br>* abstract *<br>-----<br>-/-- | 1-15<br><br><br><br><br><br><br><br><br><br><br><br>9,13 | |

The present search report has been drawn up for all claims

1

| Place of search | Date of completion of the search | Examiner |
|---|---|---|
| Munich | 28 April 2021 | Mundel, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 20 20 6809

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WENG LI-XING ET AL: "Regeneration of sugarcane elite breeding lines and engineering of stem borer resistance", PEST MANAGEMENT SCIENCE, vol. 62, no. 2, 1 January 2006 (2006-01-01), pages 178-187, XP055795879, ISSN: 1526-498X, DOI: 10.1002/ps.1144 Retrieved from the Internet: URL:https://onlinelibrary.wiley.com/doi/pdfdirect/10.1002/ps.1144> | 1-15 | |
| Y | * abstract * * 2.4. Sugarcane tissue culture and transformation; page 180 * ----- | 9,13 | |
| X | DATABASE EMBL [Online] 13 February 2018 (2018-02-13), Zhang et al.: "Ptinus sp. INB132 isoleucyl-tRNA synthetase gene, partial cds", XP055799319, Database accession no. MG069326 * sequence * ----- | 3,4 | |
| Y | CN 102 250 923 B (UNIV FUDAN; GUANGZHOU SUGARCANE INDUSTRY RES INST) 16 October 2013 (2013-10-16) * abstract * * sequence 1 * ----- | 9,13 | |

TECHNICAL FIELDS SEARCHED (IPC)

The present search report has been drawn up for all claims

1

| Place of search | Date of completion of the search | Examiner |
|---|---|---|
| Munich | 28 April 2021 | Mundel, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

# ANNEX TO THE EUROPEAN SEARCH REPORT ON EUROPEAN PATENT APPLICATION NO. EP 20 20 6809

This annex lists the patent family members relating to the patent documents cited in the above-mentioned European search report.
The members are as contained in the European Patent Office EDP file on
The European Patent Office is in no way liable for these particulars which are merely given for the purpose of information.

28-04-2021

| Patent document cited in search report | Publication date | Patent family member(s) | Publication date |
|---|---|---|---|
| CN 102250923 B | 16-10-2013 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- BR 112014023851
- WO 08021538 A
- WO 09045384 A
- WO 11012951 A


### Non-patent literature cited in the description


- *FAO Statistical Yearbook,* 2012, 233